# EUROPEAN PATENT APPLICATION

(11) **EP 4 682 163 A2**
(43) Date of publication of application: **21.01.2026**
(21) Application number: 25188887.1
(22) Date of filing: 14.02.2023
(51) Int. Cl.: C07K 14/475

(54) **METHOD OF REDUCING CST FLUCTUATION IN NEOVASCULAR AMD BY A RECOMBINANT ADENO-ASSOCIATED VIRUS**

(30) Priority: 16.02.2022 US 202263310865 P; 04.03.2022 US 202263316705 P
(62) Divisional of application: 23710626.5
(71) Applicant: Adverum Biotechnologies, Inc., Redwood City, CA 94063 (US)
(72) Inventor: DO, Joanna, Redwood City, CA 94063 (US); TURPCU, Adam, Redwood City, CA 94063 (US)
(74) Representative: EIP

(57) **Abstract**

Provided are methods for treating an ocular neovascular disease in an individual by reducing fluction of central subfoveal thickness (CST) or central retinal thickness (CRT), comprising administering a unit dose of recombinant adeno-associated virus (rAAV) particles to an eye of the individual, wherein the rAAV particles comprise: a) a nucleic acid encoding a polypeptide comprising an amino acid sequence with at least about 95% identity to the amino acid sequence of SEQ ID NO: 35 and flanked by AAV2 inverted terminal repeats (ITRs), and b) an AAV2 capsid protein comprising an amino acid sequence LGETTRP (SEQ ID NO: 14) inserted between positions 587 and 588 of the capsid protein, wherein the amino acid residue numbering corresponds to an AAV2 VP1 capsid protein.

## Description

### Cross-Reference to Related Applications

This application claims priority under 35 U.S.C. § 119(e) to U.S. Provisional Application 63/310,865 filed February 16, 2022 and U.S. Provisional Application 63/316,705 filed March 4, 2022; each of which is incorporated by reference in its entirety.

### Statement Regarding the Sequence Listing

The official copy of the Sequence Listing is submitted concurrently with the specification as an WIPO Standard ST.26 formatted XML file with file name "17234-034WO1.xml", a creation date of February 6, 2023, and a size of 49 kilobytes. This Sequence Listing filed via USPTO Patent Center is part of the specification and is incorporated in its entirety by reference herein.

### FIELD

The present disclosure relates to methods of treating ocular neovascular disease and disorders by reducing fluctuations in the central subfield thickness (CST) or central retinal thickness (CRT) in an individual that comprise administering a single unit dose of a recombinant adeno associated virus (rAAV) particles encoding an anti-VEGF agent (e.g., aflibercept) to an eye of an individual.

### BACKGROUND

Age-related macular degeneration (AMD) is a degenerative ocular disease affecting the macula, a light sensitive, small area in the center of the retina that is responsible for reading and fine vision. Conditions affecting the macula reduce central vision while leaving peripheral vision intact. In severe cases, the disease can lead to central blindness. AMD is a notable cause of vision loss in the US population among persons 65 years and older, and the estimated prevalence of any AMD among persons over 40 years of age is approximately 6.5% (Klein et al., (2011) Arch Ophthalmol, 129(1):75-80). Neovascular or exudative or wet AMD (nAMD, wAMD, or nwAMD) is an advanced form of AMD. The hallmark of wAMD is choroidal neovascularization (CNV), which is the infiltration of abnormal blood vessels in the retina from the underlying choroid layer, resulting in retinal cell damage and central blindness. This abnormal angiogenic process is modulated by growth factors, in particular, vascular endothelial growth factor (VEGF). The standard of care of wAMD is a class of molecules that bind to and sequester VEGF, such as ranibizumab (Lucentis) and aflibercept (Eylea).

Diabetic retinopathy (DR) is a major complication of diabetes mellitus, and is a leading cause of visual loss in the working age population. DR may be non-proliferative (NPDR), with no new blood vessel growth, or proliferative DR (PDR), with new abnormal blood vessel growth within the retina or choroid. Diabetic macular edema (DME) is a complication of DR and is another example of an ocular disease affecting the macula. DME affects up to 10% of people with diabetes and is caused by fluid accumulation in the macula. DME is the most frequent cause of sight loss in people with DR. Available therapies for treating DME include laser and anti-vascular endothelial growth factor (anti-VEGF) drugs such as aflibercept.

Aflibercept is a recombinant fusion protein that acts as a decoy receptor for vascular endothelial growth factor subtypes A and B (VEGF-A and VEGF-B) and placental growth factor (PGF). By binding to these ligands, aflibercept is able to prevent them from binding to vascular endothelial growth factor receptors (VEGFR), VEGFR-1 and VEGFR-2, to suppress neovascularization and decrease vascular permeability. Aflibercept consists of domain 2 of VEGFR-1 and domain 3 of VEGFR-2 fused with the Fc fragment of IgG1.

Central Subfield Thickness (CST) is the measurement of macular thickness in the central subfield area around the fovea. In nAMD, accumulation of fluid increases CST. Previous studies and treatments shows that there is a correlation between CST fluctuation and visual acuity.

Previous studies based on clinical trial data have demonstrated that greater fluctuations in retinal thickness during the course of intravitreal anti-vascular endothelial growth factor (anti-VEGF) therapy for neovascular age-related macular degeneration (nAMD) is associated with poorer visual acuity outcomes (Lai et al. J. Pers. Med. 2021, 11, 1024). Lai et al. showed that in intravitreal anti-VEGF therapy (bevacizumab, ranibizumab or aflibercept) for the treatment of nAMD, using variability in spectral-domain optical coherence tomography (OCT) central subfield thickness (CST) was calculated, a significant correlation was found between CST variability and visual acuity at 2 years, indicating that eyes with lower CST variability had better visual acuity at 2 years. Eyes with the least CST variability were associated with the highest mean visual acuity improvement at 2 years, These findings showed that eyes undergoing anti-VEGF therapy for nAMD with more stable OCT CST variability during the follow-up period were associated with better visual outcomes.Tus, data from the Comparison of AMD Treatments Trials (CATT) and Inhibit VEGF in Age-related choroidal Neovascularisation (IVAN) trials showed that greater fluctuations in CST is associated with an increased risk for fibrosis and macular atrophy which can be a cause of poorer visual acuity (VA).

Current standard of care anti-VEGF agents such as aflibercept need to be re-administered via intravitreal (IVT) injection every 4 to 8 weeks to achieve optimal therapeutic outcomes and maintain visual acuity. Compliance with such a regimen is burdensome to patients, their caregivers, and the healthcare system, and most patients fall out of compliance with the optimal regimen over time, which is correlated with vision loss (Khanani AM, *et al*.). In addition, there are complications including endophthalmitis, retinal detachments, traumatic cataract, and elevated intraocular pressure (IOP); the risks of these complications are likely to increase with repeated IVT injections (Falavarjani et al., (2013) Eye (Lond), 27(7):787-794).

There is a need in the art for therapies for ocular neovascular diseases such as wAMD, DR, or DME that are effective, reduce the risk of adverse effects, and are amenable to high long-term patient compliance. There is also a need in the art for a more effective method of reducing CST variability in this regard.

### SUMMARY OF THE DISCLOSURE

In one aspect, provided herein is a method for treating an ocular neovascular disease in an individual by reducing fluctuations central subfoveal thickness (CST), the method comprising administering a unit dose of about 6 × 10¹¹ vector genomes (vg) or less of recombinant adeno-associated virus (rAAV) particles to one eye of the individual, wherein the individual is a human, and wherein the rAAV particles comprise: (a) a nucleic acid encoding a polypeptide comprising an amino acid sequence with at least about 95% identity to the amino acid sequence of SEQ ID NO: 35 and flanked by AAV2 inverted terminal repeats (ITRs), and (b) an AAV2 capsid protein comprising, or consisting of, an amino acid sequence LGETTRP (SEQ ID NO: 14) inserted between positions 587 and 588 of the capsid protein, wherein the amino acid residue numbering corresponds to an AAV2 VP1 capsid protein. In some embodiments, the method comprises reducing retinal fluid in an eye of the individual.

In one aspect, provided herein is a method for treating an ocular neovascular disease by reducing fluctuation in central subfoveal thickness (CST) in an individual, the method comprising administering a unit dose of about 6 × 10¹¹ vector genomes (vg) or less of recombinant adeno-associated virus (rAAV) particles to one eye of the individual, wherein the individual is a human, and wherein the rAAV particles comprise: (a) a nucleic acid encoding a polypeptide comprising an amino acid sequence with at least about 95% identity to the amino acid sequence of SEQ ID NO: 35 and flanked by AAV2 inverted terminal repeats (ITRs), and (b) an AAV2 capsid protein comprising, or consisting of, an amino acid sequence LGETTRP (SEQ ID NO: 14) inserted between positions 587 and 588 of the capsid protein, wherein the amino acid residue numbering corresponds to an AAV2 VP1 capsid protein. In some embodiments, the method comprises reducing retinal fluid in an eye of the individual.

In another aspect, provided herein is a method for reducing fluctuations in the CST in an eye of an individual with an ocular neovascular disease, the method comprising administering a unit dose of rAAV particles to one eye of the individual, wherein the individual is a human, and wherein the rAAV particles comprise: (a) a nucleic acid encoding a polypeptide comprising an amino acid sequence with at least about 95% identity to the amino acid sequence of SEQ ID NO: 35 and flanked by AAV2 inverted terminal repeats (ITRs), and (b) an AAV2 capsid protein comprising, or consisting of, an amino acid sequence LGETTRP (SEQ ID NO: 14) inserted between positions 587 and 588 of the capsid protein, wherein the amino acid residue numbering corresponds to an AAV2 VP1 capsid protein. In some embodiments, the individual has received at least one treatment of an anti-VEGF agent in about last 2 to 12 weeks prior to administration of the unit dose of rAAV particles. In some embodiments, the amount or presence of retinal fluid in the one eye of the individual is refractory to prior treatment with an anti-VEGF agent. In some embodiments, the anti-VEGF agent is aflibercept. In some embodiments, the retinal fluid in the one eye is reduced by at least about 60%. In some embodiments, the retinal fluid in the one eye is reduced by about 80% compared to the level of retinal fluid in the one eye of the individual prior to administration of the rAAV to the individual. In some embodiments, the retinal fluid is subretinal fluid (SRF) or intraretinal fluid (IRF). In some embodiments, the unit dose of rAAV particles is about 6 × 10¹¹ vector genomes per eye (vg/eye) or less.

In another aspect, provided herein is a method for treating an ocular neovascular disease in an individual by reducing fluctuations in the CST, the method comprising: (a) administering an anti-VEGF agent to one eye of the individual; and (b) administering a unit dose of about 6 × 10¹¹ vector genomes (vg) or less of recombinant adeno-associated virus (rAAV) particles to the one eye of the individual after administration of the anti-VEGF agent, wherein the individual is a human, and wherein the rAAV particles comprise: (i) a nucleic acid encoding a polypeptide comprising an amino acid sequence with at least about 95% identity to the amino acid sequence of SEQ ID NO: 35 and flanked by AAV2 inverted terminal repeats (ITRs), and (ii) an AAV2 capsid protein comprising an amino acid sequence LGETTRP (SEQ ID NO: 14) inserted between positions 587 and 588 of the capsid protein, wherein the amino acid residue numbering corresponds to an AAV2 VP1 capsid protein. In some embodiments, the method comprises administering the unit dose of rAAV particles to the one eye of the individual about 1 week or about 7 days after administration of the anti-VEGF agent. In some embodiments, the method comprises administering the unit dose of rAAV particles to the one eye of the individual about 1 week to about 2 weeks after administration of the anti-VEGF agent. In some embodiments, the method comprises administering the unit dose of rAAV particles to the one eye of the individual about 1 day, about 2 days, about 3 days, about 4 days, about 5 days, about 6 days, about 7 days, about 8 days, about 9 days, about 10 days, about 11 days, about 12 days, about 13 days, about 14 days, or about 15 days after administration of the anti-VEGF agent. In some embodiments, the method comprises administering the anti-VEGF agent to the one eye of the individual on Day 1, and administering the unit dose of rAAV particles to the one eye of the individual on Day 8. In some embodiments, the anti-VEGF agent comprises aflibercept. In some embodiments, the aflibercept is administered at a dose of about 2 mg by intravitreal injection. In some embodiments, the method further comprises administering a topical steroid treatment. In some embodiments, the topical steroid treatment is a difluprednate treatment. In some embodiments, the topical steroid treatment comprises about four administrations of topical steroid per day for about four weeks, followed by about three administrations of topical steroid per day for about one week, followed by about two administrations of topical steroid per day for about one week, and followed by about one administration of topical steroid per day for about one week; timing starting with and following administration of the anti-VEGF agent. In some embodiments, the topical steroid treatment comprises about four administrations of topical steroid per day for about one month, followed by about three administrations of topical steroid per day for about one month, followed by about two administrations of topical steroid per day for about one month, and followed by about one administration of topical steroid per day for about one month; timing starting with and following administration of the anti-VEGF agent. In some embodiments, the topical steroid treatment comprises about four administrations of topical steroid per day for about one month, followed by about three administrations of topical steroid per day for about one month, followed by about two administrations of topical steroid per day for about one month, and followed by about one administration of topical steroid per day for about one month; timing starting with and following administration of the rAAV particles. In some embodiments, the topical steroid comprises difluprednate 0.05% at a dose of about 1µg to about 3 µg. In some embodiments, the topical steroid comprises difluprednate 0.05% at a dose of about 2.5µg.

In some embodiments that may be combined with any of the preceding embodiments, the unit dose of rAAV particles is about 6 × 10¹⁰ to about 2 × 10¹¹ vector genomes per eye (vg/eye). In some embodiments, the unit dose of rAAV particles is about 2 × 10¹¹ or about 6 × 10¹⁰ vector genomes per eye (vg/eye). In some embodiments that may be combined with any of the preceding embodiments, the unit dose of rAAV particles is between about 6 × 10¹⁰ to about 6 × 10¹¹ vector genomes per eye (vg/eye). In some embodiments that may be combined with any of the preceding embodiments, the unit dose of rAAV particles is between about 6 × 10¹⁰ to about 2 × 10¹¹ vector genomes per eye (vg/eye). In some embodiments that may be combined with any of the preceding embodiments, the unit dose of rAAV particles is between about 2 × 10¹¹ to about 6 × 10¹¹ vector genomes per eye (vg/eye). In some embodiments, the unit dose of rAAV particles is about 2 × 10¹¹ or about 6 × 10¹⁰ vector genomes per eye (vg/eye). In some embodiments, the unit dose of rAAV particles is about 2 × 10¹¹ vg/eye. In some embodiments, the unit dose of rAAV particles is about 6 × 10¹¹ vg/eye.

In some embodiments that may be combined with any of the preceding embodiments, the individual has one or more symptoms of an ocular neovascular disease in the contralateral eye.

In some embodiments that may be combined with any of the preceding embodiments, the methods provided herein further comprise administering a unit dose of rAAV particles to the contralateral eye of the individual. In some embodiments, the administering the unit dose of rAAV particles to the contralateral eye is up to about 2 weeks after administering the unit dose of rAAV particles to the one eye. In some embodiments, the administering the unit dose of rAAV particles to the contralateral eye is on the same day as the administering the unit dose of rAAV particles to the one eye; or the administering the unit dose of rAAV particles to the contralateral eye is between about 1 day to about 14 days after administering the unit dose of rAAV particles to the one eye. In some embodiments, the unit dose of rAAV particles administered to the contralateral eye of the individual comprises the same or less vector genomes per eye (vg/eye) than the unit dose of rAAV particles administered to the one eye of the individual. In some embodiments, the administering the unit dose of rAAV particles to the contralateral eye is at least about 2 weeks after administering the unit dose of rAAV particles to the one eye. In some embodiments, the unit dose of rAAV particles administered to the contralateral eye of the individual comprises more vector genomes per eye (vg/eye) than the unit dose of rAAV particles administered to the one eye of the individual.

In some embodiments that may be combined with any of the preceding embodiments, the nucleic acid comprises the nucleic acid sequence of SEQ ID NO: 40 or a sequence having at least 85% identity thereto.

In some embodiments that may be combined with any of the preceding embodiments, the polypeptide comprises the amino acid sequence of SEQ ID NO: 35. In some embodiments that may be combined with any of the preceding embodiments, the polypeptide comprises the amino acid sequence of SEQ ID NO: 41. In some embodiments, the polypeptide is aflibercept.

In some embodiments that may be combined with any of the preceding embodiments, the nucleic acid further comprises a first enhancer region, a promoter region, a 5'UTR region, a second enhancer region, and a polyadenylation site. In some embodiments, the nucleic acid comprises, in the 5' to 3' order: (a) a first enhancer region; (b) a promoter region; (c) a 5'UTR region; (d) a nucleic acid encoding a polypeptide comprising an amino acid sequence with at least about 95% identity to the amino acid sequence of SEQ ID NO: 35; (e) a second enhancer region; and (f) a polyadenylation site; and flanked by AAV2 inverted terminal repeats (ITRs). In some embodiments, the first enhancer region comprises a CMV sequence comprising the sequence of SEQ ID NO: 22 or a sequence having at least 85% identity thereto. In some embodiments, the promoter region comprises a CMV sequence comprising the sequence of SEQ ID NO: 23 or a sequence having at least 85% identity thereto. In some embodiments, the nucleic acid encoding a polypeptide comprises the nucleic acid sequence of SEQ ID NO: 40 or a sequence having at least 85% identity thereto. In some embodiments, the polypeptide comprises the amino acid sequence of SEQ ID NO: 35 or a sequence having at least 85% identity thereto. In some embodiments, the polypeptide comprises the amino acid sequence of SEQ ID NO: 41 or a sequence having at least 85% identity thereto. In some embodiments, the polypeptide is aflibercept. In some embodiments, the 5'UTR region comprises, in 5' to 3' order, a TPL sequence comprising the sequence of SEQ ID NO: 24 or a sequence having at least 85% identity thereto, and an eMLP sequence comprising the sequence of SEQ ID NO: 25 or a sequence having at least 85% identity thereto. In some embodiments, the second enhancer region comprises a full EES sequence comprising the sequence of SEQ ID NO: 26 or a sequence having at least 85% identity thereto. In some embodiments, the polyadenylation site comprises a HGH polyadenylation site comprising the sequence of SEQ ID NO: 27 or a sequence having at least 85% identity thereto. In some embodiments, the nucleic acid further comprises (a) a first enhancer region comprising a CMV sequence comprising the sequence of SEQ ID NO: 22 or a sequence having at least 85% identity thereto; (b) a promoter region, comprising a CMV sequence comprising the sequence of SEQ ID NO: 23 or a sequence having at least 85% identity thereto; (c) a 5'UTR region comprising, in 5' to 3' order, a TPL sequence comprising the sequence of SEQ ID NO: 24 or a sequence having at least 85% identity thereto, and an eMLP sequence comprising the sequence of SEQ ID NO: 25 or a sequence having at least 85% identity thereto; (d) a second enhancer region comprising a full EES sequence comprising the sequence of SEQ ID NO: 26 or a sequence having at least 85% identity thereto; and (e) a HGH polyadenylation site comprising the sequence of SEQ ID NO: 27 or a sequence having at least 85% identity thereto. In some embodiments, the nucleic acid comprises AAV ITRs flanking the elements.

In some embodiments that may be combined with any of the preceding embodiments, the nucleic acid comprises the sequence of SEQ ID NO: 39 or a sequence having at least 85% identity thereto.

In some embodiments that may be combined with any of the preceding embodiments, the rAAV particles comprise an AAV2 VP1 capsid protein comprising a GH loop that comprises the amino acid sequence of SEQ ID NO: 38 or an amino acid sequence having at least 90% sequence identity to SEQ ID NO: 38. In some embodiments, the rAAV particles comprise an AAV2 VP1 capsid protein comprising a GH loop that comprises an amino acid sequence having any of at least 90%, at least 91%, at least 92%, at least 93%, at least 94%, at least 95%, at least 96%, at least 97%, at least 98%, at least 99%, or 100% sequence identity to SEQ ID NO: 38.

In some embodiments that may be combined with any of the preceding embodiments, the rAAV particles comprise an AAV2 VP1 capsid protein comprising the amino acid sequence of SEQ ID NO: 37 or an amino acid sequence having at least 90% sequence identity to SEQ ID NO: 37. In some embodiments, the rAAV particles comprise an AAV2 VP1 capsid protein comprising an amino acid sequence having any of at least 90%, at least 91%, at least 92%, at least 93%, at least 94%, at least 95%, at least 96%, at least 97%, at least 98%, at least 99%, or 100% sequence identity to SEQ ID NO: 37.

In some embodiments that may be combined with any of the preceding embodiments, the AAV2 capsid protein comprises, or consists of, the amino acid sequence LGETTRP (SEQ ID NO: 14) inserted between positions 587 and 588 of the AAV2 VP1 comprising the sequence of SEQ ID NO: 13. In some embodiments, the AAV2 capsid protein comprises the amino acid sequence LALGETTRPA (SEQ ID NO: 1) inserted between positions 587 and 588 of the capsid protein, wherein the amino acid residue numbering corresponds to an AAV2 VP1 capsid protein. In some embodiments, the AAV2 capsid protein comprises the amino acid sequence LALGETTRPA (SEQ ID NO: 1) inserted between positions 587 and 588 of the AAV2 VP1 comprising the sequence of SEQ ID NO: 13. In some embodiments, the AAV2 capsid protein comprises the amino acid sequence LALGETTRPA (SEQ ID NO: 1) inserted between positions 587 and 588 of the capsid protein, wherein the amino acid residue numbering corresponds to an AAV2 VP1 capsid protein. In some embodiments, the AAV2 capsid protein comprises the amino acid sequence LALGETTRPA (SEQ ID NO: 1) inserted between positions 587 and 588 of the AAV2 VP1 comprising the sequence of SEQ ID NO: 13.

In some embodiments that may be combined with any of the preceding embodiments, the administration of the unit dose of rAAV particles to the one eye and/or the contralateral eye is by intravitreal administration.

In some embodiments that may be combined with any of the preceding embodiments, the unit dose of rAAV particles is in a pharmaceutical formulation. In some embodiments, the pharmaceutical formulation comprises the rAAV particles, sodium chloride, sodium phosphate and a surfactant. In some embodiments, the pharmaceutical formulation comprises about 150 to about 200 mM sodium chloride, about 1 to about 10 mM monobasic sodium phosphate, about 1 to about 10 mM dibasic sodium phosphate, about 0.0005% (w/v) to about 0.005% (w/v) poloxamer 188, and about 6 × 10¹³ to about 6 × 10¹⁰ vector genomes (vg) per mL (vg/mL) of the rAAV particles, wherein the pharmaceutical formulation has a pH of about 7.0 to about 7.5. In some embodiments, the pharmaceutical formulation comprises about 180 mM sodium chloride, about 5 mM monobasic sodium phosphate, about 5 mM dibasic sodium phosphate, about 6 × 10¹² vg/mL of the rAAV particles, and about 0.001% (w/v) poloxamer 188, wherein the pharmaceutical formulation has a pH of about 7.3. In some embodiments, the pharmaceutical formulation comprises about 180 mM sodium chloride, about 5 mM monobasic sodium phosphate, about 5 mM dibasic sodium phosphate, about 2 ×10¹² vg/mL of the rAAV particles, and about 0.001% (w/v) poloxamer 188, wherein the pharmaceutical formulation has a pH of about 7.3. In some embodiments, the pharmaceutical formulation comprises about 180 mM sodium chloride, about 5 mM monobasic sodium phosphate, about 5 mM dibasic sodium phosphate, about 6 ×10¹¹ vg/mL of the rAAV particles, and about 0.001% (w/v) poloxamer 188, wherein the pharmaceutical formulation has a pH of about 7.3.

In some embodiments that may be combined with any of the preceding embodiments, the unit dose of rAAV particles comprises a volume of about 25 µL to about 250 µL. In some embodiments, the unit dose of rAAV particles comprises a volume of about 100µL. In some embodiments, the unit dose of rAAV particles comprises a volume of about 30µL. In some embodiments that may be combined with any of the preceding embodiments, the unit dose of rAAV particles administered to the one eye and/or to the contralateral eye comprises a volume of about 25 µL to about 250 µL. In some embodiments, the unit dose of rAAV particles administered to the one eye and/or to the contralateral eye comprises a volume of about 100µL. In some embodiments, the unit dose of rAAV particles administered to the one eye and/or to the contralateral eye comprises a volume of about 30µL.

In some embodiments that may be combined with any of the preceding embodiments, the individual received prior treatment for the ocular neovascular disease with an anti-VEGF agent. In some embodiments, the individual has received 1 or 2 injections of an anti-VEGF agent in the one eye and/or in the contralateral eye prior to administration of the rAAV particles in the one eye and/or in the contralateral eye. In some embodiments, the individual has not received prior treatment for the ocular neovascular disease with an anti-VEGF agent. In some embodiments, the anti-VEGF agent is aflibercept.

In some embodiments that may be combined with any of the preceding embodiments, the ocular neovascular disease is wet age-related macular degeneration (AMD), retinal neovascularization, choroidal neovascularization diabetic retinopathy, proliferative diabetic retinopathy, retinal vein occlusion, central retinal vein occlusion, branched retinal vein occlusion, diabetic macular edema, diabetic retinal ischemia, ischemic retinopathy, diabetic retinal edema, or any combination thereof.

In some embodiments that may be combined with any of the preceding embodiments, the unit dose of rAAV particles is administered in combination with steroid treatment. In some embodiments, the steroid treatment is a corticosteroid treatment. In some embodiments, the steroid treatment is a systemic steroid treatment. In some embodiments, the steroid treatment is an oral steroid treatment. In some embodiments, the steroid treatment is a prednisone treatment. In some embodiments, the oral prednisone treatment comprises administering prednisone at a dose of about 60 mg per day for a total of 6 days starting at 3 days before administration of the unit dose of rAAV particles to the one eye and/or to the contralateral eye, followed by administering prednisone at a dose of about 40 mg per day for a total of 3 days, followed by administering prednisone at a dose of about 20 mg per day for a total of 2 days, and followed by administering prednisone at a dose of about 10 mg per day for a total of 2 days. In some embodiments, the steroid treatment is a topical steroid treatment. In some embodiments, the steroid treatment is a difluprednate treatment. In some embodiments, the steroid is administered before, during and/or after administration of the unit dose of rAAV particles. In some embodiments, the steroid is administered before, during and/or after administration of the unit dose of rAAV particles to the one eye and/or to the contralateral eye.

In some embodiments that may be combined with any of the preceding embodiments, the steroid treatment is a topical steroid treatment and the topical steroid treatment is a daily steroid treatment for up to about 4 weeks, up to about 6 weeks, or up to about 8 weeks from administering the unit dose of rAAV particles. In some embodiments, the topical steroid treatment comprises about four administrations of topical steroid on about week 1, about three administrations of topical steroid on about week 2, about two administrations of topical steroid on about week 3, and about one administration of topical steroid on about week 4; timing starting with and following administration of the unit dose of rAAV particles. In some embodiments, the topical steroid treatment comprises about four administrations of topical steroid per day for about 3 weeks after administration of the unit dose of rAAV particles, followed by about 3 administrations of topical steroid per day for about 1 week, followed by about 2 administrations of topical steroid per day for about 1 week, and followed by about 1 administration of topical steroid per day for about 1 week. In some embodiments, the topical steroid treatment comprises about four administrations of topical steroid per day for about four weeks, followed by about three administrations of topical steroid per day for about one week, followed by about two administrations of topical steroid per day for about one week, and followed by about one administration of topical steroid per day for about one week; timing starting at about one week prior to administration of the unit dose of rAAV particles. In some embodiments, the topical steroid treatment comprises about four administrations of topical steroid per day for about one month, followed by about three administrations of topical steroid per day for about one month, followed by about two administrations of topical steroid per day for about one month, and followed by about one administration of topical steroid per day for about one month; timing starting at about the administration of the unit dose of rAAV particles. In some embodiments, the topical steroid treatment comprises about four administrations of topical steroid per day for about one month, followed by about three administrations of topical steroid per day for about one month, followed by about two administrations of topical steroid per day for about one month, and followed by about one administration of topical steroid per day for about one month; timing starting at about one week prior to administration of the unit dose of rAAV particles. In some embodiments, the topical steroid comprises difluprednate 0.05% at a dose of about 1µg to about 3 µg. In some embodiments, the topical steroid comprises difluprednate 0.05% at a dose of about 2.5µg.

In some embodiments, the ocular neovascular disease is wet age-related macular degeneration (wAMD).

In some embodiments that may be combined with any of the preceding embodiments, the administering the unit dose of rAAV particles to the one eye and/or to the contralateral eye of the individual results in maintenance or a decrease of retinal thickness compared to the retinal thickness prior to administration of the unit dose of rAAV particles. In some embodiments, the maintenance or the decrease of retinal thickness compared to the retinal thickness prior to administration of the unit dose of rAAV particles is present at about 30 weeks, about 34 weeks, about 44 weeks, about 6 months, about 1 year, about 1.5 years, about 2 years, about 3 years, about 5 years, about 10 years, or more after administration of the unit dose of rAAV particles to the one eye and/or to the contralateral eye of the individual. In some embodiments, the administering the unit dose of rAAV particles to the one eye and/or to the contralateral eye of the individual results in a decrease in retinal thickness compared to the retinal thickness prior to administration of the unit dose of rAAV particles. In some embodiments, the decrease in retinal thickness is at least about 10% compared to the retinal thickness prior to administration of the unit dose of rAAV particles. In some embodiments, retinal thickness is central subfield thickness (CST) or central retinal thickness (CRT).

In some embodiments that may be combined with any of the preceding embodiments, the administering the unit dose of rAAV particles to the one eye and/or to the contralateral eye of the individual results in maintenance or a decrease in macular volume compared to the macular volume prior to administration of the unit dose of rAAV particles. In some embodiments, the maintenance or the decrease in macular volume compared to the macular volume prior to administration of the unit dose of rAAV particles is present at about 30 weeks, about 34 weeks, about 44 weeks, about 6 months, about 1 year, about 1.5 years, about 2 years, about 3 years, about 5 years, about 10 years, or more after administration of the unit dose of rAAV particles to the one eye and/or to the contralateral eye of the individual. In some embodiments, the administering the unit dose of rAAV particles to the one eye and/or to the contralateral eye of the individual results in a decrease in macular volume compared to the macular volume prior to administration of the unit dose of rAAV particles. In some embodiments, the decrease in macular volume is at least about 10% compared to the macular volume prior to administration of the unit dose of rAAV particles.

In some embodiments that may be combined with any of the preceding embodiments, the administering the unit dose of rAAV particles to the one eye and/or to the contralateral eye of the individual results in maintenance or an improvement of visual acuity compared to the visual acuity prior to administration of the unit dose of rAAV particles. In some embodiments, the maintenance or the improvement of visual acuity compared to the visual acuity prior to administration of the unit dose of rAAV particles is present at about 30 weeks, about 34 weeks, about 44 weeks, about 6 months, about 1 year, about 1.5 years, about 2 years, about 3 years, about 5 years, about 10 years, or more after administration of the unit dose of rAAV particles to the one eye and/or to the contralateral eye of the individual. In some embodiments, the administering the unit dose of rAAV particles to the one eye and/or to the contralateral eye of the individual results in an improvement of visual acuity compared to the visual acuity prior to administration of the unit dose of rAAV particles. In some embodiments, visual acuity is best corrected visual acuity (BCVA).

In some embodiments that may be combined with any of the preceding embodiments, administration of a single unit dose of rAAV particles to the one eye and/or to the contralateral eye of the individual provides a therapeutic benefit (e.g., treatment of an ocular neovascular disease, reduction of retinal fluid, maintenance or a decrease of retinal thickness, maintenance or a decrease in macular volume, and/or maintenance or an improvement of visual acuity). In some embodiments, administration of a single unit dose of rAAV particles to the one eye and/or to the contralateral eye of the individual provides a therapeutic benefit that is present at about 30 weeks or more, about 34 weeks or more, about 44 weeks or more, about 6 months or more, about 1 year or more, about 1.5 years or more, or about 2 years, about 3 years, about 5 years, about 10 years, or more after administration of the unit dose of rAAV particles to the one eye and/or to the contralateral eye of the individual.

In some embodiments that may be combined with any of the preceding embodiments, administration of the unit dose of rAAV particles to the one eye and/or to the contralateral eye of a plurality of individuals results in at least about 50% of the individuals in the plurality not requiring an anti-VEGF rescue treatment. In some embodiments, at least about 50% of the individuals in the plurality do not require an anti-VEGF rescue treatment for at least about 20 weeks, at least about 36 weeks, at least about 52 weeks, at least about 56 weeks, or more after administration of the unit dose of rAAV particles.

In some embodiments that may be combined with any of the preceding embodiments, administration of a single unit dose of rAAV particles to the one eye and/or to the contralateral eye of a plurality of individuals results in at least about 67% of the individuals in the plurality not requiring an anti-VEGF rescue treatment. In some embodiments, at least about 67% of the individuals in the plurality do not require an anti-VEGF rescue treatment for at least about 20 weeks, at least about 36 weeks, at least about 52 weeks, at least about 60 weeks, at least about 64 weeks, or at least about 66 weeks after administration of the unit dose of rAAV particles.

In some embodiments that may be combined with any of the preceding embodiments, administration of the unit dose of rAAV particles to the one eye and/or to the contralateral eye of a plurality of individuals results in at least about 78% of the individuals in the plurality not requiring an anti-VEGF rescue treatment. In some embodiments, at least about 78% of the individuals in the plurality do not require an anti-VEGF rescue treatment for at least about 20 weeks, at least about 36 weeks, or more after administration of the unit dose of rAAV particles.

In some embodiments that may be combined with any of the preceding embodiments, administration of the unit dose of rAAV particles to the one eye and/or to the contralateral eye of a plurality of individuals results in 100% of the individuals in the plurality not requiring an anti-VEGF rescue treatment. In some embodiments, 100% of the individuals in the plurality do not require an anti-VEGF rescue treatment for at least about 64 weeks, at least about 68 weeks, at least about 72 weeks, at least about 76 weeks, at least about 80 weeks, at least about 84 weeks, or more after administration of the unit dose of rAAV particles.

In some embodiments that may be combined with any of the preceding embodiments, administration of the unit dose of rAAV particles to the one eye and/or to the contralateral eye of a plurality of individuals results in a reduction in the annualized anti-VEGF injection rate of at least about 80%, at least about 85%, at least about 87%, at least about 90%, at least about 95%, at least about 99%, or 100% compared to the annualized anti-VEGF injection rate prior to administration of the unit dose of rAAV particles.

In some embodiments, the ocular neovascular disease is diabetic macular edema (DME). In some embodiments, the administering the unit dose of rAAV particles to the one eye and/or to the contralateral eye of the individual results in a 2-step or in a 3-step improvement in Diabetic Retinopathy Severity Scale (DRSS). In some embodiments, the administering the unit dose of rAAV particles to the one eye and/or to the contralateral eye of the individual results in a 2-step improvement in Diabetic Retinopathy Severity Scale (DRSS). In some embodiments, the administering the unit dose of rAAV particles to the one eye and/or to the contralateral eye of the individual results in a 3-step improvement in Diabetic Retinopathy Severity Scale (DRSS).

### INCORPORATION BY REFERENCE

All references cited herein, including patent applications and publications, are incorporated by reference in their entirety.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

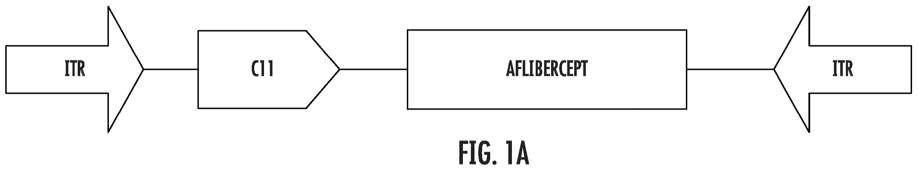

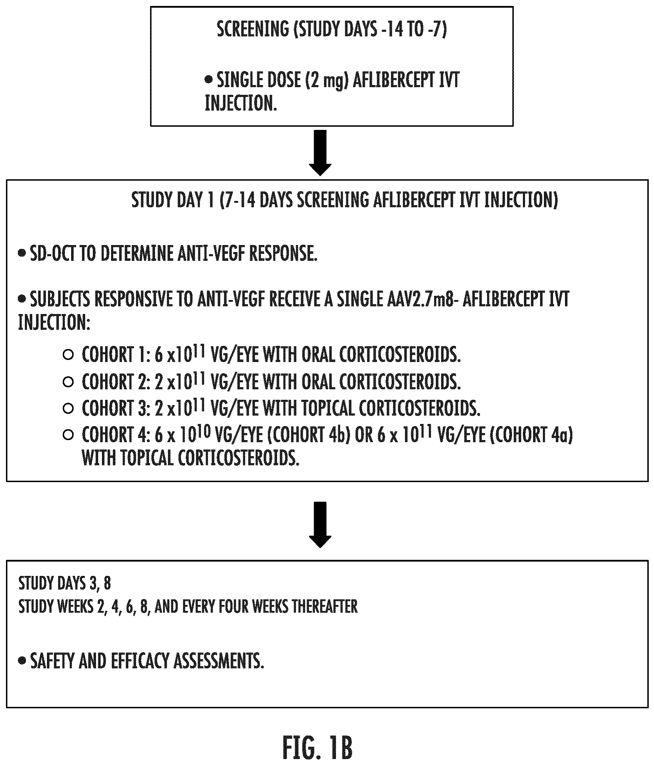

**FIGURES 1A-1B** provide schematics of the investigational medicinal product and the phase I study described in Examples 1 and 2. **FIG. 1A** is a schematic of AAV2.7m8-aflibercept. AAV2.7m8-aflibercept is a recombinant, replication-deficient adeno-associated viral (rAAV) vector containing the AAV2.7m8 protein capsid and a vector genome containing an expression cassette of a codon-optimized version of the aflibercept cDNA under the control of a ubiquitous chimeric promoter (C11). The AAV2.7m8-aflibercept vector genome also contains two AAV2 inverted terminal repeat sequences (ITR) flanking the aflibercept cDNA expression cassette. **FIG. 1B** is a diagram summarizing the study design for the phase I study described in Examples 1 and 2.

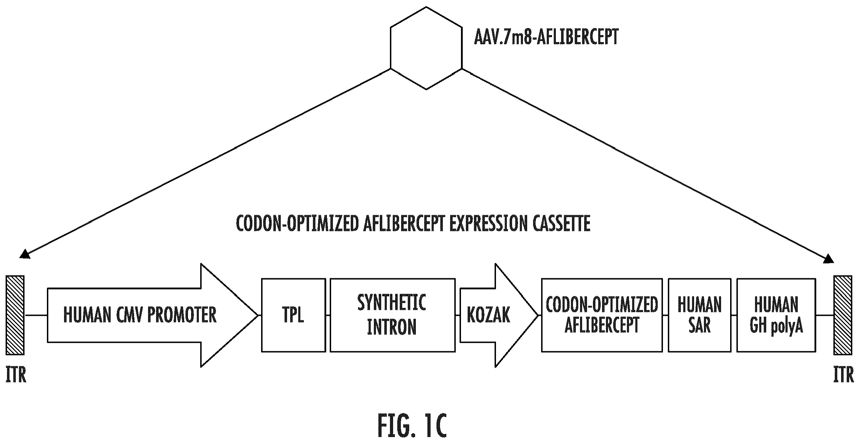

**FIGURE 1C** provides a diagram of the AAV2.7m8-aflibercept vector genome (SEQ ID NO: 39). The vector genome comprises two inverted terminal repeats (ITRs) of AAV serotype 2 (at positions 1-145 and 3772-3916 of SEQ ID NO: 39), an expression cassette comprised of the CMV promoter (at positions 180-693 of SEQ ID NO: 39), a 5' Untranslated Region (UTR) comprised of Adenovirus Tripartite Leader Sequence and Synthetic Intron (at positions 694-1314 of SEQ ID NO: 39), a Kozak sequence (at positions 1329-1340 of SEQ ID NO: 39), a codon-optimized aflibercept cDNA (at positions 1338-2714 of SEQ ID NO: 39), a 3' UTR comprised of human scaffold attachment region (at positions 2717-3527 of SEQ ID NO: 39), and human growth hormone polyadenylation/transcription stop signal (at positions 3546-3748 of SEQ ID NO: 39). AAV = adeno-associated virus; CMV = cytomegalovirus; GH = growth hormone; ITR = inverted terminal repeat; polyA = polyadenylation; SAR = scaffold-attached region; TPL = tripartite leader sequence.

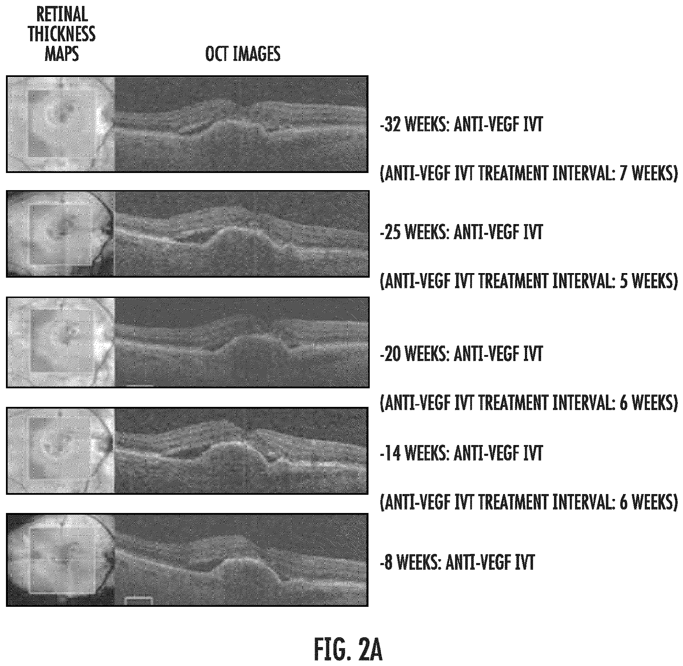

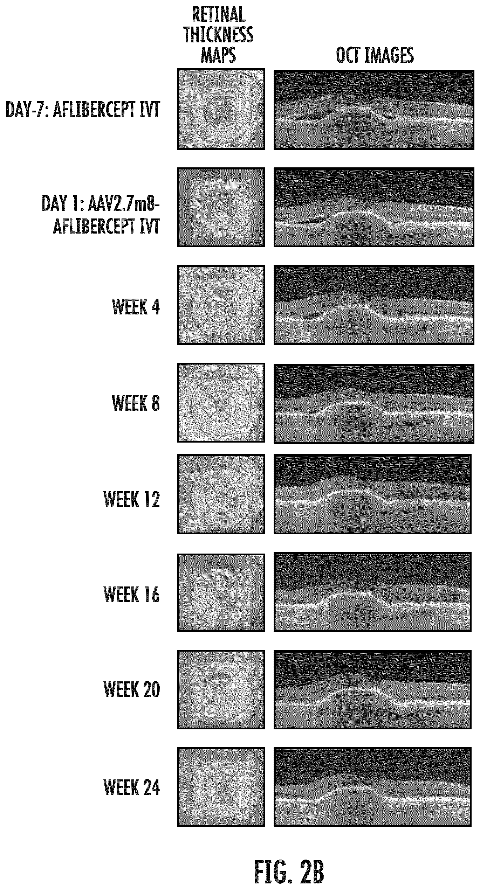

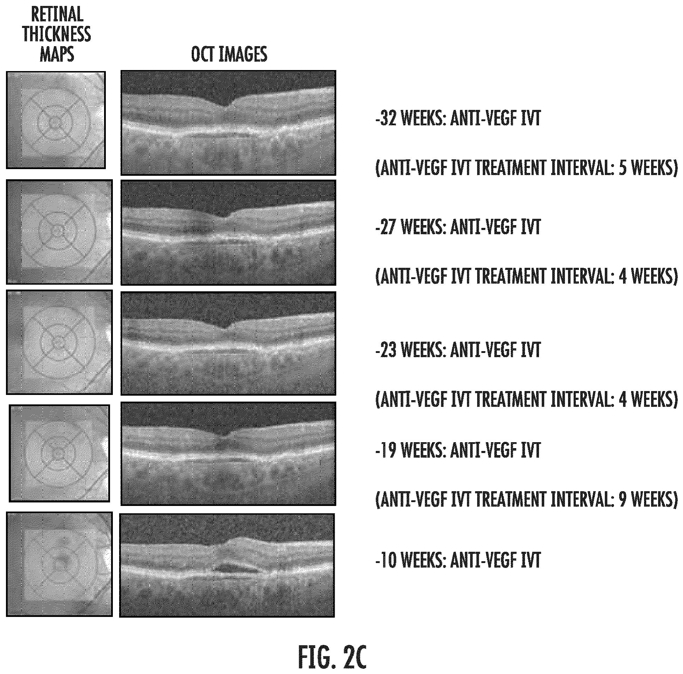

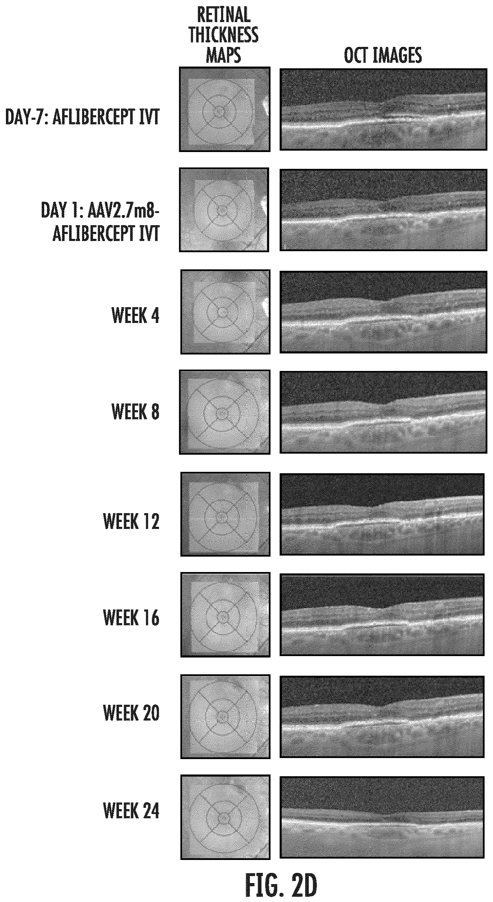

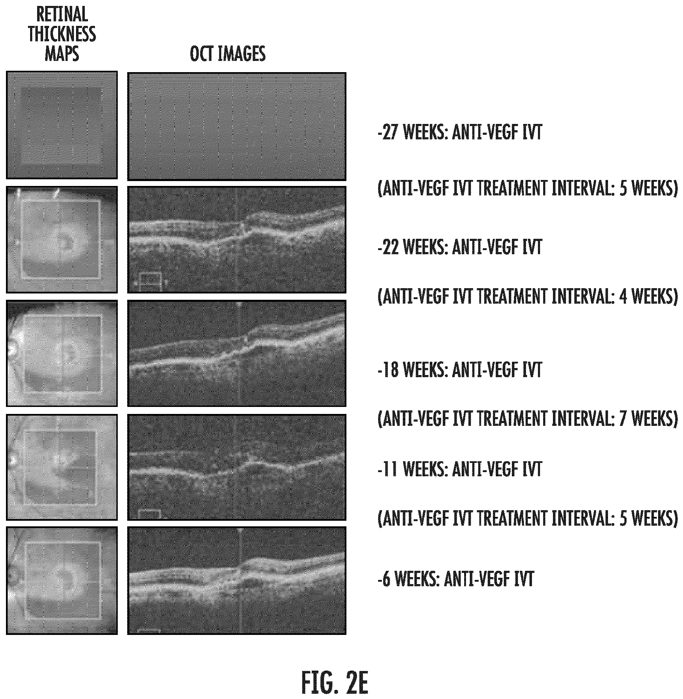

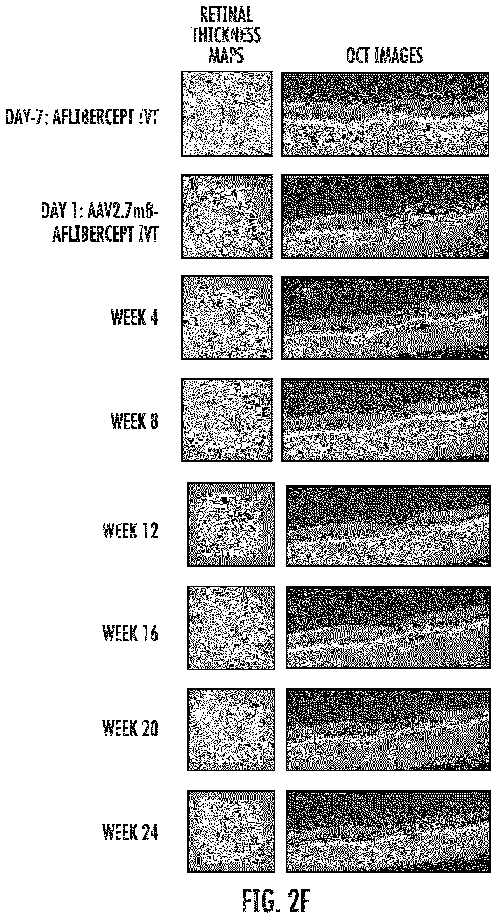

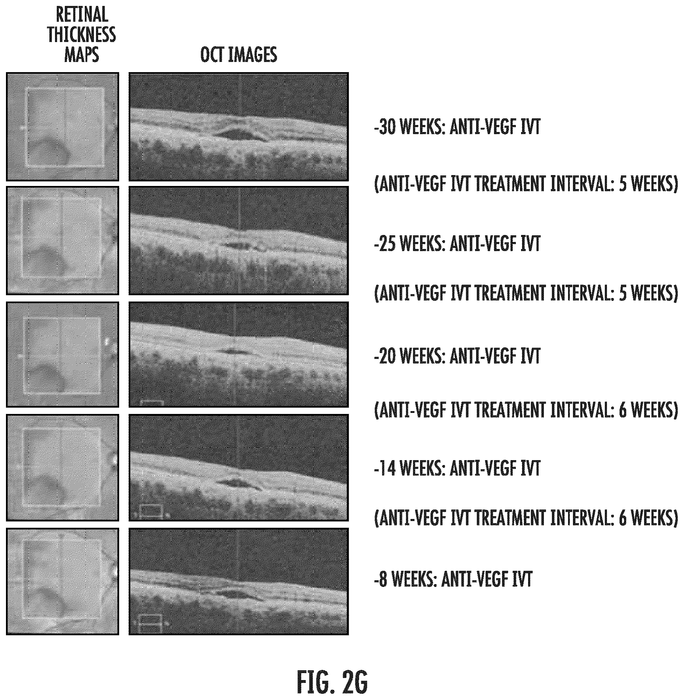

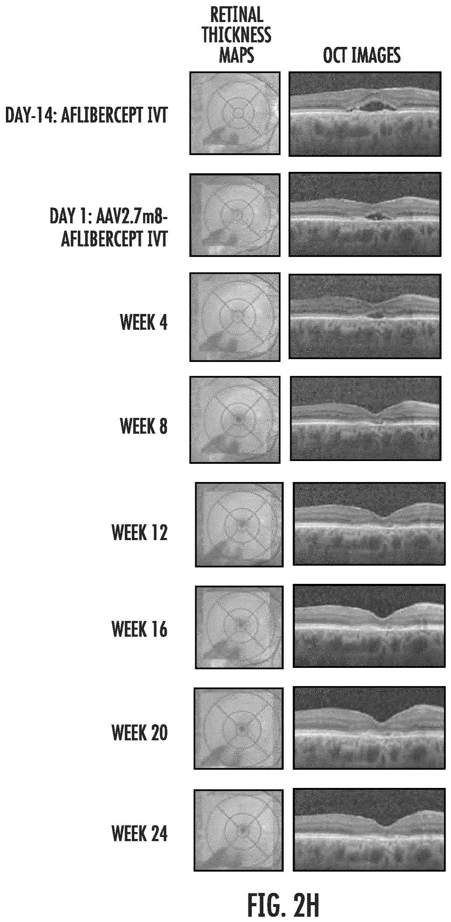

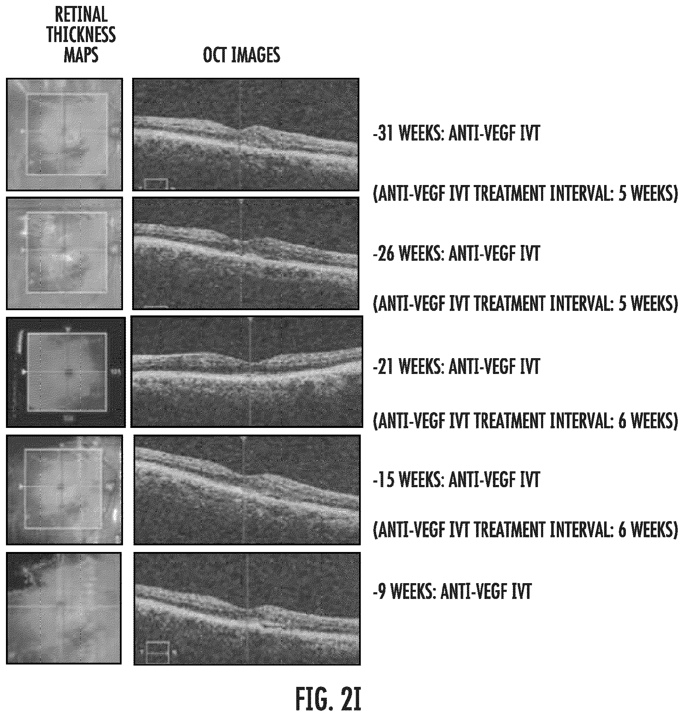

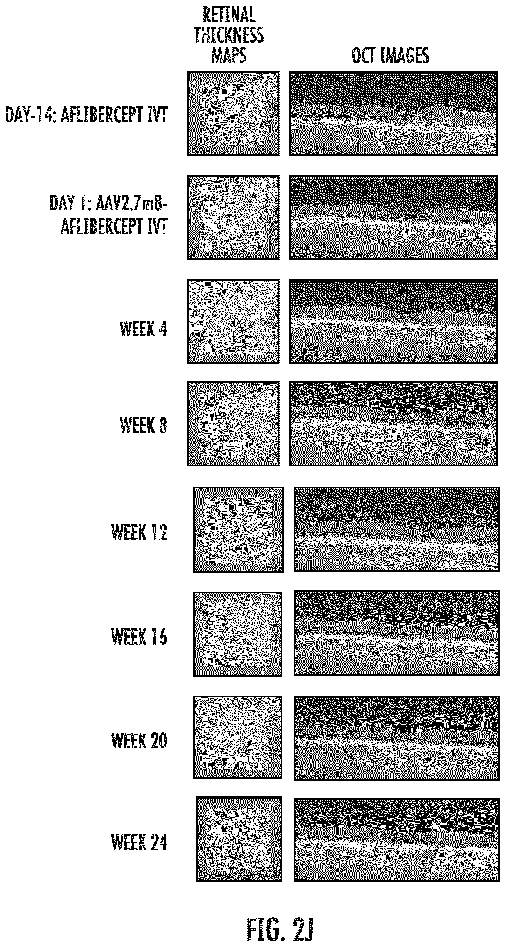

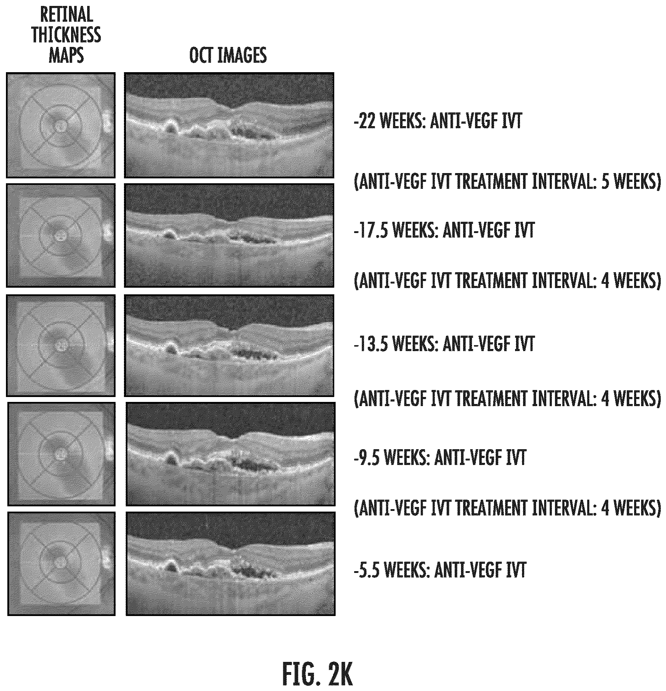

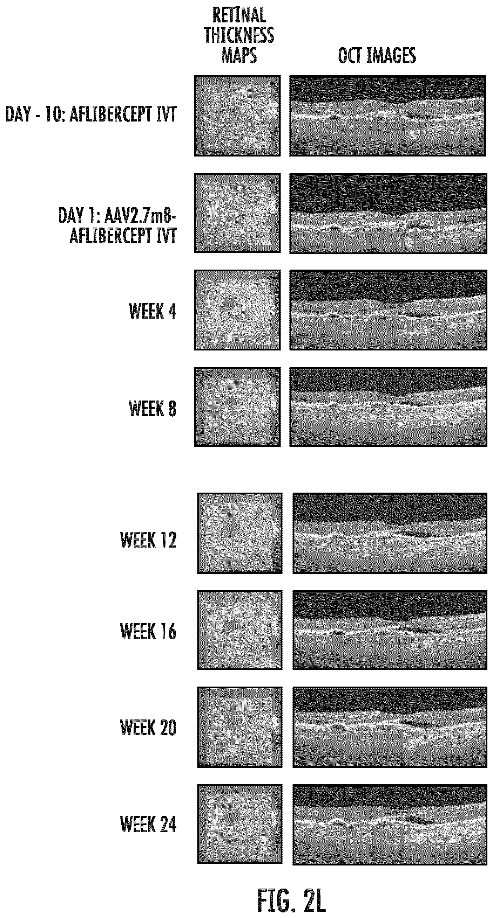

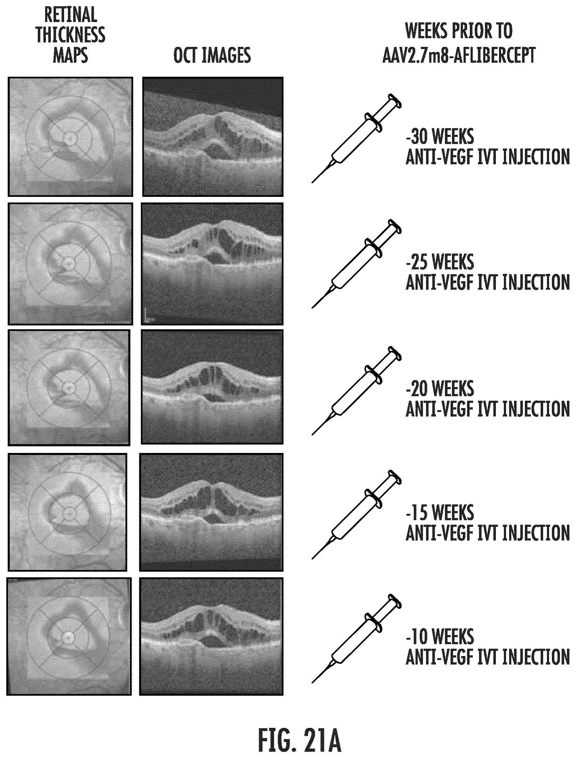

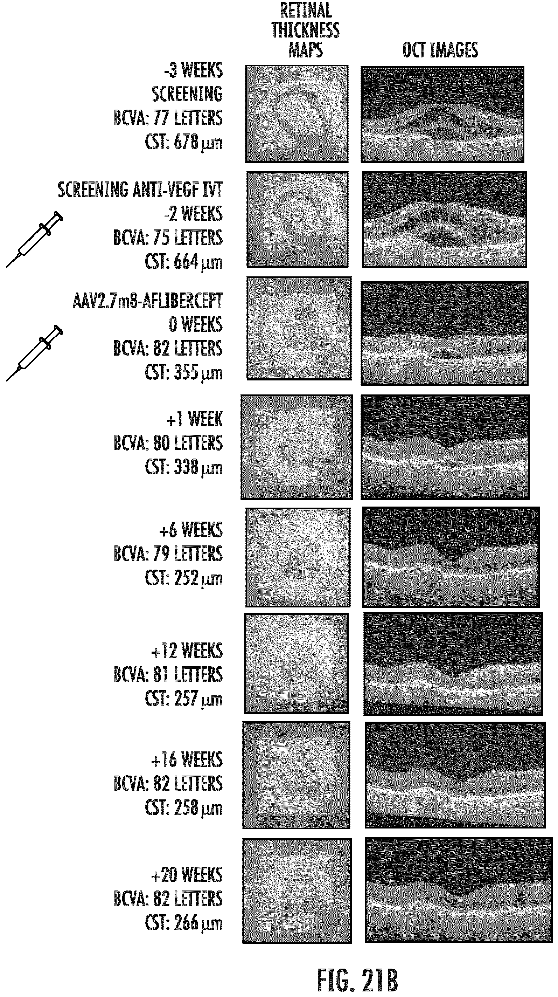

**FIGURES 2A-2L** show optical coherence tomography (OCT) images and retinal thickness maps derived from OCT images taken from subjects in Cohort 1 of the study described in Example 1. The OCT images were taken at the indicated times before and after administration of AAV2.7m8-aflibercept (Day 1). The anti-VEGF IVT treatment interval is indicated for all subjects. **FIG. 2A** provides OCT images and retinal thickness maps derived from OCT images taken from Subject 1 at five office visits at the times indicated prior to the Screening aflibercept injection. OCT images were taken immediately prior to treatment with aflibercept standard of care. Subject 1 required aflibercept IVT every 5-7 weeks and exhibited refractory subretinal fluid and a pigment epithelial detachment (PED) despite aflibercept standard of care treatment. **FIG. 2B** provides OCT images and retinal thickness maps derived from OCT images taken from Subject 1 at the Screening aflibercept injection (Day -7), at the AAV2.7m8-aflibercept injection (Day 1), and at follow-up visits at the times indicated. Subject 1 did not require any rescue injections after the AAV2.7m8-aflibercept injection. Subject 1 exhibited resolution of subretinal fluid beginning at week 4, and remained free of subretinal and intraretinal fluid (remained dry). **FIG. 2C** provides OCT images and retinal thickness maps derived from OCT images taken from Subject 2 at five office visits at the times indicated prior to the Screening aflibercept injection. OCT images were taken immediately prior to treatment with aflibercept standard of care. Subject 2 required six aflibercept IVT treatments in the 8 months prior to AAV2.7m8-aflibercept treatment to maintain retinal anatomy. **FIG. 2D** provides OCT images and retinal thickness maps derived from OCT images taken from Subject 2 at the Screening aflibercept injection (Day -7), at the AAV2.7m8-aflibercept injection (Day 1), and at follow-up visits at the times indicated. Subject 2 did not require any rescue injections after the AAV2.7m8-aflibercept injection. Subject 2 exhibited stable retinal anatomy with no subretinal or intraretinal fluid through week 24. **FIG. 2E** provides OCT images and retinal thickness maps derived from OCT images taken from Subject 3 at four office visits at the times indicated prior to the Screening aflibercept injection. OCT images taken on Week -27 are not shown. OCT images were taken immediately prior to treatment with aflibercept standard of care. Subject 3 exhibited subretinal fluid, which increased when the interval between aflibercept IVT was increased from 5 to 7 weeks. **FIG. 2F** provides OCT images and retinal thickness maps derived from OCT images taken from Subject 3 at the Screening aflibercept injection (Day -7), the AAV2.7m8-aflibercept injection (Day 1), and at follow-up visits at the times indicated. Subject 3 did not require any rescue injections after the AAV2.7m8-aflibercept injection. Subject 3 exhibited resolution of refractory subretinal fluid by week 8, and stable retinal anatomy through week 24. **FIG. 2G** provides OCT images and retinal thickness maps derived from OCT images taken from Subject 4 at five office visits at the times indicated prior to the Screening aflibercept injection. OCT images were taken immediately prior to treatment with ranibizumab 0.5 mg IVT standard of care. Subject 4 exhibited subretinal fluid that was refractory to ranibizumab IVT injections. **FIG. 2H** provides OCT images and retinal thickness maps derived from OCT images taken from Subject 4 at the Screening aflibercept injection (Day -14), the AAV2.7m8-aflibercept injection (Day 1), and at follow-up visits at the times indicated. Subject 4 did not require any rescue injections after the AAV2.7m8-aflibercept injection. Subject 4 exhibited resolution of refractory subretinal fluid by week 8, and stable retinal anatomy through week 24. **FIG. 21** provides OCT images and retinal thickness maps derived from OCT images taken from Subject 5 at five office visits at the times indicated prior to the Screening aflibercept injection. OCT images were taken immediately prior to treatment with aflibercept standard of care. **FIG. 2J** provides OCT images and retinal thickness maps derived from OCT images taken from Subject 5 at the Screening aflibercept injection (Day - 14), the AAV2.7m8-aflibercept injection (Day 1), and at follow-up visits at the times indicated. Subject 5 did not require any rescue injections after the AAV2.7m8-aflibercept injection. Subretinal fluid and PEDs present at the time of AAV2.7m8-aflibercept treatment resolved overtime, and retinal anatomy remained stable and free of subretinal or intraretinal fluid through week 24. **FIG. 2K** provides OCT images and retinal thickness maps derived from OCT images taken from Subject 6 at five office visits at the times indicated prior to the Screening aflibercept injection. OCT images were taken immediately prior to treatment with either bevacizumab 1.5 mg IVT standard of care or ranibizumab 0.5 mg IVT standard of care, as indicated. The appearance of Subject 6's retina was consistent with polypoidal choroidal vasculopathy (PCV). **FIG. 2L** provides OCT images and retinal thickness maps derived from OCT images taken from Subject 6 at the Screening aflibercept injection (Day -10), the AAV2.7m8-aflibercept injection (Day 1), and at follow-up visits at the times indicated. Subject 6 did not require any rescue injections after the AAV2.7m8-aflibercept injection. Subject 6 did not exhibit any increase in subretinal fluid and achieved some anatomical improvement through week 24. The contralateral eye of Subject 6 received standard of care aflibercept injections every 4 weeks over the course of the trial, and exhibited similar retinal morphology to the AAV2.7m8-aflibercept-treated eye.

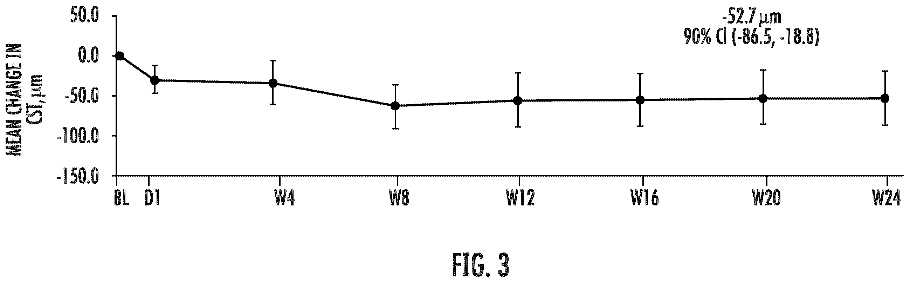

**FIGURE 3** shows the change in mean central retinal thickness (CST) for subjects in Cohort 1 of the study described in Example 1 at the indicated time points. Error bars indicate the 90% confidence interval, calculated using the T-distribution. Baseline (BL) indicates the measurement taken prior to the Screening aflibercept injection 7 to 15 days (e.g., 7-14 days) prior to AAV2.7m8-aflibercept treatment on day 1. At 24 weeks post AAV2.7m8-aflibercept treatment, subjects on average exhibited a change in CRT of -52.7 µm (90% CI -86.5, -18.8). BL = baseline; D = day; W = week. The day 1 visit occurred 7-14 days after the baseline visit.

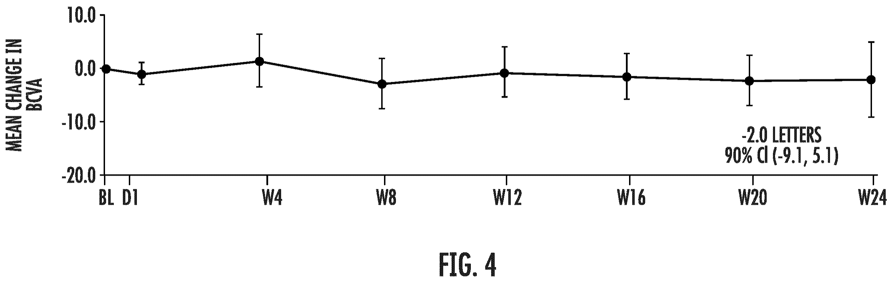

**FIGURE 4** shows the mean best corrected visual acuity (BCVA) measurements based on Early Treatment Diabetic Retinopathy Study (ETDRS) letters assessments for subjects in Cohort 1 of the study described in Example 1 at the indicated time points. Error bars indicate the 90% confidence interval, calculated using the T-distribution. Baseline (BL) indicates the measurement taken prior to the Screening aflibercept injection 7 to 15 days (e.g., 7-14 days) prior to AAV2.7m8-aflibercept treatment on day 1. At 24 weeks post AAV2.7m8-aflibercept treatment, subjects on average exhibited a change in BCVA of -2 letters (90% CI -9.1, 5.1). BL = baseline; D = day; W = week. The day 1 visit occurred 7-14 days after the baseline visit.

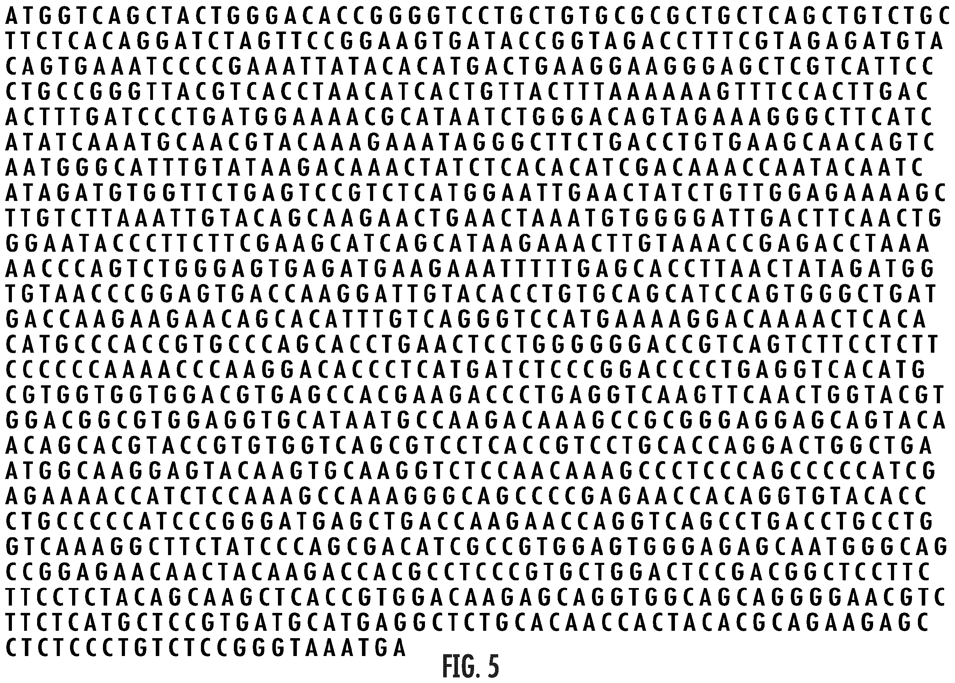

**FIGURE 5** provides the nucleic acid sequence of aflibercept (SEQ ID NO: 36).

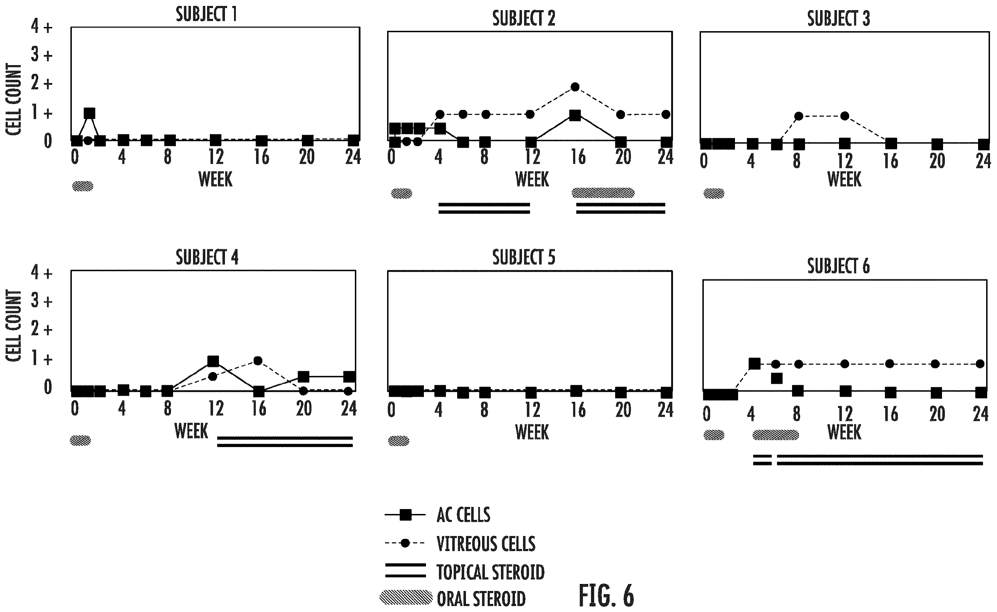

**FIGURE 6** shows plots of anterior chamber cell and vitreous cell counts following treatment with AAV2.7m8-aflibercept for subjects 1-6 of the study described in Example 1. The steroid treatment administered to each patient is indicated below each plot. Aqueous cell count categories were based on the Standardization of Uveitis Nomenclature (SUN) criteria (Jabs, DA et al., J Ophthalmol. 2005;140:509-516). Vitreous cell count categories were based on National Institutes of Health (NIH) guidelines. For aqueous cells, a cell count value of 0.5+ indicates 1-5 cells; a cell count value of 1+ indicates 6-15 cells; a cell count value of 2+ indicates 16-25 cells; a cell count value of 3+ indicates 26-50 cells; and a cell count value of 4+ indicates >50 cells. For vitreous cells, a cell count value of 0.5+ indicates 1-10 cells; a cell count value of 1+ indicates 11-20 cells; a cell count value of 2+ indicates 21-30 cells; a cell count value of 3+ indicates 31-100 cells; and a cell count value of 4+ indicates >100 cells. Rare cells were captured as 0.5+ for the analysis shown in this figure.

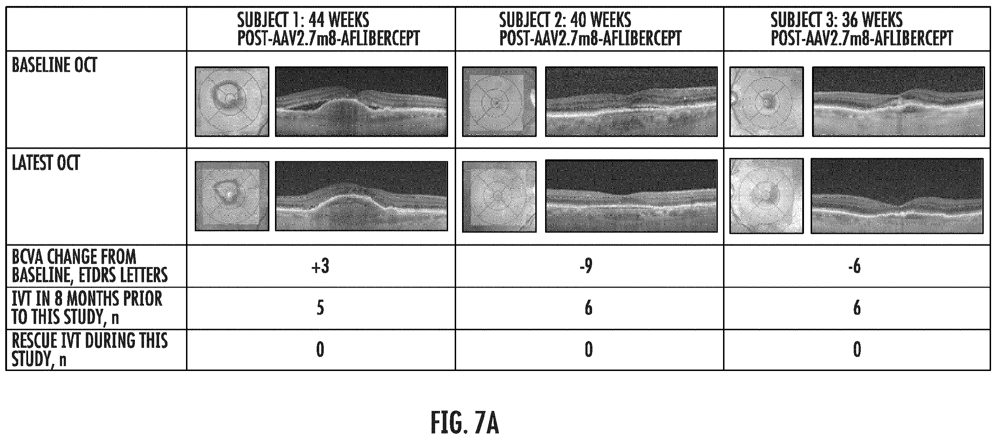

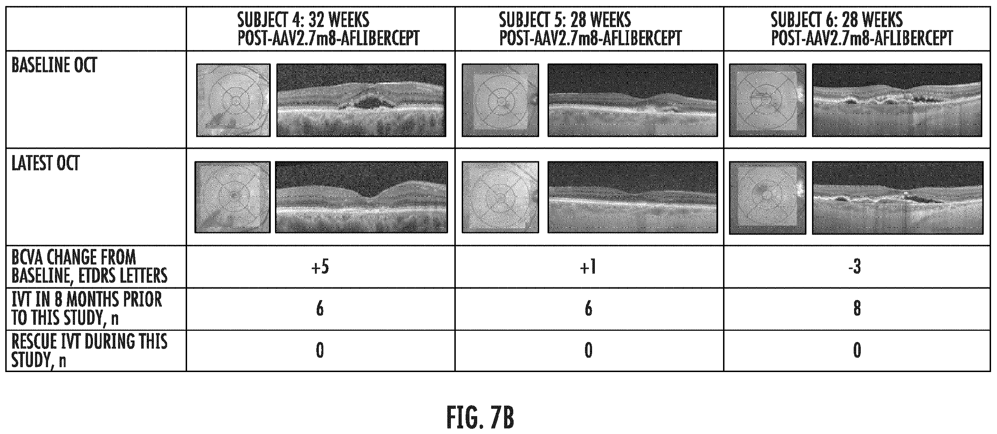

**FIGURES 7A-7B** show optical coherence tomography (OCT) images and retinal thickness maps derived from OCT images taken from subjects 1-6 in Cohort 1 of the study described in Example 1 at a median follow up time of 34 weeks. In addition, the change in BCVA from Baseline, the number of anti-VEGF IVT injections in the 8 months prior to administration of AAV2.7m8-aflibercept, and the number of administered rescue anti-VEGF IVT injections during the study are also provided for each of subjects 1-6. The actual week during which the OCT images and retinal thickness maps were obtained for each subject are indicated (Subject 1 = Week 44; Subject 2 = Week 40; Subject 3 = Week 36; Subject 4 = Week 32; Subject 5 = Week 28; and Subject 6 = Week 28). No subjects required rescue anti-VEGF IVT injections during the study and no retreatment criteria were met at any point during the follow-up period of up to 44 weeks. No subjects exhibited signs of disease re-activation on OCT imaging.

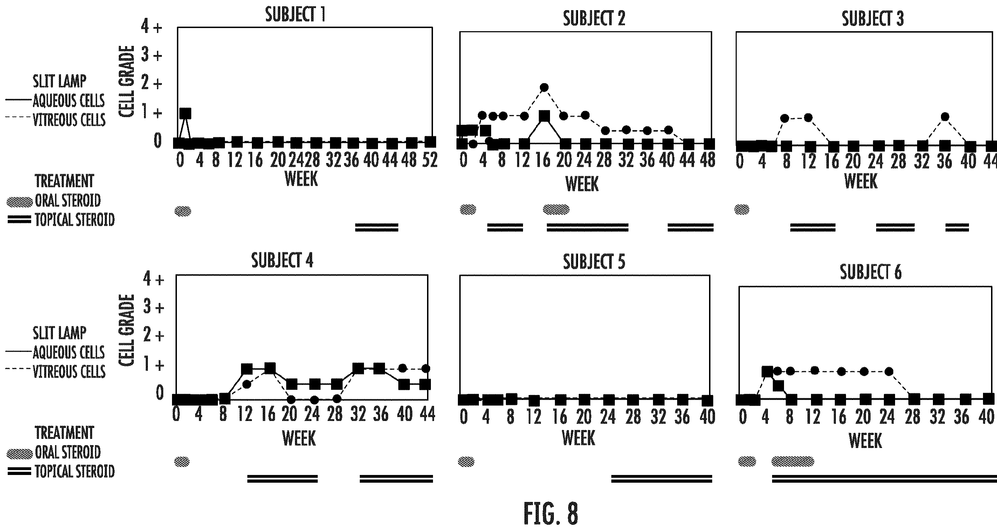

**FIGURE 8** shows plots of aqueous and vitreous cell counts following treatment with AAV2.7m8-aflibercept for subjects 1-6 in Cohort 1 of the study described in Example 1 up to a median follow-up time of 44 weeks (40-52 weeks). The steroid treatment administered to each subject is indicated below each plot. Aqueous cell grade categories were based on the Standardization of Uveitis Nomenclature (SUN) criteria (Jabs, DA et al. J Ophthalmol. 2005; 140:509-516). Vitreous cell grade categories were based on National Institutes of Health (NIH) guidelines. For aqueous cells, a cell grade of 0.5+ indicates 1-5 cells; a cell grade of 1+ indicates 6-15 cells; a cell grade of 2+ indicates 16-25 cells; a cell grade of 3+ indicates 26-50 cells; and a cell grade of 4+ indicates >50 cells. For vitreous cells, a cell grade of 0.5+ indicates 1-10 cells; a cell grade of 1+ indicates 11-20 cells; a cell grade of 2+ indicates 21-30 cells; a cell grade of 3+ indicates 31-100 cells; and a cell grade of 4+ indicates >100 cells. Rare cells were captured as a cell grade of 0.5+.

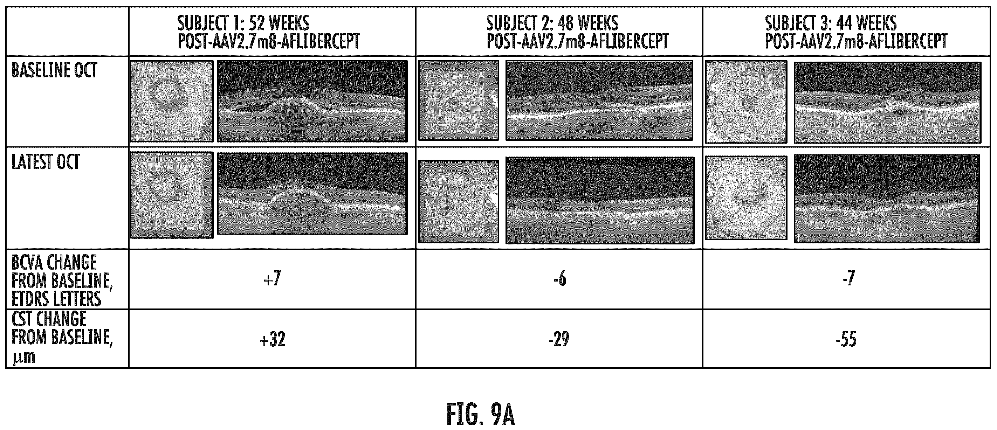

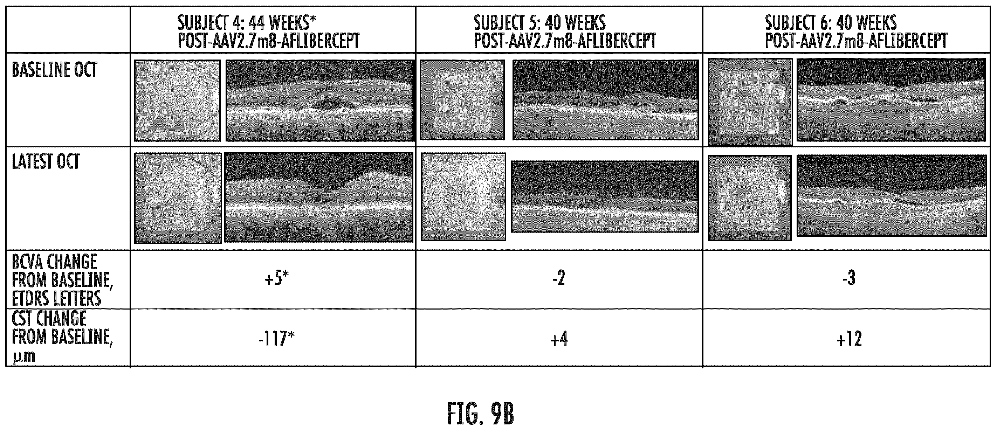

**FIGURES 9A-9B** show optical coherence tomography (OCT) images and retinal thickness maps derived from OCT images taken from subjects 1-6 in Cohort 1 of the study described in Example 1 at a median follow-up time of 44 weeks. The change in BCVA ETDRS letters and CST from Baseline are also provided. The actual weeks during which the OCT images and retinal thickness maps were obtained for each subject are indicated. The asterisk indicates that Subject 4 underwent retinal detachment repair with gas bubble injection in order to repair a spontaneous pseudophakic inferior macula off rhegmatogenous retinal detachment (RRD) **(****FIG. 9B****).** Subject 4 remains under follow-up; OCT images and BCVA values for Subject 4 correspond to the last observations prior to retinal detachment.

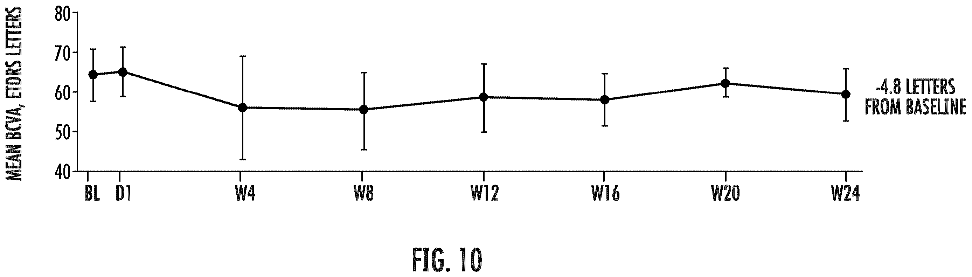

**FIGURE 10** shows the mean best corrected visual acuity (BCVA) measurements based on Early Treatment Diabetic Retinopathy Study (ETDRS) letters assessments for subjects in Cohort 2 of the study described in Examples 2 and 3 at the indicated time points. Error bars indicate the 90% confidence interval of the mean absolute BCVA calculated using the T-distribution. Baseline (BL) indicates the measurement taken prior to the Screening aflibercept injection 7 to 15 days (e.g., 7-14 days) prior to AAV2.7m8-aflibercept treatment on day 1. At 24 weeks post AAV2.7m8-aflibercept treatment, subjects exhibited a mean change in BCVA of -4.8 letters. BL = baseline; D = day; W = week. The day 1 visit occurred 7-15 days after the baseline visit.

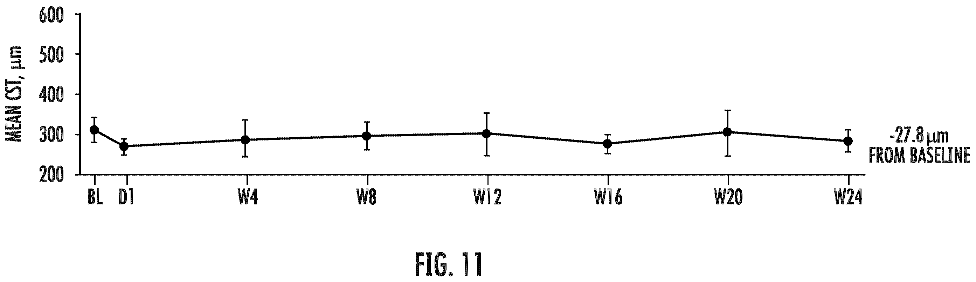

**FIGURE 11** shows the mean central retinal thickness (CST) for subjects in Cohort 2 of the study described in Examples 2 and 3 at the indicated time points. Error bars indicate the 90% confidence interval of the mean absolute CST calculated using the T-distribution. Baseline (BL) indicates the measurement taken prior to the Screening aflibercept injection 7 to 15 days (e.g., 7-14 days) prior to AAV2.7m8-aflibercept treatment on day 1. At 24 weeks post AAV2.7m8-aflibercept treatment, subjects exhibited a mean change in CST of -27.8 µm. BL = baseline; D = day; W = week. The day 1 visit occurred 7-15 days after the baseline visit.

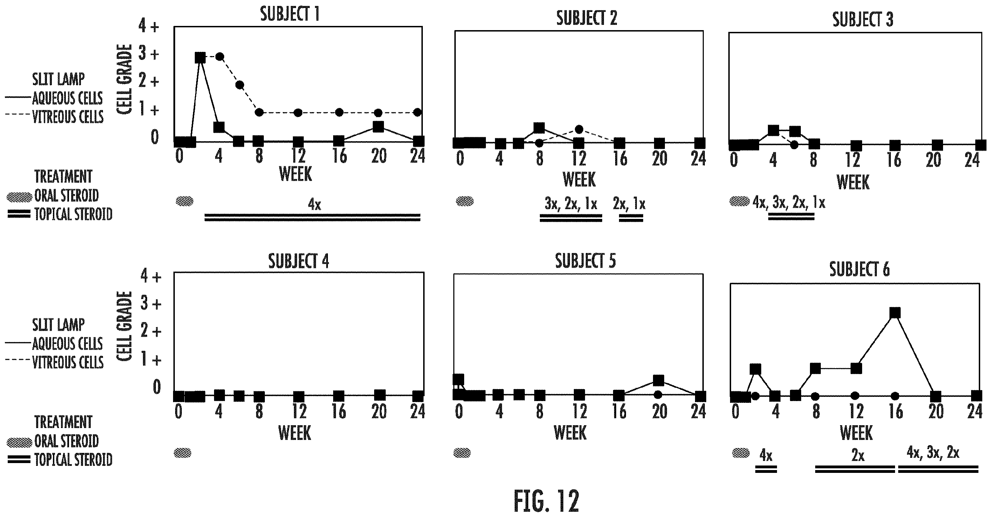

**FIGURE 12** shows plots of aqueous cell and vitreous cell counts following treatment with AAV2.7m8-aflibercept for subjects in Cohort 2 of the study described in Examples 2 and 3 up to a follow-up time of 24 weeks. The steroid treatment administered to each subject is indicated below each plot; the frequency of topical steroid (difluprednate) eye drop administration is indicated (e.g., 1x = once per day; 2x = twice per day; 3x = 3 times per day; 4x = 4 times per day). Aqueous cell grade categories were based on the Standardization of Uveitis Nomenclature (SUN) criteria (Jabs, DA et al. J Ophthalmol. 2005; 140:509-516). Vitreous cell grade categories were based on National Institutes of Health (NIH) guidelines. For aqueous cells, a cell grade of 0.5+ indicates 1-5 cells; a cell grade of 1+ indicates 6-15 cells; a cell grade of 2+ indicates 16-25 cells; a cell grade of 3+ indicates 26-50 cells; and a cell grade of 4+ indicates >50 cells. For vitreous cells, a cell grade of 0.5+ indicates 1-10 cells; a cell grade of 1+ indicates 11-20 cells; a cell grade of 2+ indicates 21-30 cells; a cell grade of 3+ indicates 31-100 cells; and a cell grade of 4+ indicates >100 cells. Rare cells were captured as a cell grade of 0.5+ for this analysis.

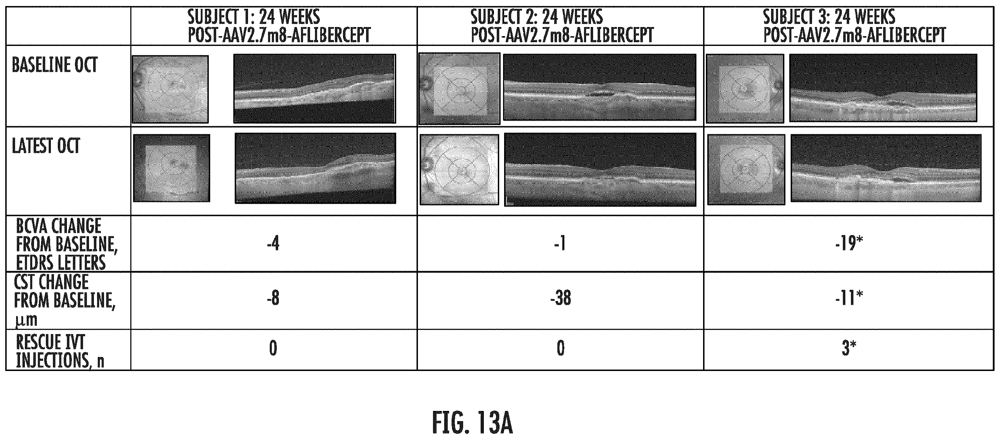

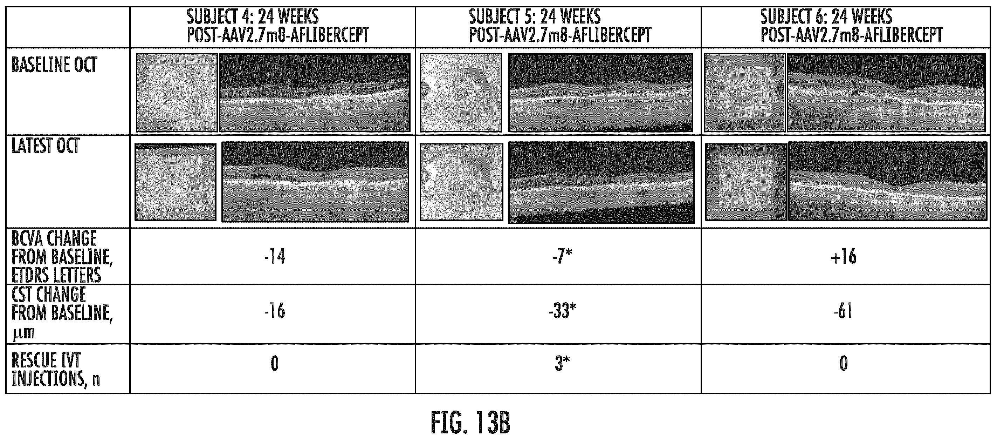

**FIGURES 13A-13B** show optical coherence tomography (OCT) images and retinal thickness maps derived from OCT images taken from subjects 1-6 in Cohort 2 of the study described in Examples 2 and 3 up to a follow-up time of 24 weeks. The change in BCVA from Baseline, the change in CST from baseline, and the number of rescue anti-VEGF injections administered during the 24-week follow up period are also provided. In **FIG. 13A****,** the asterisks indicate that Subject 3 received 3 rescue anti-VEGF injections, including at week 24, all due to loss of ≥10 letters in BCVA from baseline. This was attributed to intraretinal or subretinal fluid. In **FIG. 13B****,** the asterisks indicate that Subject 5 received 3 rescue anti-VEGF injections, the last of which occurred on week 20, due to an increase in central subfield thickness of >75µm from baseline.

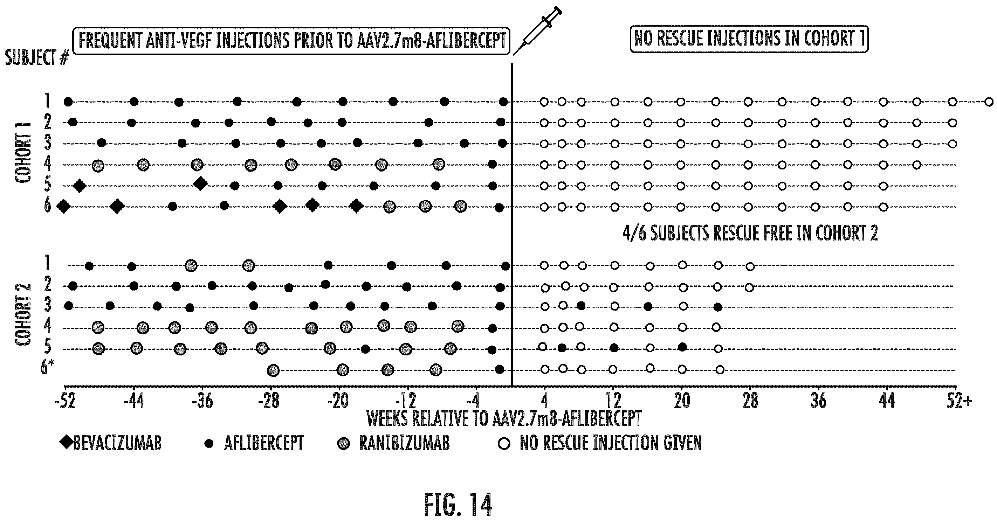

**FIGURE 14** is a Swimmer's Lane Plot showing the number of anti-VEGF injections for subjects in Cohorts 1 and 2 of the study described in Examples 1-3. The x-axis represents time in weeks relative to the time AAV2.7m8-aflibercept was administered. The y-axis shows each individual subject in Cohorts 1 and 2. The circular shapes represent anti-VEGF IVT injections administered before and after treatment with AAV2.7m8-aflibercept. The asterisk indicates that Subject 6 of Cohort 2 was diagnosed with nAMD 6.4 months prior to administration of AAV2.7m8-aflibercept. The vertical line bisecting the plot indicates day 1, when AAV2.7m8-aflibercept was administered. To the right of the bisecting vertical line are each of the subsequent 61 study visits.

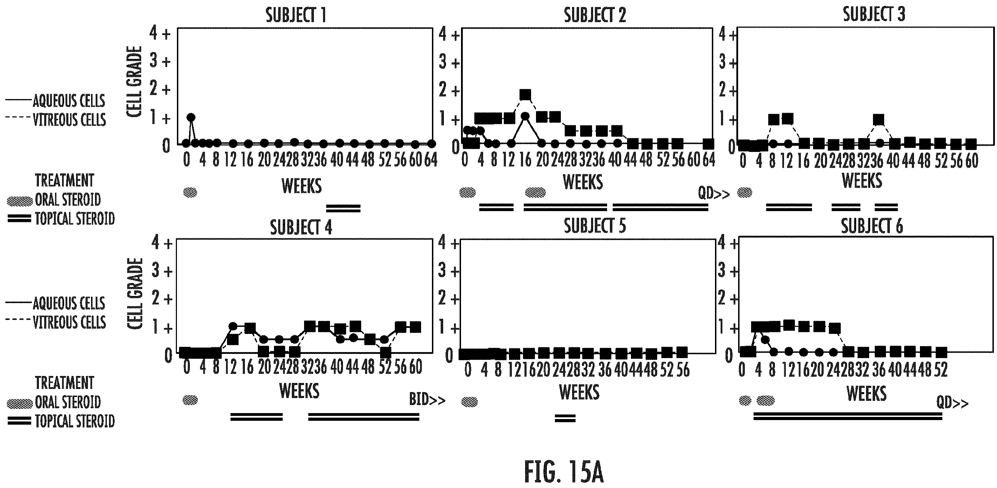

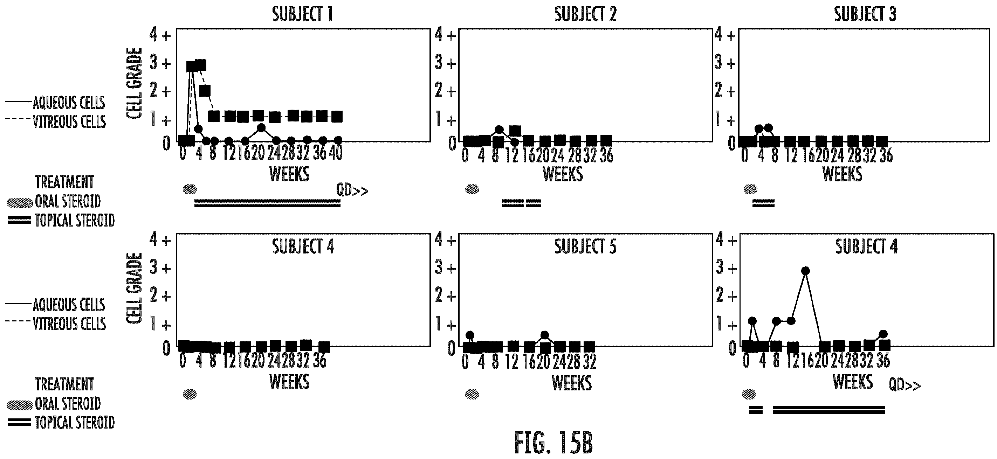

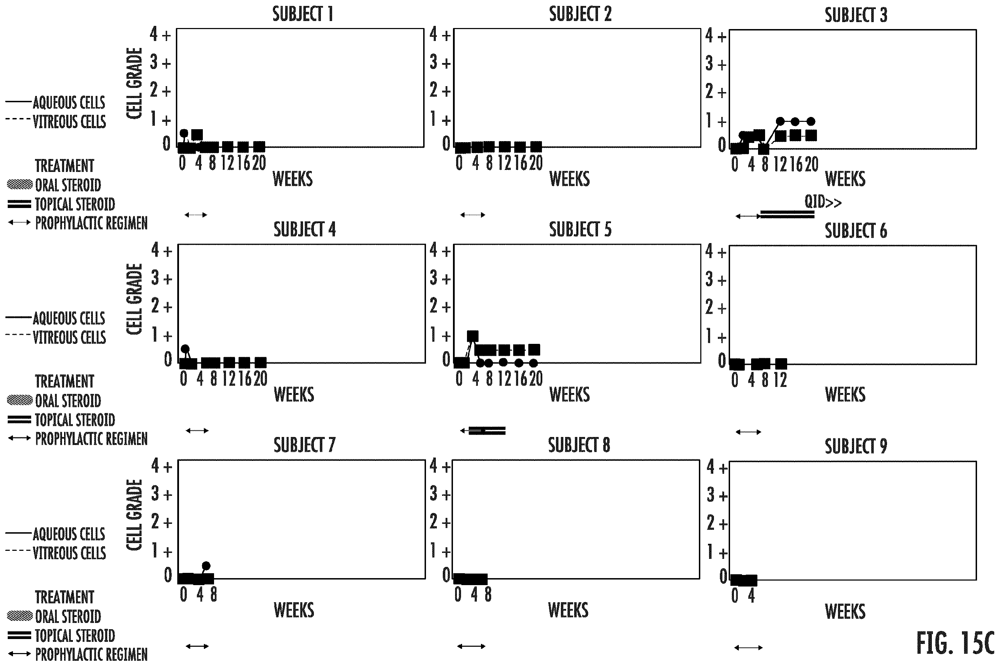

**FIGURES 15A-15C** show plots of aqueous cell and vitreous cell counts as measured by slit lamp examination following treatment with AAV2.7m8-aflibercept for subjects in Cohorts 1, 2 and 3 of the study described in Examples 1-5. **FIG. 15A** shows plots of aqueous cell and vitreous cell counts for subjects in Cohort 1 up to a follow up time of 64 weeks (median = 60 weeks; range = 52-64 weeks). **FIG. 15B** shows plots of aqueous cell and vitreous cell counts for subjects in Cohort 2 up to a follow up time of 40 weeks (median = 36 weeks; range = 32-40 weeks). **FIG. 15C** shows plots of aqueous cell and vitreous cell counts for subjects in Cohort 3 up to a follow up time of 20 weeks. In **FIGS. 15A-15C****,** the steroid treatment administered to each subject is indicated below each plot; aqueous cell grade categories were based on the Standardization of Uveitis Nomenclature (SUN) criteria: Jabs DA, et al. J Ophthalmol 2005;140:509-516; vitreous cell grade categories were based on National Institutes of Health (NIH) guidelines; aqueous cells: 0.5+ = 1-5 cells; 1+ = 6-15 cells; 2+ = 16-25 cells; 3+ = 26-50 cells; 4+ = >50 cells; vitreous cells: 0.5+ = 1-10 cells; 1+ = 11-20 cells; 2+ = 21-30 cells; 3+ = 31-100 cells; 4+ = >100 cells; rare cells were captured as 0.5+ for this analysis. QID = four times per day; TID = three time per day; BID = twice per day; QD = once per day; QOD = once every other day; "QD >>" indicates that the subject continues to receive topical steroids once per day; "BID >>" indicates that the subject continues to receive topical steroids twice per day; and "QID >>" indicates that the subject continues to receive topical steroids four times per day.

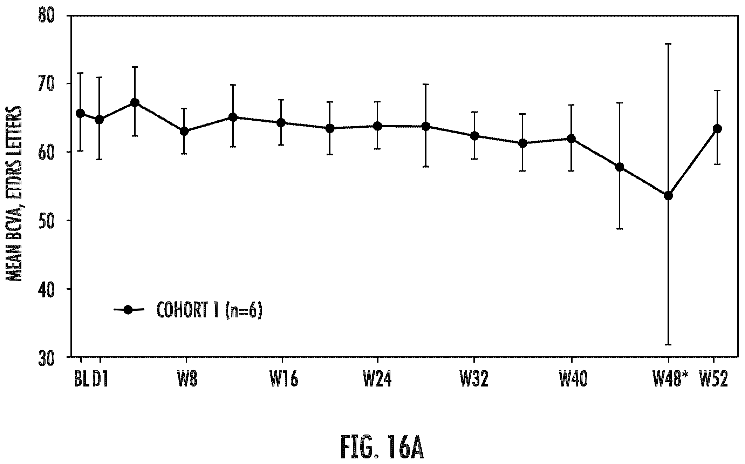

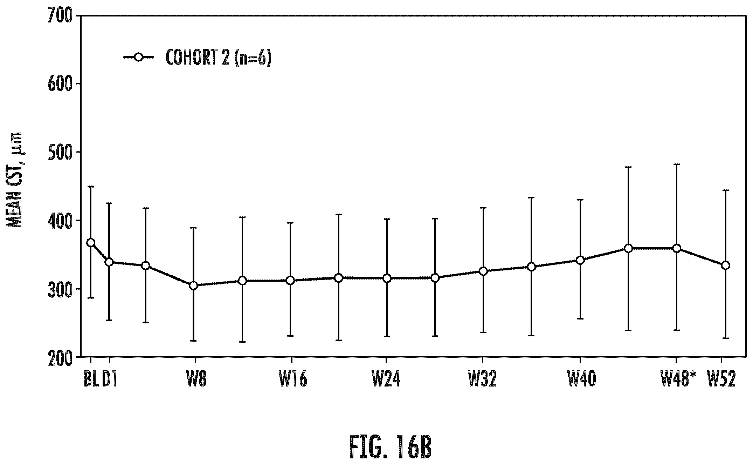

**FIGURES 16A-16B** provide the mean BCVA and mean CST for subjects in Cohort 1 of the study described in Examples 1-5 (n=6) from baseline up to Week 52. **FIG. 16A** shows the mean BCVA (ETDRS letters) from baseline up to Week 52. The asterisk indicates that one subject had low BCVA scores at Weeks 44 and 48 due to retinal detachment. **FIG. 16B** shows the mean CST (µm) from baseline up to Week 52. The asterisk indicates that one subject had no CST data at Weeks 44 and 48 due to retinal detachment. In **FIGS. 16A-16B****,** the error bars indicate the 90% confidence intervals of the mean absolute BCVA and CST values using the T-distribution; BL = baseline; D = day; W = week.

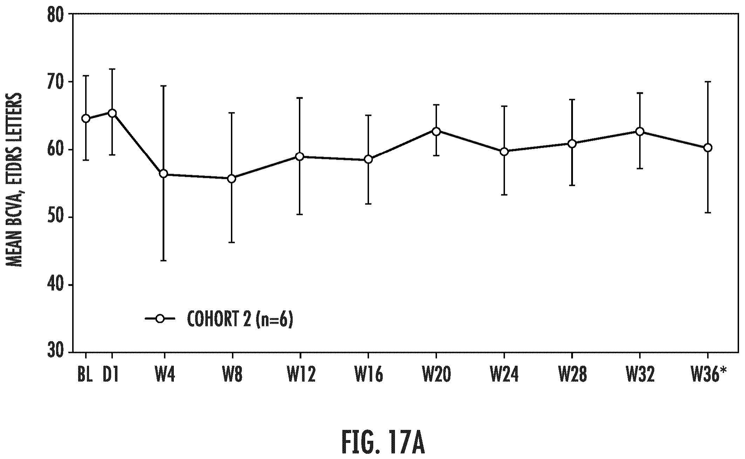

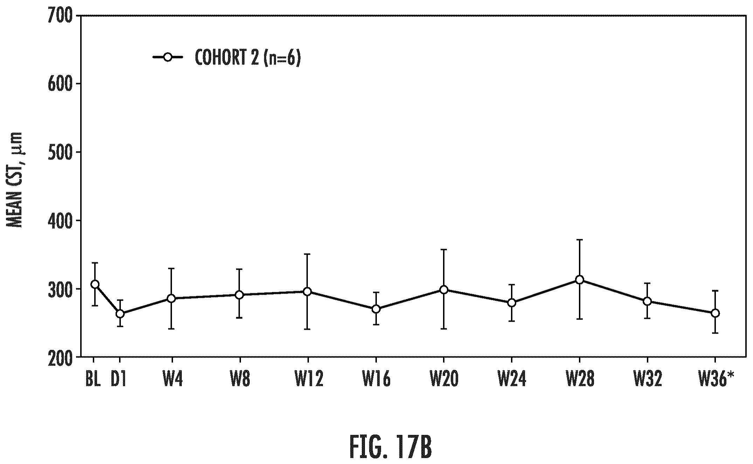

**FIGURES 17A-17B** provide the mean BCVA and mean CST for subjects in Cohort 2 of the study described in Examples 1-5 (n=6) from baseline up to Week 36. **FIG. 17A** shows the mean BCVA (ETDRS letters) from baseline up to Week 36. **FIG. 17B** shows the mean CST (µm) from baseline up to Week 36. In **FIGS. 17A-17B****,** the error bars indicate the 90% confidence intervals of the mean absolute BCVA and CST values using the T-distribution; BL = baseline; D = day; W = week; one subject missed the Week 36 visit.

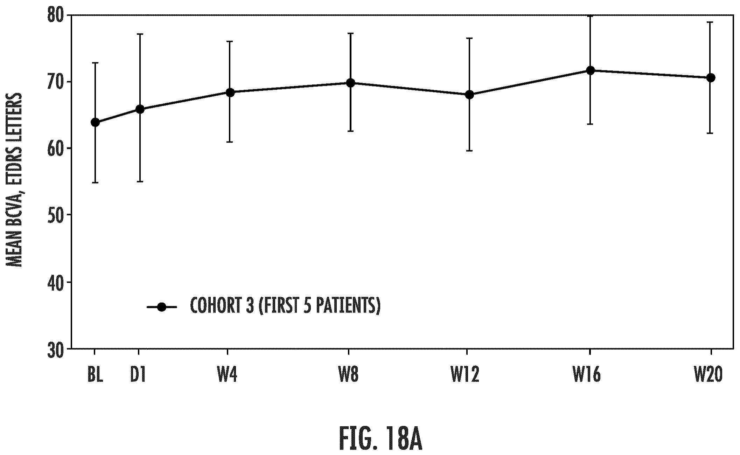

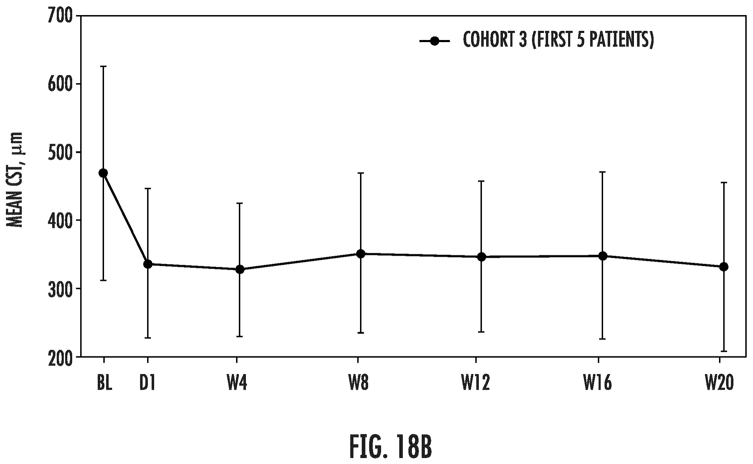

**FIGURES 18A-18B** provide the mean BCVA and mean CST for five subjects in Cohort 3 of the study described in Examples 1-5 from baseline up to Week 20. **FIG. 18A** shows the mean BCVA (ETDRS letters) from baseline up to Week 20. **FIG. 18B** shows the mean CST (µm) from baseline up to Week 20. In **FIGS. 18A-18B****,** the error bars indicate the 90% confidence intervals of the mean absolute BCVA and CST values using the T-distribution; BL = baseline; D = day; W = week; one subject missed the Week 36 visit.

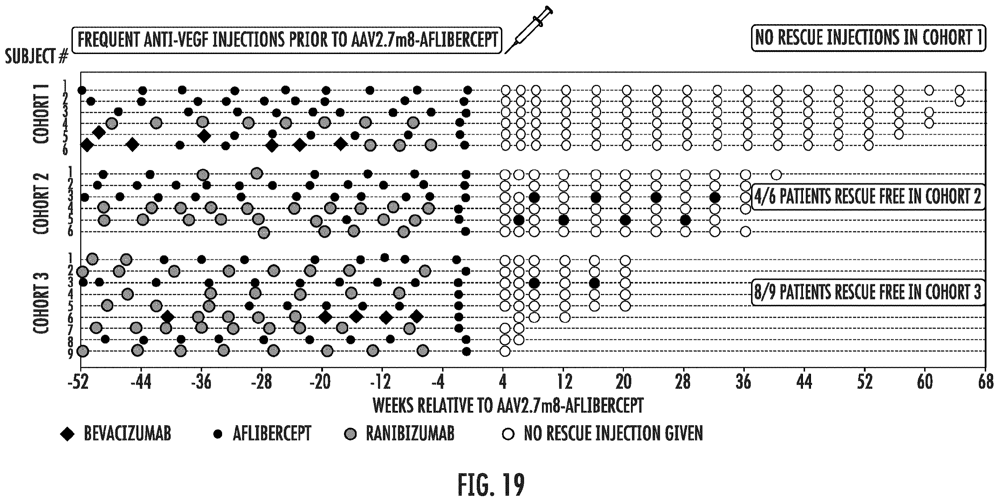

**FIGURES 19** is a Swimmer's Lane Plot showing the number of anti-VEGF injections administered to subjects in Cohorts 1, 2 and 3 of the study described in Examples 1-5. The x-axis represents time in weeks relative to the time AAV2.7m8-aflibercept was administered. The y-axis shows each individual subject in Cohorts 1-3. The circular shapes represent anti-VEGF IVT injections administered before and after treatment with AAV2.7m8-aflibercept. The vertical line bisecting the plot indicates Day 1, when AAV2.7m8-aflibercept was administered. To the right of the bisecting vertical line are each of the subsequent study visits.

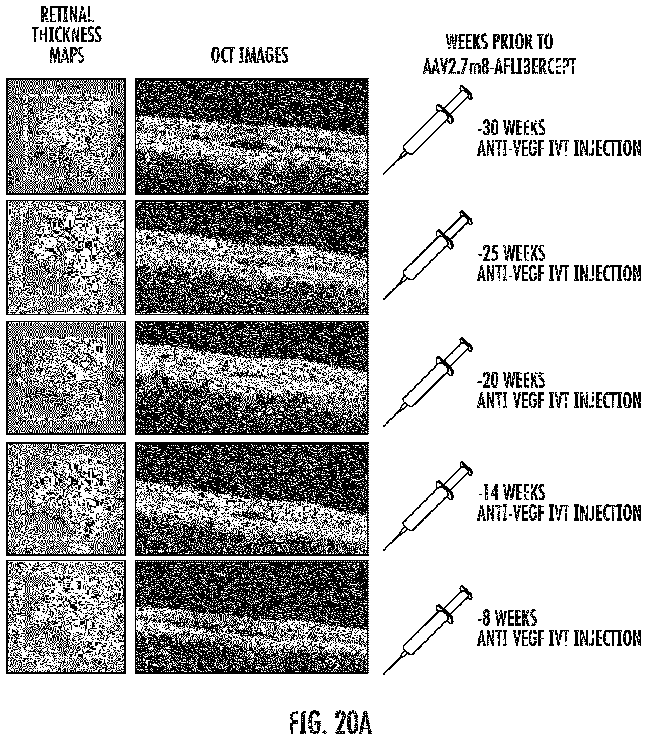

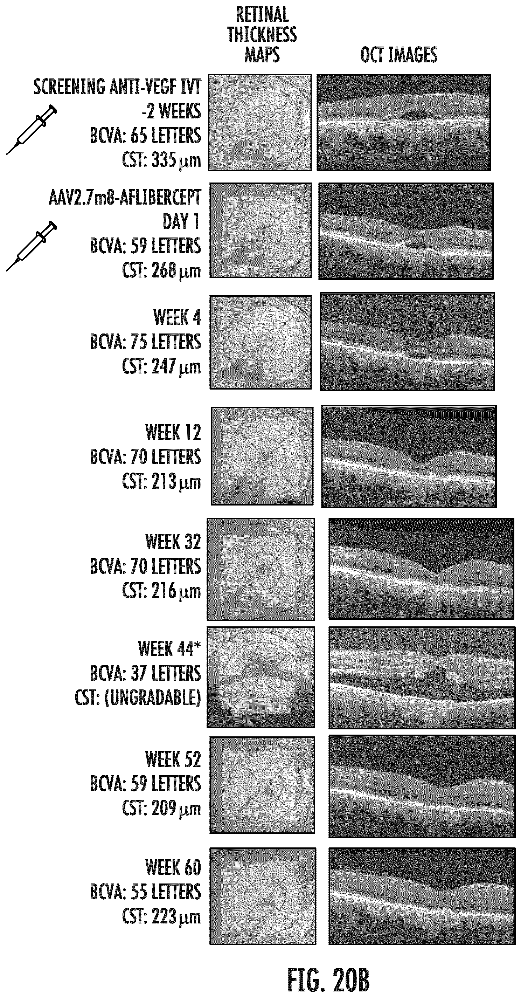

**FIGURES 20A-20B** show optical coherence tomography (OCT) images and retinal thickness maps derived from OCT images taken from Subject 4 in Cohort 1 of the study described in Examples 1-5 at the indicated times before and after administration of AAV2.7m8-aflibercept. **FIG. 20A** shows OCT images and retinal thickness maps for Subject 4 in Cohort 1 at the indicated times (weeks) prior to administration of AAV2.7m8-aflibercept, during which the subject was administered anti-VEGF IVT injections (ranibizumab). **FIG. 20B** shows OCT images and retinal thickness maps for Subject 4 in Cohort 1 at the indicated times (weeks) before and after administration of AAV2.7m8-aflibercept. The times of administration of the screening anti-VEGF IVT injection ("Screening anti-VEGF IVT") and of AAV2.7m8-aflibercept ("AA V2.7m8-aflibercept") are indicated. The BCVA (ETDRS letters) and CST (µm) at each indicated time before and after administration of AAV2.7m8-aflibercept are provided. The asterisk indicates that the subject had a retinal detachment event unrelated to AAV2.7m8-aflibercept.
**FIGURES 21A-21B** show optical coherence tomography (OCT) images and retinal thickness maps derived from OCT images taken from Subject 5 in Cohort 3 of the study described in Examples 1-5 at the indicated times before and after administration of AAV2.7m8-aflibercept. **FIG. 21A** shows OCT images and retinal thickness maps for Subject 5 in Cohort 3 at the indicated times (weeks) prior to administration of AAV2.7m8-aflibercept, during which the subject was administered anti-VEGF IVT injections (aflibercept). **FIG. 21B** shows OCT images and retinal thickness maps for Subject 5 in Cohort 3 at the indicated times (weeks) before and after administration of AAV2.7m8-aflibercept. The times of administration of the screening anti-VEGF IVT injection ("Screening anti-VEGF IVT") and of AAV2.7m8-aflibercept ("AA V2.7m8-aflibercept") are indicated. The BCVA (ETDRS letters) and CST (µm) at each indicated time before and after administration of AAV2.7m8-aflibercept are provided.

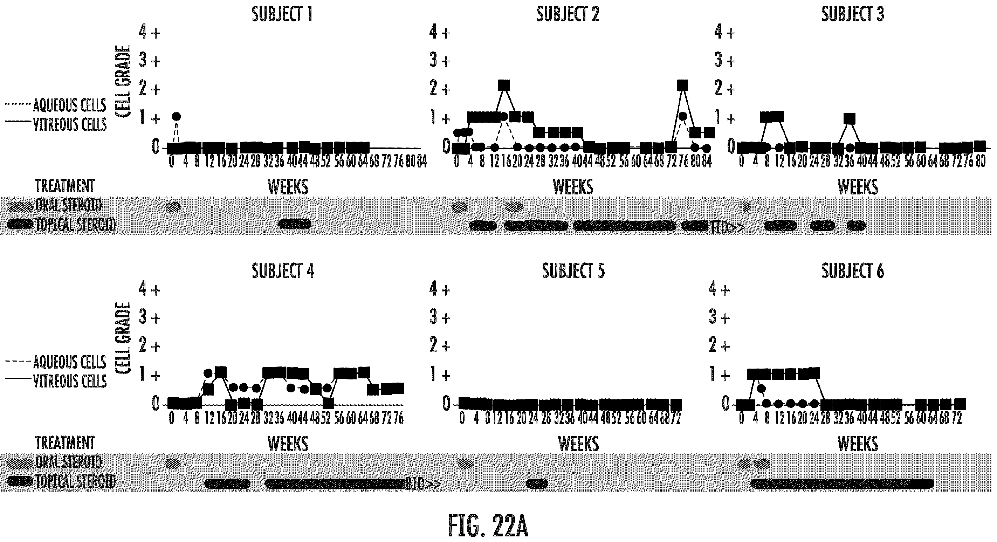

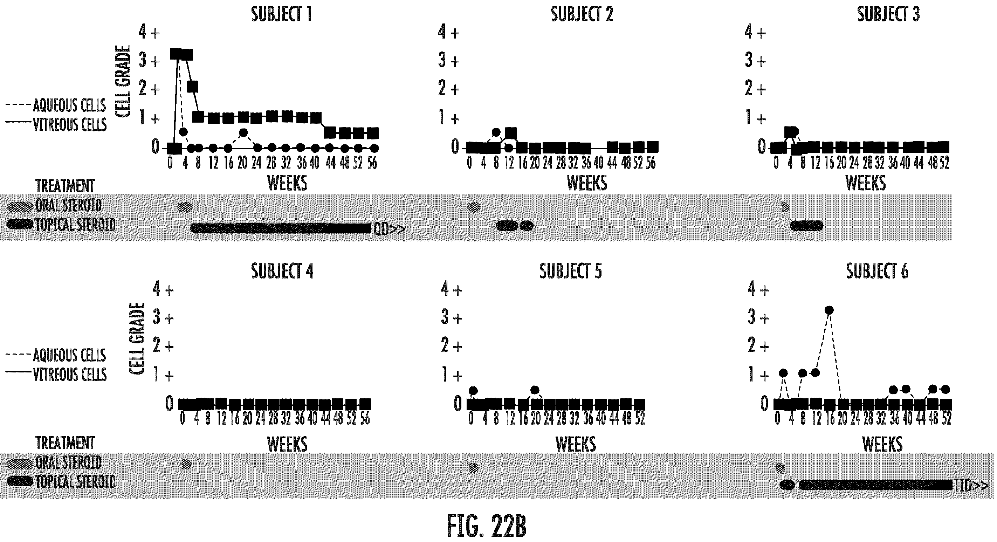

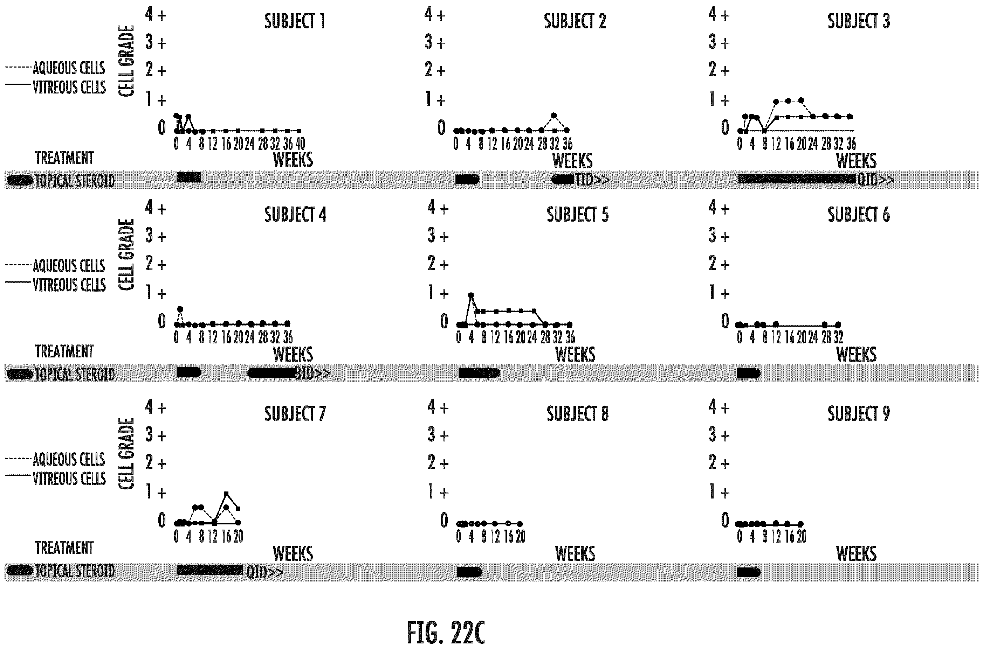

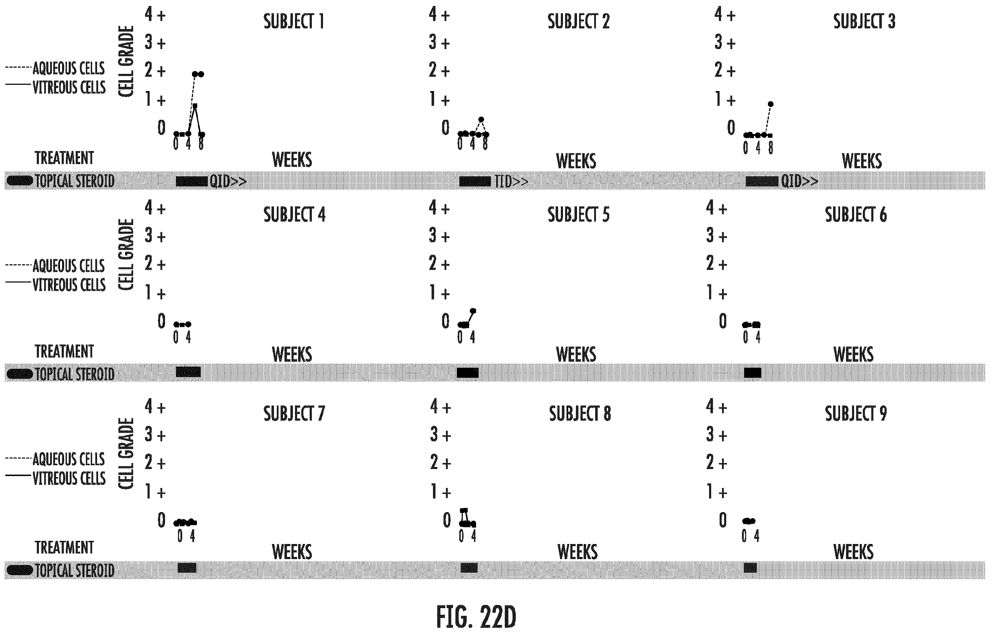

**FIGURES 22A-22D** show plots of aqueous cell and vitreous cell counts as measured by slit lamp examination following treatment with AAV2.7m8-aflibercept for subjects in Cohorts 1, 2, 3, and 4 of the study described in Examples 1-5 and 7. **FIG. 22A** shows plots of aqueous cell and vitreous cell counts for subjects in Cohort 1 up to a follow up time of 80 weeks. **FIG. 22B** shows plots of aqueous cell and vitreous cell counts for subjects in Cohort 2 up to a follow up time of 56 weeks. **FIG. 22C** shows plots of aqueous cell and vitreous cell counts for subjects in Cohort 3 up to a follow up time of 36 weeks. **FIG. 22D** shows plots of aqueous cell and vitreous cell counts for subjects in Cohort 4 up to a follow up time of 8 weeks. In **FIGS. 22A-22D****,** the steroid treatment administered to each subject is indicated below each plot; aqueous cell grade categories were based on the Standardization of Uveitis Nomenclature (SUN) criteria: Jabs DA, et al. J Ophthalmol 2005;140:509-516; vitreous cell grade categories were based on National Institutes of Health (NIH) guidelines; aqueous cells: 0.5+ = 1-5 cells; 1+ = 6-15 cells; 2+ = 16-25 cells; 3+ = 26-50 cells; 4+ = >50 cells; vitreous cells: 0.5+ = 1-10 cells; 1+ = 11-20 cells; 2+ = 21-30 cells; 3+ = 31-100 cells; 4+ = >100 cells; rare cells were captured as 0.5+ for this analysis. QID = four times per day; TID = three time per day; BID = twice per day; QD = once per day; QOD = once every other day; "QD >>" indicates that the subject continues to receive topical steroids once per day; "BID >>" indicates that the subject continues to receive topical steroids twice per day; "TID >>" indicates that the subject continues to receive topical steroids three times per day; and "QID >>" indicates that the subject continues to receive topical steroids four times per day.

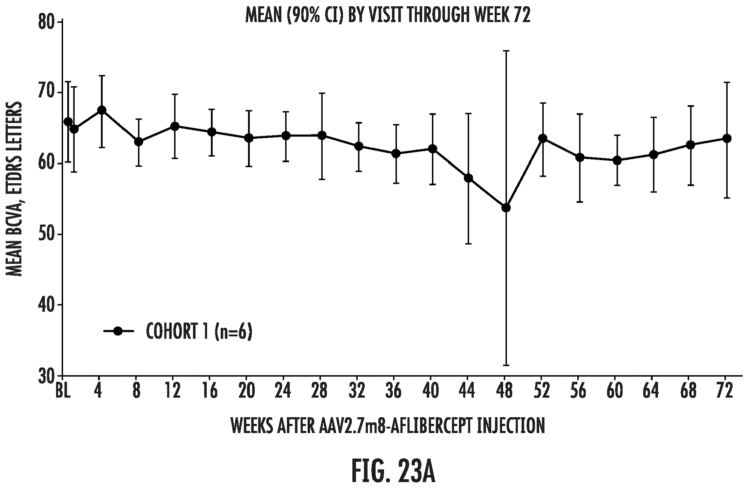

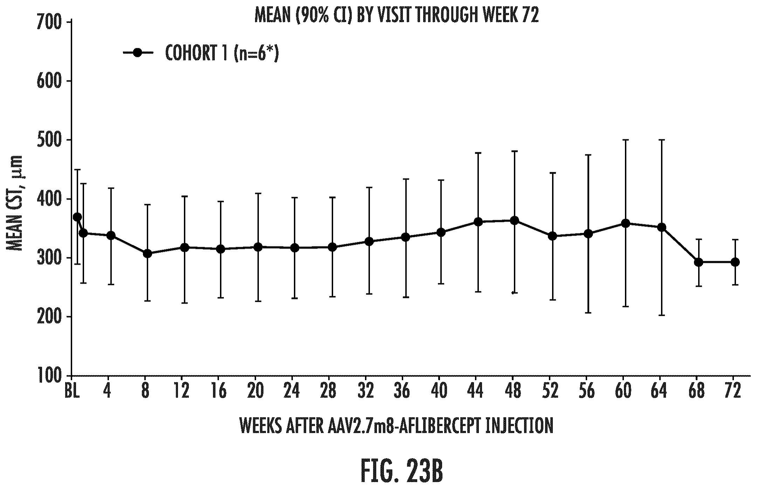

**FIGURES 23A-23B** provide the mean BCVA and mean CST for subjects in Cohort 1 of the study described in Examples 1-5 and 7 (n=6) from baseline up to Week 72. **FIG. 23A** shows the mean BCVA (ETDRS letters) from baseline up to Week 72. One subject had low BCVA scores at Weeks 44 and 48 due to retinal detachment. *n = 5 from Week 56 to Week 72. **FIG. 23B** shows the mean CST (µm) from baseline up to Week 72. One subject had no CST data at Weeks 44 and 48 due to retinal detachment. *n = 5 from Week 56 to Week 72. In **FIGS. 23A-23B****,** the error bars indicate the 90% confidence intervals of the mean absolute BCVA and CST values using the T-distribution; BL = baseline; D = day; W = week.

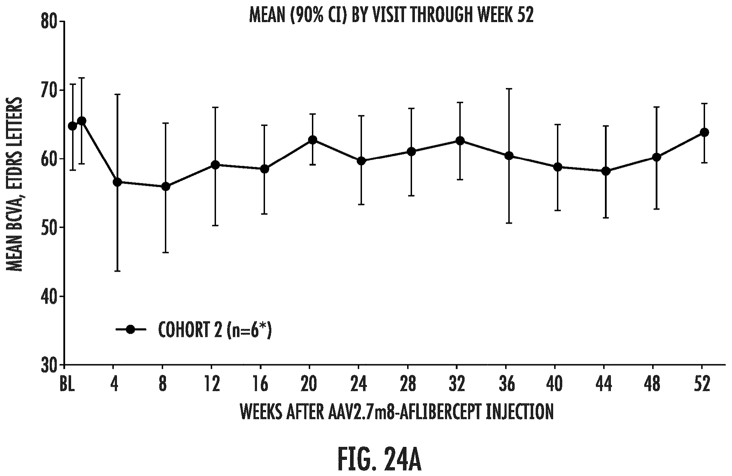

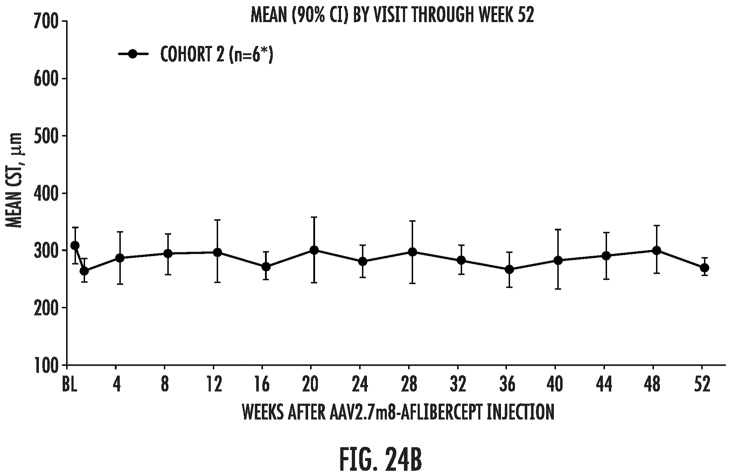

**FIGURES 24A-24B** provide the mean BCVA and mean CST for subjects in Cohort 2 of the study described in Examples 1-5 and 7 (n=6) from baseline up to Week 52. **FIG. 24A** shows the mean BCVA (ETDRS letters) from baseline up to Week 52. *n = 5 for Week 36 and Week 40. **FIG. 24B** shows the mean CST (µm) from baseline up to Week 52. *n = 5 for Week 36 and Week 40. In **FIGS. 24A-24B****,** the error bars indicate the 90% confidence intervals of the mean absolute BCVA and CST values using the T-distribution; BL = baseline; D = day; W = week.

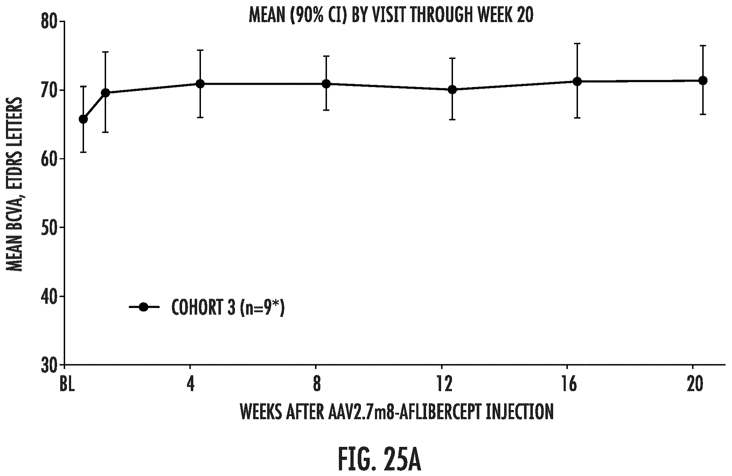

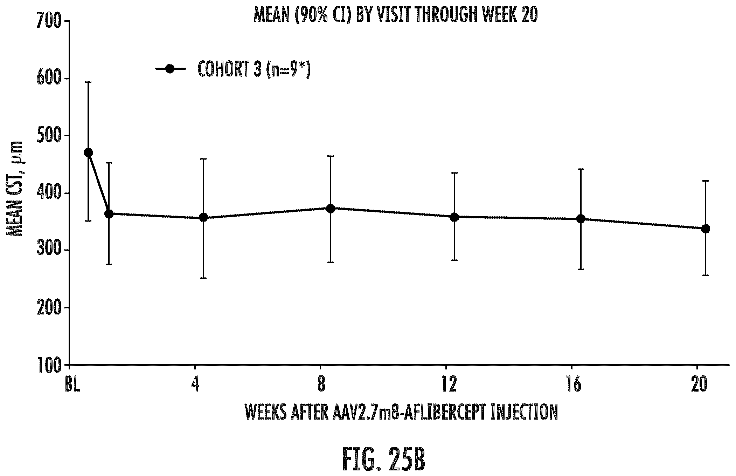

**FIGURES 25A-25B** provide the mean BCVA and mean CST for subjects in Cohort 3 of the study described in Examples 1-5 and 7 (n=9) from baseline up to Week 20. **FIG. 25A** shows the mean BCVA (ETDRS letters) from baseline up to Week 20. *n = 8 for Weeks 4, 16, and 20. **FIG. 25B** shows the mean CST (µm) from baseline up to Week 20. *n = 8 for Weeks 4, 16, and 20. In **FIGS. 25A-25B****,** the error bars indicate the 90% confidence intervals of the mean absolute BCVA and CST values using the T-distribution; BL = baseline; D = day; W = week.

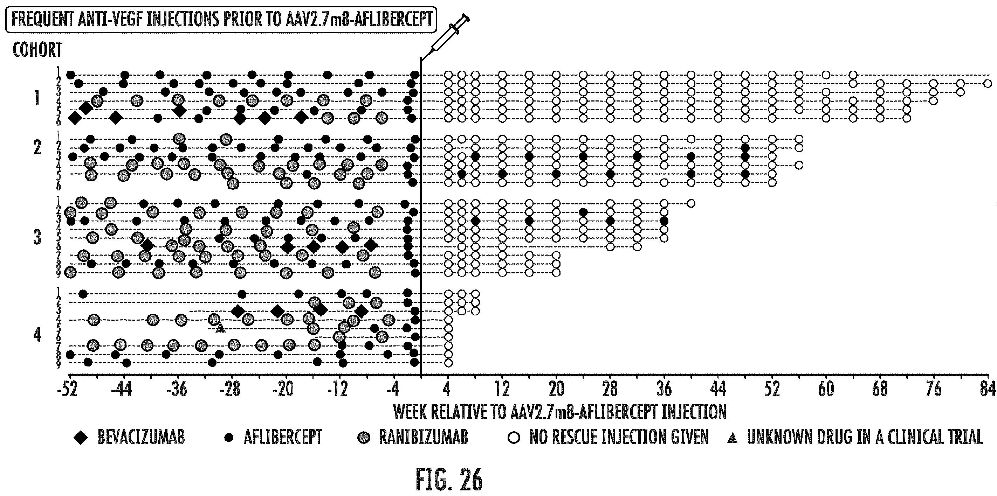

**FIGURE 26** is a Swimmer's Lane Plot showing the number of anti-VEGF injections administered to subjects in Cohorts 1, 2, 3, and 4 of the study described in Examples 1-5 and 7. The x-axis represents time in weeks relative to the time AAV2.7m8-aflibercept was administered. The y-axis shows each individual subject in Cohorts 1-4. The circular shapes represent anti-VEGF IVT injections administered before and after treatment with AAV2.7m8-aflibercept. The vertical line bisecting the plot indicates Day 1, when AAV2.7m8-aflibercept was administered. To the right of the bisecting vertical line are each of the subsequent study visits. Five subjects were diagnosed <1 year prior to AAV2.7m8-aflibercept injection (1 subject in each of Cohorts 2 and 3, and 3 subjects in Cohort 4). In Cohort 4, data before Day 1 are incomplete for Subject 2 due to relocation, and Subject 5 because the subject participated in a clinical trial with unknown medication(s) after diagnosis.

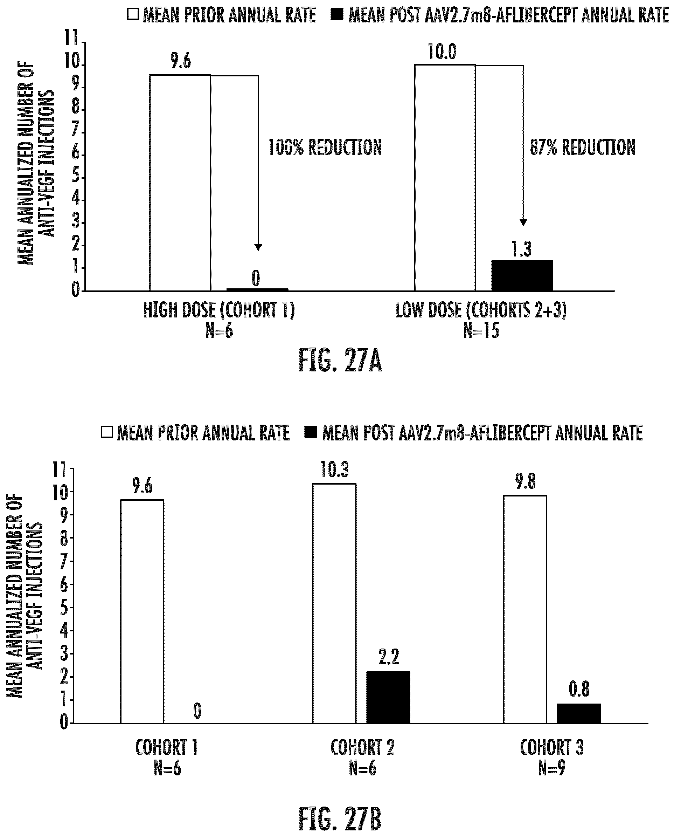

**FIGURES 27A-27B** show the mean annualized anti-VEGF injection rate for subjects in Cohorts 1-3 of the study described in Examples 1-5 and 7. **FIG. 27A** provides a comparison of the mean annualized anti-VEGF injection rates for subjects in Cohort 1 (administered the "High Dose" of 6 × 10¹¹ vg/eye; n = 6) and for subjects in Cohorts 2 and 3 (administered the "Low Dose" of 2 × 10¹¹ vg/eye; n = 15). **FIG. 27B** provides a comparison of the mean annualized anti-VEGF injection rates for subjects in each of Cohorts 1 (n = 6), 2 (n = 6), and 3 (n = 9). In **FIGS. 27A-27B****,** Annualized rate (Prior) = (number of anti-VEGF IVT injections in the 12 months prior to AAV2.7m8-aflibercept) / (days from the first anti-VEGF IVT injection in the past 12 months to the AAV2.7m8-aflibercept injection / 365.25); and Annualized rate (Post) = (number of anti-VEGF IVT injections since AA V2.7m8-aflibercept) / (days from AAV2.7m8-aflibercept to the last study follow-up / 365.25).

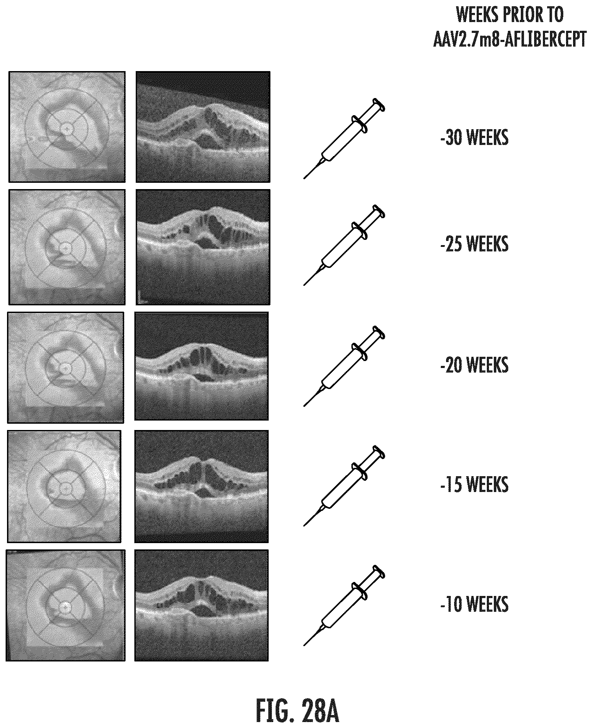

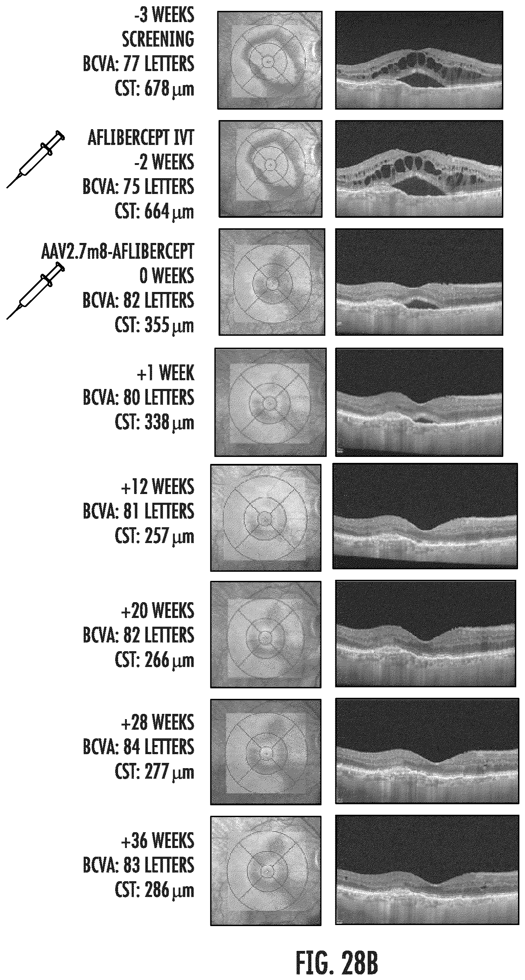

**FIGURES 28A-28B** show optical coherence tomography (OCT) images and retinal thickness maps derived from OCT images taken from Subject 5 in Cohort 3 of the study described in Examples 1-5 and 7 at the indicated times before and after administration of AAV2.7m8-aflibercept. **FIG. 28A** shows OCT images and retinal thickness maps for Subject 5 in Cohort 3 at the indicated times (weeks) prior to administration of AAV2.7m8-aflibercept, during which the subject was administered anti-VEGF IVT injections (aflibercept). **FIG. 28B** shows OCT images and retinal thickness maps for Subject 5 in Cohort 3 at the indicated times (weeks) before and after administration of AAV2.7m8-aflibercept. The times of administration of the screening anti-VEGF IVT injection ("Aflibercept IVT") and of AAV2.7m8-aflibercept are indicated. The BCVA (ETDRS letters) and CST (µm) at each indicated time before and after administration of AAV2.7m8-aflibercept are provided.

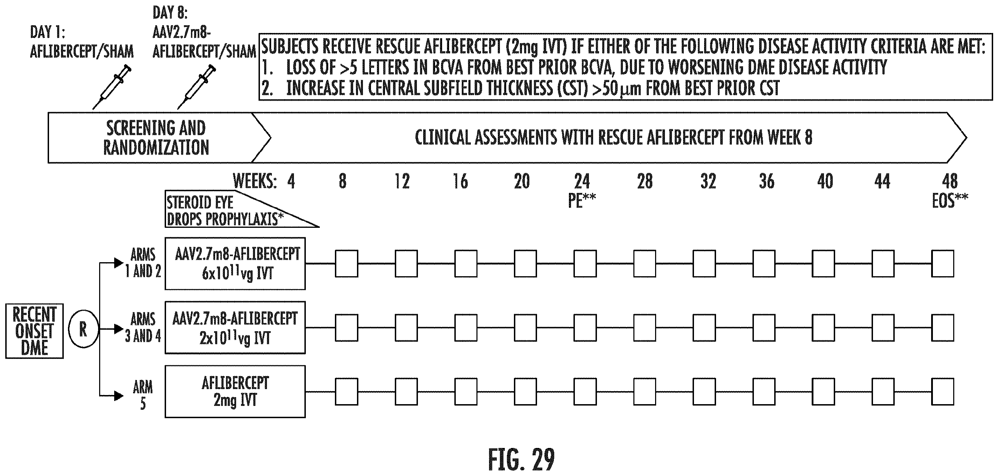

**FIGURE 29** shows a diagram of the design of the study described in Example 6, evaluating the durability of a single intravitreal (IVT) injection of AAV2.7m8-aflibercept in subjects with diabetic macular edema (DME). DRSS = Diabetic Retinopathy Severity Score; OCT= Optical Coherence Tomography; CST = Central Subfield Thickness. *All subjects receive a 7-week course of difluprednate eye drops, starting at QID and tapering to QD. **PE= Primary Endpoint assessment. ***EOS= End of Study assessment.

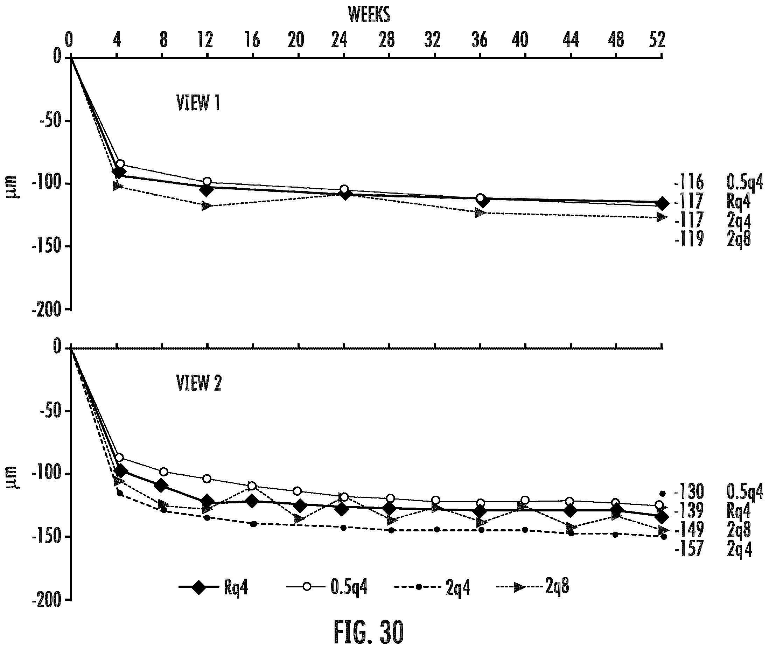

**FIGURE 30** shows two views of CST as measured using four different treatment arms. (1) Ranibizumab 0.5mg monthly (Rq4); Intravitreal Aflibercept Injection (IAI) 0.5mg monthly (0.5q4); IAI 2mg monthly (2q4); IAI 2mg every 2 month after 3 initial monthly doses (2q8).

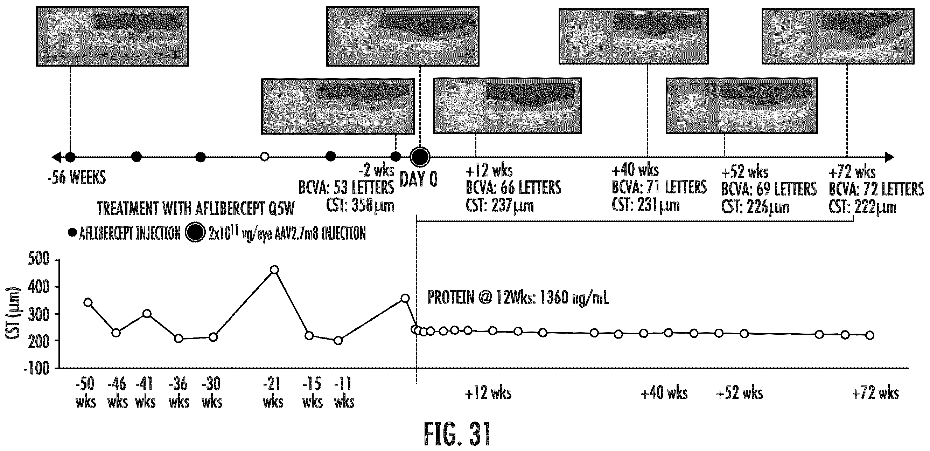

**FIGURE 31** shows case study of the results of a single intravitreal (IVT) injection of AAV2.7m8 2x10¹¹ vg/eye in eliminating fluctuations in fluid and CST. The patient was a 90-year old female with 21 IVTs prior to study with 9 IVTs in the last 12 months before injection of AAV2.7m8. OCT images are shown as well as a graph that shows the fluctuation of CST before and after administration of AAV2.7m8.

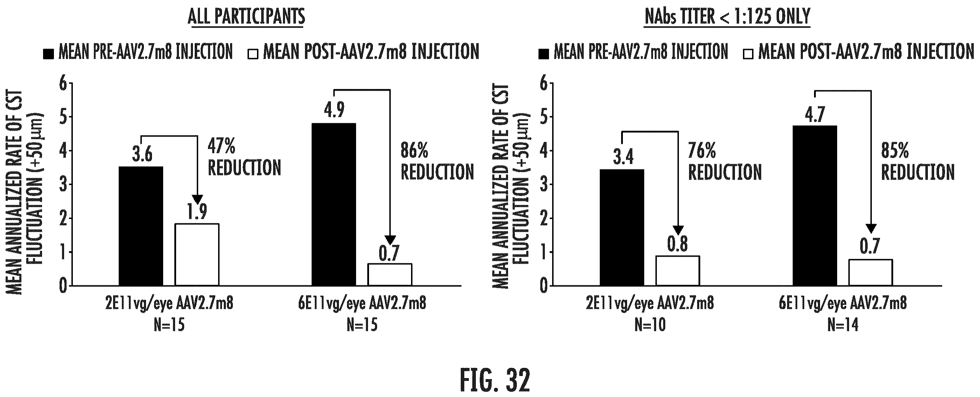

**FIGURE 32** shows an annualized rate of CST fluctuation (+ or - 50µm). A Fluctuation is defined as a change from one scheduled study week (Week A) to a later scheduled study week (Week B) that meets the following conditions: 1. The magnitude of change equals or exceeds the fluctuation criteria. 2. The direction of change from the first scheduled week (Week A) remains the same for any interim scheduled study weeks occurring between the first and second scheduled weeks (Week A and Week B). 3. The direction of change from the first scheduled week (Week A) changes at the scheduled study week immediately following the second scheduled week (Week B). Annualized Rate of Fluctuations Pre-AAV2.7m8 = (number of fluctuations since first historical observation)/(days from first historical observation to the last historical observation/365.25). Annualized Rate of Fluctuations Post- AAV2.7m8 = (number of fluctuations since AAV2.7m8 injection)/(days from AAV2.7m8 to the last study follow-up/365.25). Figure 32 also shows the reduction in CST fluctuations among subjects with documented baseline anti-AAV2.7m8 neutralizing antibody (NAbs) titer levels <1:125.

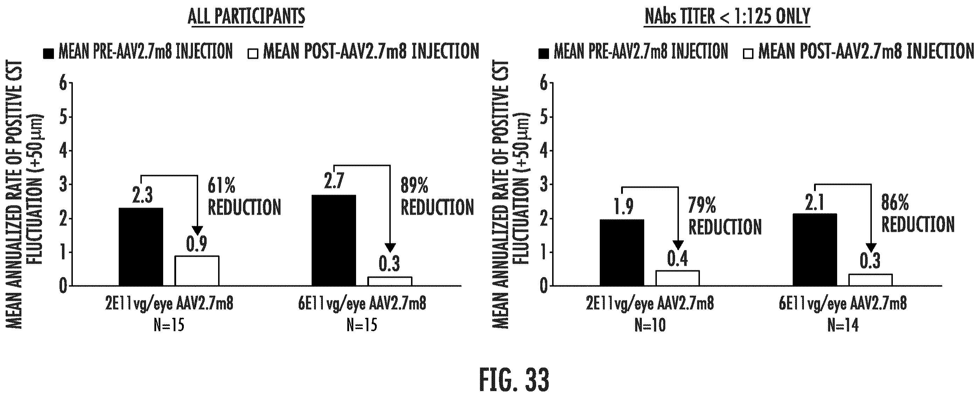

**FIGURE 33** shows an annualized rate of positive CST fluctuation (+50µm). A Fluctuation is defined as a change from one scheduled study week (Week A) to a later scheduled study week (Week B) that meets the following conditions: 1. The magnitude of change equals or exceeds the fluctuation criteria. 2. The direction of change from the first scheduled week (Week A) is positive and remains the same for any interim scheduled study weeks occurring between the first and second scheduled weeks (Week A and Week B). 3. The direction of change from the first scheduled week (Week A) changes at the scheduled study week immediately following the second scheduled week (Week B). Annualized Rate of Fluctuations Pre-AAV2.7m8 = (number of fluctuations since first historical observation)/(days from first historical observation to the last historical observation/365.25). Annualized Rate of Fluctuations Post- AAV2.7m8 = (number of fluctuations since AAV2.7m8 injection)/(days from AAV2.7m8 to the last study follow-up/365.25). Figure 33 also shows the reduction in CST fluctuations among subjects with documented baseline anti-AAV2.7m8 neutralizing antibody (NAbs) titer levels <1:125.

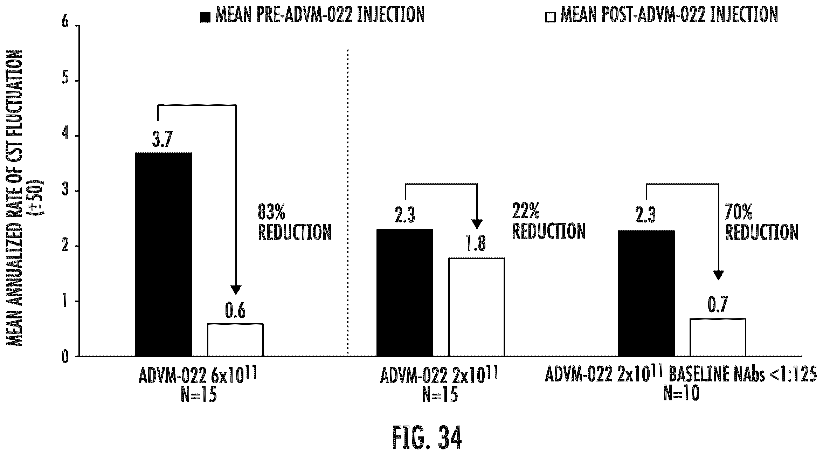

**FIGURE 34** shows an annualized rate of CST fluctuation (+ or - 50µm) over a period approximately six months longer than that shown in **FIG. 32****.** A reduction in CST fluctuation remains. Figure 34 also shows the reduction in CST fluctuations among subjects with documented baseline anti-AAV2.7m8 neutralizing antibody (NAbs) titer levels <1:125.

### DETAILED DESCRIPTION

Several aspects are described below with reference to example applications for illustration. It should be understood that numerous specific details, relationships, and methods are set forth to provide a full understanding of the features described herein. One having ordinary skill in the relevant art, however, will readily recognize that the features described herein can be practiced without one or more of the specific details or with other methods. The features described herein are not limited by the illustrated ordering of acts or events, as some acts can occur in different orders and/or concurrently with other acts or events. Furthermore, not all illustrated acts or events are required to implement a methodology in accordance with the features described herein.

### Definitions

Unless otherwise defined, all technical terms used herein have the same meaning as commonly understood by one of ordinary skill in the art.

The terminology used herein is for the purpose of describing particular examples only and is not intended to be limiting. As used herein, the singular forms "a", "an" and "the" are intended to include the plural forms as well, unless the context clearly indicates otherwise. Furthermore, to the extent that the terms "including", "includes", "having", "has", "with", or variants thereof are used in either the detailed description and/or the claims, such terms are intended to be inclusive in a manner similar to the term "comprising". The term "comprising" as used herein is synonymous with "including" or "containing", and is inclusive or openended.

Any reference to "or" herein is intended to encompass "and/or" unless otherwise stated. As used herein, the term "about" a number refers to that number plus or minus 10% of that number. The term "about" a range refers to that range minus 10% of its lowest value and plus 10% of its greatest value. Reference to "about" a value or parameter herein includes (and describes) embodiments that are directed to that value or parameter *per se.*

The term "subject", "patient", or "individual" refers to primates, such as humans and non-human primates, e.g., African green monkeys and rhesus monkeys. In some embodiments, the subject is a human.

The terms "treat," "treating", "treatment," "ameliorate" or "ameliorating" and other grammatical equivalents as used herein, refer to alleviating, abating or ameliorating an ocular neovascular disease or disorder, or symptoms of the ocular neovascular disease or disorder, preventing additional symptoms of the ocular neovascular disease or disorder, ameliorating or preventing the underlying metabolic causes of symptoms, inhibiting the ocular neovascular disease or disorder, e.g., arresting the development of the ocular neovascular disease or disorder, relieving the ocular neovascular disease or disorder, causing regression of the ocular neovascular disease or disorder, or stopping the symptoms of the ocular neovascular disease or disorder, and are intended to include prophylaxis. The terms further include achieving a therapeutic benefit and/or a prophylactic benefit. The term "therapeutic benefit" refers to eradication or amelioration of the ocular neovascular disease or disorder being treated. Also, a therapeutic benefit is achieved with the eradication or amelioration of one or more of the physiological symptoms associated with the ocular neovascular disease or disorder such that an improvement is observed in the subject, notwithstanding that, in some embodiments, the subject is still afflicted with the ocular neovascular disease or disorder. For prophylactic benefit, the pharmaceutical compositions are administered to a subject at risk of developing the ocular neovascular disease or disorder, or to a subject reporting one or more of the physiological symptoms of the ocular neovascular disease or disorder, even if a diagnosis of the disease or disorder has not been made.

The terms "administer," "administering", "administration," and the like, as used herein, can refer to the methods that are used to enable delivery of therapeutics or pharmaceutical compositions to the desired site of biological action. These methods include intravitreal or subretinal injection to an eye.

The terms "effective amount", "therapeutically effective amount" or "pharmaceutically effective amount" as used herein, can refer to a sufficient amount of at least one pharmaceutical composition or compound being administered which will relieve to some extent one or more of the symptoms of the ocular disease or disorder being treated. An "effective amount", "therapeutically effective amount" or "pharmaceutically effective amount" of a pharmaceutical composition may be administered to a subject in need thereof as a unit dose (as described in further detail elsewhere herein).

The term "pharmaceutically acceptable" as used herein, can refer to a material, such as a carrier or diluent, which does not abrogate the biological activity or properties of a compound disclosed herein, and is relatively nontoxic (i.e., when the material is administered to an individual it does not cause undesirable biological effects nor does it interact in a deleterious manner with any of the components of the composition in which it is contained).

The term "pharmaceutical composition," or simply "composition" as used herein, can refer to a biologically active compound, optionally mixed with at least one pharmaceutically acceptable chemical component, such as, though not limited to carriers, stabilizers, diluents, dispersing agents, suspending agents, thickening agents, excipients and the like.

An "AAV vector" or "rAAV vector" as used herein refers to an adeno-associated virus (AAV) vector or a recombinant AAV (rAAV) vector comprising a polynucleotide sequence not of AAV origin (e.g., a polynucleotide heterologous to AAV such as a nucleic acid sequence that encodes a therapeutic transgene, e.g., aflibercept) for transduction into a target cell or to a target tissue. In general, the heterologous polynucleotide is flanked by at least one, and generally by two, AAV inverted terminal repeat sequences (ITRs). The term rAAV vector encompasses both rAAV vector particles and rAAV vector plasmids. A rAAV vector may be either single-stranded (ssAAV) or self-complementary (scAAV).

An "AAV virus" or "AAV viral particle" or "rAAV vector particle" or "rAAV particle" refers to a viral particle comprising at least one AAV capsid protein and a polynucleotide rAAV vector. In some cases, the at least one AAV capsid protein is from a wild type AAV or is a variant AAV capsid protein (e.g., an AAV capsid protein with an insertion, e.g., an insertion of the 7m8 amino sequence as set forth below). If the particle comprises a heterologous polynucleotide (e.g., a polynucleotide other than a wild-type AAV genome such as a transgene to be delivered to a target cell or target tissue), it is referred to as a "rAAV particle", "rAAV vector particle" or a "rAAV vector". Thus, production of rAAV particles necessarily includes production of a rAAV vector, as such a vector contained within a rAAV particle.

The term "packaging" as used herein can refer to a series of intracellular events that can result in the assembly and encapsidation of a rAAV particle.

AAV "rep" and "cap" genes refer to polynucleotide sequences encoding replication and encapsidation proteins of adeno-associated virus. AAV rep and cap are referred to herein as AAV "packaging genes."

The term "polypeptide" can encompass both naturally occurring and non-naturally occurring proteins (e.g., a fusion protein), peptides, fragments, mutants, derivatives and analogs thereof. A polypeptide may be monomeric, dimeric, trimeric, or polymeric. Further, a polypeptide may comprise a number of different domains, each of which has one or more distinct activities. For the avoidance of doubt, a "polypeptide" may be any length greater two amino acids.

As used herein, "polypeptide variant" or simply "variant" refers to a polypeptide whose sequence contains an amino acid modification. In some embodiments, the modification is an insertion, duplication, deletion, rearrangement, or substitution of one or more amino acids compared to the amino acid sequence of a reference protein or polypeptide, such as a native or wild type protein. A variant may have one or more amino acid point substitutions, in which a single amino acid at a position has been changed to another amino acid, one or more insertions and/or deletions, in which one or more amino acids are inserted or deleted, respectively, in the sequence of the reference protein, and/or truncations of the amino acid sequence at either or both the amino or carboxy termini. A variant can have the same or a different biological activity compared to the reference protein, or the unmodified protein.

In some embodiments, a variant can have, for example, at least about 80%, 85%, 90%, 91%, 92%, 93%, 94%, 95%, 96%, 97%, 98%, or 99% overall sequence homology to its counterpart reference protein. In some embodiments, a variant can have at least about 90% overall sequence homology to the wild-type protein. In some embodiments, a variant exhibits at least about 95%, at least about 98%, at least about 99%, at least about 99.5%, or at least about 99.9% overall sequence identity.

As used herein, "recombinant" can refer to a biomolecule, e.g., a gene or protein, that (1) has been removed from its naturally occurring environment, (2) is not associated with all or a portion of a polynucleotide in which the gene is found in nature, (3) is operatively linked to a polynucleotide which it is not linked to in nature, or (4) does not occur in nature. The term "recombinant" can be used in reference to cloned DNA isolates, chemically synthesized polynucleotide analogs, or polynucleotide analogs that are biologically synthesized by heterologous systems, as well as proteins and/or mRNAs encoded by such nucleic acids. Thus, for example, a protein synthesized by a microorganism is recombinant, for example, if it is synthesized from an mRNA synthesized from a recombinant gene present in the cell.

The term "anti-VEGF agent" includes any therapeutic agent, including proteins, polypeptides, peptides, fusion protein, multimeric proteins, gene products, antibody, human monoclonal antibody, antibody fragment, aptamer, small molecule, kinase inhibitor, receptor or receptor fragment, or nucleic acid molecule, that can reduce, interfere with, disrupt, block and/or inhibit the activity or function of an endogenous VEGF and/or an endogenous VEGF receptor (VEGFR), or the VEGF-VEGFR interaction or pathway *in vivo.* An anti-VEGF agent can be any one of the known therapeutic agents that can reduce new blood vessel growth or formation and/or oedem, or swelling, when delivered into a cell, tissue, or a subject *in vivo,* e.g., ranibizumab, brolucizumab, or bevacizumab. In some embodiments, an anti-VEGF agent can be naturally occurring, non-naturally occurring, or synthetic. In some embodiments, an anti-VEGF agent can be derived from a naturally occurring molecule that was subsequently modified or mutated to confer an anti- VEGF activity. In some embodiments, an anti-VEGF agent is a fusion or chimeric protein. In such proteins, functional domains or polypeptides are artificially fused to a moiety or a polypeptide to make a fusion or chimeric protein that can sequester VEGF *in vivo* or function as a VEGFR decoy. In some embodiments, an anti-VEGF agent is a fusion or chimeric protein that blocks endogenous VEGFR from interacting with its ligands.

As used herein, "VEGF" can refer to any isoform of VEGF, unless required otherwise, including, but not limited to, VEGF-A, VEGF-B, VEGF-C, VEGF-D, VEGF-E, VEGF-F, or any combination, or any functional fragment or variant thereof. Unless required otherwise, "VEGF" can refer to any member of the VEGF family, including members: VEGF-A, placenta growth factor (PGF), VEGF-B, VEGF-C, and VEGF-D, or any combination, functional fragment, or variant thereof. As used herein, "VEGF receptor" or "VEGFR" or "VEGF-R" can be used to refer to any one of the receptors of VEGF, including, but not limited to, VEGFR-1 (or Flt-1), VEGFR-2 (or Flk-1/KDR), and VEGFR-3 (or Flt-4). VEGFR can be a membrane bound or soluble form, or a functional fragment or truncation of a receptor. Examples of anti-VEGF agents include, but are not limited to, ranibizumab, bevacizumab, brolucizumab, or any combination, variant, or functional fragment thereof.

"Operatively linked" or "operably linked" or "coupled" can refer to a juxtaposition of genetic elements, wherein the elements are in a relationship permitting them to operate in an expected manner. For instance, a promoter can be operatively linked to a coding region if the promoter helps initiate transcription of the coding sequence. There may be intervening residues between the promoter and coding region so long as this functional relationship is maintained.

The term "expression vector" or "expression construct" or "cassette" or "plasmid" or simply "vector" can include any type of genetic construct, including AAV or rAAV vectors, containing a nucleic acid or polynucleotide coding for a gene product in which part or all of the nucleic acid encoding sequence is capable of being transcribed and is adapted for gene therapy. The transcript can be translated into a protein. In some embodiments, the transcript is partially translated or not translated. In certain aspects, expression includes both transcription of a gene and translation of mRNA into a gene product. In other aspects, expression only includes transcription of the nucleic acid encoding genes of interest. An expression vector can also comprise control elements operatively linked to the encoding region to facilitate expression of the protein in target cells. The combination of control elements and a gene or genes to which they are operably linked for expression can sometimes be referred to as an "expression cassette," a large number of which are known and available in the art or can be readily constructed from components that are available in the art.

The term "heterologous" can refer to an entity that is genotypically distinct from that of the rest of the entity to which it is being compared. For example, a polynucleotide introduced by genetic engineering techniques into a plasmid or vector derived from a different species can be a heterologous polynucleotide. A promoter removed from its native coding sequence and operatively linked to a coding sequence with which it is not naturally found linked can be a heterologous promoter.

As used herein, "7m8" refers to the amino acid sequence LALGETTRPA (SEQ ID NO: 1).

"7m8 variant" refers to a rAAV, which can be of any serotype, with the amino acid sequence LALGETTRPA (SEQ ID NO: 1) inserted in the solvent exposed GH loop of the capsid protein.

When 7m8 is inserted in a rAAV2 (also referred to as AAV2.7m8), the amino acid sequence LALGETTRPA (SEQ ID NO: 1) is inserted into the GH loop within amino acids 570-611 of the AAV2 capsid protein, e.g., between positions 587 and 588 of the AAV2 capsid protein, VP1. In some cases, when 7m8 is inserted in a rAAV2 (also referred to as AAV2.7m8), the amino acid sequence LALGETTRPA (SEQ ID NO: 1) is inserted into the GH loop of the AAV2 capsid protein, e.g., between positions 587 and 588 of AAV2 VP1 comprising the sequence of SEQ ID NO: 13. When 7m8 is inserted in a rAAV1 (also referred to as AAV1.7m8), the amino acid sequence LALGETTRPA (SEQ ID NO: 1) is inserted into the GH loop within amino acids 571-612 of the AAV1 capsid protein, e.g., between amino acids 590 and 591 of the AAV1 capsid protein. When 7m8 is inserted in a rAAV5 (also referred to as AAV5.7m8), the amino acid sequence LALGETTRPA (SEQ ID NO: 1) is inserted into the GH loop within amino acids 560-601 of the AAV5 capsid protein, e.g., between amino acids 575 and 576 of the AAV5 capsid protein. When 7m8 is inserted in a rAAV6 (also referred to as AAV6.7m8), the amino acid sequence LALGETTRPA (SEQ ID NO: 1) is inserted into the GH loop within amino acids 571 to 612 of the AAV6 capsid protein, e.g., between amino acids 590 and 591 of the AAV6 capsid protein. When 7m8 is inserted in a rAAV7 (also referred to as AAV7.7m8), the amino acid sequence LALGETTRPA (SEQ ID NO: 1) is inserted into the GH loop within amino acids 572 to 613 of the AAV7 capsid protein, e.g., between amino acids 589 and 590 of the AAV7 capsid protein. When 7m8 is inserted in a rAAV8 (also referred to as AAV8.7m8), the amino acid sequence LALGETTRPA (SEQ ID NO: 1) is inserted into the GH loop within amino acids 573 to 614 of the AAV8 capsid protein, e.g., between amino acids 590 and 591 of the AAV8 capsid protein. When 7m8 is inserted in a rAAV9 (also referred to as AAV9.7m8), the amino acid sequence LALGETTRPA (SEQ ID NO: 1) is inserted into the GH loop of the AAV9 capsid protein, e.g., between amino acids 588 and 589 of the AAV9 capsid protein. When 7m8 is inserted in a rAAV10 (also referred to as AAV10.7m8), the amino acid sequence LALGETTRPA (SEQ ID NO: 1) is inserted into the GH loop within amino acids 573 to 614 of the AAV10 capsid protein, e.g., between amino acids 589 and 590 of the AAV10 capsid protein.

As used herein, the term "fluctuation" refers to a change in the central subfoveal or central retinal thickness from one scheduled study week (Week A) to a later scheduled study week (Week B) that meets the following conditions: 1. The magnitude of change equals or exceeds the fluctuation criteria. 2. The direction of change from the first scheduled week (Week A) remains the same for any interim scheduled study weeks occurring between the first and second scheduled weeks (Week A and Week B). 3. The direction of change from the first scheduled week (Week A) changes at the scheduled study week immediately following the second scheduled week (Week B).

As used herein "Annualized Rate of Fluctuations" is categorized and delineated into "Pre-AAV2.7m8" injection time period, which is calculated as (number of fluctuations since first historical observation)/(days from first historical observation to the last historical observation/365.25); and "Post-AAV2.7m8" injection time period, which is calculated as (number of fluctuations since AAV2.7m8 injection)/(days from AAV2.7m8 to the last study follow-up/365.25).

### Overview

Current therapies (e.g., aflibercept recombinant protein, ranibizumab recombinant protein) for ocular neovascular diseases such as wAMD require lifelong IVT administration approximately every 4-8 weeks. This can increase the risk of inflammation, infection, and other adverse effects in some patients. Further, current therapies create compliance challenges due to repeated and/or frequent trips to medical offices for administration of the therapy, especially in elderly patients, who are most affected with wAMD. Reduction in frequency of administration is associated with vision loss and deterioration of the eye disease or condition. The ability of AAV vectors to efficiently transduce target retinal cells following IVT injection has been exploited to successfully transfer therapeutic genes into photoreceptors, retinal pigment epithelium, and the inner retina to treat a variety of retinal diseases. Thus, administration of rAAV particles encoding an anti-VEGF agent (e.g., aflibercept) can provide prolonged and/or sustained release of the anti-VEGF agent *in vivo.*

Surprisingly, administration of a single low unit dose of 6 × 10¹¹ vector genomes (vg) per eye of rAAV particles encoding aflibercept to the eyes of individuals with an ocular neovascular disease led to stabilization of the disease and a robust anatomical response in all treated individuals *(See* Example 1). In addition, visual acuity was stabilized in all treated individuals and none of the individuals required rescue anti-VEGF treatment (e.g., aflibercept IVT injections) after administration of the single low unit dose of 6 × 10¹¹ vg/eye of rAAV particles encoding aflibercept. Moreover, administration of the single unit dose of rAAV particles encoding aflibercept to the eyes of individuals with an ocular neovascular disease unexpectedly caused a reduction (e.g., resolution) of symptoms, including intraretinal and subretinal fluid that were refractory to prior anti-VEGF treatments (e.g., chronic IVT injections of aflibercept, ranibizumab, or bevacizumab).

Accordingly, the present disclosure provides methods of treating an ocular neovascular disease in an individual by administering a single unit dose of 6 × 10¹¹ vg/eye or less of rAAV particles encoding an anti-VEGF agent (e.g., aflibercept). In addition, the present disclosure provides methods for reducing retinal fluid in the eye of an individual with an ocular neovascular disease by administering a single unit dose of rAAV particles encoding an anti-VEGF agent (e.g., aflibercept). The methods disclosed herein reduce or eliminate the need for repeated IVT injections while providing long-term efficacy, thereby addressing the non-compliance and non-adherence problem. In addition, the methods provided herein reduce the adverse effects associated with multiple IVT injections.

### Methods of Treatment

Provided herein is a method for treating an ocular neovascular disease in an individual, the method comprising administering a unit dose of recombinant adeno-associated virus (rAAV) particles to an eye of the individual.

Also provided herein is a method for reducing retinal fluid in the eye of an individual with an ocular neovascular disease, the method comprising administering a unit dose of rAAV particles to an eye of the individual.

Also provided herein is a method for treating an ocular neovascular disease in an individual, the method comprising administering an anti-VEGF agent (e.g., aflibercept) to an eye of the individual, and administering a unit dose of recombinant adeno-associated virus (rAAV) particles to the eye of the individual after administration of the anti-VEGF agent.

In some embodiments, the ocular neovascular disease is wet age-related macular degeneration (wAMD), retinal neovascularization, choroidal neovascularization diabetic retinopathy, proliferative diabetic retinopathy, retinal vein occlusion, central retinal vein occlusion, branched retinal vein occlusion, diabetic macular edema, diabetic retinal ischemia, ischemic retinopathy, diabetic retinal edema, or any combination thereof.

In some embodiments, the term ocular neovascular disease also encompasses VEGF-driven pre-neovascular diseases which, if left untreated, progress to a neovascular form. In some embodiments, the ocular neovascular disease is the pre-neovascular disease, non-proliferative diabetic retinopathy.

In some embodiments, the individual is a human. In some embodiments, the individual received at least one prior treatment (e.g., at least one, at least two, at least three, at least four, at least 5 or more treatments) for the ocular neovascular disease with an anti-VEGF agent (e.g., bevacizumab, brolucizumab, ranibizumab, faricimab, abicipar pegol, conbercept, OPT-302, KSI-301, injectable sunitinib maleate (GB-102), PAN-90806 (PanOptica), and/or aflibercept) in about the last 8 weeks, about the last 9 weeks, about the last 10 weeks, about the last 11 weeks, about the last 12 weeks, about the last 13 weeks, about the last 14 weeks, about the last 15 weeks, or about the last 16 weeks prior to administration of the unit dose of rAAV particles. In some embodiments, the individual demonstrated a meaningful response to a prior treatment with anti-VEGF agent (e.g., bevacizumab, brolucizumab, ranibizumab, faricimab, abicipar pegol, conbercept, OPT-302, KSI-301, injectable sunitinib maleate (GB-102), PAN-90806 (PanOptica), and/or aflibercept). In some embodiments, the anti-VEGF agent is aflibercept, a functional variant thereof, or a functional fragment thereof. In some embodiments, the anti-VEGF agent comprises a polypeptide comprising an amino acid sequence with at least about 95% identity to the amino acid sequence of SEQ ID NO: 35. In some embodiments, the retinal fluid in the eye of an individual is intraretinal fluid (IRF) and/or subretinal fluid (SRF). In some embodiments, the amount or presence of retinal fluid in the eye of the individual is refractory to prior treatment with an anti-VEGF agent (e.g., bevacizumab, brolucizumab, ranibizumab, faricimab, abicipar pegol, conbercept, OPT-302, KSI-301, injectable sunitinib maleate (GB-102), PAN-90806 (PanOptica), and/or aflibercept). In some embodiments, the anti-VEGF agent is aflibercept, a functional variant thereof, or a functional fragment thereof. In some embodiments, the anti-VEGF agent comprises a polypeptide comprising an amino acid sequence with at least about 95% identity to the amino acid sequence of SEQ ID NO: 35.

In some embodiments, the ocular neovascular disease is diabetic macular edema (DME). In some embodiments, the individual is a human. In some embodiments, the individual has type 1 or type 2 diabetes mellitus. In some embodiments, the individual has vision impairment that is due to center involving diabetic macular edema. In some embodiments, the individual has visual acuity (BCVA) of between about 78 to 50 ETDRS letters (e.g., any of 50, 51, 52, 53 54, 55, 56, 57, 58, 59, 60, 61, 62, 63, 64, 65, 66, 67, 68, 69, 70, 71, 72, 73, 74, 75, 76, 77, or 78 ETDRS letters) in the eye administered the rAAV particles prior to administration of the unit dose of rAAV particles. In some embodiments, the individual has visual acuity (Snellen equivalent) of between about 20/32 to about 20/100 in the eye administered the rAAV particles prior to administration of the unit dose of rAAV particles. In some embodiments, the individual has a central subfield thickness (CST) of ≥ 325µm using Heidelberg Spectralis^{®} with center-involving IRF (center 1 mm) in the eye administered the rAAV particles prior to administration of the unit dose of rAAV particles. In some embodiments, the individual has a decrease in vision in the eye administered the rAAV particles prior to administration of the unit dose of rAAV particles that is primarily due to diabetic macular edema. In some embodiments, the individual was diagnosed with diabetic macular edema in the eye administered the rAAV particles about 6 months or less prior to administration of the unit dose of rAAV particles, e.g., any of about 6 months, about 5 months, about 4 months, about 3 months, about 2 months, about 1 month, or less, prior to administration of the unit dose of rAAV particles. In some embodiments, the individual received 0, 1, or 2 prior treatments for DME in the eye administered the rAAV particles, e.g., 0, 1, or 2 intravitreal injections with an anti-VEGF agent, e.g., aflibercept, prior to administration of the unit dose of rAAV particles. In some embodiments, the individual received the prior treatments with an anti-VEGF agent in the eye administered the rAAV particles at least about 60 days (*i.e.*, about 2 months) prior to administration of the unit dose of rAAV particles. In some embodiments, the individual exhibited a meaningful response in central subfield thickness to the prior treatments with an anti-VEGF agent in the eye administered the rAAV particles prior to administration of the unit dose of rAAV particles, for example, at least a 10% reduction in central subfield thickness. In some embodiments, the individual did not experience an adverse reaction to the prior treatments with an anti-VEGF agent prior to administration of the unit dose of rAAV particles. In some embodiments, the individual does not have neutralizing antibodies to AAV2.7m8 prior to administration of the unit dose of rAAV particles. In some embodiments, the individual does not have an anti-AAV2.7m8 neutralizing antibody titer of greater than 1:125 prior to administration of the unit dose of rAAV particles, e.g., within about 6 months prior to administration of the unit dose of rAAV particles. In some embodiments, the individual does not have a history of allergy to aflibercept, corticosteroids, or fluorescein dye or sodium fluorescein (e.g., used in angiography) prior to administration of the unit dose of rAAV particles. In some embodiments, the individual has a history of mild allergy to aflibercept, corticosteroid, or fluorescein dye or sodium fluorescein (e.g., used in angiography) prior to administration of the unit dose of rAAV particles, wherein the allergy is amenable to treatment. In some embodiments, the individual does not have uncontrolled diabetes, e.g., HbA1C of greater than 10%, prior to administration of the unit dose of rAAV particles. In some embodiments, the individual does not have a history of diabetic ketoacidosis within about 3 months prior to administration of the unit dose of rAAV particles. In some embodiments, the individual has not initiated intensive insulin treatment, e.g., with an insulin pump or multiple daily insulin injections, prior to administration of the unit dose of rAAV particles. In some embodiments, the individual does not plan to initiate intensive insulin treatment, e.g., with an insulin pump or multiple daily insulin injections, within about 3 months after administration of the unit dose of rAAV particles. In some embodiments, the individual does not have a history of systemic autoimmune disease that requires treatment with systemic steroids or immunosuppressive treatments, e.g., methotrexate or adalimumab, prior to administration of the unit dose of rAAV particles. In some embodiments, the individual is not being administered a systemic drug known to cause macular edema, such as fingolimod, tamoxifen, chloroquine, or hydroxychloroquine, prior to administration of the unit dose of rAAV particles. In some embodiments, the individual is not being administered a systemic anti-VEGF treatment prior to administration of the unit dose of rAAV particles. In some embodiments, the individual does not have high-risk proliferative diabetic retinopathy (PDR) in the eye administered the rAAV particles prior to administration of the unit dose of rAAV particles. In some embodiments, PDR is defined as any vitreous or preretinal hemorrhage, neovascularization elsewhere >1/2-disc area within an area equivalent to standard ETDRS 7-field on clinical examination, or neovascularization of disc > 1/3-disc area on clinical examination. In some embodiments, the individual does not have focal or grid laser photocoagulation in the eye administered the rAAV particles prior to administration of the unit dose of rAAV particles. In some embodiments, the individual does not have any prior pan retinal photocoagulation (PRP) in the eye administered the rAAV particles prior to administration of the unit dose of rAAV particles. In some embodiments, the individual has not received an anti-VEGF therapy (e.g., aflibercept IVT injections) in the eye administered the rAAV particles prior to administration of the unit dose of rAAV particles. In some embodiments, the individual has not received an anti-VEGF therapy (e.g., aflibercept IVT injections) in the eye administered the rAAV particles for at least 60 days prior to administration of the unit dose of rAAV particles. In some embodiments, the individual has not received more than two anti-VEGF treatments (e.g., aflibercept IVT injections) in the eye administered the rAAV particles prior to administration of the unit dose of rAAV particles. In some embodiments, the individual does not have a history of any of anterior segment neovascularization (e.g., neovascularization of the iris [NVI] or neovascular glaucoma [NVG]), significant vitreous hemorrhage, fibrovascular proliferation, or tractional retinal detachment in the eye administered the rAAV particles prior to administration of the unit dose of rAAV particles. In some embodiments, the individual does not have structural abnormalities at the fovea (e.g., any of dense hard exudates, pigment abnormalities, foveal atrophy, vitreomacular traction or epiretinal membrane) in the eye administered the rAAV particles that contribute to macular edema or visual impairment prior to administration of the unit dose of rAAV particles. In some embodiments, structural abnormalities at the fovea are assessed on clinical examination or OCT. In some embodiments, the individual does not have a history of retinal disease other than diabetic retinopathy (e.g., age-related macular degeneration (in either eye), retinal vein occlusion, retinal arterial occlusion, or pathologic myopia) in the eye administered the rAAV particles prior to administration of the unit dose of rAAV particles. In some embodiments, the individual does not have history of ocular disease other than diabetic macular edema in the eye administered the rAAV particles, e.g., a significant cataract or macular traction, or evidence of posterior subcapsular cataract, prior to administration of the unit dose of rAAV particles. In some embodiments, the individual does not have history of cataract extraction or Yttrium Aluminum Garnet (YAG) capsulotomy in the eye administered the rAAV particles within at least about 3 months prior to administration of the unit dose of rAAV particles. In some embodiments, the individual does not have a history of retinal detachment (with or without repair) in the eye administered the rAAV particles prior to administration of the unit dose of rAAV particles. In some embodiments, the individual does not have a history of any of trabeculectomy, glaucoma shunt, or minimally invasive glaucoma surgery (MIGS) in the eye administered the rAAV particles prior to administration of the unit dose of rAAV particles. In some embodiments, the individual does not have a history of vitrectomy or other filtration surgery in the eye administered the rAAV particles prior to administration of the unit dose of rAAV particles. In some embodiments, the individual does not have aphakia or presence of an anterior chamber intraocular lens in the eye administered the rAAV particles prior to administration of the unit dose of rAAV particles. In some embodiments, the individual does not have uncontrolled ocular hypertension or glaucoma in the eye administered the rAAV particles, e.g., IOP >22 mmHg despite treatment with anti-glaucoma medication or current use of >2 IOP lowering medications, prior to administration of the unit dose of rAAV particles. In some embodiments, the individual does not have a history of intraocular or periocular steroid treatment for any ocular condition (e.g., IVT Triesence, Iluvien or Ozurdex) in the eye administered the rAAV particles prior to administration of the unit dose of rAAV particles. In some embodiments, the individual has not had refractive surgery in the eye administered the rAAV particles within at least about 90 days prior to administration of the unit dose of rAAV particles. In some embodiments, the individual does not have previous penetrating keratoplasty, endothelial keratoplasty, or ocular radiation in the eye administered the rAAV particles prior to administration of the unit dose of rAAV particles. In some embodiments, the individual has not had any prior vitreoretinal surgery in the eye administered the rAAV particles prior to administration of the unit dose of rAAV particles. In some embodiments, the individual does not have a history of uveitis or intraocular inflammation, e.g., grade trace or above except mild anticipated postoperative inflammation that resolved, prior to administration of the unit dose of rAAV particles. In some embodiments, the individual does not have a history of IOP elevation that is related to topical steroid administration prior to administration of the unit dose of rAAV particles. In some embodiments, the individual does not have a history of ocular Herpes Simplex Virus (HSV), Varicella-zoster virus (VZV), or Cytomegalovirus (CMV), including viral uveitis, retinitis, or keratitis prior to administration of the unit dose of rAAV particles. In some embodiments, the individual does not have evidence of any of external ocular infection, including conjunctivitis, chalazion, or significant blepharitis prior to administration of the unit dose of rAAV particles. In some embodiments, the individual does not have history of ocular toxoplasmosis prior to administration of the unit dose of rAAV particles.

In some embodiments, the unit dose is expressed as the number of vector genomes (vg). In some embodiments, the unit dose is about 6 × 10¹¹ vector genomes (vg) or less of the rAAV particles. In some embodiments, the unit dose is expressed as the number of vector genomes (vg) per eye (vg/eye). In some embodiments, the unit dose is about 6 × 10¹¹ vg/eye or less of the rAAV particles. In some embodiments, the unit dose of rAAV particles is about 6 × 10¹⁰ to about 2 × 10¹¹ vg/eye. In some embodiments, the unit dose of rAAV particles is about 2 × 10¹¹ or about 6 × 10¹⁰ vg/eye.

In some embodiments, the unit dose of rAAV particles is administered to one eye of the individual. In some embodiments, the one eye of the individual is the right eye or the left eye. In some embodiments, the one eye of the individual is the right eye. In some embodiments, the one eye of the individual is the left eye. In some embodiments, the methods provided herein further comprise administering a unit dose of rAAV particles to the contralateral eye of the individual. In some embodiments, the one eye of the individual is the right eye and the contralateral eye is the left eye. In some embodiments, the one eye of the individual is the left eye and the contralateral eye is the right eye.

In some embodiments, the administering the unit dose of rAAV particles to the contralateral eye of the individual is up to about 2 weeks (e.g., about 0 days, 1 day, 2 days, 3 days, 4 days, 5 days, 6 days, 7 days, 8 days, 9 days, 10 days, 11 days, 12 days, 13 days, or 14 days) after administering the unit dose of rAAV particles to the one eye. In some embodiments, the unit dose of rAAV particles administered to the contralateral eye of the individual is about the same as (e.g., less than 1% higher or lower, less than 5% higher or lower, less than 10% higher or lower, or less than 20% higher or lower) or lower (e.g., about 5%, about 10%, about 20%, about 30%, about 40%, about 50%, about 60%, about 70%, about 80%, or about 90% lower) than the unit dose of rAAV particles administered to the one eye of the individual.

In some embodiments, the administering the unit dose of rAAV particles to the contralateral eye is at least about 2 weeks (e.g., at least about 2 weeks, at least about 3 weeks, at least about 4 weeks, at least about 1 month, at least about 2 months, at least about 3 months, at least about 4 months, at least about 5 months, at least about 6 months, at least about 7 months, at least about 8 months, at least about 9 months, at least about 10 months, at least about 11 months, at least about 12 months, at least about 1 year, at least about 2 years, at least about 3 years, at least about 4 years, at least about 5 years, or more) after administering the unit dose of rAAV particles to the one eye. In some embodiments, the unit dose of rAAV particles administered to the contralateral eye of the individual is higher (e.g., any of about 5%, about 10%, about 20%, about 30%, about 40%, about 50%, about 60%, about 70%, about 80%, about 90%, about 100%, about 125%, about 150%, about 175%, about 200%, about 225%, about 250%, about 275%, about 300%, or more, higher) than the unit dose of rAAV particles administered to the one eye of the individual.

In some embodiments, the rAAV particles comprise a) a nucleic acid encoding a polypeptide comprising an amino acid sequence with at least about 95%, at least about 96%, at least about 97%, at least about 98%, at least about 99%, at least about 99.99%, or 100% identity to the amino acid sequence of SEQ ID NO: 35 and flanked by AAV2 inverted terminal repeats (ITRs), and b) an AAV2 capsid protein comprising an amino acid sequence LGETTRP (SEQ ID NO: 14) inserted between positions 587 and 588 of the capsid protein, wherein the amino acid residue numbering corresponds to an AAV2 VP1 capsid protein. The sequence of SEQ ID NO: 35 is provided below:

SDTGRPFVEMYSEIPEIIHMTEGRELVIPCRVTSPNITVTLKKFPLDTLIPDGKRIIWDSRKGFIISNATYKE
IGLLTCEATVNGHLYKTNYLTHRQTNTIIDVVLSPSHGIELSVGEKLVLNCTARTELNVGIDFNWEYPSSKHQ
HKKLVNRDLKTQSGSEMKKFLSTLTIDGVTRSDQGLYTCAASSGLMTKKNSTFVRVHEKDKTHTCPPCPAPEL
LGG[?]SVFLFPPKPKDTLMISRTPEVTCVVVDVSHEDPEVKFNWYVDGVEVHNAKTKPREEQYNSTYRVVSVLT
VLHQDWLNGKEYKCKVSNKALPAPIEKTISKAKGQPREPQVYTLPPSRDELTKNQVSLTCLVKGFYPSDIAVE
WESNGQPENNYKTTPPVLDSDGSFFLYSKLTVDKSRWQQGNVFSCSVMHEALHNHYTQKSLSLSPG (SEQ
ID NO: 35)

In some embodiments, the rAAV particles comprise a) a nucleic acid encoding a polypeptide comprising an amino acid sequence with at least about 95% identity to the amino acid sequence of SEQ ID NO: 35 and flanked by AAV2 inverted terminal repeats (ITRs), and b) an AAV2 capsid protein comprising an amino acid sequence LGETTRP (SEQ ID NO: 14) inserted between positions 587 and 588 of the capsid protein, wherein the amino acid residue numbering corresponds to an AAV2 VP1 capsid protein.

In some embodiments, the rAAV particles comprise a nucleic acid encoding a polypeptide comprising an amino acid sequence with at least about 80%, at least about 85%, at least about 90%, at least about 95%, at least about 96%, at least about 97%, at least about 98%, at least about 99%, at least about 99.99%, or 100% identity to the amino acid sequence of SEQ ID NO: 35 and flanked by AAV2 inverted terminal repeats (ITRs). In some embodiments, the rAAV particles comprise a nucleic acid encoding a polypeptide comprising an amino acid sequence with at least about 95% identity to the amino acid sequence of SEQ ID NO: 35 and flanked by AAV2 inverted terminal repeats (ITRs). In some embodiments, the polypeptide comprises the amino acid sequence of SEQ ID NO: 35. In some embodiments, the polypeptide is aflibercept or a functional variant thereof or functional fragment thereof.

In some embodiments, the rAAV particles comprise a nucleic acid comprising a codon-optimized sequence encoding an amino acid sequence with at least about 80%, at least about 85%, at least about 90%, at least about 95%, at least about 96%, at least about 97%, at least about 98%, at least about 99%, at least about 99.99%, or 100% identity to the amino acid sequence of SEQ ID NO: 35 and flanked by AAV2 inverted terminal repeats (ITRs). In some embodiments, the rAAV particles comprise a nucleic acid comprising a codon-optimized sequence encoding an amino acid sequence with at least about 95% identity to the amino acid sequence of SEQ ID NO: 35 and flanked by AAV2 inverted terminal repeats (ITRs). In some embodiments, the rAAV particles comprise a nucleic acid comprising a codon-optimized sequence encoding an amino acid sequence with 100% identity to the amino acid sequence of SEQ ID NO: 35 and flanked by AAV2 inverted terminal repeats (ITRs).

In some embodiments, the rAAV particles comprise a nucleic acid comprising the cDNA sequence of aflibercept or a functional variant thereof or functional fragment thereof and flanked by AAV2 inverted terminal repeats (ITRs). In some embodiments, the rAAV particles comprise a nucleic acid comprising a codon-optimized cDNA sequence of aflibercept or a functional variant thereof or functional fragment thereof and flanked by AAV2 inverted terminal repeats (ITRs). In some embodiments, the rAAV particles comprise a nucleic acid comprising the nucleic acid sequence of SEQ ID NO: 36.

In some embodiments, the nucleic acid further comprises (a) a first enhancer region comprising a CMV sequence; (b) a promoter region comprising a CMV sequence; (c) a 5'UTR region comprising, in the 5' to 3' order, a TPL sequence and an eMLP sequence; (d) a second enhancer region comprising a full EES sequence; and (e) a HGH polyadenylation site. In some embodiments, the enhancer region comprising a CMV sequence comprises the sequence of SEQ ID NO: 22. In some embodiments, the promoter region comprising a CMV sequence comprises the sequence of SEQ ID NO: 23. In some embodiments, the TPL sequence comprises the sequence of SEQ ID NO: 24. In some embodiments, the eMLP sequence comprises the sequence of SEQ ID NO: 25. In some embodiments, the second enhancer region comprising a full EES sequence comprises the sequence of SEQ ID NO: 26. In some embodiments, the HGH polyadenylation site comprises the sequence of SEQ ID NO: 27.

In some embodiments, the rAAV particles comprise an AAV2 capsid protein comprising the amino acid sequence LGETTRP (SEQ ID NO: 14) inserted between positions 587 and 588 of the AAV2 VP1 comprising the sequence of SEQ ID NO: 13. The sequence of SEQ ID NO: 13 is provided below:













 (SEQ ID NO: 13)

In some embodiments, the rAAV particles comprise an AAV2 capsid protein comprising the amino acid sequence LALGETTRPA (SEQ ID NO: 1) inserted between positions 587 and 588 of the capsid protein, wherein the amino acid residue numbering corresponds to an AAV2 VP1 capsid protein. In some embodiments, the rAAV particles comprise an AAV2 capsid protein comprising the amino acid sequence LALGETTRPA (SEQ ID NO: 1) inserted between positions 587 and 588 of the AAV2 VP1 comprising the sequence of SEQ ID NO: 13.

In some embodiments, the rAAV particles comprise an AAV2 capsid protein comprising any of the following amino acid sequences inserted between positions 587 and 588 of the capsid protein, wherein the amino acid residue numbering corresponds to an AAV2 VP1 capsid protein: LALGETTRPA (SEQ ID NO: 1); LANETITRPA (SEQ ID NO: 2), LAKAGQANNA (SEQ ID NO: 3), LAKDPKTTNA (SEQ ID NO: 4), KDTDTTR (SEQ ID NO: 5), RAGGSVG (SEQ ID NO: 6), AVDTTKF (SEQ ID NO: 7), STGKVPN (SEQ ID NO: 8), LAKDTDTTRA (SEQ ID NO: 9), LARAGGSVGA (SEQ ID NO: 10), LAAVDTTKFA (SEQ ID NO: 11), LASTGKVPNA (SEQ ID NO: 12), LGETTRP (SEQ ID NO: 14), NETITRP (SEQ ID NO: 15), KAGQANN (SEQ ID NO: 16), KDPKTTN (SEQ ID NO: 17), KDTDTTR (SEQ ID NO: 18), RAGGSVG (SEQ ID NO: 19), AVDTTKF (SEQ ID NO: 20), and STGKVPN (SEQ ID NO: 21). In some embodiments, the rAAV particles comprise an AAV2 capsid protein comprising any of the following amino acid sequences inserted between positions 587 and 588 of the AAV2 VP1 comprising the sequence of SEQ ID NO: 13: LALGETTRPA (SEQ ID NO: 1); LANETITRPA (SEQ ID NO: 2), LAKAGQANNA (SEQ ID NO: 3), LAKDPKTTNA (SEQ ID NO: 4), KDTDTTR (SEQ ID NO: 5), RAGGSVG (SEQ ID NO: 6), AVDTTKF (SEQ ID NO: 7), STGKVPN (SEQ ID NO: 8), LAKDTDTTRA (SEQ ID NO: 9), LARAGGSVGA (SEQ ID NO: 10), LAAVDTTKFA (SEQ ID NO: 11), LASTGKVPNA (SEQ ID NO: 12), LGETTRP (SEQ ID NO: 14), NETITRP (SEQ ID NO: 15), KAGQANN (SEQ ID NO: 16), KDPKTTN (SEQ ID NO: 17), KDTDTTR (SEQ ID NO: 18), RAGGSVG (SEQ ID NO: 19), AVDTTKF (SEQ ID NO: 20), and STGKVPN (SEQ ID NO: 21).

In some embodiments, the administration of the unit dose of rAAV particles to the one eye and/or to the contralateral eye of the individual is by intravitreal (IVT) injection, intraocular administration, or intraretinal injection. In some embodiments, the administration of the unit dose of rAAV particles to the one eye and/or to the contralateral eye of the individual is by intravitreal (IVT) injection.

In some embodiments, the unit dose of rAAV particles is in a pharmaceutical formulation. In some embodiments, the pharmaceutical formulation comprises the rAAV particles, one or more osmotic or ionic strength agents, one or more buffering agents, one or more surfactants, and one or more solvents. In some embodiments, the osmotic or ionic strength agent is sodium chloride. In some embodiments, the one or more buffering agents are sodium phosphate monobasic and/or sodium phosphate dibasic. In some embodiments, the surfactant is Poloxamer 188. In some embodiments, the solvent is water. In some embodiments, the pharmaceutical formulation comprises the rAAV particles, sodium chloride, sodium phosphate and a surfactant. In some embodiments, the pharmaceutical formulation comprises about 1×10¹⁰ vg/mL to about 1×10¹³ vg/mL of rAAV particles. In some embodiments, the pharmaceutical formulation comprises about 6×10¹¹ vg/mL to about 6×10¹² vg/mL of rAAV particles. In some embodiments, the pharmaceutical formulation comprises about 150 mM to about 200 mM sodium chloride (e.g., any of about 150 mM, about 160 mM, about 170 mM, about 180 mM, about 190 mM, or about 200 mM). In some embodiments, the pharmaceutical formulation comprises about 1 mM to about 10 mM monobasic sodium phosphate (e.g., about 1mM, about 2 mM, about 3 mM, about 4 mM, about 5 mM, about 6 mM, about 7 mM, about 8 mM, about 9 mM, or about 10 mM). In some embodiments, the pharmaceutical formulation comprises about 1 mM to about 10 mM dibasic sodium phosphate (e.g., about 1mM, about 2 mM, about 3 mM, about 4 mM, about 5 mM, about 6 mM, about 7 mM, about 8 mM, about 9 mM, or about 10 mM). In some embodiments, the pharmaceutical formulation comprises about 0.0005% (w/v) to about 0.005% (w/v) poloxamer 188 (e.g., any of about 0.0005% (w/v), 0.0006% (w/v) , 0.0007% (w/v) , 0.0008% (w/v) , 0.0009% (w/v), 0.001% (w/v) , 0.002% (w/v) , 0.003% (w/v) , 0.004% (w/v), or about 0.005% (w/v)). In some embodiments, the pharmaceutical formulation has a pH of about 7.0 to about 7.5 (e.g., any of about 7.0, about 7.1, about 7.2, about 7.3, about 7.4, or about 7.5). In some embodiments, the pharmaceutical formulation comprises about 6×10¹² vg/mL of rAAV particles, about 180 mM sodium chloride, about 5 mM monobasic sodium phosphate, about 5 mM dibasic sodium phosphate, and about 0.001% (w/v) poloxamer 188, wherein the pharmaceutical formulation has a pH of about 7.3. In some embodiments, the pharmaceutical formulation comprises about 6×10¹¹ vg/mL of rAAV particles, about 180 mM sodium chloride, about 5 mM monobasic sodium phosphate, about 5 mM dibasic sodium phosphate, and about 0.001% (w/v) poloxamer 188, wherein the pharmaceutical formulation has a pH of about 7.3.

In some embodiments, the unit dose of rAAV particles comprises a volume of between about 25 µL to about 250 µL (e.g., any of about 25 µL, about 30 µL, about 40 µL, about 50 µL, about 60 µL, about 70 µL, about 80 µL, about 90 µL, about 100 µL, about 110 µL, about 120 µL, about 130 µL, about 140 µL, about 150 µL, about 160 µL, about 170 µL, about 180 µL, about 190 µL, about 200 µL, about 210 µL, about 220 µL, about 230 µL, about 240 µL, or about 250 µL). In some embodiments, the concentration of rAAV particles in the pharmaceutical formulation is adjusted such that the volume of the unit dose of rAAV particles administered to an eye of the individual is between about 25 µL to about 250 µL. In some embodiments, the unit dose of rAAV particles comprises a volume of about 100 µL. In some embodiments, the unit dose of rAAV particles comprises a volume of about 30 µL.

In some embodiments, the unit dose of rAAV particles is administered in combination with steroid treatment. In some embodiments, the steroid treatment is a corticosteroid treatment. In some embodiments, the steroid treatment is a systemic steroid treatment. In some embodiments, the steroid treatment is an oral steroid treatment. In some embodiments, the steroid treatment is a prednisone treatment. In some embodiments, the steroid treatment is an ophthalmic steroid treatment. In some embodiments, the ophthalmic steroid treatment is a topical steroid treatment (e.g., a drop), a periocular steroid treatment (e.g., subtenons, subconjunctival), an intravitreal steroid treatment, or a superchoroidal steroid treatment. In some embodiments, the topical steroid treatment is a difluprednate treatment, a medrysone treatment, a loteprednol treatment, a prednisolone treatment, a fluocinolone treatment, a triamcinolone treatment, a rimexolone treatment, a dexamethasone treatment, a fluorometholone treatment, a fluocinolone treatment, a rimexolone treatment, or a prednisone treatment. In some embodiments, the topical steroid treatment is a difluprednate treatment. In some embodiments, the steroid treatment is administered before, during, and/or after administration of the unit dose of rAAV particles. In some embodiments, the steroid treatment is administered before administration of the unit dose of rAAV particles. In some embodiments, the steroid treatment is administered during administration of the unit dose of rAAV particles. In some embodiments, the steroid treatment is administered after administration of the unit dose of rAAV particles. In some embodiments, the steroid treatment is administered before and during administration of the unit dose of rAAV particles. In some embodiments, the steroid treatment is administered before and after administration of the unit dose of rAAV particles. In some embodiments, the steroid treatment is administered during, and after administration of the unit dose of rAAV particles. In some embodiments, the steroid treatment is administered before, during, and after administration of the unit dose of rAAV particles.

In some embodiments, the steroid treatment is an ophthalmic steroid treatment (e.g., difluprednate). In some embodiments, the ophthalmic steroid treatment (e.g., difluprednate) is a daily steroid treatment for up to about 4 weeks, about 6 weeks, or about 8 weeks from administering the unit dose of rAAV particles. In some embodiments, the ophthalmic steroid treatment comprises about four administrations of ophthalmic steroid on about week 1, about three administrations of ophthalmic steroid on about week 2, about two administrations of ophthalmic steroid on about week 3, and about one administration of ophthalmic steroid on about week 4; timing starting with and following administration of the unit dose of rAAV particles. In some embodiments, the ophthalmic steroid is about 0.005% to about 0.5% difluprednate. In some embodiments, the ophthalmic steroid is any of about 0.005%, about 0.006%, about 0.007%, about 0.008%, about 0.009%, about 0.01%, about 0.02%, about 0.03%, about 0.4%, about 0.05%, about 0.06%, about 0.07%, about 0.08%, about 0.09%, or about 0.1% difluprednate. In some embodiments, the ophthalmic steroid is difluprednate 0.05%. In some embodiments, a dose of difluprednate 0.05% is one drop of ophthalmic solution. In some embodiments, one drop is about 50 µl (e.g., about 25 µl to about 50 µl, about 50 µl to about 100 µl). In some embodiments, a dose of difluprednate comprises about 1 µg to about 5 µg, or about 2 µg to about 3 µg, or about 2.5 µg difluprednate. In some embodiments, a dose of difluprednate comprises about 2.5 µg difluprednate.

In some embodiments, the unit dose of rAAV particles is administered in combination with steroid treatment. In some embodiments, the steroid treatment is a corticosteroid treatment. In some embodiments, the steroid treatment is a systemic steroid treatment. In some embodiments, the steroid treatment is an oral steroid treatment. In some embodiments, the steroid treatment is a prednisone treatment. In some embodiments, the steroid treatment is an ophthalmic steroid treatment. In some embodiments, the ophthalmic steroid treatment is a topical steroid treatment (e.g., a drop), a periocular steroid treatment (e.g., subtenons, subconjunctival), an intravitreal steroid treatment, or a superchoroidal steroid treatment. In some embodiments, the ophthalmic steroid treatment is a glucocorticoid including, but not limited to, an anti-inflammatory glucocorticoid. In some embodiments, the topical steroid treatment is a glucocorticoid including but not limited to, an anti-inflammatory glucocorticoid. In some embodiments, the topical steroid treatment is a difluprednate treatment, a medrysone treatment, a loteprednol treatment, a prednisolone treatment, a fluocinolone treatment, a triamcinolone treatment, a rimexolone treatment, a dexamethasone treatment, a fluorometholone treatment, a fluocinolone treatment, a rimexolone treatment, or a prednisone treatment. Anti-inflammatory glucocorticoids may include, but are not limited to, difluprednate, dexamethasone, prednisolone, triamcinolone, fluorometholone, rimexolone, fluocinolone, loteprednol and generics thereof. In some embodiments, the topical steroid treatment is a difluprednate treatment. By "dexamethasone" is intended dexamethasone and a generic dexamethasone and pharmaceutical compositions comprising dexamethasone a generic dexamethasone. Pharmaceutical compositions comprising dexamethasone include, but are not limited to, Ozurdex^{™}, Maxidex^{™}, Decadron^{™}, Dexamethasone Intensol^{™}, Ocu-Dex^{™}, Dexycu^{™}, Dextenza^{™} and Zodex^{™}. Ozurdex^{™} is a pharmaceutical composition comprising dexamethasone. By "difluprednate" is intended difluprednate, a generic difluprednate, and pharmaceutical compositions comprising difluprednate or a generic difluprednate. Pharmaceutical compositions comprising difluprednate include, but are not limited to, Durezol^{™} and difluprednate emulsions. By "triamcinolone" is intended triamcinolone, a generic triamcinolone, and pharmaceutical compositions comprising triamcinolone, or a generic triamcinolone. Pharmaceutical compositions comprising triamcinolone include, but are not limited to, Triesence^{™}, Xipere^{™}, and Trivaris^{™}. In some embodiments, the steroid treatment is administered before, during, and/or after administration of the unit dose of rAAV particles. In some embodiments, the steroid treatment is administered before administration of the unit dose of rAAV particles. In some embodiments, the steroid treatment is administered during administration of the unit dose of rAAV particles. In some embodiments, the steroid treatment is administered after administration of the unit dose of rAAV particles. In some embodiments, the steroid treatment is administered before and during administration of the unit dose of rAAV particles. In some embodiments, the steroid treatment is administered before and after administration of the unit dose of rAAV particles. In some embodiments, the steroid treatment is administered during, and after administration of the unit dose of rAAV particles. In some embodiments, the steroid treatment is administered before, during, and after administration of the unit dose of rAAV particles.

In some embodiments, the steroid treatment is an ophthalmic steroid treatment (e.g., difluprednate). In some embodiments, the ophthalmic steroid treatment (e.g., difluprednate) is a daily topical steroid treatment for up to about 4 weeks, about 6 weeks, or about 8 weeks from administering the unit dose of rAAV particles. In some embodiments, the topical steroid treatment comprises about four administrations of topical steroid on about week 1, about three administrations of topical steroid on about week 2, about two administrations of topical steroid on about week 3, and about one administration of topical steroid on about week 4; timing starting with and following administration of the unit dose of rAAV particles. In some embodiments, the topical steroid treatment comprises about four administrations of topical steroid *(i.e.,* QID) per day for about 3 weeks after administration of the unit dose of rAAV particles, followed by about 3 administrations of topical steroid per day (*i.e.,* TID) for about 1 week, followed by about 2 administrations of topical steroid per day (*i.e.,* BID) for about 1 week, and followed by about 1 administration of topical steroid per day (*i.e.,* QD) for about 1 week. In some embodiments, the topical steroid comprises difluprednate 0.05% at a dose of about 1µg to about 3 µg. In some embodiments, the topical steroid comprises difluprednate 0.05% at a dose of about 2.5µg. In some embodiments, the topical steroid is about 0.005% to about 0.5% difluprednate. In some embodiments, the topical steroid is any of about 0.005%, about 0.006%, about 0.007%, about 0.008%, about 0.009%, about 0.01%, about 0.02%, about 0.03%, about 0.04%, about 0.05%, about 0.06%, about 0.07%, about 0.08%, about 0.09%, or about 0.1% difluprednate. In some embodiments, the topical steroid is difluprednate 0.05%. In some embodiments, a dose of difluprednate 0.05% is one drop of ophthalmic solution. In some embodiments, one drop is about 50 µl (e.g., about 25 µl to about 50 µl, about 50 µl to about 100 µl). In some embodiments, a dose of difluprednate comprises about 1 µg to about 5 µg, or about 2 µg to about 3 µg, or about 2.5 µg difluprednate. In some embodiments, a dose of difluprednate comprises about 2.5 µg difluprednate.

In some embodiments, the retinal fluid in the eye of the individual (e.g., SRF and/or IRF) is reduced by more than any of about 5%, about 10%, about 15%, about 20%, about 30%, about 40%, about 50%, about 60%, about 70%, about 80%, about 90%, or about 100% after administration of the unit dose of rAAV particles to the one eye and/or to the contralateral of the individual. In some embodiments, the retinal fluid in the eye of the individual (e.g., SRF and/or IRF) is reduced by more than any of about 5%, about 10%, about 15%, about 20%, about 30%, about 40%, about 50%, about 60%, about 70%, about 80%, about 90%, or about 100% after administration of the unit dose of rAAV particles to the one eye and/or to the contralateral of the individual compared to the level of retinal fluid in the eye of the individual prior to administration of the unit dose or rAAV particles. In some embodiments, the retinal fluid in the eye of the individual (e.g., SRF and/or IRF) is reduced by about 100% after administration of the unit dose of rAAV particles to the one eye and/or to the contralateral of the individual compared to the level of retinal fluid in the eye of the individual prior to administration of the unit dose or rAAV particles.

In some embodiments, the methods provided herein further comprise monitoring the level of retinal fluid (e.g., SRF and/or IRF) in the one eye and/or the contralateral eye of the individual after administration of the unit dose of rAAV particles. In some embodiments, the reduction of retinal fluid (e.g., SRF and/or IRF) in the eye is first observed any of about 1 day, about 3 days, about 8 days, about 2 weeks, about 4 weeks, about 6 weeks, about 8 weeks, about 12 weeks, about 16 weeks, about 20 weeks, about 24 weeks, about 28 weeks, about 32 weeks, about 36 weeks, about 40 weeks, about 44 weeks, about 48 weeks, about 52 weeks, about 56 weeks, about 60 weeks, about 64 weeks, about 68 weeks, about 72 weeks, about 76 weeks, about 80 weeks, about 84 weeks, about 88 weeks, about 92 weeks, about 96 weeks, about 100 weeks, about 104 weeks, or more after administration of the unit dose of rAAV particles. In some embodiments, the reduction of retinal fluid (e.g., SRF and/or IRF) in the eye continues or is maintained for at least 1 week, at least 2 weeks, at least 4 weeks, at least 6 weeks, at least 8 weeks, at least 12 weeks, at least 16 weeks, at least 20 weeks, at least 24 weeks, at least 28 weeks, at least 32 weeks, at least 36 weeks, at least 40 weeks, at least 44 weeks, at least 48 weeks, at least 52 weeks, at least 56 weeks, at least 60 weeks, at least 64 weeks, at least 68 weeks, at least 72 weeks, at least 76 weeks, at least 80 weeks, at least 84 weeks, at least 88 weeks, at least 92 weeks, at least 96 weeks, at least 100 weeks, at least 104 weeks, or more after administration of the unit dose of rAAV particles.

In some embodiments, the reduction of retinal fluid (e.g., SRF and/or IRF) in the eye is determined by any method known in the art. In some embodiments, the reduction of retinal fluid (e.g., SRF and/or IRF) in the eye is determined by optical coherence tomography (OCT), spectral domain OCT (SD-OCT), OCT angiography, fluorescein angiography, or by direct retinal observation. In some embodiments, the reduction of retinal fluid (e.g., SRF and/or IRF) in the eye is determined by optical coherence tomography (OCT). In some embodiments, the reduction of retinal fluid (e.g., SRF and/or IRF) in the eye is determined by spectral domain OCT (SD-OCT). In some embodiments, the reduction of retinal fluid (e.g., SRF and/or IRF) in the eye is determined by OCT angiography. In some embodiments, the reduction of retinal fluid (e.g., SRF and/or IRF) in the eye is determined by fluorescein angiography. In some embodiments, the reduction of retinal fluid (e.g., SRF and/or IRF) in the eye is determined by direct retinal observation.

In some embodiments, treatment of an ocular neovascular disease in an individual after administration of the unit dose of rAAV particles to the one eye and/or the contralateral eye is assessed based on the level of retinal fluid (e.g., intraretinal fluid (IRF) and/or subretinal fluid (SRF)) compared the level of retinal fluid (e.g., SRF and/or IRF) prior to administration of the unit dose of rAAV particles to the one eye and/or the contralateral eye (e.g., as described above). In some embodiments, the retinal fluid is subretinal fluid (SRF) or intraretinal fluid (IRF). In some embodiments, the retinal fluid is subretinal fluid (SRF). In some embodiments, the retinal fluid is intraretinal fluid (IRF). In some embodiments, treatment of an ocular neovascular disease in an individual after administration of the unit dose of rAAV particles to the one eye and/or the contralateral eye is determined if a reduction in retinal fluid (e.g., IRF and/or SRF) is observed after administration of the unit dose of rAAV particles to the one eye and/or the contralateral eye compared to the levels of retinal fluid (e.g., IRF and/or SRF) prior to administration of the unit dose of rAAV particles in the one eye and/or the contralateral eye (e.g., as described above). In some embodiments, the ocular neovascular disease is wAMD.

In some embodiments, the administering the unit dose of rAAV particles to the one eye and/or to the contralateral eye of the individual results in maintenance or a decrease of retinal thickness compared to the retinal thickness prior to administration of the unit dose of rAAV particles. In some embodiments, the administering the unit dose of rAAV particles to the one eye and/or to the contralateral eye of the individual results in a decrease of retinal thickness compared to the retinal thickness prior to administration of the unit dose of rAAV particles. In some embodiments, retinal thickness is central subfield thickness (CST) or central retinal thickness (CRT). In some embodiments, the administering the unit dose of rAAV particles to the one eye and/or to the contralateral eye of the individual results in a decrease of retinal thickness of more than any of about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 99%, or about 100% compared to the retinal thickness prior to administration of the unit dose of rAAV particles. In some embodiments, the retinal thickness (e.g., CST or CRT) is determined by OCT or SD-OCT.

In some embodiments, the administering the unit dose of rAAV particles to the one eye and/or to the contralateral eye of the individual results in a decrease of central retinal thickness (CRT) or central subfield thickness (CST) of about 10 µm to about 100 µm (e.g., more than any of about 10 µm, about 15 µm, about 20 µm, about 25 µm, about 30 µm, about 35 µm, about 40 µm, about 45 µm, about 50 µm, about 55 µm, about 60 µm, about 65 µm, about 70 µm, about 75 µm, about 80 µm, about 85 µm, about 90 µm, about 95 µm, about 100 µm, or more). In some embodiments, the administering the unit dose of rAAV particles to the one eye and/or to the contralateral eye of the individual results in a decrease of central retinal thickness (CRT) or central subfield thickness (CST) of about 5 µm to about 50 µm (e.g., any of about 5 µm, about 10 µm, about 15 µm, about 20 µm, about 25 µm, about 30 µm, about 35 µm, about 40 µm, about 45 µm, or about 50 µm). In some embodiments, the administering the unit dose of rAAV particles to the one eye and/or to the contralateral eye of the individual results in a decrease of central retinal thickness (CRT) or central subfield thickness (CST) of about 5 µm to about 40 µm (e.g., any of about 5 µm, about 10 µm, about 15 µm, about 20 µm, about 25 µm, about 30 µm, about 35 µm, or about 40 µm). In some embodiments, the administering the unit dose of rAAV particles to the one eye and/or to the contralateral eye of the individual results in a decrease of central retinal thickness (CRT) or central subfield thickness (CST) of about 5 µm to about 30 µm (e.g., any of about 5 µm, about 6 µm, about 7 µm, about 8 µm, about 9 µm, about 10 µm, about 11 µm, about 12 µm, about 13 µm, about 14 µm, about 15 µm, about 16 µm, about 17 µm, about 18 µm, about 19 µm, about 20 µm, about 21 µm, about 22 µm, about 23 µm, about 24 µm, about 25 µm, about 26 µm, about 27 µm, about 28 µm, about 29 µm, or about 30 µm). In some embodiments, the administering the unit dose of rAAV particles to the one eye and/or to the contralateral eye of the individual results in a decrease of central retinal thickness (CRT) or central subfield thickness (CST) of about 5 µm to about 25 µm (e.g., any of about 5 µm, about 6 µm, about 7 µm, about 8 µm, about 9 µm, about 10 µm, about 11 µm, about 12 µm, about 13 µm, about 14 µm, about 15 µm, about 16 µm, about 17 µm, about 18 µm, about 19 µm, about 20 µm, about 21 µm, about 22 µm, about 23 µm, about 24 µm, or about 25 µm). In some embodiments, the administering the unit dose of rAAV particles to the one eye and/or to the contralateral eye of the individual results in a decrease of central retinal thickness (CRT) or central subfield thickness (CST) of about 5 µm to about 20 µm (e.g., any of about 5 µm, about 6 µm, about 7 µm, about 8 µm, about 9 µm, about 10 µm, about 11 µm, about 12 µm, about 13 µm, about 14 µm, about 15 µm, about 16 µm, about 17 µm, about 18 µm, about 19 µm, or about 20 µm). In some embodiments, the administering the unit dose of rAAV particles to the one eye and/or to the contralateral eye of the individual results in a decrease of central retinal thickness (CRT) or central subfield thickness (CST) of between about 18 µm to about 75 µm. In some embodiments, the administering the unit dose of rAAV particles to the one eye and/or to the contralateral eye of the individual results in a decrease of central retinal thickness (CRT) or central subfield thickness (CST) of about 18.5 µm. In some embodiments, the administering the unit dose of rAAV particles to the one eye and/or to the contralateral eye of the individual results in a decrease of central retinal thickness (CRT) or central subfield thickness (CST) of about 21.0 µm. In some embodiments, the administering the unit dose of rAAV particles to the one eye and/or to the contralateral eye of the individual results in a decrease of central retinal thickness (CRT) or central subfield thickness (CST) of about 8.3 µm. In some embodiments, the administering the unit dose of rAAV particles to the one eye and/or to the contralateral eye of the individual results in a decrease of central retinal thickness (CRT) or central subfield thickness (CST) of about 25.5 µm. In some embodiments, the administering the unit dose of rAAV particles to the one eye and/or to the contralateral eye of the individual results in a decrease of central retinal thickness (CRT) or central subfield thickness (CST) of about 24.8 µm. In some embodiments, the administering the unit dose of rAAV particles to the one eye and/or to the contralateral eye of the individual results in a decrease of central retinal thickness (CRT) or central subfield thickness (CST) of about 75 µm. In some embodiments, the administering the unit dose of rAAV particles to the one eye and/or to the contralateral eye of the individual results in a decrease of central retinal thickness (CRT) or central subfield thickness (CST) of about 100 µm or more (e.g., any of about 100 µm or more, about 110 µm or more, about 120 µm or more, about 130 µm or more, about 140 µm or more, about 150 µm or more, about 160 µm or more, about 170 µm or more, about 180 µm or more, about 190 µm or more, or about 200 µm or more). In some embodiments, the administering the unit dose of rAAV particles to the one eye and/or to the contralateral eye of the individual results in a decrease of central retinal thickness (CRT) or central subfield thickness (CST) of between about 10 µm to about 200 µm (e.g., any of about 10 µm, about 20 µm, about 30 µm, about 40 µm, about 50 µm, about 60 µm, about 70 µm, about 80 µm, about 90 µm, about 100 µm, about 110 µm, about 120 µm, about 130 µm, about 140 µm, about 150 µm, about 160 µm, about 170 µm, about 180 µm, about 190 µm, or about 200 µm). In some embodiments, the administering the unit dose of rAAV particles to the one eye and/or to the contralateral eye of the individual results in a decrease of central retinal thickness (CRT) or central subfield thickness (CST) of between about 50 µm to about 200 µm (e.g., any of about 50 µm, about 60 µm, about 70 µm, about 80 µm, about 90 µm, about 100 µm, about 110 µm, about 120 µm, about 130 µm, about 140 µm, about 150 µm, about 160 µm, about 170 µm, about 180 µm, about 190 µm, or about 200 µm). In some embodiments, the administering the unit dose of rAAV particles to the one eye and/or to the contralateral eye of the individual results in a decrease of central retinal thickness (CRT) or central subfield thickness (CST) of between about 60 µm to about 200 µm (e.g., any of about 60 µm, about 70 µm, about 80 µm, about 90 µm, about 100 µm, about 110 µm, about 120 µm, about 130 µm, about 140 µm, about 150 µm, about 160 µm, about 170 µm, about 180 µm, about 190 µm, or about 200 µm). In some embodiments, the administering the unit dose of rAAV particles to the one eye and/or to the contralateral eye of the individual results in a decrease of central retinal thickness (CRT) or central subfield thickness (CST) of between about 70 µm to about 200 µm (e.g., any of about 70 µm, about 80 µm, about 90 µm, about 100 µm, about 110 µm, about 120 µm, about 130 µm, about 140 µm, about 150 µm, about 160 µm, about 170 µm, about 180 µm, about 190 µm, or about 200 µm). In some embodiments, the administering the unit dose of rAAV particles to the one eye and/or to the contralateral eye of the individual results in a decrease of central retinal thickness (CRT) or central subfield thickness (CST) of between about 80 µm to about 200 µm (e.g., any of about 80 µm, about 90 µm, about 100 µm, about 110 µm, about 120 µm, about 130 µm, about 140 µm, about 150 µm, about 160 µm, about 170 µm, about 180 µm, about 190 µm, or about 200 µm). In some embodiments, the administering the unit dose of rAAV particles to the one eye and/or to the contralateral eye of the individual results in a decrease of central retinal thickness (CRT) or central subfield thickness (CST) of between about 90 µm to about 200 µm (e.g., any of about 90 µm, about 100 µm, about 110 µm, about 120 µm, about 130 µm, about 140 µm, about 150 µm, about 160 µm, about 170 µm, about 180 µm, about 190 µm, or about 200 µm). In some embodiments, the administering the unit dose of rAAV particles to the one eye and/or to the contralateral eye of the individual results in a decrease of central retinal thickness (CRT) or central subfield thickness (CST) of between about 100 µm to about 200 µm (e.g., any of about 100 µm, about 110 µm, about 120 µm, about 130 µm, about 140 µm, about 150 µm, about 160 µm, about 170 µm, about 180 µm, about 190 µm, or about 200 µm). In some embodiments, the administering the unit dose of rAAV particles to the one eye and/or to the contralateral eye of the individual results in a decrease of central retinal thickness (CRT) or central subfield thickness (CST) of between about 110 µm to about 200 µm (e.g., any of about 110 µm, about 120 µm, about 130 µm, about 140 µm, about 150 µm, about 160 µm, about 170 µm, about 180 µm, about 190 µm, or about 200 µm). In some embodiments, the administering the unit dose of rAAV particles to the one eye and/or to the contralateral eye of the individual results in a decrease of central retinal thickness (CRT) or central subfield thickness (CST) of between about 115 µm to about 200 µm (e.g., any of about 115 µm, about 120 µm, about 125 µm, about 130 µm, about 135 µm, about 140 µm, about 145 µm, about 150 µm, about 155 µm, about 160 µm, about 165 µm, about 170 µm, about 175 µm, about 180 µm, about 185 µm, about 190 µm, about 195 µm, or about 200 µm). In some embodiments, the administering the unit dose of rAAV particles to the one eye and/or to the contralateral eye of the individual results in a decrease of central retinal thickness (CRT) or central subfield thickness (CST) of between about 120 µm to about 200 µm (e.g., any of about 120 µm, about 125 µm, about 130 µm, about 135 µm, about 140 µm, about 145 µm, about 150 µm, about 155 µm, about 160 µm, about 165 µm, about 170 µm, about 175 µm, about 180 µm, about 185 µm, about 190 µm, about 195 µm, or about 200 µm). In some embodiments, the administering the unit dose of rAAV particles to the one eye and/or to the contralateral eye of the individual results in a decrease of central retinal thickness (CRT) or central subfield thickness (CST) of between about 125 µm to about 200 µm (e.g., any of about 125 µm, about 130 µm, about 135 µm, about 140 µm, about 145 µm, about 150 µm, about 155 µm, about 160 µm, about 165 µm, about 170 µm, about 175 µm, about 180 µm, about 185 µm, about 190 µm, about 195 µm, or about 200 µm). In some embodiments, the administering the unit dose of rAAV particles to the one eye and/or to the contralateral eye of the individual results in a decrease of central retinal thickness (CRT) or central subfield thickness (CST) of between about 130 µm to about 200 µm (e.g., any of about 130 µm, about 135 µm, about 140 µm, about 145 µm, about 150 µm, about 155 µm, about 160 µm, about 165 µm, about 170 µm, about 175 µm, about 180 µm, about 185 µm, about 190 µm, about 195 µm, or about 200 µm). In some embodiments, the administering the unit dose of rAAV particles to the one eye and/or to the contralateral eye of the individual results in a decrease of central retinal thickness (CRT) or central subfield thickness (CST) of between about 135 µm to about 200 µm (e.g., any of about 135 µm, about 140 µm, about 145 µm, about 150 µm, about 155 µm, about 160 µm, about 165 µm, about 170 µm, about 175 µm, about 180 µm, about 185 µm, about 190 µm, about 195 µm, or about 200 µm). In some embodiments, the administering the unit dose of rAAV particles to the one eye and/or to the contralateral eye of the individual results in a decrease of central retinal thickness (CRT) or central subfield thickness (CST) of between about 140 µm to about 200 µm (e.g., any of about 140 µm, about 145 µm, about 150 µm, about 155 µm, about 160 µm, about 165 µm, about 170 µm, about 175 µm, about 180 µm, about 185 µm, about 190 µm, about 195 µm, or about 200 µm). In some embodiments, the administering the unit dose of rAAV particles to the one eye and/or to the contralateral eye of the individual results in a decrease of central retinal thickness (CRT) or central subfield thickness (CST) of between about 145 µm to about 200 µm (e.g., any of about 145 µm, about 150 µm, about 155 µm, about 160 µm, about 165 µm, about 170 µm, about 175 µm, about 180 µm, about 185 µm, about 190 µm, about 195 µm, or about 200 µm). In some embodiments, the administering the unit dose of rAAV particles to the one eye and/or to the contralateral eye of the individual results in a decrease of central retinal thickness (CRT) or central subfield thickness (CST) of between about 150 µm to about 200 µm (e.g., any of about 150 µm, about 155 µm, about 160 µm, about 165 µm, about 170 µm, about 175 µm, about 180 µm, about 185 µm, about 190 µm, about 195 µm, or about 200 µm). In some embodiments, the administering the unit dose of rAAV particles to the one eye and/or to the contralateral eye of the individual results in a decrease of central retinal thickness (CRT) or central subfield thickness (CST) of between about 155 µm to about 200 µm (e.g., any of about 155 µm, about 160 µm, about 165 µm, about 170 µm, about 175 µm, about 180 µm, about 185 µm, about 190 µm, about 195 µm, or about 200 µm). In some embodiments, the administering the unit dose of rAAV particles to the one eye and/or to the contralateral eye of the individual results in a decrease of central retinal thickness (CRT) or central subfield thickness (CST) of between about 160 µm to about 200 µm (e.g., any of about 160 µm, about 165 µm, about 170 µm, about 175 µm, about 180 µm, about 185 µm, about 190 µm, about 195 µm, or about 200 µm). In some embodiments, the administering the unit dose of rAAV particles to the one eye and/or to the contralateral eye of the individual results in a decrease of central retinal thickness (CRT) or central subfield thickness (CST) of between about 165 µm to about 200 µm (e.g., any of about 165 µm, about 170 µm, about 175 µm, about 180 µm, about 185 µm, about 190 µm, about 195 µm, or about 200 µm). In some embodiments, the administering the unit dose of rAAV particles to the one eye and/or to the contralateral eye of the individual results in a decrease of central retinal thickness (CRT) or central subfield thickness (CST) of between about 170 µm to about 200 µm (e.g., any of about 170 µm, about 175 µm, about 180 µm, about 185 µm, about 190 µm, about 195 µm, or about 200 µm). In some embodiments, the administering the unit dose of rAAV particles to the one eye and/or to the contralateral eye of the individual results in a decrease of central retinal thickness (CRT) or central subfield thickness (CST) of between about 175 µm to about 200 µm (e.g., any of about 175 µm, about 180 µm, about 185 µm, about 190 µm, about 195 µm, or about 200 µm). In some embodiments, the administering the unit dose of rAAV particles to the one eye and/or to the contralateral eye of the individual results in a decrease of central retinal thickness (CRT) or central subfield thickness (CST) of between about 180 µm to about 200 µm (e.g., any of about 180 µm, about 185 µm, about 190 µm, about 195 µm, or about 200 µm). In some embodiments, the administering the unit dose of rAAV particles to the one eye and/or to the contralateral eye of the individual results in a decrease of central retinal thickness (CRT) or central subfield thickness (CST) of about 21.0 µm compared to the central retinal thickness (CRT) or central subfield thickness (CST) prior to administration of the unit dose of rAAV particles. In some embodiments, the administering the unit dose of rAAV particles to the one eye and/or to the contralateral eye of the individual results in a decrease of central retinal thickness (CRT) or central subfield thickness (CST) of about 8.3 µm compared to the central retinal thickness (CRT) or central subfield thickness (CST) prior to administration of the unit dose of rAAV particles. In some embodiments, the administering the unit dose of rAAV particles to the one eye and/or to the contralateral eye of the individual results in a decrease of central retinal thickness (CRT) or central subfield thickness (CST) of about 26.2 µm compared to the central retinal thickness (CRT) or central subfield thickness (CST) prior to administration of the unit dose of rAAV particles. In some embodiments, the administering the unit dose of rAAV particles to the one eye and/or to the contralateral eye of the individual results in a decrease of central retinal thickness (CRT) or central subfield thickness (CST) of about 24.8 µm compared to the central retinal thickness (CRT) or central subfield thickness (CST) prior to administration of the unit dose of rAAV particles. In some embodiments, the administering the unit dose of rAAV particles to the one eye and/or to the contralateral eye of the individual results in a decrease of central retinal thickness (CRT) or central subfield thickness (CST) of about 40.8 µm compared to the central retinal thickness (CRT) or central subfield thickness (CST) prior to administration of the unit dose of rAAV particles. In some embodiments, the administering the unit dose of rAAV particles to the one eye and/or to the contralateral eye of the individual results in a decrease of central retinal thickness (CRT) or central subfield thickness (CST) of about 30.0 µm compared to the central retinal thickness (CRT) or central subfield thickness (CST) prior to administration of the unit dose of rAAV particles. In some embodiments, the administering the unit dose of rAAV particles to the one eye and/or to the contralateral eye of the individual results in a decrease of central retinal thickness (CRT) or central subfield thickness (CST) of about 118.6 µm compared to the central retinal thickness (CRT) or central subfield thickness (CST) prior to administration of the unit dose of rAAV particles. In some embodiments, the administering the unit dose of rAAV particles to the one eye and/or to the contralateral eye of the individual results in a decrease of central retinal thickness (CRT) or central subfield thickness (CST) of about 119.0 µm compared to the central retinal thickness (CRT) or central subfield thickness (CST) prior to administration of the unit dose of rAAV particles. In some embodiments, the administering the unit dose of rAAV particles to the one eye and/or to the contralateral eye of the individual results in a decrease of central retinal thickness (CRT) or central subfield thickness (CST) of about 137.8 µm compared to the central retinal thickness (CRT) or central subfield thickness (CST) prior to administration of the unit dose of rAAV particles. In some embodiments, the administering the unit dose of rAAV particles to the one eye and/or to the contralateral eye of the individual results in a decrease of central retinal thickness (CRT) or central subfield thickness (CST) of about 152.7 µm compared to the central retinal thickness (CRT) or central subfield thickness (CST) prior to administration of the unit dose of rAAV particles. In some embodiments, the administering the unit dose of rAAV particles to the one eye and/or to the contralateral eye of the individual results in a decrease of central retinal thickness (CRT) or central subfield thickness (CST) of about 153.3 µm compared to the central retinal thickness (CRT) or central subfield thickness (CST) prior to administration of the unit dose of rAAV particles. In some embodiments, the administering the unit dose of rAAV particles to the one eye and/or to the contralateral eye of the individual results in a decrease of central retinal thickness (CRT) or central subfield thickness (CST) of about 149.8 µm compared to the central retinal thickness (CRT) or central subfield thickness (CST) prior to administration of the unit dose of rAAV particles.

In some embodiments, the administering the unit dose of rAAV particles to the one eye and/or to the contralateral eye of the individual results in a change in the central retinal thickness (CRT) or central subfield thickness (CST) of between about -200 µm to about +40 µm (e.g., any about -200 µm, about -180 µm, about - 160 µm, about -140 µm, about -120 µm, about -100 µm, about -80 µm, about -60 µm, about -40 µm, about -20 µm, about 0 µm, about +5 µm, about +10 µm, about +15 µm, about +20 µm, about +25 µm, about +30 µm, about +35 µm, or about +40 µm) compared to the retinal thickness prior to administration of the unit dose of rAAV particles. In some embodiments, the administering the unit dose of rAAV particles to the one eye and/or to the contralateral eye of the individual results in a decrease in the central retinal thickness (CRT) or central subfield thickness (CST) of any of about 8 µm, about 11 µm, about 16 µm, about 29 µm, about 33 µm, about 38 µm, about 55 µm, about 61 µm, or about 117 µm. In some embodiments, the administering the unit dose of rAAV particles to the one eye and/or to the contralateral eye of the individual results in a decrease in the central retinal thickness (CRT) or central subfield thickness (CST) of any of about 27.8 µm or about 30.8 µm. In some embodiments, the administering the unit dose of rAAV particles to the one eye and/or to the contralateral eye of the individual results in an increase in the central retinal thickness (CRT) or central subfield thickness (CST) of any of about 4 µm, about 12 µm, or about 32 µm. In some embodiments, the administering the unit dose of rAAV particles to the one eye and/or to the contralateral eye of the individual results in a change in the central retinal thickness (CRT) or central subfield thickness (CST) of about -21.0 µm compared to the central retinal thickness (CRT) or central subfield thickness (CST) prior to administration of the unit dose of rAAV particles. In some embodiments, the administering the unit dose of rAAV particles to the one eye and/or to the contralateral eye of the individual results in a change in the central retinal thickness (CRT) or central subfield thickness (CST) of about -8.3 µm compared to the central retinal thickness (CRT) or central subfield thickness (CST) prior to administration of the unit dose of rAAV particles. In some embodiments, the administering the unit dose of rAAV particles to the one eye and/or to the contralateral eye of the individual results in a change in the central retinal thickness (CRT) or central subfield thickness (CST) of about -26.2 µm compared to the central retinal thickness (CRT) or central subfield thickness (CST) prior to administration of the unit dose of rAAV particles. In some embodiments, the administering the unit dose of rAAV particles to the one eye and/or to the contralateral eye of the individual results in a change in the central retinal thickness (CRT) or central subfield thickness (CST) of about -24.8 µm compared to the central retinal thickness (CRT) or central subfield thickness (CST) prior to administration of the unit dose of rAAV particles. In some embodiments, the administering the unit dose of rAAV particles to the one eye and/or to the contralateral eye of the individual results in a change in the central retinal thickness (CRT) or central subfield thickness (CST) of about -40.8 µm compared to the central retinal thickness (CRT) or central subfield thickness (CST) prior to administration of the unit dose of rAAV particles. In some embodiments, the administering the unit dose of rAAV particles to the one eye and/or to the contralateral eye of the individual results in a change in the central retinal thickness (CRT) or central subfield thickness (CST) of about -30.0 µm compared to the central retinal thickness (CRT) or central subfield thickness (CST) prior to administration of the unit dose of rAAV particles. In some embodiments, the administering the unit dose of rAAV particles to the one eye and/or to the contralateral eye of the individual results in a change in the central retinal thickness (CRT) or central subfield thickness (CST) of about -118.6 µm compared to the central retinal thickness (CRT) or central subfield thickness (CST) prior to administration of the unit dose of rAAV particles. In some embodiments, the administering the unit dose of rAAV particles to the one eye and/or to the contralateral eye of the individual results in a change in the central retinal thickness (CRT) or central subfield thickness (CST) of about -119.0 µm compared to the central retinal thickness (CRT) or central subfield thickness (CST) prior to administration of the unit dose of rAAV particles. In some embodiments, the administering the unit dose of rAAV particles to the one eye and/or to the contralateral eye of the individual results in a change in the central retinal thickness (CRT) or central subfield thickness (CST) of about -137.8 µm compared to the central retinal thickness (CRT) or central subfield thickness (CST) prior to administration of the unit dose of rAAV particles. In some embodiments, the administering the unit dose of rAAV particles to the one eye and/or to the contralateral eye of the individual results in a change in the central retinal thickness (CRT) or central subfield thickness (CST) of about -152.7 µm compared to the central retinal thickness (CRT) or central subfield thickness (CST) prior to administration of the unit dose of rAAV particles. In some embodiments, the administering the unit dose of rAAV particles to the one eye and/or to the contralateral eye of the individual results in a change in the central retinal thickness (CRT) or central subfield thickness (CST) of about -153.3 µm compared to the central retinal thickness (CRT) or central subfield thickness (CST) prior to administration of the unit dose of rAAV particles. In some embodiments, the administering the unit dose of rAAV particles to the one eye and/or to the contralateral eye of the individual results in a change in the central retinal thickness (CRT) or central subfield thickness (CST) of about -149.8 µm compared to the central retinal thickness (CRT) or central subfield thickness (CST) prior to administration of the unit dose of rAAV particles.

In some embodiments, the change (e.g., the decrease) in the central retinal thickness (CRT) or central subfield thickness (CST) compared to the central retinal thickness (CRT) or central subfield thickness (CST) prior to administration of the unit dose of rAAV particles is present at any of about 1 day, about 1 week, about 2 weeks, about 4 weeks, about 8 weeks, about 16 weeks, about 24 weeks, about 30 weeks, about 32 weeks, about 34 weeks, about 40 weeks, about 44 weeks, about 48 weeks, about 52 weeks, or more after administration of the unit dose of rAAV particles to the one eye and/or to the contralateral eye of the individual. In some embodiments, the change (e.g., the decrease) in the central retinal thickness (CRT) or central subfield thickness (CST) compared to the central retinal thickness (CRT) or central subfield thickness (CST) prior to administration of the unit dose of rAAV particles is present at any of about 1 day, about 1 week, about 2 weeks, about 4 weeks, about 8 weeks, about 12 weeks, about 16 weeks, about 20 weeks, about 24 weeks, about 28 weeks, about 32 weeks, about 36 weeks, about 40 weeks, about 44 weeks, about 48 weeks, about 52 weeks, about 56 weeks, about 60 weeks, about 64 weeks, about 68 weeks, about 72 weeks, about 76 weeks, about 80 weeks, about 84 weeks, about 88 weeks, about 92 weeks, about 96 weeks, about 100 weeks, about 104 weeks, about 108 weeks, or more after administration of the unit dose of rAAV particles to the one eye and/or to the contralateral eye of the individual.

In some embodiments, the administering the unit dose of rAAV particles to the one eye and/or to the contralateral eye of the individual results in a decrease of central retinal thickness (CRT) or central subfield thickness (CST) of more than any of about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 99%, or about 100% compared to the retinal thickness prior to administration of the unit dose of rAAV particles. In some embodiments, the administering the unit dose of rAAV particles to the one eye and/or to the contralateral eye of the individual results in a decrease of central retinal thickness (CRT) or central subfield thickness (CST) of at least about 10% compared to the retinal thickness prior to administration of the unit dose of rAAV particles. In some embodiments, the administering the unit dose of rAAV particles to the one eye and/or to the contralateral eye of the individual results in a decrease of central retinal thickness (CRT) or central subfield thickness (CST) of about 15% or more compared to the retinal thickness prior to administration of the unit dose of rAAV particles.

In some embodiments, the administering the unit dose of rAAV particles to the one eye and/or to the contralateral eye of the individual results in an increase of central retinal thickness (CRT) or central subfield thickness (CST) of less than about 40 µm (e.g., any of less than about 40 µm, less than about 35 µm, less than about 30 µm, less than about 25 µm, less than about 20 µm, less than about 15 µm, less than about 10 µm, less than about 5 µm, less than about 1 µm, or less) compared to the retinal thickness prior to administration of the unit dose of rAAV particles. In some embodiments, the administering the unit dose of rAAV particles to the one eye and/or to the contralateral eye of the individual results in an increase of central retinal thickness (CRT) or central subfield thickness (CST) of about 32 µm or less compared to the retinal thickness prior to administration of the unit dose of rAAV particles.

In some embodiments, the maintenance, the decrease, or the increase of retinal thickness compared to the retinal thickness prior to administration of the unit dose of rAAV particles is present at about 30 weeks or more after administration of the unit dose of rAAV particles to the one eye and/or to the contralateral eye of the individual. In some embodiments, the maintenance, the decrease, or the increase of retinal thickness compared to the retinal thickness prior to administration of the unit dose of rAAV particles is present at any of about 30 weeks, about 34 weeks, about 44 weeks, about 6 months, about 1 year, about 1.5 years, about 2 years, about 3 years, about 5 years, about 10 years, or more, after administration of the unit dose of rAAV particles to the one eye and/or to the contralateral eye of the individual.

In some embodiments, the administering the unit dose of rAAV particles to the one eye and/or to the contralateral eye of the individual results in maintenance or a decrease in macular volume compared to the macular volume prior to administration of the unit dose of rAAV particles. In some embodiments, the administering the unit dose of rAAV particles to the one eye and/or to the contralateral eye of the individual results in a decrease in macular volume compared to the macular volume prior to administration of the unit dose of rAAV particles. In some embodiments, the administering the unit dose of rAAV particles to the one eye and/or to the contralateral eye of the individual results in a decrease in macular volume of more than any of about 5%, about 10%, about 15%, about 20%, about 25%, about 30%, about 35%, about 40%, about 45%, or about 50% compared to the macular volume prior to administration of the unit dose of rAAV particles. In some embodiments, the administering the unit dose of rAAV particles to the one eye and/or to the contralateral eye of the individual results in a decrease in macular volume of at least about 10% compared to the macular volume prior to administration of the unit dose of rAAV particles. In some embodiments, the macular volume is determined by OCT or SD-OCT. In some embodiments, the administering the unit dose of rAAV particles to the one eye and/or to the contralateral eye of the individual results in a decrease in macular volume of at least about 10% compared to the macular volume prior to administration of the unit dose of rAAV particles. In some embodiments, the administering the unit dose of rAAV particles to the one eye and/or to the contralateral eye of the individual results in a decrease in macular volume of about 15% or more compared to the macular volume prior to administration of the unit dose of rAAV particles. In some embodiments, the macular volume is determined by OCT or SD-OCT.

In some embodiments, the maintenance or the decrease in macular volume compared to the macular volume prior to administration of the unit dose of rAAV particles is present at about 30 weeks or more after administration of the unit dose of rAAV particles to the one eye and/or to the contralateral eye of the individual. In some embodiments, the maintenance or the decrease in macular volume compared to the macular volume prior to administration of the unit dose of rAAV particles is present at any of about 30 weeks, about 34 weeks, about 44 weeks, about 6 months, about 1 year, about 1.5 years, about 2 years, about 3 years, about 5 years, about 10 years, or more after administration of the unit dose of rAAV particles to the one eye and/or to the contralateral eye of the individual.

In some embodiments, the administering the unit dose of rAAV particles to the one eye and/or to the contralateral eye of the individual results in maintenance or an improvement of visual acuity compared to the visual acuity prior to administration of the unit dose of rAAV particles. In some embodiments, the administering the unit dose of rAAV particles to the one eye and/or to the contralateral eye of the individual results in an improvement of visual acuity compared to the visual acuity prior to administration of the unit dose of rAAV particles. In some embodiments, the administering the unit dose of rAAV particles to the one eye and/or to the contralateral eye of the individual results in an improvement of visual acuity of more than any of about 5%, about 10%, about 20%, about 30%, about 40%, about 50%, about 60%, about 70%, about 80%, about 90%, about 100%, about 125%, about 150%, about 175%, about 200%, about 225%, about 250%, about 275%, about 300%, or more, compared to the visual acuity prior to administration of the unit dose of rAAV particles. In some embodiments, visual acuity is best corrected visual acuity (BCVA). In some embodiments, the administering the unit dose of rAAV particles to the one eye and/or to the contralateral eye of the individual results in an improvement of BCVA compared to the BCVA prior to administration of the unit dose of rAAV particles. In some embodiments, BCVA is expressed as an ETDRS score, which corresponds to the number of letters correctly read (Vitale et al., (2016) JAMA Opthalmol 134(9):1041:1047).

In some embodiments, the administering the unit dose of rAAV particles to the one eye and/or to the contralateral eye of the individual results in an improvement of BCVA of at least 15 ETDRS letters (Vitale et al., (2016) JAMA Opthalmol 134(9):1041:1047) (e.g., at least about 15, at least about 20, at least about 30, at least about 40, at least about 50, at least about 60, or about 70 letters) compared to the BCVA prior to administration of the unit dose of rAAV particles. In some embodiments, the administering the unit dose of rAAV particles to the one eye and/or to the contralateral eye of the individual results in an improvement of BCVA of between about 1 to about 15 (e.g., any of about 1, about 2, about 3, about 4, about 5, about 6, about 7, about 8, about 9, about 10, about 11, about 12, about 13, about 14, or about 15) ETDRS letters compared to the BCVA prior to administration of the unit dose of rAAV particles. In some embodiments, the administering the unit dose of rAAV particles to the one eye and/or to the contralateral eye of the individual results in an improvement of BCVA of about 5 ETDRS letters compared to the BCVA prior to administration of the unit dose of rAAV particles. In some embodiments, the administering the unit dose of rAAV particles to the one eye and/or to the contralateral eye of the individual results in an improvement of BCVA of any of about 1, about 2, about 3, about 4, about 5, about 6, or about 7 ETDRS letters compared to the BCVA prior to administration of the unit dose of rAAV particles. In some embodiments, the administering the unit dose of rAAV particles to the one eye and/or to the contralateral eye of the individual results in an improvement of BCVA of about 3 ETDRS letters or more compared to the BCVA prior to administration of the unit dose of rAAV particles. In some embodiments, the administering the unit dose of rAAV particles to the one eye and/or to the contralateral eye of the individual results in an improvement of BCVA of about 4 ETDRS letters or more compared to the BCVA prior to administration of the unit dose of rAAV particles. In some embodiments, the administering the unit dose of rAAV particles to the one eye and/or to the contralateral eye of the individual results in an improvement of BCVA of about 5.1 ETDRS letters or more compared to the BCVA prior to administration of the unit dose of rAAV particles. In some embodiments, the administering the unit dose of rAAV particles to the one eye and/or to the contralateral eye of the individual results in an improvement of BCVA of about 6.4 ETDRS letters or more compared to the BCVA prior to administration of the unit dose of rAAV particles. In some embodiments, the administering the unit dose of rAAV particles to the one eye and/or to the contralateral eye of the individual results in an improvement of BCVA of about 6.8 ETDRS letters compared to the BCVA prior to administration of the unit dose of rAAV particles. In some embodiments, the administering the unit dose of rAAV particles to the one eye and/or to the contralateral eye of the individual results in an improvement of BCVA of about 8.8 ETDRS letters compared to the BCVA prior to administration of the unit dose of rAAV particles. In some embodiments, the administering the unit dose of rAAV particles to the one eye and/or to the contralateral eye of the individual results in an improvement of BCVA of about 2.3 ETDRS letters compared to the BCVA prior to administration of the unit dose of rAAV particles.

In some embodiments, the administering the unit dose of rAAV particles to the one eye and/or to the contralateral eye of the individual results in maintenance of BCVA, wherein the individual loses fewer than 15 ETDRS letters (Vitale et al., (2016) JAMA Opthalmol 134(9):1041:1047) (e.g., any of 15 or less, 14 or less, 13 or less, 12 or less, 11 or less, 10 or less, 9 or less, 8 or less, 7 or less, 6 or less, 5 or less, 4 or less, 3 or less, 2 or less, 1, or 0 letters) compared to the BCVA prior to administration of the unit dose of rAAV particles. In some embodiments, the administering the unit dose of rAAV particles to the one eye and/or to the contralateral eye of the individual results in maintenance of BCVA, wherein the individual loses about 2 letters compared to the BCVA prior to administration of the unit dose of rAAV particles. In some embodiments, the administering the unit dose of rAAV particles to the one eye and/or to the contralateral eye of the individual results in maintenance of BCVA, wherein the individual loses any of about 1, about 2, about 3, about 4, about 5, about 6, about 7, about 8, or about 9 ETDRS letters compared to the BCVA prior to administration of the unit dose of rAAV particles. In some embodiments, the administering the unit dose of rAAV particles to the one eye and/or to the contralateral eye of the individual results in maintenance of BCVA, wherein the individual loses 0 letters compared to the BCVA prior to administration of the unit dose of rAAV particles. In some embodiments, the administering the unit dose of rAAV particles to the one eye and/or to the contralateral eye of the individual results in maintenance of BCVA, wherein the individual loses about 1 letter compared to the BCVA prior to administration of the unit dose of rAAV particles. In some embodiments, the administering the unit dose of rAAV particles to the one eye and/or to the contralateral eye of the individual results in maintenance of BCVA, wherein the individual loses about 2.7 letters compared to the BCVA prior to administration of the unit dose of rAAV particles. In some embodiments, the administering the unit dose of rAAV particles to the one eye and/or to the contralateral eye of the individual results in maintenance of BCVA, wherein the individual loses about 2.8 letters compared to the BCVA prior to administration of the unit dose of rAAV particles. In some embodiments, the administering the unit dose of rAAV particles to the one eye and/or to the contralateral eye of the individual results in maintenance of BCVA, wherein the individual loses about 2 letters or less compared to the BCVA prior to administration of the unit dose of rAAV particles. In some embodiments, the administering the unit dose of rAAV particles to the one eye and/or to the contralateral eye of the individual results in maintenance of BCVA, wherein the individual loses about 3.2 letters or less compared to the BCVA prior to administration of the unit dose of rAAV particles. In some embodiments, the administering the unit dose of rAAV particles to the one eye and/or to the contralateral eye of the individual results in maintenance of BCVA, wherein the individual loses between about 15 to about 0 letters (e.g., any of about 15, about 14, about 13, about 12, about 11, about 10, about 9, about 8, about 7, about 6, about 5, about 4, about 3, about 2, about 1, or 0 letters) compared to the BCVA prior to administration of the unit dose of rAAV particles. In some embodiments, the administering the unit dose of rAAV particles to the one eye and/or to the contralateral eye of the individual results in maintenance of BCVA, wherein the individual loses between about 10 to about 0 letters (e.g., any of about 10, about 9, about 8, about 7, about 6, about 5, about 4, about 3, about 2, about 1, or 0 letters) compared to the BCVA prior to administration of the unit dose of rAAV particles. In some embodiments, the administering the unit dose of rAAV particles to the one eye and/or to the contralateral eye of the individual results in maintenance of BCVA, wherein the individual loses between about 5 to about 0 letters (e.g., any of about 5, about 4, about 3, about 2, about 1, or 0 letters) compared to the BCVA prior to administration of the unit dose of rAAV particles. In some embodiments, the administering the unit dose of rAAV particles to the one eye and/or to the contralateral eye of the individual results in maintenance of BCVA, wherein the individual loses between about 4 to about 0 letters (e.g., any of about 4, about 3, about 2, about 1, or 0 letters) compared to the BCVA prior to administration of the unit dose of rAAV particles. In some embodiments, the administering the unit dose of rAAV particles to the one eye and/or to the contralateral eye of the individual results in maintenance of BCVA, wherein the individual loses between about 3 to about 0 letters (e.g., any of about 3, about 2, about 1, or 0 letters) compared to the BCVA prior to administration of the unit dose of rAAV particles. In some embodiments, the administering the unit dose of rAAV particles to the one eye and/or to the contralateral eye of the individual results in maintenance of BCVA, wherein the individual loses between about 2 to about 0 letters (e.g., any of about 2, about 1, or 0 letters) compared to the BCVA prior to administration of the unit dose of rAAV particles. In some embodiments, the administering the unit dose of rAAV particles to the one eye and/or to the contralateral eye of the individual results in maintenance of BCVA, wherein the individual loses between about 1 to about 0 letters compared to the BCVA prior to administration of the unit dose of rAAV particles.

In some embodiments, the administering the unit dose of rAAV particles to the one eye and/or to the contralateral eye of the individual results in a change in BCVA of between about -20 to +7 or more (e.g., any of -20, -19, -18, -17, -16, -15, -14, -13, -12, -11, -10, -9, -8, -7, -6, -5, -4, -3, -2, -1, 0, +1, +2, +3, +4, +5, +6, +7, +8, +9, +10, +11, +12, +13, +14, +15, +16, +17, +18, +19, +20, or more) ETDRS letters, compared to the BCVA prior to administration of the unit dose of rAAV particles. In some embodiments, the administering the unit dose of rAAV particles to the one eye and/or to the contralateral eye of the individual results in an increase in BCVA of about any of 16, 7 or 5 ETDRS letters compared to the BCVA prior to administration of the unit dose of rAAV particles. In some embodiments, the administering the unit dose of rAAV particles to the one eye and/or to the contralateral eye of the individual results in a decrease in BCVA of about any of 19, 14, 7, 6, 5, 4, 3, 2, or 1 ETDRS letters compared to the BCVA prior to administration of the unit dose of rAAV particles. In some embodiments, the administering the unit dose of rAAV particles to the one eye and/or to the contralateral eye of the individual results in a decrease in BCVA of about 4.8 or about 0.8 ETDRS letters compared to the BCVA prior to administration of the unit dose of rAAV particles. In some embodiments, the administering the unit dose of rAAV particles to the one eye and/or to the contralateral eye of the individual results in a decrease in BCVA of about 2 ETDRS letters or less compared to the BCVA prior to administration of the unit dose of rAAV particles. In some embodiments, the administering the unit dose of rAAV particles to the one eye and/or to the contralateral eye of the individual results in a decrease in BCVA of about 3.2 ETDRS letters or less compared to the BCVA prior to administration of the unit dose of rAAV particles.

In some embodiments, the administering the unit dose of rAAV particles to the one eye and/or to the contralateral eye of the individual results in a change in BCVA of between about -15 to +7 or more (e.g., any of -15, -14, -13, -12, -11, -10, -9, -8, -7, -6, -5, -4, -3, -2, -1, 0, +1, +2, +3, +4, +5, +6, +7, +8, +9, +10, +11, +12, +13, +14, +15, +16, +17, +18, +19, +20, or more) ETDRS letters, compared to the BCVA prior to administration of the unit dose of rAAV particles. In some embodiments, the administering the unit dose of rAAV particles to the one eye and/or to the contralateral eye of the individual results in a change in BCVA of between about -10 to +7 or more (e.g., any of -10, -9, -8, -7, -6, -5, -4, -3, -2, -1, 0, +1, +2, +3, +4, +5, +6, +7, +8, +9, +10, +11, +12, +13, +14, +15, +16, +17, +18, +19, +20, or more) ETDRS letters, compared to the BCVA prior to administration of the unit dose of rAAV particles. In some embodiments, the administering the unit dose of rAAV particles to the one eye and/or to the contralateral eye of the individual results in a change in BCVA of between about -5 to +7 or more (e.g., any of -5, -4, -3, -2, -1, 0, +1, +2, +3, +4, +5, +6, +7, +8, +9, +10, +11, +12, +13, +14, +15, +16, +17, +18, +19, +20, or more) ETDRS letters, compared to the BCVA prior to administration of the unit dose of rAAV particles. In some embodiments, the administering the unit dose of rAAV particles to the one eye and/or to the contralateral eye of the individual results in a change in BCVA of between about -4 to +7 or more (e.g., any of -4, -3, -2, -1, 0, +1, +2, +3, +4, +5, +6, +7, +8, +9, +10, +11, +12, +13, +14, +15, +16, +17, +18, +19, +20, or more) ETDRS letters, compared to the BCVA prior to administration of the unit dose of rAAV particles. In some embodiments, the administering the unit dose of rAAV particles to the one eye and/or to the contralateral eye of the individual results in a change in BCVA of between about -3 to +7 or more (e.g., any of -3, -2, -1, 0, +1, +2, +3, +4, +5, +6, +7, +8, +9, +10, +11, +12, +13, +14, +15, +16, +17, +18, +19, +20, or more) ETDRS letters, compared to the BCVA prior to administration of the unit dose of rAAV particles. In some embodiments, the administering the unit dose of rAAV particles to the one eye and/or to the contralateral eye of the individual results in a change in BCVA of between about -2 to +7 or more (e.g., any of -2, -1, 0, +1, +2, +3, +4, +5, +6, +7, +8, +9, +10, +11, +12, +13, +14, +15, +16, +17, +18, +19, +20, or more) ETDRS letters, compared to the BCVA prior to administration of the unit dose of rAAV particles. In some embodiments, the administering the unit dose of rAAV particles to the one eye and/or to the contralateral eye of the individual results in a change in BCVA of between about -1 to +7 or more (e.g., any of -1, 0, +1, +2, +3, +4, +5, +6, +7, +8, +9, +10, +11, +12, +13, +14, +15, +16, +17, +18, +19, +20, or more) ETDRS letters, compared to the BCVA prior to administration of the unit dose of rAAV particles. In some embodiments, the administering the unit dose of rAAV particles to the one eye and/or to the contralateral eye of the individual results in a change in BCVA of between about 0 to +7 or more (e.g., any of 0, +1, +2, +3, +4, +5, +6, +7, +8, +9, +10, +11, +12, +13, +14, +15, +16, +17, +18, +19, +20, or more) ETDRS letters, compared to the BCVA prior to administration of the unit dose of rAAV particles. In some embodiments, the administering the unit dose of rAAV particles to the one eye and/or to the contralateral eye of the individual results in a change in BCVA of between about +1 to +7 or more (e.g., any of +1, +2, +3, +4, +5, +6, +7, +8, +9, +10, +11, +12, +13, +14, +15, +16, +17, +18, +19, +20, or more) ETDRS letters, compared to the BCVA prior to administration of the unit dose of rAAV particles. In some embodiments, the administering the unit dose of rAAV particles to the one eye and/or to the contralateral eye of the individual results in a change in BCVA of between about +2 to +7 or more (e.g., any of +2, +3, +4, +5, +6, +7, +8, +9, +10, +11, +12, +13, +14, +15, +16, +17, +18, +19, +20, or more) ETDRS letters, compared to the BCVA prior to administration of the unit dose of rAAV particles. In some embodiments, the administering the unit dose of rAAV particles to the one eye and/or to the contralateral eye of the individual results in a change in BCVA of between about +3 to +7 or more (e.g., any of +3, +4, +5, +6, +7, +8, +9, +10, +11, +12, +13, +14, +15, +16, +17, +18, +19, +20, or more) ETDRS letters, compared to the BCVA prior to administration of the unit dose of rAAV particles. In some embodiments, the administering the unit dose of rAAV particles to the one eye and/or to the contralateral eye of the individual results in a change in BCVA of between about +4 to +7 or more (e.g., any of +4, +5, +6, +7, +8, +9, +10, +11, +12, +13, +14, +15, +16, +17, +18, +19, +20, or more) ETDRS letters, compared to the BCVA prior to administration of the unit dose of rAAV particles. In some embodiments, the administering the unit dose of rAAV particles to the one eye and/or to the contralateral eye of the individual results in a change in BCVA of between about +5 to +7 or more (e.g., any of +5, +6, +7, +8, +9, +10, +11, +12, +13, +14, +15, +16, +17, +18, +19, +20, or more) ETDRS letters, compared to the BCVA prior to administration of the unit dose of rAAV particles. In some embodiments, the administering the unit dose of rAAV particles to the one eye and/or to the contralateral eye of the individual results in a change in BCVA of about +6 or about +7 ETDRS letters, compared to the BCVA prior to administration of the unit dose of rAAV particles.

In some embodiments, administration of the unit dose of rAAV particles to the one eye and/or to the contralateral eye of the individual results in transient inflammation (e.g., inflammation driven by aqueous cells and/or vitreous cells, aqueous flare, posterior synchiae, poor pupil dilation). In some embodiments, administration of the unit dose of rAAV particles to the one eye and/or to the contralateral eye of the individual results in inflammation (e.g., inflammation driven by aqueous cells and/or vitreous cells, aqueous flare, posterior synchiae, poor pupil dilation) that is improved after administration of oral and/or topical steroid treatment and/or mydryatics. In some embodiments, administration of the unit dose of rAAV particles to the one eye and/or to the contralateral eye of the individual results in inflammation (e.g., inflammation driven by aqueous cells and/or vitreous cells) that resolves after administration of oral and/or topical steroid treatment. Inflammation (e.g., inflammation driven by aqueous cells and/or vitreous cells, aqueous flare, posterior synchiae, poor pupil dilation) may be measured using any method known in the art, such as the slit lamp exam.

In some embodiments, the maintenance or the improvement of visual acuity (e.g., BCVA) compared to the visual acuity prior to administration of the unit dose of rAAV particles is present at any of about 1 day, about 1 week, about 2 weeks, about 4 weeks, about 8 weeks, about 12 weeks, about 16 weeks, about 20 weeks, about 24 weeks, about 28 weeks, about 32 weeks, about 36 weeks, about 40 weeks, about 44 weeks, about 48 weeks, about 52 weeks, about 56 weeks, about 60 weeks, about 64 weeks, about 68 weeks, about 72 weeks, about 76 weeks, about 80 weeks, about 84 weeks, about 88 weeks, about 92 weeks, about 96 weeks, about 100 weeks, about 104 weeks, about 108 weeks, or more, after administration of the unit dose of rAAV particles to the one eye and/or to the contralateral eye of the individual. In some embodiments, the maintenance or the improvement of visual acuity (e.g., BCVA) compared to the visual acuity prior to administration of the unit dose of rAAV particles is present at about 30 weeks or more after administration of the unit dose of rAAV particles to the one eye and/or to the contralateral eye of the individual. In some embodiments, the maintenance or the improvement of visual acuity (e.g., BCVA) compared to the visual acuity prior to administration of the unit dose of rAAV particles is present at any of about 30 weeks, about 34 weeks, about 44 weeks, about 6 months, about 1 year, about 1.5 years, about 2 years, about 3 years, about 5 years, about 10 years, or more, after administration of the unit dose of rAAV particles to the one eye and/or to the contralateral eye of the individual.

In some embodiments, treatment of an ocular neovascular disease in an individual after administration of the unit dose of rAAV particles in the one eye and/or the contralateral eye is assessed based on best corrected visual acuity (BCVA) in the one eye and/or the contralateral eye. In some embodiments, BCVA is expressed as an ETDRS score, which corresponds to the number of letters correctly read (Vitale et al., (2016) JAMA Opthalmol 134(9):1041:1047). In some embodiments, an individual is determined to have maintenance of vision and/or visual acuity if the individual loses fewer than 15 letters in an ETDRS score (e.g., any of 15 or less, 14 or less, 13 or less, 12 or less, 11 or less, 10 or less, 9 or less, 8 or less, 7 or less, 6 or less, 5 or less, 4 or less, 3 or less, 2 or less, 1, or 0 letters) compared to prior to administration of the unit dose of rAAV particles in the one eye and/or the contralateral eye. In some embodiments, an individual is determined to have an improvement of vision and/or visual acuity if the individual gains at least 15 letters (e.g., any of at least about 15, at least about 20, at least about 30, at least about 40, at least about 50, at least about 60, or about 70 letters) comparted to prior to administration of the unit dose of rAAV particles in the one eye and/or the contralateral eye.

In some embodiments, treatment of an ocular neovascular disease in an individual after administration of the unit dose of rAAV particles in the one eye and/or the contralateral eye is assessed based on central subfield thickness (CST) or central retinal thickness (CRT) in the one eye and/or the contralateral eye. In some embodiments, CST or CRT is determined by SD-OCT. In some embodiments, treatment of an ocular neovascular disease in an individual after administration of the unit dose of rAAV particles in the one eye and/or the contralateral eye is determined if the CST or CRT assessed by SD-OCT is decreased after administration of the unit dose of rAAV particles in the one eye and/or the contralateral eye compared to prior to administration of the unit dose of rAAV particles in the one eye and/or the contralateral eye. In some embodiments, treatment of an ocular neovascular disease in an individual after administration of the unit dose of rAAV particles in the one eye and/or the contralateral eye is determined if the CST or CRT assessed by SD-OCT is maintained after administration of the unit dose of rAAV particles in the one eye and/or the contralateral eye compared to prior to administration of the unit dose of rAAV particles in the one eye and/or the contralateral eye.

In some embodiments, treatment of an ocular neovascular disease in an individual after administration of the unit dose of rAAV particles in the one eye and/or the contralateral eye is assessed based on macular volume in the one eye and/or the contralateral eye. In some embodiments, macular volume is determined by SD-OCT. In some embodiments, treatment of an ocular neovascular disease in an individual after administration of the unit dose of rAAV particles in the one eye and/or the contralateral eye is determined if the macular volume assessed by SD-OCT is decreased after administration of the unit dose of rAAV particles in the one eye and/or the contralateral eye compared to prior to administration of the unit dose of rAAV particles in the one eye and/or the contralateral eye. In some embodiments, treatment of an ocular neovascular disease in an individual after administration of the unit dose of rAAV particles in the one eye and/or the contralateral eye is determined if the macular volume assessed by SD-OCT is maintained after administration of the unit dose of rAAV particles in the one eye and/or the contralateral eye compared to prior to administration of the unit dose of rAAV particles in the one eye and/or the contralateral eye.

In some embodiments, treatment of an ocular neovascular disease in an individual after administration of the unit dose of rAAV particles in the one eye and/or the contralateral eye is assessed based on retinal thickness (e.g., central retinal thickness (CRT) or central subfield thickness (CST)) and macular volume in the one eye and/or the contralateral eye. In some embodiments, CST and macular volume are determined by SD-OCT. In some embodiments, treatment of an ocular neovascular disease in an individual after administration of the unit dose of rAAV particles in the one eye and/or the contralateral eye is determined if the CST and macular volume assessed by SD-OCT are decreased after administration of the unit dose of rAAV particles in the one eye and/or the contralateral eye compared to prior to administration of the unit dose of rAAV particles in the one eye and/or the contralateral eye. In some embodiments, treatment of an ocular neovascular disease in an individual after administration of the unit dose of rAAV particles in the one eye and/or the contralateral eye is determined if the CST and macular volume assessed by SD-OCT are maintained after administration of the unit dose of rAAV particles in the one eye and/or the contralateral eye compared to prior to administration of the unit dose of rAAV particles in the one eye and/or the contralateral eye.

In some embodiments, treatment of an ocular neovascular disease in an individual after administration of the unit dose of rAAV particles in the one eye and/or the contralateral eye is assessed based on the number of rescue therapy treatments (e.g., aflibercept injections) required by the individual after administration of the unit dose of rAAV particles in the one eye and/or the contralateral eye. In some embodiments, treatment of an ocular neovascular disease in an individual after administration of the unit dose of rAAV particles in the one eye and/or the contralateral eye is determined if an individual requires less than one rescue therapy treatment (e.g., aflibercept injection) any of every 4 weeks, every 5 weeks, every 6 weeks, every 7 weeks, every 8 weeks, every 9 weeks, every 10 weeks, or more, after administration of the unit dose of rAAV particles in the one eye and/or the contralateral eye.

In some embodiments, treatment of an ocular neovascular disease in an individual after administration of the unit dose of rAAV particles in the one eye and/or the contralateral eye is determined if an individual does not require any rescue therapy treatment (e.g., aflibercept injection) for any of at least 1 week, at least 2 weeks, at least 3 weeks, at least 4 weeks, at least 5 weeks, at least 6 weeks, at least 7 weeks, at least 8 weeks, at least 9 weeks, at least 10 weeks, at least 15 weeks, at least 20 weeks, at least 30 weeks, at least 40 weeks, at least 50 weeks, at least 60 weeks, at least 70 weeks, at least 80 weeks, at least 90 weeks, at least 100 weeks, at least 110 weeks, or more, after administration of the unit dose of rAAV particles in the one eye and/or the contralateral eye.

In some embodiments, the individual does not require any rescue therapy treatment (e.g., aflibercept injection) for any of at least about 24 months, at least about 23 months, at least about 22 months, at least about 21 months, at least about 20 months, at least about 19 months, at least about 18 months, at least about 17 months, at least about 16 months, at least about 15 months, at least about 14 months, at least about 13 months, at least about 12 months, at least about 11 months, at least about 10 months, at least about 9 months, at least about 8 months, at least about 7 months, at least about 6 months, at least about 5 months, at least about 4 months, at least about 3 months, at least about 2 months, at least about 1 month, at least about 3 weeks, at least about 2 weeks, or at least about 1 week after administration of the unit dose of rAAV particles in the one eye and/or the contralateral eye. In some embodiments, the individual does not require any rescue therapy treatment (e.g., aflibercept injection) for at least about 12 months after administration of the unit dose of rAAV particles in the one eye and/or the contralateral eye. In some embodiments, the individual does not require any rescue therapy treatment (e.g., aflibercept injection) for at least about 10 months after administration of the unit dose of rAAV particles in the one eye and/or the contralateral eye. In some embodiments, the individual does not require any rescue therapy treatment (e.g., aflibercept injection) for at least about 7 months after administration of the unit dose of rAAV particles in the one eye and/or the contralateral eye. In some embodiments, the individual does not require any rescue therapy treatment (e.g., aflibercept injection) for at least about 6 months after administration of the unit dose of rAAV particles in the one eye and/or the contralateral eye. In some embodiments, the individual does not require any rescue therapy treatment (e.g., aflibercept injection) for at least about 2 months after administration of the unit dose of rAAV particles in the one eye and/or the contralateral eye. In some embodiments, the individual does not require any rescue therapy treatment (e.g., aflibercept injection) for at least about 1 month after administration of the unit dose of rAAV particles in the one eye and/or the contralateral eye.

In some embodiments, administration of a single unit dose of rAAV particles to the one eye and/or to the contralateral eye of a plurality of individuals results in at least about 50% (e.g., any of at least about 50%, at least about 55%, at least about 60%, at least about 65%, at least about 70%, at least about 75%, at least about 80%, at least about 85%, at least about 90%, at least about 95%, at least about 99%, or 100%) of the individuals in the plurality not requiring an anti-VEGF rescue treatment (e.g., aflibercept injection). In some embodiments, administration of a single unit dose of rAAV particles to the one eye and/or to the contralateral eye of a plurality of individuals results in at least about 67% (e.g., any of at least about 67%, at least about 70%, at least about 75%, at least about 80%, at least about 85%, at least about 90%, at least about 95%, at least about 99%, or 100%) of the individuals in the plurality not requiring an anti-VEGF rescue treatment (e.g., aflibercept injection). In some embodiments, administration of a single unit dose of rAAV particles to the one eye and/or to the contralateral eye of a plurality of individuals results in at least about 50% of the individuals in the plurality not requiring an anti-VEGF rescue treatment (e.g., aflibercept injection). In some embodiments, administration of a single unit dose of rAAV particles to the one eye and/or to the contralateral eye of a plurality of individuals results in at least about 78% of the individuals in the plurality not requiring an anti-VEGF rescue treatment (e.g., aflibercept injection). In some embodiments, administration of a single unit dose of rAAV particles to the one eye and/or to the contralateral eye of a plurality of individuals results in at least about 80% of the individuals in the plurality not requiring an anti-VEGF rescue treatment (e.g., aflibercept injection). In some embodiments, administration of a single unit dose of rAAV particles to the one eye and/or to the contralateral eye of a plurality of individuals results in at least about 82% of the individuals in the plurality not requiring an anti-VEGF rescue treatment (e.g., aflibercept injection). In some embodiments, administration of a single unit dose of rAAV particles to the one eye and/or to the contralateral eye of a plurality of individuals results in 100% of the individuals in the plurality not requiring an anti-VEGF rescue treatment (e.g., aflibercept injection).

In some embodiments, administration of a single unit dose of rAAV particles to the one eye and/or to the contralateral eye of a plurality of individuals results in at least about 50% (e.g., any of at least about 50%, at least about 55%, at least about 60%, at least about 65%, at least about 70%, at least about 75%, at least about 80%, at least about 85%, at least about 90%, at least about 95%, at least about 99%, or 100%) of the individuals in the plurality not requiring an anti-VEGF rescue treatment (e.g., aflibercept injection) for at least about 4 weeks after administration of the rAAV particles, e.g., any of at least about 4 weeks, at least about 8 weeks, at least about 12 weeks, at least about 16 weeks, at least about 20 weeks, at least about 24 weeks, at least about 28 weeks, at least about 32 weeks, at least about 36 weeks, at least about 40 weeks, at least about 44 weeks, at least about 48 weeks, at least about 52 weeks, at least about 56 weeks, at least about 60 weeks, at least about 64 weeks, at least about 68 weeks, at least about 72 weeks, at least about 76 weeks, at least about 80 weeks, at least about 84 weeks, at least about 88 weeks, at least about 92 weeks, at least about 96 weeks, at least about 100 weeks, at least about 104 weeks, at least about 108 weeks, or more after administration of the rAAV particles. In some embodiments, administration of a single unit dose of rAAV particles to the one eye and/or to the contralateral eye of a plurality of individuals results in at least about 50% of the individuals in the plurality not requiring an anti-VEGF rescue treatment (e.g., aflibercept injection) for about 52 weeks or more, or about 56 weeks or more, after administration of the rAAV particles. In some embodiments, administration of a single unit dose of rAAV particles to the one eye and/or to the contralateral eye of a plurality of individuals results in at least about 67% (e.g., any of at least about 67%, at least about 70%, at least about 75%, at least about 80%, at least about 85%, at least about 90%, at least about 95%, at least about 99%, or 100%) of the individuals in the plurality not requiring an anti-VEGF rescue treatment (e.g., aflibercept injection) for at least about 20 weeks after administration of the rAAV particles, e.g., any of at least about 20 weeks, at least about 24 weeks, at least about 28 weeks, at least about 32 weeks, at least about 36 weeks, at least about 40 weeks, at least about 44 weeks, at least about 48 weeks, at least about 52 weeks, at least about 56 weeks, at least about 60 weeks, at least about 64 weeks, at least about 66 weeks, or more after administration of the rAAV particles. In some embodiments, administration of a single unit dose of rAAV particles to the one eye and/or to the contralateral eye of a plurality of individuals results in at least about 78% of the individuals in the plurality not requiring an anti-VEGF rescue treatment (e.g., aflibercept injection) for at least about 4 weeks after administration of the rAAV particles, e.g., any of at least about 4 weeks, at least about 8 weeks, at least about 12 weeks, at least about 16 weeks, at least about 20 weeks, at least about 24 weeks, at least about 28 weeks, at least about 32 weeks, at least about 36 weeks, at least about 40 weeks, at least about 44 weeks, at least about 48 weeks, at least about 52 weeks, at least about 56 weeks, at least about 60 weeks, at least about 64 weeks, at least about 68 weeks, at least about 72 weeks, at least about 76 weeks, at least about 80 weeks, at least about 84 weeks, at least about 88 weeks, at least about 92 weeks, at least about 96 weeks, at least about 100 weeks, at least about 104 weeks, at least about 108 weeks, or more, after administration of the rAAV particles. In some embodiments, administration of a single unit dose of rAAV particles to the one eye and/or to the contralateral eye of a plurality of individuals results in at least about 78% of the individuals in the plurality not requiring an anti-VEGF rescue treatment (e.g., aflibercept injection) for about 20 weeks or more, or about 36 weeks or more, after administration of the rAAV particles. In some embodiments, administration of a single unit dose of rAAV particles to the one eye and/or to the contralateral eye of a plurality of individuals results in at least about 80% of the individuals in the plurality not requiring an anti-VEGF rescue treatment (e.g., aflibercept injection) for at least about 20 weeks after administration of the rAAV particles, e.g., any of at least about 20 weeks, at least about 24 weeks, at least about 28 weeks, at least about 32 weeks, at least about 36 weeks, at least about 40 weeks, at least about 44 weeks, at least about 48 weeks, at least about 52 weeks, at least about 56 weeks, at least about 60 weeks, at least about 64 weeks, at least about 66 weeks, or more after administration of the rAAV particles. In some embodiments, administration of a single unit dose of rAAV particles to the one eye and/or to the contralateral eye of a plurality of individuals results in at least about 82% of the individuals in the plurality not requiring an anti-VEGF rescue treatment (e.g., aflibercept injection) for at least about 20 weeks after administration of the rAAV particles, e.g., any of at least about 20 weeks, at least about 24 weeks, at least about 28 weeks, at least about 32 weeks, at least about 36 weeks, at least about 40 weeks, at least about 44 weeks, at least about 48 weeks, at least about 52 weeks, at least about 56 weeks, at least about 60 weeks, at least about 64 weeks, at least about 66 weeks, or more, after administration of the rAAV particles. In some embodiments, administration of a single unit dose of rAAV particles to the one eye and/or to the contralateral eye of a plurality of individuals results in 100% of the individuals in the plurality not requiring an anti-VEGF rescue treatment (e.g., aflibercept injection) for at least about 20 weeks after administration of the rAAV particles, e.g., any of at least about 20 weeks, at least about 24 weeks, at least about 28 weeks, at least about 32 weeks, at least about 36 weeks, at least about 40 weeks, at least about 44 weeks, at least about 48 weeks, at least about 52 weeks, at least about 56 weeks, at least about 60 weeks, at least about 64 weeks, at least about 66 weeks, or more, after administration of the rAAV particles. In some embodiments, administration of a single unit dose of rAAV particles to the one eye and/or to the contralateral eye of a plurality of individuals results in 100% of the individuals in the plurality not requiring an anti-VEGF rescue treatment (e.g., aflibercept injection) for at least about 4 weeks after administration of the rAAV particles, e.g., any of any of at least about 4 weeks, at least about 8 weeks, at least about 12 weeks, at least about 16 weeks, at least about 20 weeks, at least about 24 weeks, at least about 28 weeks, at least about 32 weeks, at least about 36 weeks, at least about 40 weeks, at least about 44 weeks, at least about 48 weeks, at least about 52 weeks, at least about 56 weeks, at least about 60 weeks, at least about 64 weeks, at least about 68 weeks, at least about 72 weeks, at least about 76 weeks, at least about 80 weeks, at least about 84 weeks, at least about 88 weeks, at least about 92 weeks, at least about 96 weeks, at least about 100 weeks, at least about 104 weeks, at least about 108 weeks, or more after administration of the rAAV particles. In some embodiments, administration of a single unit dose of rAAV particles to the one eye and/or to the contralateral eye of a plurality of individuals results in 100% of the individuals in the plurality not requiring an anti-VEGF rescue treatment (e.g., aflibercept injection) for any of about 64 weeks or more, 72 weeks or more, or 84 weeks or more, after administration of the rAAV particles.

In some embodiments, administration of a unit dose of rAAV particles in the one eye and/or the contralateral eye of a plurality of individuals results in about 78% or less (e.g., any of about 78% or less, about 75% or less, about 70% or less, about 65% or less, about 60% or less, about 55% or less, about 50% or less, about 45% or less, about 40% or less, about 35% or less, about 30% or less, about 25% or less, about 20% or less, about 15% or less, about 10% or less, about 5% or less, about 2.5% or less, about 1% or less, or about 0.5% or less) of the individuals in the plurality requiring any rescue treatment (e.g., aflibercept injection) in the one eye and/or the contralateral eye. In some embodiments, administration of a unit dose of rAAV particles in the one eye and/or the contralateral eye of a plurality of individuals results in about 50% or less (e.g., any of about 50% or less, about 45% or less, about 40% or less, about 35% or less, about 30% or less, about 25% or less, about 20% or less, about 15% or less, about 10% or less, about 5% or less, about 2.5% or less, about 1% or less, or about 0.5% or less) of the individuals in the plurality requiring any rescue treatment (e.g., aflibercept injection) in the one eye and/or the contralateral eye. In some embodiments, administration of a unit dose of rAAV particles in the one eye and/or the contralateral eye of a plurality of individuals results in less than about 30% (e.g., less than any of about 30%, about 25%, about 20%, about 15%, about 10%, about 5%, about 2.5%, about 1%, or about 0.5%) of the individuals in the plurality requiring any rescue treatment (e.g., aflibercept injection) in the one eye and/or the contralateral eye. In some embodiments, administration of a unit dose of rAAV particles in the one eye and/or the contralateral eye of a plurality of individuals results in less than about 30% of the individuals in the plurality requiring any rescue treatment (e.g., aflibercept injection) in the one eye and/or the contralateral eye. In some embodiments, administration of a unit dose of rAAV particles in the one eye and/or the contralateral eye of a plurality of individuals results in less than about 20% of the individuals in the plurality requiring any rescue treatment (e.g., aflibercept injection) in the one eye and/or the contralateral eye. In some embodiments, administration of a unit dose of rAAV particles in the one eye and/or the contralateral eye of a plurality of individuals results in 0% of the individuals in the plurality requiring any rescue treatment (e.g., aflibercept injection) in the one eye and/or the contralateral eye.

In some embodiments, administration of a unit dose of rAAV particles in the one eye and/or the contralateral eye of a plurality of individuals results in about 78% or less (e.g., any of about 78% or less, about 75% or less, about 70% or less, about 65% or less, about 60% or less, about 55% or less, about 50% or less, about 45% or less, about 40% or less, about 35% or less, about 30% or less, about 25% or less, about 20% or less, about 15% or less, about 10% or less, about 5% or less, about 2.5% or less, about 1% or less, or about 0.5% or less) of the individuals in the plurality requiring any rescue treatment (e.g., aflibercept injection) for at least about 4 weeks after administration of the rAAV particles, e.g., any of at least about 4 weeks, at least about 8 weeks, at least about 12 weeks, at least about 16 weeks, at least about 20 weeks, at least about 24 weeks, at least about 28 weeks, at least about 32 weeks, at least about 36 weeks, at least about 40 weeks, at least about 44 weeks, at least about 48 weeks, at least about 52 weeks, at least about 56 weeks, at least about 60 weeks, at least about 64 weeks, at least about 68 weeks, at least about 72 weeks, at least about 76 weeks, at least about 80 weeks, at least about 84 weeks, at least about 88 weeks, at least about 92 weeks, at least about 96 weeks, at least about 100 weeks, at least about 104 weeks, at least about 108 weeks, or more, after administration of the rAAV particles. In some embodiments, administration of a unit dose of rAAV particles in the one eye and/or the contralateral eye of a plurality of individuals results in less than about 30% (e.g., less than any of about 30%, about 25%, about 20%, about 15%, about 10%, about 5%, about 2.5%, about 1%, or about 0.5%) of the individuals in the plurality requiring any rescue treatment (e.g., aflibercept injection) for at least about 20 weeks after administration of the rAAV particles, e.g., any of at least about 20 weeks, at least about 24 weeks, at least about 28 weeks, at least about 32 weeks, at least about 36 weeks, at least about 40 weeks, at least about 44 weeks, at least about 48 weeks, at least about 52 weeks, at least about 56 weeks, at least about 60 weeks, at least about 64 weeks, at least about 66 weeks, or more, after administration of the rAAV particles. In some embodiments, administration of a unit dose of rAAV particles in the one eye and/or the contralateral eye of a plurality of individuals results in less than about 30% of the individuals in the plurality requiring any rescue treatment (e.g., aflibercept injection) in the one eye and/or the contralateral eye for at least about 20 weeks after administration of the rAAV particles, e.g., any of at least about 20 weeks, at least about 24 weeks, at least about 28 weeks, at least about 32 weeks, at least about 36 weeks, at least about 40 weeks, at least about 44 weeks, at least about 48 weeks, at least about 52 weeks, at least about 56 weeks, at least about 60 weeks, at least about 64 weeks, at least about 66 weeks, or more, after administration of the rAAV particles. In some embodiments, administration of a unit dose of rAAV particles in the one eye and/or the contralateral eye of a plurality of individuals results in less than about 20% of the individuals in the plurality requiring any rescue treatment (e.g., aflibercept injection) in the one eye and/or the contralateral eye for at least about 20 weeks after administration of the rAAV particles, e.g., any of at least about 20 weeks, at least about 24 weeks, at least about 28 weeks, at least about 32 weeks, at least about 36 weeks, at least about 40 weeks, at least about 44 weeks, at least about 48 weeks, at least about 52 weeks, at least about 56 weeks, at least about 60 weeks, at least about 64 weeks, at least about 66 weeks, or more after administration of the rAAV particles. In some embodiments, administration of a unit dose of rAAV particles in the one eye and/or the contralateral eye of a plurality of individuals results in 0% of the individuals in the plurality requiring any rescue treatment (e.g., aflibercept injection) in the one eye and/or the contralateral eye for at least about 20 weeks after administration of the rAAV particles, e.g., any of at least about 20 weeks, at least about 24 weeks, at least about 28 weeks, at least about 32 weeks, at least about 36 weeks, at least about 40 weeks, at least about 44 weeks, at least about 48 weeks, at least about 52 weeks, at least about 56 weeks, at least about 60 weeks, at least about 64 weeks, at least about 66 weeks, or more after administration of the rAAV particles. In some embodiments, administration of a unit dose of rAAV particles in the one eye and/or the contralateral eye of a plurality of individuals results in 0% of the individuals in the plurality requiring any rescue treatment (e.g., aflibercept injection) in the one eye and/or the contralateral eye for at least about 4 weeks after administration of the rAAV particles, e.g., any of at least about 4 weeks, at least about 8 weeks, at least about 12 weeks, at least about 16 weeks, at least about 20 weeks, at least about 24 weeks, at least about 28 weeks, at least about 32 weeks, at least about 36 weeks, at least about 40 weeks, at least about 44 weeks, at least about 48 weeks, at least about 52 weeks, at least about 56 weeks, at least about 60 weeks, at least about 64 weeks, at least about 68 weeks, at least about 72 weeks, at least about 76 weeks, at least about 80 weeks, at least about 84 weeks, at least about 88 weeks, at least about 92 weeks, at least about 96 weeks, at least about 100 weeks, at least about 104 weeks, at least about 108 weeks, or more, after administration of the rAAV particles.

In some embodiments, administration of the unit dose of rAAV particles to the one eye and/or to the contralateral eye of a plurality of individuals results in a reduction in the mean annualized anti-VEGF injection rate of any of at least about 80%, at least about 85%, at least about 87%, at least about 90%, at least about 95%, at least about 99%, or 100%, compared to the mean annualized anti-VEGF injection rate prior to administration of the unit dose of rAAV particles. In some embodiments, administration of the unit dose of rAAV particles to the one eye and/or to the contralateral eye of a plurality of individuals results in a reduction in the mean annualized anti-VEGF injection rate of about 87% or more compared to the mean annualized anti-VEGF injection rate prior to administration of the unit dose of rAAV particles. In some embodiments, administration of the unit dose of rAAV particles to the one eye and/or to the contralateral eye of a plurality of individuals results in a reduction in the mean annualized anti-VEGF injection rate of 100% compared to the mean annualized anti-VEGF injection rate prior to administration of the unit dose of rAAV particles.

In some embodiments, the mean annualized anti-VEGF injection rate prior to administration of the unit dose of rAAV particles is calculated according to the formula:
Annualized rate prior to administration of the unit dose of rAAV particles = (number of anti-VEGF injections in 12 months prior to administration of the unit dose of rAAV particles) / (days from the first anti-VEGF injection in the past 12 months prior to administration of the unit dose of rAAV particles to the administration of the unit dose of rAAV particles / 365.25)

In some embodiments, the mean annualized anti-VEGF injection rate after administration of the unit dose of rAAV particles is calculated according to the formula:
Annualized rate after administration of the unit dose of rAAV particles = (number of anti-VEGF injections since administration of the unit dose of rAAV particles) / (days from administration of the unit dose of rAAV particles / 365.25).

In some embodiments, an individual is determined to require a rescue treatment (e.g., anti-VEGF intravitreal injection, such as aflibercept injection) after administration of the rAAV particles if the individual exhibits loss of 10 or more letters in BCVA (e.g., using the ETDRS protocol) in the one eye and/or the contralateral eye administered the rAAV particles that is attributed to intraretinal or subretinal fluid (e.g., as determined by SD-OCT) compared to the BCVA in the one eye and/or the contralateral eye administered the rAAV particles prior to administration of the rAAV particles. In some embodiments, an individual is determined to require a rescue treatment (e.g., anti-VEGF intravitreal injection, such as aflibercept injection) after administration of the rAAV particles if the individual exhibits an increase in central subfield thickness (e.g., CST or CRT) greater than 75 µm in the one eye and/or the contralateral eye administered the rAAV particles compared to the central subfield thickness in the one eye and/or the contralateral eye administered the rAAV particles prior to administration of the rAAV particles, e.g., as determined by SD-OCT. In some embodiments, an individual is determined to require a rescue treatment (e.g., anti-VEGF intravitreal injection, such as aflibercept injection) after administration of the rAAV particles if the individual exhibits vision-threatening hemorrhage due to AMD in the one eye and/or the contralateral eye administered the rAAV particles.

In some embodiments, a rescue treatment comprises administration of a standard of care anti-VEGF therapy. Such standard of care anti-VEGF therapy comprises one or more anti-VEGF treatments (e.g., anti-VEGF intravitreal injections). In some embodiments, a rescue treatment comprises one or more aflibercept IVT injections. In some embodiments, a rescue treatment comprises one or more aflibercept IVT injections comprising about 2 mg of aflibercept.

In some embodiments, treatment of an ocular neovascular disease in an individual after administration of the unit dose of rAAV particles in the one eye and/or the contralateral eye is assessed based on the level of retinal fluid compared the level of retinal fluid prior to administration of the unit dose of rAAV particles in the one eye and/or the contralateral eye. In some embodiments, treatment of an ocular neovascular disease in an individual after administration of the unit dose of rAAV particles in the one eye and/or the contralateral eye is determined if a reduction in retinal fluid is observed after administration of the unit dose of rAAV particles in the one eye and/or the contralateral eye compared to the level of retinal fluid prior to administration of the unit dose of rAAV particles in the one eye and/or the contralateral eye. In some embodiments, the ocular neovascular disease is wAMD.

In some embodiments, treatment of an ocular neovascular disease in an individual after administration of the unit dose of rAAV particles in the one eye and/or the contralateral eye is assessed based on the resolution of pigment epithelial detachment (PED) compared to PED prior to administration of the unit dose of rAAV particles in the one eye and/or the contralateral eye. In some embodiments, treatment of an ocular neovascular disease in an individual after administration of the unit dose of rAAV particles in the one eye and/or the contralateral eye is determined if resolution of PED after administration of the unit dose of rAAV particles in the one eye and/or the contralateral eye is observed, compared to PED prior to administration of the unit dose of rAAV particles in the one eye and/or the contralateral eye. In some embodiments, the ocular neovascular disease is wAMD.

In some embodiments, treatment of an ocular neovascular disease in an individual after administration of the unit dose of rAAV particles in the one eye and/or the contralateral eye is assessed based on choroidal neovascularization (CNV) lesion growth as determined by fluorescein angiography. In some embodiments, treatment of an ocular neovascular disease in an individual after administration of the unit dose of rAAV particles in the one eye and/or the contralateral eye is determined if CNV lesions shrink (e.g., by more than any of about 5%, about 10%, about 20%, about 30%, about 40%, about 50%, about 60%, about 70%, about 80%, or 100%) after administration of the unit dose of rAAV particles in the one eye and/or the contralateral eye compared to CNV lesions present prior to administration of the unit dose of rAAV particles in the one eye and/or the contralateral eye. In some embodiments, treatment of an ocular neovascular disease in an individual after administration of the unit dose of rAAV particles in the one eye and/or the contralateral eye is determined if CNV lesions do not grow (e.g., grow less than any of about 1%, about 2%, about 3%, about 4%, about 5%, about 6%, about 7%, about 8%, about 9%, about 10%, about 15%, or about 20%) after administration of the unit dose of rAAV particles in the one eye and/or the contralateral eye compared to CNV lesions present prior to administration of the unit dose of rAAV particles in the one eye and/or the contralateral eye. In some embodiments, the ocular neovascular disease is wAMD.

In some embodiments, treatment of an ocular neovascular disease in an individual after administration of the unit dose of rAAV particles in the one eye and/or the contralateral eye is assessed based on the anatomical features of the one eye and/or the contralateral eye based on any methods known in the art (e.g., SD-OCT, OCT, fluorescein angiography, digital color fundus photography, etc.). In some embodiments, treatment of an ocular neovascular disease in an individual after administration of the unit dose of rAAV particles in the one eye and/or the contralateral eye is determined if an improvement in anatomical features of the one eye and/or the contralateral eye is observed after administration of the unit dose of rAAV particles in the one eye and/or the contralateral eye. In some embodiments, the ocular neovascular disease is wAMD.

In some embodiments, treatment of an ocular neovascular disease in an individual after administration of the unit dose of rAAV particles in the one eye and/or the contralateral eye is assessed based on ophthalmologic examination, intraocular pressure (e.g., using a Goldmann applanation tonometer or Tono-pen), indirect ophthalmoscopy, examination of the one eye and/or the contralateral eye and adnexa, eyelid and/or pupil responsiveness, belpharoptosis, abnormal pupil shape, unequal pupils, abnormal reaction to light, afferent pupillary defects, slit-lamp examination (including of the eyelids, conjunctiva, cornea, lens, iris, and anterior chamber), posterior segment abnormalities of the vitreous, optic nerve, peripheral retina, and retinal vasculature, SD-OCT, fluorescein angiography, digital color fundus photography (including images of the retina, optic disc, and/or macula), aqueous humor sampling, vitreous humor sampling, OCT-angiography (OCT-A), refraction and/or visual acuity (BCVA). In some embodiments, SD-OCT is performed to evaluate retinal thickness (e.g., central retinal thickness or central subfield thickness), macular volume, and/or the presence of fluid (e.g., subretinal fluid or intraretinal fluid). In some embodiments, the ocular neovascular disease is wAMD.

The unit dose of rAAV particles may be administered to the one eye and/or to the contralateral eye of the individual by any method known in the art. For example, the unit dose of rAAV particles may be administered to the one eye and/or to the contralateral eye of the individual intraocularly, or by intravitreal injection. In some embodiments, the administration of the unit dose of rAAV particles to the one eye and/or to the contralateral eye of the individual is intraocular. In some embodiments, the administration of the unit dose of rAAV particles to the one eye and/or to the contralateral eye of the individual is by intravitreal injection (IVT) or subretinal injection. In some embodiments, the administration of the unit dose of rAAV particles to the one eye and/or to the contralateral eye of the individual is by IVT injection. In some embodiments, aseptic technique is employed to administer a unit dose of rAAV particles by intravitreal injection. In some embodiments, aseptic technique with providone-iodine is employed to administer a unit dose of rAAV particles by intravitreal injection.

In some embodiments, the individual has not received a prior treatment for an ocular neovascular disease. In some embodiments, the individual has not received a prior treatment in the one eye and/or the contralateral eye for an ocular neovascular disease. In some embodiments, the individual has not received a prior treatment with an anti-VEGF agent (e.g., bevacizumab, brolucizumab, ranibizumab, faricimab, abicipar pegol, conbercept, OPT-302, KSI-301, injectable sunitinib maleate (GB-102), PAN-90806 (PanOptica), and/or aflibercept). In some embodiments, the individual has not received a prior treatment with an anti-VEGF agent (e.g., bevacizumab, brolucizumab, ranibizumab, faricimab, abicipar pegol, conbercept, OPT-302, KSI-301, injectable sunitinib maleate (GB-102), PAN-90806 (PanOptica), and/or aflibercept) in the one eye and/or the contralateral eye. In some embodiments, the individual has not received a prior aflibercept treatment. In some embodiments, the individual has not received a prior aflibercept treatment in the one eye and/or the contralateral eye.

### Steroid Treatments

In some embodiments, the unit dose of rAAV particles is administered in combination with steroid treatment. In some embodiments, the steroid treatment is a corticosteroid treatment. Exemplary corticosteroids include, without limitation, aclometasone, amcinomide, beclometasone, betamethasone, budesonide, ciclesonide, clobetasol, clobetasone, clocortolone, cloprednol, cortivazol, deflazacort, deoxycorticosterone, desonide desoximetasone, dexamethasone, diflorasone, diflucortolone, difluprednate, fluclorolone, fludrocortisone, fludroxycortide, flumetasone, flunisolide, fluocinolone acetonide, fluocinonide, fluocortin, fluocortolone, fluorometholone, fluperolone, fluticasone, fuprednidene, formocortal, halcinonide, halometasone, hydrocortisone aceponate, hydrocortisone buteprate, hydrocortisone butyrate, loteprednol, medrysone, meprednisone, methylprednisolone, methylprednisolone aceponate, mometasone furoate, paramethasone, prednicarbate, prednisone, prednisolone, prednylidene, remexolone, tixocortol, triamcinolone, and ulobetasol. In some embodiments, the steroid treatment is a systemic steroid treatment. In some embodiments, the steroid treatment is an oral steroid treatment. In some embodiments, the steroid treatment is an ophthalmic steroid treatment. In some embodiments, the ophthalmic steroid treatment is a topical steroid treatment (e.g. a drop), a periocular steroid treatment (e.g., subtenons, subconjunctival), an intravitreal steroid treatment, or a superchoroidal steroid treatment. In some embodiments, the topical steroid treatment is a difluprednate treatment, a medrysone treatment, a loteprednol treatment, a prednisolone treatment, a fluocinolone treatment, a triamcinolone treatment, a rimexolone treatment, a dexamethasone treatment, a fluorometholone treatment, a fluocinolone treatment, a rimexolone treatment, or a prednisone treatment. In some embodiments, the ophthalmic steroid treatment is a difluprednate treatment. In some embodiments, the steroid treatment is a prednisone treatment. In some embodiments, the steroid treatment is a difluprednate treatment.

In some embodiments, the steroid treatment comprises a systemic steroid treatment and a topical steroid treatment. In some embodiments, the systemic steroid treatment is an oral steroid treatment. In some embodiments, the systemic steroid treatment is a prednisone treatment. In some embodiments, the topical steroid treatment is a difluprednate treatment. In some embodiments, the systemic steroid treatment and the topical steroid treatment are administered simultaneously (e.g., on the same day). In some embodiments, the systemic steroid treatment and the topical steroid treatment are administered separately (e.g., on different days).

In some embodiments, the steroid is administered before, during, and/or after administration of the unit dose of rAAV particles. In some embodiments, the steroid is administered before, during, and after administration of the unit dose of rAAV particles. In some embodiments, the steroid is administered during, and after administration of the unit dose of rAAV particles. In some embodiments, the steroid is administered before administration of the unit dose of rAAV particles. In some embodiments, the steroid is administered during administration of the unit dose of rAAV particles. In some embodiments, the steroid is administered before and during administration of the unit dose of rAAV particles. In some embodiments, the steroid is administered after administration of the unit dose of rAAV particles. In some embodiments, the steroid is administered during and after administration of the unit dose of rAAV particles. In some embodiments, the steroid is administered before and/or after administration of the unit dose of rAAV particles. In some embodiments, the steroid is administered before and after administration of the unit dose of rAAV particles.

In some embodiments, the steroid treatment is a systemic steroid treatment. In some embodiments, the systemic steroid treatment is an oral steroid treatment.

In some embodiments, the steroid treatment is an oral prednisone treatment. In some embodiments, the oral prednisone treatment is initiated prior to administration of the unit dose of rAAV particles. In some embodiments, an initial oral prednisone treatment is administered at a dose of any of about 40 mg, about 45 mg, about 50 mg, about 55 mg, about 60 mg, about 65 mg, or about 70 mg of prednisone per day any of about 7 days, about 6 days, about 5 days, about 4 days, about 3 days, about 2 days, about 1 day, or 0 days before administration of the unit dose of rAAV particles, and is continued for any of about 3 days, about 4 days, about 5 days, about 6 days, about 7 days, about 8 days, about 9 days, or about 10 days, or more. In some embodiments, an initial oral prednisone treatment is administered at a dose of about 60 mg of prednisone per day about 3 days before administration of the unit dose of rAAV and is continued for about 3 days.

In some embodiments, the initial oral prednisone treatment is followed by an oral prednisone treatment dose taper. In some embodiments, the oral prednisone treatment dose taper is administered at a dose of any of about 20 mg, about 25 mg, about 30 mg, about 35 mg, about 40 mg, about 45 mg, or about 50 mg of prednisone per day for a total of any of about 1 day, about 2 days, about 3 days, about 4 days, about 5 days, about 6 days, or about 7 days, followed by a dose of about 10 mg, about 15 mg, about 20 mg or about 25 mg of prednisone per day for any of about 1 day, about 2 days, about 3 days, or about 4 days, followed by a dose of about 5 mg, about 10 mg, or about 15 mg of prednisone per day for about 1 day, about 2 days, about 3 days, or about 4 days. In some embodiments, the prednisone dose taper is administered at a dose of any of about 40 mg of prednisone per day for 3 days, followed by a dose of about 20 mg of prednisone per day for 2 days, followed by a dose of about 10 mg of prednisone per day for 2 days.

In some embodiments, an initial oral prednisone treatment is initiated 3 days before administration of the unit dose of rAAV particles at a dose of 60 mg of prednisone per day for a total of 6 days, followed by a dose of 40 mg of prednisone per day for a total of 3 days, followed by a dose of 20 mg of prednisone per day for 2 days, followed by a dose of 10 mg of prednisone per day for 2 days.

In some embodiments, the steroid treatment is an ophthalmic steroid treatment. In some embodiments, the ophthalmic steroid treatment is a difluprednate treatment. In some embodiments, the steroid treatment is administered before, during, and/or after administration of the unit dose of rAAV particles. In some embodiments, the steroid treatment is administered before administration of the unit dose of rAAV particles. In some embodiments, the steroid treatment is administered during administration of the unit dose of rAAV particles. In some embodiments, the steroid treatment is administered after administration of the unit dose of rAAV particles. In some embodiments, the steroid treatment is administered before and during administration of the unit dose of rAAV particles. In some embodiments, the steroid treatment is administered before and after administration of the unit dose of rAAV particles. In some embodiments, the steroid treatment is administered during, and after administration of the unit dose of rAAV particles. In some embodiments, the steroid treatment is administered before, during, and after administration of the unit dose of rAAV particles.

In some embodiments, the steroid treatment is an ophthalmic steroid treatment, e.g., a topical steroid treatment. In some embodiments, the ophthalmic steroid treatment, e.g., a topical steroid treatment, is a daily steroid treatment for up to 4 weeks, up to 6 weeks, up to 8 weeks, up to 3 months, up to 4 months, up to 5 months, or up to 6 months after administration of the unit dose of rAAV particles. In some embodiments, the topical steroid treatment comprises about four administrations of topical steroid on about week 1, about three administrations of topical steroid on about week 2, about two administrations of topical steroid on about week 3, and about one administration of topical steroid on about week 4; timing starting with and following administration of the unit dose of rAAV particles. In some embodiments, the topical steroid treatment comprises about four administrations of topical steroid per day (*i.e.,* QID) for about 3 weeks after administration of the unit dose of rAAV particles, followed by about 3 administrations of topical steroid per day (*i.e.,* TID) for about 1 week, followed by about 2 administrations of topical steroid per day (*i.e.,* BID) for about 1 week, and followed by about 1 administration of topical steroid per day (*i.e.,* QD) for about 1 week. In some embodiments, the ophthalmic steroid treatment is extended at the discretion of the treating physician.

In some embodiments, the ophthalmic steroid is about 0.005% to about 0.5% difluprednate. In some embodiments, the ophthalmic steroid is any of about 0.005%, about 0.006%, about 0.007%, about 0.008%, about 0.009%, about 0.01%, about 0.02%, about 0.03%, about 0.4%, about 0.05%, about 0.06%, about 0.07%, about 0.08%, about 0.09%, or about 0.1% difluprednate. In some embodiments, the ophthalmic steroid is difluprednate 0.05%. In some embodiments, a dose of difluprednate 0.05% is one drop of ophthalmic solution. In some embodiments, one drop is about 50 µl (e.g., about 25 µl to about 50 µl, or about 50 µl to about 100 µl). In some embodiments, a dose of difluprednate comprises about 1 µg to about 5 µg, or about 2 µg to about 3 µg, or about 2.5 µg difluprednate. In some embodiments, a dose of difluprednate comprises about 2.5 µg difluprednate.

In some embodiments, the topical steroid treatment comprises a 7-week topical steroid treatment, e.g., 0.05% difluprednate. In some embodiments, the topical steroid treatment comprises about four administrations of topical steroid per day (*i.e.,* QID) for about four weeks, followed by about three administrations of topical steroid per day (*i.e.,* TID) for about one week, followed by about two administrations of topical steroid per day (*i.e.,* BID) for about one week, and followed by about one administration of topical steroid per day (*i.e.,* QD) for about one week; timing starting at about one week prior to administration of the unit dose of rAAV particles. In some embodiments, the topical steroid treatment comprises about four administrations of topical steroid per day (*i.e.,* QID) for about 28 days, followed by about three administrations of topical steroid per day (*i.e.,* TID) for about 7 days, followed by about two administrations of topical steroid per day (*i.e.,* BID) for about 7 days, and followed by about one administration of topical steroid per day (*i.e.,* QD) for about 7 days; timing starting at about 7 days prior to administration of the unit dose of rAAV particles. In some embodiments, the topical steroid treatment comprises about four administrations of topical steroid per day (*i.e.,* QID) on Day 1 to about Day 28, followed by about three administrations of topical steroid per day (*i.e.,* TID) on about Day 29 to about Day 35, followed by about two administrations of topical steroid per day (*i.e.,* BID) on about Day 36 to about Day 42, and followed by about one administration of topical steroid per day (*i.e.,* QD) on about Day 43 to about Day 49; timing starting at Day 1. In some embodiments, the topical steroid treatment is continued if inflammation is present.

In some embodiments, the methods of treatment provided herein comprise administering an anti-VEGF agent (e.g., an aflibercept IVT injection) to one eye of the individual prior to administration of the unit dose of rAAV particles to the one eye of the individual. In some embodiments, the anti-VEGF agent is administered about 7 days or about 1 week prior to administration of the unit dose of rAAV particles. In some embodiments, the anti-VEGF agent is administered on about Day 1 and the unit dose of rAAV particles is administered on about Day 8. In some embodiments, the topical steroid treatment comprises a 7-week topical steroid treatment, e.g., 0.05% difluprednate. In some embodiments, the topical steroid treatment comprises about four administrations of topical steroid per day (*i.e.,* QID) for about four weeks, followed by about three administrations of topical steroid per day (*i.e.,* TID) for about one week, followed by about two administrations of topical steroid per day (*i.e.,* BID) for about one week, and followed by about one administration of topical steroid per day (*i.e.,* QD) for about one week; timing starting with and following administration of the anti-VEGF agent. In some embodiments, the topical steroid treatment comprises about four administrations of topical steroid per day (*i.e.,* QID) for about 28 days, followed by about three administrations of topical steroid per day (*i.e.,* TID) for about 7 days, followed by about two administrations of topical steroid per day (*i.e.,* BID) for about 7 days, and followed by about one administration of topical steroid per day (*i.e.,* QD) for about 7 days; timing starting with and following administration of the anti-VEGF agent. In some embodiments, the topical steroid treatment comprises about four administrations of topical steroid per day (*i.e.,* QID) on Day 1 to about Day 28, followed by about three administrations of topical steroid per day (*i.e.,* TID) on about Day 29 to about Day 35, followed by about two administrations of topical steroid per day (*i.e.,* BID) on about Day 36 to about Day 42, and followed by about one administration of topical steroid per day (*i.e.,* QD) on about Day 43 to about Day 49; timing starting at Day 1. In some embodiments, the topical steroid treatment is continued if inflammation is present.

In some embodiments, the topical steroid treatment comprises a 4-month topical steroid treatment, e.g., 0.05% difluprednate. In some embodiments, the topical steroid treatment comprises about four administrations of topical steroid per day (*i.e.,* QID) for about one month, followed by about three administrations of topical steroid per day (*i.e.,* TID) for about one month, followed by about two administrations of topical steroid per day (*i.e.,* BID) for about one month, and followed by about one administration of topical steroid per day (*i.e.,* QD) for about one month; timing starting at about one week prior to administration of the unit dose of rAAV particles. In some embodiments, the topical steroid treatment comprises about four administrations of topical steroid per day (*i.e.,* QID) for about 30 days, followed by about three administrations of topical steroid per day (*i.e.,* TID) for about 30 days, followed by about two administrations of topical steroid per day (*i.e.,* BID) for about 30 days, and followed by about one administration of topical steroid per day (*i.e.,* QD) for about 30 days; timing starting at about 7 days prior to administration of the unit dose of rAAV particles. In some embodiments, the topical steroid treatment comprises about four administrations of topical steroid per day (*i.e.,* QID) on Day 1 to about Day 30, followed by about three administrations of topical steroid per day (*i.e.,* TID) on about Day 31 to about Day 60, followed by about two administrations of topical steroid per day (*i.e.,* BID) on about Day 61 to about Day 90, and followed by about one administration of topical steroid per day (*i.e.,* QD) on about Day 91 to about Day 120; timing starting at Day 1. In some embodiments, the topical steroid treatment is continued if inflammation is present.

In some embodiments, the methods of treatment provided herein comprise administering an anti-VEGF agent (e.g., an aflibercept IVT injection) to one eye of the individual prior to administration of the unit dose of rAAV particles to the one eye of the individual. In some embodiments, the anti-VEGF agent is administered about 7 days or about 1 week prior to administration of the unit dose of rAAV particles. In some embodiments, the anti-VEGF agent is administered on about Day 1 and the unit dose of rAAV particles is administered on about Day 8. In some embodiments, the topical steroid treatment comprises a 4-month topical steroid treatment, e.g., 0.05% difluprednate. In some embodiments, the topical steroid treatment comprises about four administrations of topical steroid per day (*i.e.,* QID) for about one month, followed by about three administrations of topical steroid per day (*i.e.,* TID) for about one month, followed by about two administrations of topical steroid per day (*i.e.,* BID) for about one month, and followed by about one administration of topical steroid per day (*i.e.,* QD) for about one month; timing starting with and following administration of the anti-VEGF agent. In some embodiments, the topical steroid treatment comprises about four administrations of topical steroid per day (*i.e.,* QID) for about 30 days, followed by about three administrations of topical steroid per day (*i.e.,* TID) for about 30 days, followed by about two administrations of topical steroid per day (*i.e.,* BID) for about 30 days, and followed by about one administration of topical steroid per day (*i.e.,* QD) for about 30 days; timing starting with and following administration of the anti-VEGF agent. In some embodiments, the topical steroid treatment comprises about four administrations of topical steroid per day (*i.e.,* QID) on Day 1 to about Day 30, followed by about three administrations of topical steroid per day (*i.e.,* TID) on about Day 31 to about Day 60, followed by about two administrations of topical steroid per day (*i.e.,* BID) on about Day 61 to about Day 90, and followed by about one administration of topical steroid per day (*i.e.,* QD) on about Day 91 to about Day 120; timing starting at Day 1. In some embodiments, the topical steroid treatment is continued if inflammation is present.

### Vectors for Delivering Transgenes to Target Cells

In some embodiments, the recombinant adeno-associated virus (rAAV) particles comprise a recombinant viral vector derived from adeno-associated virus (AAV) that has been altered so that it is replication-defective in the subject (e.g., a human or a non-human primate). In some embodiments, the adeno-associated virus (AAV) is a recombinant AAV (rAAV).

AAV or rAAV are small non-enveloped single-stranded DNA viruses. rAAVs are non-pathogenic human parvoviruses and can be made to be dependent on helper viruses, including adenovirus, herpes simplex virus, vaccinia virus and CMV, for replication.

Exposure to wild type (wt) AAV is not associated or known to cause any human pathologies and is common in the general population, making AAV or rAAV a suitable delivery system for gene therapy. AAV and rAAV used for gene therapy for delivery of an anti-VEGF agent, e.g., aflibercept, can be of any serotype. In some embodiments, the methods of the disclosure provide for use of any suitable AAV serotype, including AAV1, AAV2, AAV2.5, AAV3, AAV4, AAV5, AAV6, AAV7, AAV8, AAV9, AAV10, AAV11, AAV12, rh10, AAV-DJ, and any hybrid or chimeric AAV thereof. In some embodiments, the serotype used is based on tropism of the virus, or infectivity of a target cell of interest. In some embodiments, several AAV vectors may be generated to allow selection of the most optimal serotype for use with an anti-VEGF agent transgene (e.g., aflibercept transgene).

In some embodiments, the methods of the present disclosure provide for the use of pseudotyped AAV. Pseudotyped AAV particles comprise AAV genome inverted terminal repeats (ITRs) of one AAV serotype encapsidated by an AAV capsid of another AAV serotype. Typically, pseudotyped AAV is designated as "AAV#/#", where the first "#" indicates the AAV ITR serotype and the second "#" indicates capsid serotype. For example, an AAV particle comprising AAV2 ITRs and an AAV1 capsid would be designated "AAV2/1".

In some embodiments, the rAAV particles comprise a nucleic acid, e.g., a heterologous nucleic acid. In some embodiments, the nucleic acid encodes a transgene, e.g., an anti-VEGF agent (e.g., aflibercept). In some embodiments, the encoded transgene, e.g., anti-VEGF agent, is under the transcriptional control of a promoter that initiates transcription of the nucleic acid. In some embodiments, the promoter is a "ubiquitous" promoter. In some embodiments, the promoter is a "strong" or constitutively active promoter, e.g., a cytomegalovirus (CMV) promoter, an elongation factor 1 alpha (EFla) promoter, a glyceraldehyde 3-phosphate dehydrogenase (GAPDH) promoter, or a connexin36 (or "Cx36") promoter. In some embodiments, the promoter is a tissue-specific promoter that is activated in specific tissues or cells, such as retinal cells, to reduce potential toxicity or undesirable effects to non-targeted cells. In some aspects, several AAV vectors may be generated to allow selection of the most optimal serotype and promoter for use with the anti-VEGF agent transgene (e.g., aflibercept transgene). In some embodiments, the nucleic acid is flanked by AAV inverted terminal repeats (ITRs). In some embodiments, the nucleic acid is flanked by AAV2 ITRs.

In some embodiments, the AAV vector comprises a polynucleotide cassette for enhanced expression of a transgene (e.g., an anti-VEGF agent such as aflibercept) in a target cell (e.g., a retinal cell). In some embodiments, the polynucleotide cassette comprises in 5' to 3' order: (a) a first enhancer region comprising a CMV sequence (SEQ ID NO: 22); (b) a promoter region, comprising a CMV sequence (SEQ ID NO: 23); (c) a 5'UTR region comprising, in 5' to 3' order, TPL and eMLP sequences (SEQ ID NO: 24 and SEQ ID NO: 25, respectively); (d) a coding sequence encoding a peptide or polypeptide (e.g., an anti-VEGF agent such as aflibercept); (e) a second enhancer region comprising a full EES sequence (SEQ ID NO: 26); and (f) a HGH polyadenylation site (SEQ ID NO: 27). In certain of these embodiments, the polynucleotide cassette comprises one or more sequences selected from SEQ ID NOs: 28-32, or a sequence with at least 85% identity thereto. In certain of these embodiments, the 5' arm of the polynucleotide cassette comprises or consists of SEQ ID NO: 33 or a sequence with at least 85% identity thereto. In certain of these embodiments, the 3' arm of the polynucleotide cassette comprises or consists of SEQ ID NO: 34 or a sequence with at least 85% identity thereto. The nucleic acid sequences of SEQ ID NOs: 22-34 are provided below: 
ACTTACGGTA AATGGCCCGC CTGGCTGACC GCCCAACGAC CCCCGCCCAT TGACGTCAAT
AATGACGTAT GTTCCCATAG TAACGCCAAT AGGGACTTTC CATTGACGTC AATGGGTGGA
GTATTTACGG TAAACTGCCC ACTTGGCAGT ACATCAAGTG TATCATATGC CAAGTCCGCC
CCCTATTGAC GTCAATGACG GTAAATGGCC CGCCTGGCAT TATGCCCAGT ACATGACCTT
ACGGGACTTT CCTACTTGGC AGTACATCTA CGTATTAGTC ATCGCTATTA CCA (SEQ ID NO:
22)
 
TGCTGATGCG GTTTTGGCAG TACACCAATG GGCGTGGATA GCGGTTTGAC TCACGGGGAT
TTCCAAGTCT CCACCCCATT GACGTCAATG GGAGTTTGTT TTGGCACCAA AATCAACGGG
ACTTTCCAAA ATGTCGTAAT AACCCCGCCC CGTTGACGCA AATGGGCGGT AGGCGTGTAC
GGTGGGAGGT CTATATAAGC AGAGCTCGTT TAGTGAACCG (SEQ ID NO: 23)
 
CTCACTCTCT TCCGCATCGC TGTCTGCGAG GGCCAGCTGT TGGGCTCGCG GTTGAGGACA
AACTCTTCGC GGTCTTTCCA GTACTCTTGG ATCGGAAACC CGTCGGCCTC CGAACGGTAC
TCCGCCACCG AGGGACCTGA GCGAGTCCGC ATCGACCGGA TCGGAAAACC TCTCGAGAAA
GGCGTCTAAC CAGTCACAGT CGCAAGGTAG GCTGAGCACC GTGGCGGGCG GCAGCGGGTG
GCGGTCGGGG TTGTTTCTGG CGGAGGTGCT GCTGATGATG TAATTAAAGT AGGCGGTCTT
GAGACGGCGG ATGGTCGA (SEQ ID NO: 24)
 
CCAGCTGTTG GGGTGAGTAC TCCCTCTCAA AAGCGGGCAT TACTTCTGCG CTAAGATTGT
CAGTTTCCAA AAACGAGGAG GATTTGATAT TCACCTGGCC CG (SEQ ID NO: 25)
 
CTGTTCTCAT CACATCATAT CAAGGTTATA TACCATCAAT ATTGCCACAG ATGTTACTTA
GCCTTTTAAT ATTTCTCTAA TTTAGTGTAT ATGCAATGAT AGTTCTCTGA TTTCTGAGAT
TGAGTTTCTC ATGTGTAATG ATTATTTAGA GTTTCTCTTT CATCTGTTCA AATTTTTGTC
TAGTTTTATT TTTTACTGAT TTGTAAGACT TCTTTTTATA ATCTGCATAT TACAATTCTC
TTTACTGGGG TGTTGCAAAT ATTTTCTGTC ATTCTATGGC CTGACTTTTC TTAATGGTTT
TTTAATTTTA AAAATAAGTC TTAATATTCA TGCAATCTAA TTAACAATCT TTTCTTTGTG
GTTAGGACTT TGAGTCATAA GAAATTTTTC TCTACACTGA AGTCATGATG GCATGCTTCT
ATATTATTTT CTAAAAGATT TAAAGTTTTG CCTTCTCCAT TTAGACTTAT AATTCACTGG
AATTTTTTTG TGTGTATGGT ATGACATATG GGTTCCCTTT TATTTTTTAC ATATAAATAT
ATTTCCCTGT TTTTCTAAAA AAGAAAAAGA TCATCATTTT CCCATTGTAA AATGCCATAT
TTTTTTCATA GGTCACTTAC ATATATCAAT GGGTCTGTTT CTGAGCTCTA CTCTATTTTA
TCAGCCTCAC TGTCTATCCC CACACATCTC ATGCTTTGCT CTAAATCTTG ATATTTAGTG
GAACATTCTT TCCCATTTTG TTCTACAAGA ATATTTTTGT TATTGTCTTT GGGCTTTCTA
TATACATTTT GAAATGAGGT TGACAAGTTA (SEQ ID NO: 26)
 
CTGCCCGGGT GGCATCCCTG TGACCCCTCC CCAGTGCCTC TCCTGGCCCT GGAAGTTGCC
ACTCCAGTGC CCACCAGCCT TGTCCTAATA AAATTAAGTT GCATCATTTT GTCTGACTAG
 
GTGTCCTTCT ATAATATTAT GGGGTGGAGG GGGGTGGTAT GGAGCAAGGG GCCCAAGTTG
GGAAGAAACC TGTAGGGCCT GC (SEQ ID NO: 27)
 
AGGCGGTCTT GAGACGGCGG ATGGTCGAGG TGAGGTGTGG CAGGCTTGAG ATCCAGCTGT
TGGGGTGA (SEQ ID NO: 28)
 
CGCTGTTTTG ACCTCCATAG TGGACACCGG GACCGATCCA GCCTCCGCGT CTCAGGGGAG
ATCTCGTTTA GTGAACCGTC AGATCCTCAC TCTCTTCCGC ATCGCTGTCT GCGAGGGCCA
GCTGTTGGG (SEQ ID NO: 29)
TTGATATTCA CCTGGCCCGA TCTGGCCATA CACTTG (SEQ ID NO: 30)
CCCAGGTCCA AGTTTAAACG CC (SEQ ID NO: 31)

TCTTTGGGCT TTCTATATAC ATTTTGAAAT GAGGTTGACA AGTTACCTAG GAAAACTGTC
TTCCTGCCCG GGTGGCA (SEQ ID NO: 32)

CTCTGGAGAC GACTTACGGT AAATGGCCCG CCTGGCTGAC CGCCCAACGA CCCCCGCCCA
TTGACGTCAA TAATGACGTA TGTTCCCATA GTAACGCCAA TAGGGACTTT CCATTGACGT
CAATGGGTGG AGTATTTACG GTAAACTGCC CACTTGGCAG TACATCAAGT GTATCATATG
CCAAGTCCGC CCCCTATTGA CGTCAATGAC GGTAAATGGC CCGCCTGGCA TTATGCCCAG
TACATGACCT TACGGGACTT TCCTACTTGG CAGTACATCT ACGTATTAGT CATCGCTATT
ACCATGCTGA TGCGGTTTTG GCAGTACACC AATGGGCGTG GATAGCGGTT TGACTCACGG
GGATTTCCAA GTCTCCACCC CATTGACGTC AATGGGAGTT TGTTTTGGCA CCAAAATCAA
CGGGACTTTC CAAAATGTCG TAATAACCCC GCCCCGTTGA CGCAAATGGG CGGTAGGCGT
GTACGGTGGG AGGTCTATAT AAGCAGAGCT CGTTTAGTGA ACCGTCAGAT CGCCTGGAGA
GGCCATCCAC GCTGTTTTGA CCTCCATAGT GGACACCGGG ACCGATCCAG CCTCCGCGTC
TCAGGGGAGA TCTCGTTTAG TGAACCGTCA GATCCTCACT CTCTTCCGCA TCGCTGTCTG
CGAGGGCCAG CTGTTGGGCT CGCGGTTGAG GACAAACTCT TCGCGGTCTT TCCAGTACTC
TTGGATCGGA AACCCGTCGG CCTCCGAACG GTACTCCGCC ACCGAGGGAC CTGAGCGAGT
CCGCATCGAC CGGATCGGAA AACCTCTCGA GAAAGGCGTC TAACCAGTCA CAGTCGCAAG
GTAGGCTGAG CACCGTGGCG GGCGGCAGCG GGTGGCGGTC GGGGTTGTTT CTGGCGGAGG
TGCTGCTGAT GATGTAATTA AAGTAGGCGG TCTTGAGACG GCGGATGGTC GAGGTGAGGT
GTGGCAGGCT TGAGATCCAG CTGTTGGGGT GAGTACTCCC TCTCAAAAGC GGGCATTACT
TCTGCGCTAA GATTGTCAGT TTCCAAAAAC GAGGAGGATT TGATATTCAC CTGGCCCGAT
CTGGCCATAC ACTTGAGTGA CAATGACATC CACTTTGCCT TTCTCTCCAC AGGTGTCCAC
TCCCAGGTCC AAGTTTAAAC GCCGCCACCA TG (SEQ ID NO: 33)

ACTGTTCTCA TCACATCATA TCAAGGTTAT ATACCATCAA TATTGCCACA GATGTTACTT
AGCCTTTTAA TATTTCTCTA ATTTAGTGTA TATGCAATGA TAGTTCTCTG ATTTCTGAGA
TTGAGTTTCT CATGTGTAAT GATTATTTAG AGTTTCTCTT TCATCTGTTC AAATTTTTGT
CTAGTTTTAT TTTTTACTGA TTTGTAAGAC TTCTTTTTAT AATCTGCATA TTACAATTCT
CTTTACTGGG GTGTTGCAAA TATTTTCTGT CATTCTATGG CCTGACTTTT CTTAATGGTT
TTTTAATTTT AAAAATAAGT CTTAATATTC ATGCAATCTA ATTAACAATC TTTTCTTTGT
GGTTAGGACT TTGAGTCATA AGAAATTTTT CTCTACACTG AAGTCATGAT GGCATGCTTC
TATATTATTT TCTAAAAGAT TTAAAGTTTT GCCTTCTCCA TTTAGACTTA TAATTCACTG
GAATTTTTTT GTGTGTATGG TATGACATAT GGGTTCCCTT TTATTTTTTA CATATAAATA
TATTTCCCTG TTTTTCTAAA AAAGAAAAAG ATCATCATTT TCCCATTGTA AAATGCCATA
TTTTTTTCAT AGGTCACTTA CATATATCAA TGGGTCTGTT TCTGAGCTCT ACTCTATTTT
ATCAGCCTCA CTGTCTATCC CCACACATCT CATGCTTTGC TCTAAATCTT GATATTTAGT
GGAACATTCT TTCCCATTTT GTTCTACAAG AATATTTTTG TTATTGTCTT TGGGCTTTCT
ATATACATTT TGAAATGAGG TTGACAAGTT ACCTAGGAAA ACTGTCTTCC TGCCCGGGTG
GCATCCCTGT GACCCCTCCC CAGTGCCTCT CCTGGCCCTG GAAGTTGCCA CTCCAGTGCC

CACCAGCCTT GTCCTAATAA AATTAAGTTG CATCATTTTG TCTGACTAGG TGTCCTTCTA
TAATATTATG GGGTGGAGGG GGTGGTATGG AGCAAGGGGC CCAAGTTGGG AAGAAACCTG
GTAGGGCCTG CGAAGACAGT CAG (SEQ ID NO: 34)

In some embodiments, the polynucleotide cassette comprises or consists of SEQ ID NO: 39 or a sequence with at least 85% identity thereto.

gcgcgctcgctcgctcactgaggccgcccgggcaaagcccgggcgtcgggcgacctttggtcgcccggcctca
gtgagcgagcgagcgcgcagagagggagtggccaactccatcactaggggttccttgtagttaatgattaacc
cgccatgctacttatctacgtactctggagacgacttacggtaaatggcccgcctggctgaccgcccaacgac
ccccgcccattgacgtcaataatgacgtatgttcccatagtaacgccaatagggactttccattgacgtcaat
gggtggagtatttacggtaaactgcccacttggcagtacatcaagtgtatcatatgccaagtccgccccctat
tgacgtcaatgacggtaaatggcccgcctggcattatgcccagtacatgaccttacgggactttcctacttgg
cagtacatctacgtattagtcatcgctattaccatgctgatgcggttttggcagtacaccaatgggcgtggat
agcggtttgactcacggggatttccaagtctccaccccattgacgtcaatgggagtttgttttggcaccaaaa
tcaacgggactttccaaaatgtcgtaataaccccgccccgttgacgcaaatgggcggtaggcgtgtacggtgg
gaggtctatataagcagagctcgtttagtgaaccgtcagatcgcctggagaggccatccacgctgttttgacc
tccatagtggacaccgggaccgatccagcctccgcgtctcaggggagatctcgtttagtgaaccgtcagatcc
tcactctcttccgcatcgctgtctgcgagggccagctgttgggctcgcggttgaggacaaactcttcgcggtc
tttccagtactcttggatcggaaacccgtcggcctccgaacggtactccgccaccgagggacctgagcgagtc
cgcatcgaccggatcggaaaacctctcgagaaaggcgtctaaccagtcacagtcgcaaggtaggctgagcacc
gtggcgggcggcagcgggtggcggtcggggttgtttctggcggaggtgctgctgatgatgtaattaaagtagg
cggtcttgagacggcggatggtcgaggtgaggtgtggcaggcttgagatccagctgttggggtgagtactccc
tctcaaaagcgggcattacttctgcgctaagattgtcagtttccaaaaacgaggaggatttgatattcacctg
gcccgatctggccatacacttgagtgacaatgacatccactttgcctttctctccacaggtgtccactcccag
gtccaagtttaaacgccgccaccatggtgtcatactgggatactggagtcttgctttgtgccctgctgtcctg
cctcctcctgactggctccagctcgggctcagataccggtcgccccttcgtggagatgtactccgagatcccg
gaaattatccacatgactgaggggcgcgaacttgtgatcccctgccgggtcaccagcccgaacattactgtga
ctttgaagaagttccccctggacaccctgattccggatgggaagagaattatctgggattcacggaagggatt
catcatcagcaacgcgacctacaaggaaattggcctcctcacttgcgaagccactgtgaacggacacttgtac
aagaccaactacctgacccaccgccagaccaacaccatcatcgacgtcgtcctgtccccttcgcacgggatcg
agctctcggtgggagagaagttggtgcttaactgcaccgcccggacggaactgaatgtgggaatcgacttcaa
ctgggaatacccgtccagcaagcatcagcataagaagctggtgaaccgggacctcaagactcagtccggcagc
gaaatgaagaagttcctgtcgaccctcactattgacggagtgaccagatccgaccagggcctctacacttgcg
ccgcttccagcggactcatgaccaagaagaacagcactttcgtgagggtgcatgagaaggacaagacccacac
gtgtccgccgtgcccagccccagagctgctgggaggcccttccgtgttcctgtttccgcccaagccaaaggat
accctgatgatctcaaggacccctgaggtcacatgcgtcgtggtggatgtgtcgcacgaggaccctgaagtca
aattcaattggtatgtggacggagtggaagtccacaacgcgaaaaccaagccgagagaagaacagtacaattc
cacctaccgggtggtgtcggtgctgactgtgctgcaccaggactggctcaacggaaaggagtacaagtgcaag
gtgtccaacaaggctctgcccgcacctattgaaaagaccatctccaaggccaagggtcaacctcgcgagcctc
aggtgtacactctgcctccaagccgggacgaactgactaagaaccaagtctctctgacctgtttggtgaaggg
cttctacccgtcagacatcgcagtggagtgggagtcaaacggtcagccggagaacaactacaaaacaaccccc
cccgtgctggactccgacggctccttcttcctgtactccaagcttaccgtggataagagccgctggcaacagg
gcaacgtgttttcctgctccgtcatgcacgaagccctgcacaaccattatacccagaagtccctgtcgctgtc
ccccgggaaatagtgactgttctcatcacatcatatcaaggttatataccatcaatattgccacagatgttac

ttagccttttaatatttctctaatttagtgtatatgcaatgatagttctctgatttctgagattgagtttctc
atgtgtaatgattatttagagtttctctttcatctgttcaaatttttgtctagttttattttttactgatttg
taagacttctttttataatctgcatattacaattctctttactggggtgttgcaaatattttctgtcattcta
tggcctgacttttcttaatggttttttaattttaaaaataagtcttaatattcatgcaatctaattaacaatc
ttttctttgtggttaggactttgagtcataagaaatttttctctacactgaagtcatgatggcatgcttctat
attattttctaaaagatttaaagttttgccttctccatttagacttataattcactggaatttttttgtgtgt
atggtatgacatatgggttcccttttattttttacatataaatatatttccctgtttttctaaaaaagaaaaa
gatcatcattttcccattgtaaaatgccatatttttttcataggtcacttacatatatcaatgggtctgtttc
tgagctctactctattttatcagcctcactgtctatccccacacatctcatgctttgctctaaatcttgatat
ttagtggaacattctttcccattttgttctacaagaatatttttgtta
ttgtctttgggctttctatatacattttgaaatgaggttgacaagttacctaggaaaactgtcttcctgcccg
ggtggcatccctgtgacccctccccagtgcctctcctggccctggaagttgccactccagtgcccaccagcct
tgtcctaataaaattaagttgcatcattttgtctgactaggtgtccttctataatattatggggtggagggg
ggtggtatggagcaaggggcccaagttgggaagaaacctgtagggcctgcgtacgtagataagtagcatggcgg
gttaatcattaactacaaggaacccctagtgatggagttggccactccctctctgcgcgctcgctcgctcact
gaggccgggcgaccaaaggtcgcccgacgcccgggctttgcccgggcggcctcagtgagcgagcgagcgcgc
(SEQ ID NO: 39)

SEQ ID NO: 39 shown above comprises, in the 5' to 3' direction, an inverted terminal repeat (ITR) of AAV serotype 2 comprising nucleotides 1-145 of SEQ ID NO: 39; a CMV promoter comprising nucleotides 180-693 of SEQ ID NO: 39; a 5' Untranslated Region (UTR), including an Adenovirus Tripartite Leader Sequence and Synthetic Intron, and comprising nucleotides 694-1314 of SEQ ID NO: 39; a Kozak sequence comprising nucleotides 1329-1340 of SEQ ID NO: 39; a codon-optimized aflibercept cDNA sequence comprising nucleotides 1338-2714 of SEQ ID NO: 39; a 3' UTR including a human scaffold attachment region and comprising nucleotides 2717-3527 of SEQ ID NO: 39; a human growth hormone polyadenylation/transcription stop signal comprising nucleotides 3546-3748 of SEQ ID NO: 39; and an inverted terminal repeat (ITR) of AAV serotype 2 comprising nucleotides 3772-3916 of SEQ ID NO: 39. *See, e.g.,* **FIG. 1C****.**

Additional polynucleotide cassettes for enhanced expression of a transgene (e.g., a transgene encoding an anti-VEGF agent such as aflibercept) in a target cell (such as a retinal cell) are disclosed in WO2018/170473, the contents of which related to polynucleotide cassettes for enhanced expression of a transgene in a target cell are incorporated herein by reference.

In some embodiments, the rAAV particles comprise a variant capsid protein having increased infectivity of target cells, e.g. retinal cells, are used to increase transduction of retinal cells or to increase targeting of gene delivery to retinal cells in an individual. In some embodiments, the rAAV particle comprises an amino acid modification in a capsid protein GH loop/loop IV of the AAV capsid protein. In some embodiments, the site of modification is a solvent-accessible portion of the GH loop/loop IV of the AAV capsid protein. For a description of the GH loop/loop IV of AAV capsid, see, e.g., van Vliet et al. (2006) Mol. Ther. 14:809; Padron et al. (2005) J. Virol. 79:5047; and Shen et al. (2007) Mol. Ther. 15:1955. Several AAV capsid variants are known, including the 7m8 variant. In some embodiments, a rAAV particle comprises a variant AAV capsid protein that comprises an insertion of from 5 amino acids to 11 amino acids, e.g., 7 amino acid sequence, in the GH loop of a capsid protein relative to a corresponding parental AAV capsid protein, and wherein the variant capsid protein confers increased infectivity of a retinal cell compared to the infectivity of the retinal cell by an AAV particle comprising the corresponding parental or unmodified AAV capsid protein. In some embodiments, any one of the following amino acid sequences can be inserted in the GH loop of a capsid protein: LALGETTRPA (SEQ ID NO: 1); LANETITRPA (SEQ ID NO: 2), LAKAGQANNA (SEQ ID NO: 3), LAKDPKTTNA (SEQ ID NO: 4), KDTDTTR (SEQ ID NO: 5), RAGGSVG (SEQ ID NO: 6), AVDTTKF (SEQ ID NO: 7), STGKVPN (SEQ ID NO: 8), LAKDTDTTRA (SEQ ID NO: 9), LARAGGSVGA (SEQ ID NO: 10), LAAVDTTKFA (SEQ ID NO: 11), and LASTGKVPNA (SEQ ID NO: 12), LGETTRP (SEQ ID NO: 14), NETITRP (SEQ ID NO: 15), KAGQANN (SEQ ID NO: 16), KDPKTTN (SEQ ID NO: 17), KDTDTTR (SEQ ID NO: 18), RAGGSVG (SEQ ID NO: 19), AVDTTKF (SEQ ID NO: 20), and STGKVPN (SEQ ID NO: 21). In some embodiments, any one of the amino acid sequences set forth in SEQ ID NOs: 1-12 and 14-21 is inserted in the solvent-exposed GH loop of VP1 capsid protein in a rAAV. Additional details regarding amino acid sequences that can be inserted into the GH loop of a capsid protein, e.g., to facilitate transduction of a nucleic acid of interest to a retinal cell following IVT injection, are provided in WO2012145601, US9587282, US10202657, and US10214785, the contents of which related to amino acid sequences that can be inserted into the GH loop of a capsid protein are incorporated herein by reference.

In some embodiments, the rAAV particles comprise an AAV capsid protein, e.g., an AAV2 capsid protein, that includes any one of the following amino acid sequences: LALGETTRPA (SEQ ID NO: 1); LANETITRPA (SEQ ID NO: 2), LAKAGQANNA (SEQ ID NO: 3), LAKDPKTTNA (SEQ ID NO: 4), KDTDTTR (SEQ ID NO: 5), RAGGSVG (SEQ ID NO: 6), AVDTTKF (SEQ ID NO: 7), STGKVPN (SEQ ID NO: 8), LAKDTDTTRA (SEQ ID NO: 9), LARAGGSVGA (SEQ ID NO: 10), LAAVDTTKFA (SEQ ID NO: 11), and LASTGKVPNA (SEQ ID NO: 12), LGETTRP (SEQ ID NO: 14), NETITRP (SEQ ID NO: 15), KAGQANN (SEQ ID NO: 16), KDPKTTN (SEQ ID NO: 17), KDTDTTR (SEQ ID NO: 18), RAGGSVG (SEQ ID NO: 19), AVDTTKF (SEQ ID NO: 20), and STGKVPN (SEQ ID NO: 21) inserted at the following positions: between positions 587 and 588 of the AAV2 capsid protein; between amino acids 590 and 591 of the AAV1 capsid protein; between amino acids 575 and 576 of the AAV5 capsid protein; between amino acids 590 and 591 of the AAV6 capsid protein; between amino acids 589 and 590 of the AAV7 capsid protein; between amino acids 590 and 591 of the AAV8 capsid protein; between amino acids 588 and 589 of the AAV9 capsid protein; or between amino acids 589 and 590 of the AAV10 capsid protein. In some embodiments, the rAAV particles comprise AAV2 capsid proteins comprising an amino acid sequence LALGETTRPA (SEQ ID NO: 1) inserted between positions 587 and 588 of the capsid protein, wherein the amino acid residue numbering corresponds to an AAV2 VP1 capsid protein. In some embodiments, the rAAV particles comprise AAV2 capsid proteins comprising the amino acid sequence LALGETTRPA (SEQ ID NO: 1) inserted between positions 587 and 588 of the AAV2 VP1 comprising the sequence of SEQ ID NO: 13.

In some embodiments, rAAV particles comprise the 7m8 variant capsid protein from AAV2 comprising the amino acid sequence LALGETTRPA (SEQ ID NO: 1) inserted in the GH loop of the AAV2 VP1 protein between positions 587 and 588 of the AAV2 VP1. In some embodiments, the rAAV particles comprise an AAV2 VP1 capsid protein comprising a GH loop that comprises the amino acid sequence of SEQ ID NO: 38 or an amino acid sequence having at least 90% sequence identity to SEQ ID NO: 38. In some embodiments, the rAAV particles comprise an AAV2 VP1 capsid protein comprising a GH loop that comprises an amino acid sequence having any of at least 90%, at least 91%, at least 92%, at least 93%, at least 94%, at least 95%, at least 96%, at least 97%, at least 98%, at least 99%, or 100% sequence identity to SEQ ID NO: 38.

FSYTFEDVPFHSSYAHSQSLDRLMNPLIDQYLYYLSRTNTPSGTTTQSRLQFSQAGASDI
RDQSRNWLPGPCYRQQRVSKTSADNNNSEYSWTGATKYHLNGRDSLVNPGPAMASHKDDE
EKFFPQSGVLIFGKQGSEKTNVDIEKVMITDEEEIRTTNPVATEQYGSVSTNLQRGNLAL
GETTRPARQAATADVNTQGVLPGMVWQDRDVYLQGPIWAKIPHTDGHFHPSPLMGGFGLK
HPPPQILIKN (SEQ ID NO: 38)

In some embodiments, rAAV particles comprise the 7m8 variant capsid protein from AAV2 comprising the amino acid sequence LALGETTRPA (SEQ ID NO: 1) inserted between positions 587 and 588 of the AAV2 VP1. The sequence of the 7m8 variant capsid protein from AAV2 comprising the amino acid sequence LALGETTRPA (SEQ ID NO: 1) is inserted between positions 587 and 588 of the AAV2 VP1 is provided below:

MAADGYLPDWLEDTLSEGIRQWWKLKPGPPPPKPAERHKDDSRGLVLPGYKYLGPFNGLDKGEPV
NEADAAALEHDKAYDRQLDSGDNPYLKYNHADAEFQERLKEDTSFGGNLGRAVFQAKKRVLEPLG
LVEEPVKTAPGKKRPVEHSPVEPDSSSGTGKAGQQPARKRLNFGQTGDADSVPDPQPLGQPPAAPSG
LGTNTMATGSGAPMADNNEGADGVGNSSGNWHCDSTWMGDRVITTSTRTWALPTYNNHLYKQIS
SQSGASNDNHYFGYSTPWGYFDFNRFHCHFSPRDWQRLINNNWGFRPKRLNFKLFNIQVKEVTQND
GTTTIANNLTSTVQVFTDSEYQLPYVLGSAHQGCLPPFPADVFMVPQYGYLTLNNGSQAVGRSSFYC
LEYFPSQMLRTGNNFTFSYTFEDVPFHSSYAHSQSLDRLMNPLIDQYLYYLSRTNTPSGTTTQSRLQF
SQAGASDIRDQSRNWLPGPCYRQQRVSKTSADNNNSEYSWTGATKYHLNGRDSLVNPGPAMASHK
DDEEKFFPQSGVLIFGKQGSEKTNVDIEKVMITDEEEIRTTNPVATEQYGSVSTNLQRGNLALGETTR
PARQAATADVNTQGVLPGMVWQDRDVYLQGPIWAKIPHTDGHFHPSPLMGGFGLKHPPPQILIKNT
PVPANPSTTFSAAKFASFITQYSTGQVSVEIEWELQKENSKRWNPEIQYTSNYNKSVNVDFTVDTNG
VYSEPRPIGTRYLTRNL (SEQ ID NO: 37)

In some embodiments, the rAAV particles comprise a capsid protein VP1 comprising the amino acid sequence LGETTRP (SEQ ID NO: 14) inserted between positions 587 and 588 of the capsid protein, wherein the amino acid residue numbering corresponds to an AAV2 VP1 capsid protein. In some embodiments, the rAAV particles comprise a capsid protein VP2 comprising the amino acid sequence LGETTRP (SEQ ID NO: 14) inserted between positions 587 and 588 of the capsid protein, wherein the amino acid residue numbering corresponds to an AAV2 VP1 capsid protein. In some embodiments, the rAAV particles comprise a capsid protein VP3 comprising the amino acid sequence LGETTRP (SEQ ID NO: 14) inserted between positions 587 and 588 of the capsid protein, wherein the amino acid residue numbering corresponds to an AAV2 VP1 capsid protein. In some embodiments, the rAAV particles comprise capsid proteins VP1, VP2, and VP3, wherein each of VP1, VP2, and VP3 comprise the amino acid sequence LGETTRP (SEQ ID NO: 14) inserted between positions 587 and 588 of the capsid protein, wherein the amino acid residue numbering corresponds to an AAV2 VP1 capsid protein.

In some embodiments, the rAAV particles comprise a capsid protein VP1 comprising the amino acid sequence LALGETTRPA (SEQ ID NO: 1) inserted between positions 587 and 588 of the capsid protein, wherein the amino acid residue numbering corresponds to an AAV2 VP1 capsid protein. In some embodiments, the rAAV particles comprise a capsid protein VP2 comprising the amino acid sequence LALGETTRPA (SEQ ID NO: 1) inserted between positions 587 and 588 of the capsid protein, wherein the amino acid residue numbering corresponds to an AAV2 VP1 capsid protein. In some embodiments, the rAAV particles comprise a capsid protein VP3 comprising the amino acid sequence LALGETTRPA (SEQ ID NO: 1) inserted between positions 587 and 588 of the capsid protein, wherein the amino acid residue numbering corresponds to an AAV2 VP1 capsid protein. In some embodiments, the rAAV particles comprise capsid proteins VP1, VP2, and VP3, wherein each of VP1, VP2, and VP3 comprise the amino acid sequence LALGETTRPA (SEQ ID NO: 1) inserted between positions 587 and 588 of the capsid protein, wherein the amino acid residue numbering corresponds to an AAV2 VP1 capsid protein.

In some embodiments, a recombinant virus and/or plasmid used to generate a rAAV virus comprises other transcriptional or regulatory elements, such as a poly A (polyadenylation) sequence, untranslated regions (UTRs), 3' UTRs, or termination sequences. In some embodiments, more than one gene is expressed from the vector or plasmid using internal ribosome entry site (IRES) or similar element that allows co-expression of two or more proteins or create multigene, or polycistronic mRNA.

In some embodiments, the rAAV and/or plasmid used to generate the rAAV comprises one or more of the following nucleic acid elements: a first ITR sequence; a promoter sequence; an intron sequence; a first UTR sequence; a heterologous nucleic acid encoding an anti-VEGF agent (e.g., aflibercept); a second UTR sequence; a polyA sequence; and a second ITR sequence. In some embodiments, linker sequence(s) are inserted between two or more of the nucleic acid elements. In some embodiments, the heterologous nucleic acid encodes a therapeutic polypeptide, e.g., encodes aflibercept (or a functional fragment or functional variant thereof).

In some embodiments, the vector is a targeted vector, especially a targeted rAAV (e.g., AAV2.7m8) that shows higher infectivity of a specific cell, such as a retinal cell (e.g., a photoreceptor, a retinal ganglion cell, a Müller cell, a bipolar cell, an amacrine cell, a horizontal cell, or a retinal pigmented epithelium cell). Viral vectors for use in the disclosure can include those that exhibit low toxicity and/or low immunogenicity in an individual and expresses therapeutically effective quantities of the anti-VEGF agent (e.g., aflibercept) in an individual, e.g., a human. Any suitable method known in the art can be used in the biochemical purification of recombinant viruses (e.g., rAAV), e.g., for the preparation of pharmaceutical compositions described elsewhere herein. Recombinant AAV viruses can be harvested directly from cells, or from the culture media comprising cells. Virus can be purified using various biochemical means, such as gel filtration, filtration, chromatography, affinity purification, gradient ultracentrifugation, or size exclusion methods. In some embodiments, the virus is lyophilized.

In some embodiments, the rAAV particles comprise a 7m8 variant capsid protein, e.g., rAAV2.7m8, and a nucleic acid sequence that encodes an anti-VEGF agent (e.g., aflibercept, or a functional fragment or functional variant thereof). In some embodiments, the rAAV particles (e.g., the 7m8 variant) have an increase in retinal cell infectivity of any of at least 5%, at least 10%, at least 20%, at least 30%, at least 40%, at least 50%, at least 60%, at least 70%, at least 80%, at least 90%, or at least 100% as compared to an AAV particle comprising the corresponding parental or unmodified AAV capsid protein. In some embodiments, the increase in infectivity of retinal cells is an increase of any of between 5% to 100%, between 5% to 95%, between 5% to 90%, between 5% to 85%, between 5% to 80%, between 5% to 75%, between 5% to 70%, between 5% to 65%, between 5% to 60%, between 5% to 55%, between 5% to 50%, between 5% to 45%, between 5% to 40%, between 5% to 35%, between 5% to 30%, between 5% to 25%, between 5% to 20%, between 5% to 15%, or between 5% to 10%, as compared to an AAV particle comprising the corresponding parental or unmodified AAV capsid protein.

In some embodiments, the increase in retinal cell infectivity of a rAAV variant, e.g., rAAV2.7m8, is any of at least 1-fold, at least 1.1-fold, at least 1.2-fold, at least 1.3-fold, at least 1.4-fold, at least 1.5-fold, at least 1.6-fold, at least 1.7-fold, at least 1.8-fold, at least 1.9-fold, or at least 2-fold compared to an AAV particle comprising the corresponding parental or unmodified AAV capsid protein. In some embodiments, the increase in infectivity is any of at least 2-fold, at least 3-fold, at least 4-fold, at least 5-fold, at least 6-fold, at least 7-fold, at least 8-fold, at least 9-fold, or at least 10-fold, as compared to an AAV particle comprising the corresponding parental AAV capsid protein. In some embodiments, the increase in infectivity is any of at least 15-fold, at least 20-fold, at least 25-fold, at least 30-fold, at least 35-fold, at least 40-fold, at least 45-fold, at least 50-fold, at least 55-fold, at least 60-fold, at least 65-fold, at least 70-fold, at least 75-fold, at least 80-fold, at least 85-fold, at least 90-fold, or at least 100-fold compared to an AAV particle comprising the corresponding parental or unmodified AAV capsid protein.

In some embodiments, the increase in retinal cell infectivity of a rAAV variant, e.g., rAAV2.7m8, is between 10-fold to 100-fold, between 10-fold to 95-fold, between 10-fold to 90-fold, between 10-fold to 85-fold, between 10-fold to 80-fold, between 10-fold to 75-fold, between 10-fold to 70-fold, between 10-fold to 65-fold, between 10-fold to 60-fold, between 10-fold to 55-fold, between 10-fold to 50-fold, between 10-fold to 45-fold, between 10-fold to 40-fold, between 10-fold to 35-fold, between 10-fold to 30-fold, between 10-fold to 25-fold, between 10-fold to 20-fold, or between 10-fold to 15-fold, as compared to an AAV particle comprising the corresponding parental or unmodified AAV capsid protein.

In some embodiments, the increase in retinal cell infectivity is between 2-fold to 20-fold, between 2-fold to 19-fold, between 2-fold to 18-fold, between 2-fold to 17-fold, between 2-fold to 16-fold, between 2-fold to 15-fold, between 2-fold to 14-fold, between 2-fold to 13-fold, between 2-fold to 12-fold, between 2-fold to 11-fold, between 2-fold to 10-fold, between 2-fold to 9-fold, between 2-fold to 8-fold, between 2-fold to 7-fold, between 2-fold to 6-fold, between 2-fold to 5-fold, between 2-fold to 4-fold, or between 2-fold to 3-fold, as compared to an AAV particle comprising the corresponding parental or unmodified AAV capsid protein.

In some embodiments, an amino acid modification of a capsid protein described herein can confer an increase in an ability to cross an internal limiting membrane (ILM) in an eye of an individual, e.g., a human, as compared to the ability of an AAV particle comprising the corresponding parental or unmodified AAV capsid protein to cross the ILM in the eye of the subject. In some embodiments, the increase in the ability to cross the ILM of a rAAV variant, e.g., rAAV2.7m8, is an increase of any of at least 5%, at least 10%, at least 20%, at least 30%, at least 40%, at least 50%, at least 60%, at least 70%, at least 80%, at least 90%, or at least 100% as compared to an AAV particle comprising the corresponding parental or unmodified AAV capsid protein. In some embodiments, the increase in the ability to cross the ILM is an increase of between 5% to 100%, between 5% to 95%, between 5% to 90%, between 5% to 85%, between 5% to 80%, between 5% to 75%, between 5% to 70%, between 5% to 65%, between 5% to 60%, between 5% to 55%, between 5% to 50%, between 5% to 45%, between 5% to 40%, between 5% to 35%, between 5% to 30%, between 5% to 25%, between 5% to 20%, between 5% to 15%, or between 5% to 10%, as compared to the parental or unmodified AAV capsid protein.

In some embodiments, the increase in the ability to cross the ILM of a rAAV variant, e.g., rAAV2.7m8, is any of at least 1-fold, at least 1.1-fold, at least 1.2-fold, at least 1.3-fold, at least 1.4-fold, at least 1.5-fold, at least 1.6-fold, at least 1.7-fold, at least 1.8-fold, at least 1.9-fold, or at least 2-fold compared to an AAV particle comprising the corresponding parental AAV capsid protein. In some embodiments, the increase in the ability to cross the ILM is any of at least 2-fold, at least 3-fold, at least 4-fold, at least 5-fold, at least 6-fold, at least 7-fold, at least 8-fold, at least 9-fold, or at least 10-fold, as compared to an AAV particle comprising the corresponding parental AAV capsid protein. In some embodiments, the increase in the ability to cross the ILM is any of at least 15-fold, at least 20-fold, at least 25-fold, at least 30-fold, at least 35-fold, at least 40-fold, at least 45-fold, at least 50-fold, at least 55-fold, at least 60-fold, at least 65-fold, at least 70-fold, at least 75-fold, at least 80-fold, at least 85-fold, at least 90-fold, or at least 100-fold compared to an AAV particle comprising the corresponding parental or unmodified AAV capsid protein.

In some embodiments, the increase in the ability to cross the ILM of a rAAV variant, e.g., rAAV2.7m8, is between 10-fold to 100-fold, between 10-fold to 95-fold, between 10-fold to 90-fold, between 10-fold to 85-fold, between 10-fold to 80-fold, between 10-fold to 75-fold, between 10-fold to 70-fold, between 10-fold to 65-fold, between 10-fold to 60-fold, between 10-fold to 55-fold, between 10-fold to 50-fold, between 10-fold to 45-fold, between 10-fold to 40-fold, between 10-fold to 35-fold, between 10-fold to 30-fold, between 10-fold to 25-fold, between 10-fold to 20-fold, or between 10-fold to 15-fold as compared to an AAV particle comprising the corresponding parental or unmodified AAV capsid protein.

In some embodiments, the increase in the ability to cross the ILM of a rAAV variant, e.g., rAAV2.7m8, is between 2-fold to 20-fold, between 2-fold to 19-fold, between 2-fold to 18-fold, between 2-fold to 17-fold, between 2-fold to 16-fold, between 2-fold to 15-fold, between 2-fold to 14-fold, between 2-fold to 13-fold, between 2-fold to 12-fold, between 2-fold to 11-fold, between 2-fold to 10-fold, between 2-fold to 9-fold, between 2-fold to 8-fold, between 2-fold to 7-fold, between 2-fold to 6-fold, between 2-fold to 5-fold, between 2-fold to 4-fold, or between 2-fold to 3-fold, as compared to an AAV particle comprising the corresponding parental or unmodified AAV capsid protein.

In some embodiments, rAAV.7m8 comprising nucleic acid encoding aflibercept is used for gene therapy. In some embodiments, AAV2 or rAAV2 is used to deliver a nucleic acid sequence encoding an anti-VEGF agent (e.g., aflibercept) into an eye or retinal cells of a subject via intravitreal or subretinal injection. In some embodiments, AAV2 or rAAV2 is used to deliver a nucleic acid sequence encoding an anti-VEGF agent (e.g., aflibercept) into an eye or retinal cells of a subject via intravitreal injection. In some embodiments, rAAV2.7m8 is used to deliver the nucleic acid sequence of the anti-VEGF agent (e.g., aflibercept) into the retinal cells of a subject. In some embodiments, the heterologous nucleic acid (e.g., a nucleic acid that encodes an anti-VEGF agent such as aflibercept) integrates into the target cell genome (e.g., retinal cell genome), resulting in long-term expression of, e.g., the anti-VEGF agent (such as aflibercept), in the target cell. In some embodiments, the viral vector delivers a plasmid or other extrachromosomal genetic element that comprises the heterologous nucleic acid (e.g., a nucleic acid that encodes an anti-VEGF agent such as aflibercept) to the target cell (e.g., retinal cell).

In some embodiments, the rAAV particles comprise a nucleic acid encoding a polypeptide comprising an amino acid sequence with any of at least about 95%, at least about 96%, at least about 97%, at least about 98%, at least about 99%, or at least about 100% identity to the amino acid sequence of SEQ ID NO: 35 and flanked by AAV2 inverted terminal repeats (ITRs). In some embodiments, the rAAV particles comprise a nucleic acid encoding a polypeptide comprising an amino acid sequence with at least about 95% identity to the amino acid sequence of SEQ ID NO: 35 and flanked by AAV2 inverted terminal repeats (ITRs). In some embodiments, the rAAV particles comprise a nucleic acid encoding a polypeptide comprising the amino acid sequence of SEQ ID NO: 35 and flanked by AAV2 inverted terminal repeats (ITRs). In some embodiments, the rAAV particles comprise a nucleic acid encoding a polypeptide comprising the amino acid sequence of SEQ ID NO: 35. In some embodiments, the rAAV particles comprise a nucleic acid encoding aflibercept and flanked by AAV2 inverted terminal repeats (ITRs). The sequence of SEQ ID NO: 35 is provided below:

SDTGRPFVEMYSEIPEIIHMTEGRELVIPCRVTSPNITVTLKKFPLDTLIPDGKRIIWDSRKGFIISNATYKE
IGLLTCEATVNGHLYKTNYLTHRQTNTIIDVVLSPSHGIELSVGEKLVLNCTARTELNVGIDFNWEYPSSKHQ
HKKLVNRDLKTQSGSEMKKFLSTLTIDGVTRSDQGLYTCAASSGLMTKKNSTFVRVHEKDKTHTCPPCPAPEL
LGG[?]SVFLFPPKPKDTLMISRTPEVTCVVVDVSHEDPEVKFNWYVDGVEVHNAKTKPREEQYNSTYRVVSVLT
VLHQDWLNGKEYKCKVSNKALPAPIEKTISKAKGQPREPQVYTLPPSRDELTKNQVSLTCLVKGFYPSDIAVE
WESNGQPENNYKTTPPVLDSDGSFFLYSKLTVDKSRWQQGNVFSCSVMHEALHNHYTQKSLSLSPG (SEQ
ID NO: 35)

In some embodiments, the rAAV particles comprise a nucleic acid with any of at least about 75%, at least about 80%, at least about 81%, at least about 82%, at least about 83%, at least about 84%, at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, at least about 99%, at least about 99.9%, or at least about 100% sequence homology to the nucleic acid sequence of SEQ ID NO: 36, and wherein the nucleic acid is flanked by AAV2 inverted terminal repeats (ITRs). The sequence of SEQ ID NO: 36 is provided in **FIG. 5****.** In some embodiments, the rAAV particles comprise a nucleic acid with any of at least about 75%, at least about 80%, at least about 81%, at least about 82%, at least about 83%, at least about 84%, at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, at least about 99%, at least about 99.9%, or at least about 100% sequence homology to the nucleic acid sequence of aflibercept (e.g., SEQ ID NO: 36), and wherein the nucleic acid is flanked by AAV2 inverted terminal repeats (ITRs). In some embodiments, the nucleic acid sequence of aflibercept is derived from its amino acid sequence. In some embodiments, the nucleic acid sequence of aflibercept is codon optimized to improve its expression in a subject. In some embodiments, the rAAV particles comprise a nucleic acid with any of at least about 75%, at least about 80%, at least about 81%, at least about 82%, at least about 83%, at least about 84%, at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, at least about 99%, at least about 99.9%, or 100% sequence homology to the nucleic acid sequence of SEQ ID NO: 40, and wherein the nucleic acid is flanked by AAV2 inverted terminal repeats (ITRs). In some embodiments, the rAAV particles comprise a nucleic acid comprising the nucleic acid sequence of SEQ ID NO: 40. In some embodiments, the rAAV particles comprise a nucleic acid comprising the nucleic acid sequence of SEQ ID NO: 40, and wherein the nucleic acid is flanked by AAV2 inverted terminal repeats (ITRs).

atggtgtcatactgggatactggagtcttgctttgtgccctgctgtcctgcctcctcctgactggctccagct
cgggctcagataccggtcgccccttcgtggagatgtactccgagatcccggaaattatccacatgactgaggg
gcgcgaacttgtgatcccctgccgggtcaccagcccgaacattactgtgactttgaagaagttccccctggac
accctgattccggatgggaagagaattatctgggattcacggaagggattcatcatcagcaacgcgacctaca
aggaaattggcctcctcacttgcgaagccactgtgaacggacacttgtacaagaccaactacctgacccaccg
ccagaccaacaccatcatcgacgtcgtcctgtccccttcgcacgggatcgagctctcggtgggagagaagttg
gtgcttaactgcaccgcccggacggaactgaatgtgggaatcgacttcaactgggaatacccgtccagcaagc
atcagcataagaagctggtgaaccgggacctcaagactcagtccggcagcgaaatgaagaagttcctgtcgac
cctcactattgacggagtgaccagatccgaccagggcctctacacttgcgccgcttccagcggactcatgacc
aagaagaacagcactttcgtgagggtgcatgagaaggacaagacccacacgtgtccgccgtgcccagccccag
agctgctgggaggcccttccgtgttcctgtttccgcccaagccaaaggat
accctgatgatctcaaggacccctgaggtcacatgcgtcgtggtggatgtgtcgcacgaggaccctgaagtca
aattcaattggtatgtggacggagtggaagtccacaacgcgaaaaccaagccgagagaagaacagtacaattc
cacctaccgggtggtgtcggtgctgactgtgctgcaccaggactggctcaacggaaaggagtacaagtgcaag
gtgtccaacaaggctctgcccgcacctattgaaaagaccatctccaaggccaagggtcaacctcgcgagcctc
aggtgtacactctgcctccaagccgggacgaactgactaagaaccaagtctctctgacctgtttggtgaaggg
cttctacccgtcagacatcgcagtggagtgggagtcaaacggtcagccggagaacaactacaaaacaaccccc
cccgtgctggactccgacggctccttcttcctgtactccaagcttaccgtggataagagccgctggcaacagg
gcaacgtgttttcctgctccgtcatgcacgaagccctgcacaaccattatacccagaagtccctgtcgctgtc
ccccgggaaatag (SEQ ID NO: 40)

In some embodiments, the rAAV particles comprise a nucleic acid encoding a polypeptide comprising an amino acid sequence with any of at least about 95%, at least about 96%, at least about 97%, at least about 98%, at least about 99%, or 100% identity to the amino acid sequence of SEQ ID NO: 41 and flanked by AAV2 inverted terminal repeats (ITRs). In some embodiments, the rAAV particles comprise a nucleic acid encoding a polypeptide comprising an amino acid sequence with at least about 95% identity to the amino acid sequence of SEQ ID NO: 41 and flanked by AAV2 inverted terminal repeats (ITRs). In some embodiments, the rAAV particles comprise a nucleic acid encoding a polypeptide comprising the amino acid sequence of SEQ ID NO: 41 and flanked by AAV2 inverted terminal repeats (ITRs). In some embodiments, the rAAV particles comprise a nucleic acid encoding a polypeptide comprising the amino acid sequence of SEQ ID NO: 41.

MVSYWDTGVLLCALLSCLLLTGSSSGSDTGRPFVEMYSEIPEIIHMTEGRELVIPCRVTSPNITVTLKKFPLD
TLIPDGKRIIWDSRKGFIISNATYKEIGLLTCEATVNGHLYKTNYLTHRQTNTIIDVVLSPSHGIELSVGEKL
VLNCTARTELNVGIDFNWEYPSSKHQHKKLVNRDLKTQSGSEMKKFLSTLTIDGVTRSDQGLYTCAASSGLMT
KKNSTFVRVHEKDKTHTCPPCPAPELLGGPSVFLFPPKPKDTLMISRTPEVTCVVVDVSHEDPEVKFNWYVDG
VEVHNAKTKPREEQYNSTYRVVSVLTVLHQDWLNGKEYKCKVSNKALPAPIEKTISKAKGQPREPQVYTLPPS
RDELTKNQVSLTCLVKGFYPSDIAVEWESNGQPENNYKTTPPVLDSDGSFFLYSKLTVDKSRWQQGNVFSCSV
MHEALHNHYTQKSLSLSPGK (SEQ ID NO: 41)

In some embodiments, the nucleic acid sequence of aflibercept is codon-optimized for expression in a primate or a human subject. Construction of a synthetic gene corresponding to the aflibercept amino acid sequence has been described in literature, e.g., Kanda A, Noda K, Saito W, Ishida S. Aflibercept Traps Galectin-1, an Angiogenic Factor Associated with Diabetic Retinopathy. Scientific Reports 5:17946 (2015) (describing "VEGF-Trap_{R1R2} (corresponding to aflibercept) cDNA was generated as a synthetic gene by IDT (Coralville, IA)"). Given the available amino acid sequence of aflibercept, any method known in the art can be used to generate the cDNA of aflibercept for use in a gene therapy or a rAAV described herein.

Codon optimization can be achieved with any method known in the art. Codon optimization refers to a process of modifying a nucleic acid sequence for enhanced expression of a gene in target or host cells of interest, e.g., human retinal cells, by replacing at least one codon (e.g., about or more than 1, 2, 3, 4, 5, 10, 15, 20, 25, 50, 100 or more codons) of a native sequence with codons that are used more frequently or are most frequently used in the host cell while maintaining the native amino acid sequence. Codon usage tables are readily available, including for examples, GenScript Codon Usage Frequency Table Tool at www(dot)genscript(dot)com/tools/codon-frequency-table; Codon Usage Database at www(dot)kazusa(dot)or(dot)jp/codon/; and Nakamura, Y., et al. "Codon usage tabulated from the international DNA sequence databases: status for the year 2000" Nucl. Acids Res. 28:292 (2000).

Homology refers to the percent conservation of residues of an alignment between two sequences, including, but not limited to functional fragments, sequences comprising insertions, deletions, substitutions, pseudofragments, pseudogenes, splice variants or artificially optimized sequences.

In some embodiments, the rAAV particles comprise a nucleic acid encoding aflibercept. In some embodiments, the polypeptide is aflibercept.

As used herein, "aflibercept" refers to a polypeptide or protein sequence, or a functional fragment or variant or mutant thereof, with any of at least 75%, 80%, 81%, 82%, 83%, 84%, 85%, 86%, 87%, 88%, 89%, 90%, 91%, 92%, 93%, 94%, 95%, 96%, 97%, 98%, 99% or more, or 100% homology to the aflibercept amino acid sequence identified above (SEQ ID NO: 35). Homology refers to the percent conservation of residues of an alignment between two sequences, including, but not limited to functional fragments, sequences comprising insertions, deletions, substitutions, pseudofragments, pseudogenes, splice variants or artificially optimized sequences.

In some embodiments, the amino acid sequence of aflibercept is any of at least 75%, 80%, 81%, 82%, 83%, 84%, 85%, 86%, 87%, 88%, 89%, 90%, 91%, 92%, 93%, 94%, 95%, 96%, 97%, 98%, 99%, 99.9%, or 100% homologous to the aflibercept amino acid sequence of SEQ ID NO: 35. In some embodiments, the nucleic acid sequence encoding aflibercept disclosed herein is compared to the corresponding cDNA sequence of the aflibercept amino acid sequence identified above, and shows any of at least 75%, 80%, 81%, 82%, 83%, 84%, 85%, 86%, 87%, 88%, 89%, 90%, 91%, 92%, 93%, 94%, 95%, 96%, 97%, 98%, 99%, 99.9%, or 100% sequence homology between the nucleic acid sequences of aflibercept (e.g., SEQ ID NO: 36). In some embodiments, aflibercept is any of at least 80%, 85%, 90%, 91%, 92%, 93%, 94%, 95%, 96%, 97%, 98%, 99%, 99.9%, or 100% spatially homologous to aflibercept (e.g., in terms of its secondary, tertiary, and quaternary structure or conformation). In some embodiments, aflibercept is any of at most 80%, 85%, 90%, 91%, 92%, 93%, 94%, 95%, 96%, 97%, 98%, 99%, 99.9%, or 100% spatially homologous to the aflibercept used in the standard of care (e.g., secondary, tertiary, and quaternary structure or conformation).

In some embodiments, the aflibercept gene product, or aflibercept transgene, as included in a gene therapy based on a rAAV, comprises a capsid variant as disclosed herein (e.g., the 7m8 variant), encodes a protein, fusion protein, or polypeptide that has any of at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or at least 100% homology to the above amino acid sequence of SEQ ID NO: 35, or between the corresponding cDNA sequences of aflibercept (e.g., cDNA of aflibercept sequence used in a gene therapy compared to SEQ ID NO: 36). In some embodiments, the methods compositions disclosed herein comprise a functional fragment of aflibercept, or a variant or mutant thereof. In some embodiments, the nucleic acid sequence of aflibercept is modified or codon-optimized to enhance its activity, expression, stability, and/or solubility in vivo.

Aflibercept is a 115 kDa fusion protein, which can be glycosylated. Aflibercept comprises an IgG backbone fused to extracellular VEGF receptor sequences of the human VEGFR-1 and VEGFR-2, and functions like a soluble decoy receptor by binding VEGF-A with a greater affinity than its natural or endogenous receptors. See, for example, Stewart MW. Aflibercept (VEGF Trap-eye): the newest anti-VEGF drug. Br. J. Ophthalmol. 2012 Sep;96(9):1157-8. Aflibercept's high affinity for VEGF interferes or disrupts subsequent binding and activation of native or endogenous VEGF receptors. Reduced VEGF activity can lead to decreased angiogenesis and vascular permeability. Inhibition of placental growth factor PIGF and VEGF-B by aflibercept may also contribute to the treatment of ocular diseases or disorders characterized by abnormal (e.g., excessive) angiogenesis and/or neovascularization. PIGF has been associated with angiogenesis and certain ocular diseases or disorders, such as wet AMD, may be associated with elevated levels of PIGF. VEGF-B overexpression can be associated with breakdown of the blood-retinal barrier and retinal angiogenesis. Thus, inhibition of VEGF-A, VEGF-B, and PIGF may all contribute to the efficacy of aflibercept.

### Methods for Preparation of Vectors for Delivering Transgenes to Target Cells

In some embodiments, the rAAV particles are manufactured using any method known in the art. In some embodiments, the rAAV particles are manufactured using a baculovirus expression vector system in Sf9 cells. Sf9 cells are an insect cell culture cell line commonly used for recombinant protein production using baculovirus. In some embodiments, the rAAV particles are manufactured using two baculoviruses in Sf9 cells. In some embodiments, the rAAV particles are manufactured using two baculoviruses in Sf9 cells, wherein a first baculovirus encodes the genes for AAV2 Rep and AAV2.7m8 Cap proteins and a second baculovirus encodes an anti-VEGF agent. In some embodiments, the rAAV particles are manufactured using two baculoviruses in Sf9 cells, wherein a first baculovirus encodes the genes for AAV2 Rep and AAV2.7m8 Cap proteins and a second baculovirus encodes an aflibercept (e.g., human aflibercept) cDNA expression cassette. In some embodiments, the rAAV particles are manufactured using two baculoviruses in Sf9 cells, wherein a first baculovirus encodes the genes for AAV2 Rep and AAV2.7m8 Cap proteins and a second baculovirus comprises a nucleic acid encoding a polypeptide comprising an amino acid sequence with at least about 95% identity to the amino acid sequence of SEQ ID NO: 35 and flanked by AAV2 inverted terminal repeats (ITRs). In some embodiments, the polypeptide comprises the amino acid sequence of SEQ ID NO: 35. In some embodiments, the polypeptide is aflibercept.

### Doses

In some embodiments, the unit dose of rAAV particles is administered to one eye of the individual. In some embodiments, the one eye of the individual is the right eye or the left eye. In some embodiments, the one eye of the individual is the right eye. In some embodiments, the one eye of the individual is the left eye. In some embodiments, the methods provided herein further comprise administering a unit dose of rAAV particles to the contralateral eye of the individual. In some embodiments, the one eye of the individual is the right eye and the contralateral eye is the left eye. In some embodiments, the one eye of the individual is the left eye and the contralateral eye is the right eye.

In some embodiments, the administering the unit dose of rAAV particles to the contralateral eye is at least about 2 weeks (e.g., at least about 2 weeks, at least about 3 weeks, at least about 4 weeks, at least about 1 month, at least about 2 months, at least about 3 months, at least about 4 months, at least about 5 months, at least about 6 months, at least about 7 months, at least about 8 months, at least about 9 months, at least about 10 months, at least about 11 months, at least about 12 months, at least about 1 year, at least about 2 years, at least about 3 years, at least about 4 years, at least about 5 years, or more) after administering the unit dose of rAAV particles to the one eye. In some embodiments, the administering the unit dose of rAAV particles to the contralateral eye is at least about 2 weeks after administering the unit dose of rAAV particles to the one eye and the unit dose of rAAV particles administered to the contralateral eye of the individual is higher (e.g., any of about 5%, about 10%, about 20%, about 30%, about 40%, about 50%, about 60%, about 70%, about 80%, about 90%, about 100%, about 125%, about 150%, about 175%, about 200%, about 225%, about 250%, about 275%, about 300%, or more, higher) than the unit dose of rAAV particles administered to the one eye of the individual.

In some embodiments, the administering the unit dose of rAAV particles to the contralateral eye of the individual is up to about 1 week, up to about 2 weeks, up to about 3 weeks, or up to about 4 weeks after administering the unit dose of rAAV particles to the one eye. In some embodiments, the administering the unit dose of rAAV particles to the contralateral eye of the individual is up to about 2 weeks (e.g., about 0 days, 1 day, 2 days, 3 days, 4 days, 5 days, 6 days, 7 days, 8 days, 9 days, 10 days, 11 days, 12 days, 13 days, or 14 days) after administering the unit dose of rAAV particles to the one eye. In some embodiments, the administering the unit dose of rAAV particles to the contralateral eye of the individual is up to about 2 weeks (e.g., about 0 days, 1 day, 2 days, 3 days, 4 days, 5 days, 6 days, 7 days, 8 days, 9 days, 10 days, 11 days, 12 days, 13 days, or 14 days) after administering the unit dose of rAAV particles to the one eye and the unit dose of rAAV particles administered to the contralateral eye of the individual is about the same as (e.g., less than 1% higher or lower, less than 5% higher or lower, less than 10% higher or lower, or less than 20% higher or lower), or lower (e.g., about 5%, about 10%, about 20%, about 30%, about 40%, about 50%, about 60%, about 70%, about 80%, or about 90% lower) than the unit dose of rAAV particles administered to the one eye of the individual. In some embodiments, the administering the unit dose of rAAV particles to the contralateral eye of the individual is up to about 2 weeks after administering the unit dose of rAAV particles to the one eye and the unit dose of rAAV particles administered to the contralateral eye of the individual is about the same (e.g., less than 1% higher or lower, less than 5% higher or lower, less than 10% higher or lower, or less than 20% higher or lower) as the unit dose of rAAV particles administered to the one eye of the individual. In some embodiments, the administering the unit dose of rAAV particles to the contralateral eye of the individual is up to about 2 weeks after administering the unit dose of rAAV particles to the one eye and the unit dose of rAAV particles administered to the contralateral eye of the individual is lower (e.g., about 5%, about 10%, about 20%, about 30%, about 40%, about 50%, about 60%, about 70%, about 80%, or about 90% lower) than the unit dose of rAAV particles administered to the one eye of the individual.

In some embodiments, the unit dose of rAAV particles is administered to one eye and/or to the contralateral eye of the individual. In some embodiments, the unit dose of rAAV particles is expressed as the number of vector genomes (vg). In some embodiments, the unit dose is about 6 × 10¹¹ vector genomes (vg) or less of the rAAV particles. In some embodiments, the unit dose is about 1×10¹⁰ to about 2×10¹⁰, between about 2×10¹⁰ to about 3×10¹⁰, between about 3×10¹⁰ to about 4×10¹⁰, between about 4×10¹⁰ to about 5×10¹⁰, between about 5×10¹⁰ to about 6×10¹⁰, between about 6×10¹⁰ to about 7×10¹⁰, between about 7×10¹⁰ to about 8×10¹⁰, between about 8×10¹⁰ to about 9×10¹⁰, between about 9×10¹⁰ to about 10×10¹⁰, between about 1×10¹¹ to about 2×10¹¹, between about 2×10¹¹ to about 3×10¹¹, between about 3x¹⁰11 to about 4×10¹¹, between about 4×10¹¹ to about 5×10¹¹, or between about 5×10¹¹ to about 6×10¹¹ vg of the rAAV particles, including any value within these ranges, of the rAAV particles. In some embodiments, the unit dose is about 6×10¹⁰ vector genomes (vg) to about 2×10¹¹ vg of the rAAV particles. In some embodiments, the unit dose is about 6×10¹⁰ vg to about 2×10¹¹ vg, about 7×10¹⁰ vg to about 2×10¹¹ vg, about 8×10¹⁰ vg to about 2×10¹¹ vg, about 9×10¹⁰ vg to about 2×10¹¹ vg, about 10×10¹⁰ vg to about 2×10¹¹ vg, or about 1×10¹¹ vg to about 2×10¹¹ vg of the rAAV particles. In some embodiments, the unit dose is about 6×10¹⁰ vg to about 2×10¹¹ vg of the rAAV particles. In some embodiments, the unit dose is about 6×10¹⁰ vg to about 7×10¹⁰ vg, about 7×10¹⁰ vg to about 8×10¹⁰ vg, about 8×10¹⁰ vg to about 9×10¹⁰ vg, about 9×10¹⁰ vg to about 10×10¹⁰ vg, about 10×10¹⁰ vg to about 1×10¹¹ vg, or about 1×10¹¹ vg to about 2×10¹¹ vg of the rAAV particles. In some embodiments, the unit dose is about 6×10¹⁰ vg, about 7×10¹⁰ vg, about 8×10¹⁰ vg, about 9×10¹⁰ vg, about 10×10¹⁰ vg, about 1×10¹¹ vg, or about 2×10¹¹ vg of the rAAV particles. In some embodiments, the unit dose is about 6×10¹⁰ vg or about 2×10¹¹ vg of the rAAV particles. In some embodiments, the unit dose is about 6×10¹⁰ vg of the rAAV particles. In some embodiments, the unit dose is about 6 × 10¹⁰ vg, about 2 × 10¹¹ vg, or about 6× 10¹¹ vg. In some embodiments, the unit dose is about 6 × 10¹⁰ vg. In some embodiments, the unit dose is about 2 × 10¹¹ vg. In some embodiments, the unit dose is about 6× 10¹¹ vg.

In some embodiments, the unit dose of rAAV particles is administered to one eye and/or to the contralateral eye of the individual. In some embodiments, the unit dose is expressed as the number of vector genomes (vg) per eye (vg/eye). In some embodiments, the unit dose is about 6 × 10¹¹ vg/eye or less of the rAAV particles. In some embodiments, the unit dose is about 1×10¹⁰ to about 2×10¹⁰, between about 2×10¹⁰ to about 3×10¹⁰, between about 3×10¹⁰ to about 4×10¹⁰, between about 4×10¹⁰ to about 5×10¹⁰,between about 5×10¹⁰ to about 6×10¹⁰, between about 6×10¹⁰ to about 7×10¹⁰, between about 7×10¹⁰ to about 8×10¹⁰, between about 8×10¹⁰ to about 9×10¹⁰, between about 9×10¹⁰ to about 10×10¹⁰, between about 1×10¹¹ to about 2×10¹¹, between about 2×10¹¹ to about 3×10¹¹, between about 3×10¹¹ to about 4×10¹¹, between about 4×10¹¹ to about 5×10¹¹, or between about 5×10¹¹ to about 6×10¹¹ vg/eye of the rAAV particles, including any value within these ranges, of the rAAV particles. In some embodiments, the unit dose is about 6×10¹⁰ vg/eye to about 2×10¹¹ vg/eye of the rAAV particles. In some embodiments, the unit dose is about 6×10¹⁰ vg/eye to about 2×10¹¹ vg/eye, about 7×10¹⁰ vg/eye to about 2×10¹¹ vg/eye, about 8×10¹⁰ vg/eye to about 2×10¹¹ vg/eye, about 9×10¹⁰ vg/eye to about 2×10¹¹ vg/eye, about 10×10¹⁰ vg/eye to about 2×10¹¹ vg/eye, or about 1×10¹¹ vg/eye to about 2×10¹¹ vg/eye of the rAAV particles. In some embodiments, the unit dose is about 6×10¹⁰ vg/eye to about 2×10¹¹ vg/eye of the rAAV particles. In some embodiments, the unit dose is about 6×10¹⁰ vg/eye to about 7×10¹⁰ vg/eye, about 7×10¹⁰ vg/eye to about 8×10¹⁰ vg/eye, about 8×10¹⁰ vg/eye to about 9×10¹⁰ vg/eye, about 9×10¹⁰ vg/eye to about 10×10¹⁰ vg/eye, about 10×10¹⁰ vg/eye to about 1×10¹¹ vg/eye, or about 1×10¹¹ vg/eye to about 2×10¹¹ vg/eye of the rAAV particles. In some embodiments, the unit dose is about 6×10¹⁰ vg/eye, about 7×10¹⁰ vg/eye, about 8×10¹⁰ vg/eye, about 9×10¹⁰ vg/eye, about 10×10¹⁰ vg/eye, about 1×10¹¹ vg/eye, or about 2×10¹¹ vg/eye of the rAAV particles. In some embodiments, the unit dose is about 6×10¹⁰ vg/eye or about 2×10¹¹ vg/eye of the rAAV particles. In some embodiments, the unit dose is about 6×10¹⁰ vg/eye of the rAAV particles. In some embodiments, the unit dose is about 6 × 10¹⁰ vg/eye, about 2 × 10¹¹ vg/eye, or about 6× 10¹¹ vg/eye. In some embodiments, the unit dose is about 6 × 10¹⁰ vg/eye. In some embodiments, the unit dose is about 2 × 10¹¹ vg/eye. In some embodiments, the unit dose is about 6× 10¹¹ vg/eye.

In some embodiments, the unit dose of rAAV particles is administered to one eye and/or to the contralateral eye of the individual. In some embodiments, E is a shorthand for base 10 for exponentiation, and xEy refers to x multiplied by base 10 to the y power/exponent. In some embodiments, the unit dose is expressed as the number of vector genomes (vg). In some embodiments, the unit dose is about 6E¹¹ vector genomes (vg) or less of the rAAV particles. In some embodiments, the unit dose is about 1E¹⁰ to about 2E¹⁰, between about 2E¹⁰ to about 3E¹⁰, between about 3E¹⁰ to about 4E¹⁰, between about 4E¹⁰ to about 5E¹⁰, between about 5E¹⁰ to about 6E¹⁰, between about 6E¹⁰ to about 7E¹⁰, between about 7E¹⁰ to about 8E¹⁰, between about 8E¹⁰ to about 9E¹⁰, between about 9E¹⁰ to about 10E¹⁰, between about 1E¹¹ to about 2E¹¹, between about 2E¹¹ to about 3E¹¹, between about 3E¹¹ to about 4E¹¹, between about 4E¹¹ to about 5E¹¹, or between about 5E¹¹ to about 6E¹¹ vg of the rAAV particles, including any value within these ranges, of the rAAV particles. In some embodiments, the unit dose is about 6E¹⁰ vector genomes (vg) to about 2E¹¹ vg of the rAAV particles. In some embodiments, the unit dose is about 6E¹⁰ vg to about 2E¹¹ vg, about 7E¹⁰ vg to about 2E¹¹ vg, about 8E¹⁰ vg to about 2E¹¹ vg, about 9E¹⁰ vg to about 2E¹¹ vg, about 10E¹⁰ vg to about 2E¹¹ vg, or about 1E¹¹ vg to about 2E¹¹ vg of the rAAV particles. In some embodiments, the unit dose is about 6E¹⁰ vg to about 2E¹¹ vg of the rAAV particles. In some embodiments, the unit dose is about 6E¹⁰ vg to about 7E¹⁰ vg, about 7E¹⁰ vg to about 8E¹⁰ vg, about 8E¹⁰ vg to about 9E¹⁰ vg, about 9E¹⁰ vg to about 10E¹⁰ vg, about 10E¹⁰ vg to about 1E¹¹ vg, or about 1E¹¹ vg to about 2E¹¹ vg of the rAAV particles. In some embodiments, the unit dose is about 6E¹⁰ vg, about 7E¹⁰ vg, about 8E¹⁰ vg, about 9E¹⁰ vg, about 10E¹⁰ vg, about 1E¹¹ vg, or about 2E¹¹ vg of the rAAV particles. In some embodiments, the unit dose is about 6E¹⁰ vg or about 2E¹¹ vg of the rAAV particles. In some embodiments, the unit dose is about 6E¹⁰ vg of the rAAV particles. In some embodiments, the unit dose is about 6E¹⁰ vg, about 2E¹¹ vg, or about 6E¹¹ vg. In some embodiments, the unit dose is about 6E¹⁰ vg. In some embodiments, the unit dose is about 2E¹¹ vg. In some embodiments, the unit dose is about 6E¹¹ vg.

In some embodiments, the unit dose of rAAV particles is administered to one eye and/or to the contralateral eye of the individual. In some embodiments, the unit dose is expressed as the number of vector genomes (vg) per eye (vg/eye). In some embodiments, the unit dose is about 6E¹¹ vg/eye or less of the rAAV particles. In some embodiments, the unit dose is about 1E¹⁰ to about 2E¹⁰, between about 2E¹⁰ to about 3E¹⁰, between about 3E¹⁰ to about 4E¹⁰, between about 4E¹⁰ to about 5E¹⁰, between about 5E¹⁰ to about 6E¹⁰, between about 6E¹⁰ to about 7E¹⁰, between about 7E¹⁰ to about 8E¹⁰, between about 8E¹⁰ to about 9E¹⁰, between about 9E¹⁰ to about 10E¹⁰, between about 1E¹¹ to about 2E¹¹, between about 2E¹¹ to about 3E¹¹, between about 3E¹¹ to about 4E¹¹, between about 4E¹¹ to about 5E¹¹, or between about 5E¹¹ to about 6E¹¹ vg/eye of the rAAV particles, including any value within these ranges, of the rAAV particles. In some embodiments, the unit dose is about 6E¹⁰ vg/eye to about 2E¹¹ vg/eye of the rAAV particles. In some embodiments, the unit dose is about 6E¹⁰ vg/eye to about 2E¹¹ vg/eye, about 7E¹⁰ vg/eye to about 2E¹¹ vg/eye, about 8E¹⁰ vg/eye to about 2E¹¹ vg/eye, about 9E¹⁰ vg/eye to about 2E¹¹ vg/eye, about 10E¹⁰ vg/eye to about 2E¹¹ vg/eye, or about 1E¹¹ vg/eye to about 2E¹¹ vg/eye of the rAAV particles. In some embodiments, the unit dose is about 6E¹⁰ vg/eye to about 2E¹¹ vg/eye of the rAAV particles. In some embodiments, the unit dose is about 6E¹⁰ vg/eye to about 7E¹⁰ vg/eye, about 7E¹⁰ vg/eye to about 8E¹⁰ vg/eye, about 8E¹⁰ vg/eye to about 9E¹⁰ vg/eye, about 9E¹⁰ vg/eye to about 10E¹⁰ vg/eye, about 10E¹⁰ vg/eye to about 1E¹¹ vg/eye, or about 1E¹¹ vg/eye to about 2E¹¹ vg/eye of the rAAV particles. In some embodiments, the unit dose is about 6E¹⁰ vg/eye, about 7E¹⁰ vg/eye, about 8E¹⁰ vg/eye, about 9E¹⁰ vg/eye, about 10E¹⁰ vg/eye, about 1E¹¹ vg/eye, or about 2E¹¹ vg/eye of the rAAV particles. In some embodiments, the unit dose is about 6E¹⁰ vg/eye or about 2E¹¹ vg/eye of the rAAV particles. In some embodiments, the unit dose is about 6E¹⁰ vg/eye of the rAAV particles. In some embodiments, the unit dose is about 6E¹⁰ vg/eye, about 2E¹¹ vg/eye, or about 6E¹¹ vg/eye. In some embodiments, the unit dose is about 6E¹⁰ vg/eye. In some embodiments, the unit dose is about 2E¹¹ vg/eye. In some embodiments, the unit dose is about 6E¹¹ vg/eye.

In some embodiments, the unit dose of rAAV particles is administered to one eye and/or to the contralateral eye of the individual. In some embodiments, the unit dose of rAAV particles is a unit dose sufficient to cause expression of the therapeutic protein (e.g., an anti-VEGF agent such as aflibercept) in the vitreous fluid. In some embodiments, the unit dose of rAAV particles is a unit dose sufficient to achieve a concentration of the therapeutic protein (e.g., an anti-VEGF agent such as aflibercept) at about any one of 3, 3.5, 4, 4.5, 5, 5.5, 6, 6.5,7, 7.5, 8, 8.5, 9, 9.5, 10 µg/ml, or more, including any range in between these values, in the vitreous fluid. In some embodiments, the unit dose of rAAV particles is a unit dose sufficient to cause expression of aflibercept in the vitreous fluid. In some embodiments, the unit dose of rAAV particles is a unit dose sufficient to achieve a concentration of aflibercept at about any one of 3, 3.5, 4, 4.5, 5, 5.5, 6, 6.5,7, 7.5, 8, 8.5, 9, 9.5, 10 µg/ml, or more, including any range in between these values, in the vitreous fluid.

In some embodiments, the unit dose of rAAV particles administered to the one eye and/or to the contralateral eye of the individual is a unit dose sufficient to cause expression of the therapeutic protein (e.g., an anti-VEGF agent such as aflibercept) in the aqueous fluid. In some embodiments, the unit dose of rAAV particles is a unit dose sufficient to achieve a concentration of the therapeutic protein (e.g., an anti-VEGF agent such as aflibercept) of at least about 0.5, 0.55, 0.6, 0.65, 0.7, 0.75, 0.8, 0.85, 0.9, 0.95, 1.0 µg/ml, or more, including any range in between these values, in the aqueous fluid. In some embodiments, the unit dose of rAAV particles is a unit dose sufficient to cause expression of aflibercept in the aqueous fluid. In some embodiments, the unit dose of rAAV particles is a unit dose sufficient to achieve a concentration of aflibercept of at least about 0.5, 0.55, 0.6, 0.65, 0.7, 0.75, 0.8, 0.85, 0.9, 0.95, 1.0 µg/ml, or more, including any range in between these values, in the aqueous fluid.

In some embodiments, the unit dose of rAAV particles is administered to one eye and/or to the contralateral eye of the individual. In some embodiments, the unit dose of rAAV particles is a unit dose sufficient to cause expression of the therapeutic protein (e.g., an anti-VEGF agent such as aflibercept) in the retina. In some embodiments, the unit dose of rAAV particles is a unit dose sufficient to achieve a concentration of the therapeutic protein (e.g., an anti-VEGF agent such as aflibercept) of at least about 3, 3.5, 4, 4.5, 5, 5.5, 6, 6.5,7, 7.5, 8, 8.5, 9, 9.5, 10 µg/g, or more, including any range in between these values, in the retina. In some embodiments, the unit dose of rAAV particles is a unit dose sufficient to cause expression of aflibercept in the retina. In some embodiments, the unit dose of rAAV particles is a unit dose sufficient to achieve a concentration of aflibercept of at least about 3, 3.5, 4, 4.5, 5, 5.5, 6, 6.5,7, 7.5, 8, 8.5, 9, 9.5, 10 µg/g, or more, including any range in between these values, in the retina.

In some embodiments, the unit dose of rAAV particles is administered to one eye and/or to the contralateral eye of the individual. In some embodiments, the unit dose of rAAV particles is a unit dose sufficient to cause expression of the therapeutic protein (e.g., an anti-VEGF agent such as aflibercept) in the choroid. In some embodiments, the unit dose of rAAV particles is a unit dose sufficient to achieve a concentration of the therapeutic protein (e.g., an anti-VEGF agent such as aflibercept) at about any one of 3, 3.5, 4, 4.5, 5, 5.5, 6, 6.5,7, 7.5, 8, 8.5, 9, 9.5, 10 µg/g, or more, including any range in between these values, in the choroid. In some embodiments, the unit dose of rAAV particles is a unit dose sufficient to cause expression of aflibercept in the choroid. In some embodiments, the unit dose of rAAV particles is a unit dose sufficient to achieve a concentration of aflibercept at about any one of 3, 3.5, 4, 4.5, 5, 5.5, 6, 6.5,7, 7.5, 8, 8.5, 9, 9.5, 10 µg/g, or more, including any range in between these values, in the choroid.

In some embodiments, the unit dose of rAAV particles is administered to one eye and/or to the contralateral eye of the individual. In some embodiments, the unit dose of rAAV particles is a therapeutically effective dose.

In some embodiments, the unit dose of rAAV particles is a therapeutically effective dose if the unit dose is sufficient to cause maintenance or a decrease of retinal thickness compared to the retinal thickness prior to administration of the unit dose of rAAV particles. In some embodiments, the unit dose of rAAV particles is a therapeutically effective dose if the unit dose is sufficient to cause a decrease of retinal thickness compared to the retinal thickness prior to administration of the unit dose of rAAV particles. In some embodiments, retinal thickness is central subfield thickness (CST) or central retinal thickness (CRT). In some embodiments, the unit dose of rAAV particles is a therapeutically effective dose if the unit dose is sufficient to cause a decrease of retinal thickness of more than any of about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 99%, or about 100% compared to the retinal thickness prior to administration of the unit dose of rAAV particles. In some embodiments, the retinal thickness (e.g., CST or CRT) is determined by OCT or SD-OCT.

In some embodiments, the unit dose of rAAV particles is a therapeutically effective dose if the unit dose is sufficient to cause maintenance or a decrease in macular volume compared to the macular volume prior to administration of the unit dose of rAAV particles. In some embodiments, the unit dose of rAAV particles is a therapeutically effective dose if the unit dose is sufficient to cause a decrease in macular volume compared to the macular volume prior to administration of the unit dose of rAAV particles. In some embodiments, the unit dose of rAAV particles is a therapeutically effective dose if the unit dose is sufficient to cause a decrease in macular volume of more than any of about 5%, about 10%, about 15%, about 20%, about 25%, about 30%, about 35%, about 40%, about 45%, about 50%, about 55%, about 60%, or about 65% compared to the macular volume prior to administration of the unit dose of rAAV particles. In some embodiments, the macular volume is determined by OCT or SD-OCT.

In some embodiments, the unit dose of rAAV particles is a therapeutically effective dose if the unit dose is sufficient to cause maintenance or an improvement of visual acuity compared to the visual acuity prior to administration of the unit dose of rAAV particles. In some embodiments, the unit dose of rAAV particles is a therapeutically effective dose if the unit dose is sufficient to cause an improvement of visual acuity compared to the visual acuity prior to administration of the unit dose of rAAV particles. In some embodiments, the unit dose of rAAV particles is a therapeutically effective dose if the unit dose is sufficient to cause an improvement of visual acuity of more than any of about 5%, about 10%, about 20%, about 30%, about 40%, about 50%, about 60%, about 70%, about 80%, about 90%, about 100%, about 125%, about 150%, about 175%, about 200%, about 225%, about 250%, about 275%, about 300%, or more compared to the visual acuity prior to administration of the unit dose of rAAV particles. In some embodiments, visual acuity is best corrected visual acuity (BCVA). In some embodiments, the unit dose of rAAV particles is a therapeutically effective dose if the unit dose is sufficient to cause an improvement of BCVA compared to the BCVA prior to administration of the unit dose of rAAV particles. In some embodiments, BCVA is expressed as an ETDRS score, which corresponds to the number of letters correctly read (Vitale et al., (2016) JAMA Opthalmol 134(9):1041:1047). In some embodiments, the unit dose of rAAV particles is a therapeutically effective dose if the unit dose is sufficient to cause an improvement of BCVA of at least 15 ETDRS letters (Vitale et al., (2016) JAMA Opthalmol 134(9):1041:1047) (e.g., at least about 15, at least about 20, at least about 30, at least about 40, at least about 50, at least about 60, or about 70 letters) compared to the BCVA prior to administration of the unit dose of rAAV particles. In some embodiments, the unit dose of rAAV particles is a therapeutically effective dose if the unit dose is sufficient to cause maintenance of BCVA, wherein the individual loses fewer than 15 ETDRS letters (Vitale et al., (2016) JAMA Opthalmol 134(9):1041:1047) (e.g., 15 or less, 14 or less, 13 or less, 12 or less, 11 or less, 10 or less, 9 or less, 8 or less, 7 or less, 6 or less, 5 or less, 4 or less, 3 or less, 2 or less, 1, or 0 letters) compared to the BCVA prior to administration of the unit dose of rAAV particles.

In some embodiments, the unit dose of rAAV particles is a therapeutically effective dose if, after administration of the unit dose of rAAV particles, the individual is determined to have maintenance of vision. In some embodiments, the unit dose of rAAV particles is a therapeutically effective dose if, after administration of the unit dose of rAAV particles, the individual is determined have an improvement of vision. In some embodiments, the unit dose of rAAV particles is a therapeutically effective dose if, after administration of the unit dose of rAAV particles, the CST or CRT assessed by SD-OCT is decreased compared to prior to administration of the unit dose of rAAV particles. In some embodiments, the unit dose of rAAV particles is a therapeutically effective dose if, after administration of the unit dose of rAAV particles, the CST or CRT assessed by SD-OCT is maintained compared to prior to administration of the unit dose of rAAV particles.

In some embodiments, the unit dose of rAAV particles is a therapeutically effective dose if, after administration of the unit dose of rAAV particles, the macular volume is decreased compared to prior to administration of the unit dose of rAAV particles. In some embodiments, the unit dose of rAAV particles is a therapeutically effective dose if, after administration of the unit dose of rAAV particles, the macular volume is maintained compared to prior to administration of the unit dose of rAAV particles.

In some embodiments, the unit dose of rAAV particles is a therapeutically effective dose if, after administration of the unit dose of rAAV particles, the retinal thickness (e.g., central retinal thickness (CRT) or central subfield thickness (CST)) and macular volume are decreased compared to prior to administration of the unit dose of rAAV particles. In some embodiments, the unit dose of rAAV particles is a therapeutically effective dose if, after administration of the unit dose of rAAV particles, the retinal thickness (e.g., central retinal thickness (CRT) or central subfield thickness (CST)) and macular volume are maintained compared to prior to administration of the unit dose of rAAV particles.

In some embodiments, the unit dose of rAAV particles is a therapeutically effective dose if, after administration of the unit dose of rAAV particles, the individual requires less than one rescue therapy treatment (e.g., aflibercept injection) about any of every 4 weeks, every 5 weeks, every 6 weeks, every 7 weeks, every 8 weeks, every 9 weeks, every 10 weeks, or more after administration of the unit dose of rAAV particles in the one eye and/or the contralateral eye. In some embodiments, the unit dose of rAAV particles is a therapeutically effective dose if, after administration of the unit dose of rAAV particles, the individual does not require any rescue therapy treatment (e.g., aflibercept injection) for at least about any of 1 week, at least 2 weeks, at least 3 weeks, at least 4 weeks, at least 5 weeks, at least 6 weeks, at least 7 weeks, at least 8 weeks, at least 9 weeks, at least 10 weeks, at least 15 weeks, at least 20 weeks, at least 30 weeks, at least 40 weeks, at least 50 weeks, at least 60 weeks, at least 70 weeks, at least 80 weeks, at least 90 weeks, at least 100 weeks, at least 110 weeks, or more.

In some embodiments, the unit dose of rAAV particles is a therapeutically effective dose if, after administration of the unit dose of rAAV particles, the individual is determined to have a reduction in retinal fluid compared to the level of retinal fluid prior to administration of the unit dose of rAAV particles in the one eye and/or the contralateral eye. In some embodiments, the unit dose of rAAV particles is a therapeutically effective dose if, after administration of the unit dose of rAAV particles, the individual is determined to have maintenance in retinal fluid compared to the level of retinal fluid prior to administration of the unit dose of rAAV particles in the one eye and/or the contralateral eye. In some embodiments, the unit dose of rAAV particles is a therapeutically effective dose if, after administration of the unit dose of rAAV particles, the individual is determined to have a reduction in IRF and/or SRF in the one eye and/or the contralateral eye compared to the levels of IRF and/or SRF prior to administration of the unit dose of rAAV particles in the one eye and/or the contralateral eye.

In some embodiments, the unit dose of rAAV particles is a therapeutically effective dose if, after administration of the unit dose of rAAV particles, the individual is determined to have a resolution of pigment epithelial detachment (PED) compared to PED prior to administration of the unit dose of rAAV particles in the one eye and/or the contralateral eye.

In some embodiments, the unit dose of rAAV particles is a therapeutically effective dose if, after administration of the unit dose of rAAV particles, CNV lesions shrink compared to CNV lesions present prior to administration of the unit dose of rAAV particles in the one eye and/or the contralateral eye. In some embodiments, the unit dose of rAAV particles is a therapeutically effective dose if, after administration of the unit dose of rAAV particles, CNV lesions shrink by more than any of about 5%, 10%, 20%, 30%, 40%, 50%, 60%, 70%, 80%, 90%, or 100% compared to CNV lesions present prior to administration of the unit dose of rAAV particles in the one eye and/or the contralateral eye. In some embodiments, the unit dose of rAAV particles is a therapeutically effective dose if, after administration of the unit dose of rAAV particles, CNV lesions do not grow compared to CNV lesions present prior to administration of the unit dose of rAAV particles in the one eye and/or the contralateral eye. In some embodiments, the unit dose of rAAV particles is a therapeutically effective dose if, after administration of the unit dose of rAAV particles, CNV lesions do not grow by more than about any of about 1%, 2%, 3%, 4%, 5%, 6%, 7%, 8%, 9%, 10%, 15%, or 20% compared to CNV lesions present prior to administration of the unit dose of rAAV particles in the one eye and/or the contralateral eye.

In some embodiments, the unit dose of rAAV particles is a therapeutically effective dose if, after administration of the unit dose of rAAV particles, the individual is determined to have an improvement in anatomical features of the one eye and/or the contralateral eye compared to the anatomical features prior to administration of the unit dose of rAAV particles. In some embodiments, the unit dose of rAAV particles is a therapeutically effective dose if, after administration of the unit dose of rAAV particles, the individual is determined to have a stabilization and/or maintenance of anatomical features of the one eye and/or the contralateral eye compared to the anatomical features prior to administration of the unit dose of rAAV particles.

In some embodiments, the unit dose of rAAV particles is therapeutically effective if administration of the dose to the one eye and/or the contralateral eye of the individual reduces, stops, or prevents at least one symptom of the ocular neovascular disease or disorder. In the cases of ocular neovascular diseases or disorders characterized by abnormal (e.g., excessive) angiogenesis, such symptoms include, but are not limited to, e.g., visual distortions (such as impaired color vision, blurred vision, deterioration of central vision) and vision loss. In some embodiments, the unit dose of rAAV particles administered to the one eye and/or to the contralateral eye of the individual is a therapeutically effective dose if administration of the unit dose to the one eye and/or to the contralateral eye of the individual results in the maintenance, partial resolution, or complete resolution of one or more clinical features of the ocular neovascular disease. For example, the unit dose of rAAV particles administered to the one eye and/or to the contralateral eye of the individual is therapeutically effective if administration of the dose to the one eye and/or to the contralateral eye of the individual results in complete resolution, partial resolution or maintenance of the ocular neovascular disease as measured by any method known in the art. In some embodiments, the unit dose of rAAV particles administered to the one eye and/or to the contralateral eye of the individual is therapeutically effective if administration of the dose to the one eye and/or to the contralateral eye of the individual results in complete resolution, partial resolution or maintenance of the ocular neovascular disease as assessed by best corrected visual acuity (BCVA) (e.g., based on an ETDRS score; Vitale et al., (2016) JAMA Opthalmol 134(9):1041:1047), central retinal thickness as determined by SD-OCT, the number of rescue therapy treatments (e.g., aflibercept injections) required by the individual after administration of the unit dose of rAAV particles in the one eye and/or the contralateral eye, the presence of intraretinal fluid (IRF) and/or subretinal fluid (SRF), the resolution of pigment epithelial detachment (PED), choroidal neovascularization (CNV) lesion growth, anatomical features based on any methods known in the art (e.g., SD-OCT, OCT, fluorescein angiography, digital color fundus photography, etc.). In some embodiments, the unit dose of rAAV particles administered to the one eye and/or to the contralateral eye of the individual is therapeutically effective if administration of the dose to the one eye and/or to the contralateral eye of the individual results in complete resolution, partial resolution or maintenance of the ocular neovascular disease as assessed by ophthalmologic examination, intraocular pressure (e.g., using a Goldmann applanation tonometer or Tono-pen), indirect ophthalmoscopy, examination of the one eye and/or the contralateral eye and adnexa, eyelid and/or pupil responsiveness, belpharoptosis, abnormal pupil shape, unequal pupils, abnormal reaction to light, afferent pupillary defects, slit-lamp examination (including of the eyelids, conjunctiva, cornea, lens, iris, and anterior chamber), posterior segment abnormalities of the vitreous, optic nerve, peripheral retina, and retinal vasculature, SD-OCT, fluorescein angiography, digital color fundus photography (including images of the retina, optic disc, and/or macula), aqueous humor sampling, vitreous humor sampling, OCT-angiography (OCT-A), refraction, and visual acuity (BCVA).

In some embodiments, the unit dose of rAAV particles administered to the one eye of the individual is the same as the unit dose of rAAV particles administered to the contralateral eye of the individual. In some embodiments, the unit dose of rAAV particles administered to the one eye of the individual is different from the unit dose of rAAV particles administered to the contralateral eye of the individual. In some embodiments, the unit dose of rAAV particles administered to the one eye of the individual is higher, e.g., more than any of about 10%, about 20%, about 30%, about 40%, about 50%, about 60%, about 70%, about 80%, about 90%, about 100%, about 125%, about 150%, about 175%, about 200%, about 225%, about 250%, about 275%, about 300% or more, than the unit dose of rAAV particles administered to the contralateral eye of the individual. In some embodiments, the unit dose of rAAV particles administered to the contralateral eye of the individual is higher, e.g., more than any of about 10%, about 20%, about 30%, about 40%, about 50%, about 60%, about 70%, about 80%, about 90%, about 100%, about 125%, about 150%, about 175%, about 200%, about 225%, about 250%, about 275%, about 300% or more, than the unit dose of rAAV particles administered to the one eye of the individual. In some embodiments, the unit dose of rAAV particles is expressed as the number of vector genomes (vg) per eye (vg/eye). In some embodiments, the unit dose of rAAV particles is about 6 × 10¹¹ vg/eye or less of the rAAV particles. In some embodiments, the unit dose of rAAV particles is about 1×10¹⁰ to about 2×10¹⁰, between about 2×10¹⁰ to about 3×10¹⁰, between about 3×10¹⁰ to about 4×10¹⁰, between about 4×10¹⁰ to about 5×10¹⁰,between about 5×10¹⁰ to about 6×10¹⁰, between about 6×10¹⁰ to about 7×10¹⁰, between about 7×10¹⁰ to about 8×10¹⁰, between about 8×10¹⁰ to about 9×10¹⁰, between about 9×10¹⁰ to about 10×10¹⁰, between about 1×10¹¹ to about 2×10¹¹, between about 2×10¹¹ to about 3×10¹¹, between about 3×10¹¹ to about 4×10¹¹, between about 4×10¹¹ to about 5×10¹¹, or between about 5×10¹¹ to about 6×10¹¹ vg/eye of the rAAV particles, including any value within these ranges, of the rAAV particles. In some embodiments, the unit dose of rAAV particles is about 6×10¹⁰ vg/eye to about 2×10¹¹ vg/eye of the rAAV particles. In some embodiments, the unit dose of rAAV particles is about 6×10¹⁰ vg/eye to about 2×10¹¹ vg/eye, about 7×10¹⁰ vg/eye to about 2×10¹¹ vg/eye, about 8×10¹⁰ vg/eye to about 2×10¹¹ vg/eye, about 9×10¹⁰ vg/eye to about 2×10¹¹ vg/eye, about 10×10¹⁰ vg/eye to about 2×10¹¹ vg/eye, or about 1×10¹¹ vg/eye to about 2×10¹¹ vg/eye of the rAAV particles. In some embodiments, the unit dose of rAAV particles is about 6×10¹⁰ vg/eye to about 2×10¹¹ vg/eye of the rAAV particles. In some embodiments, the unit dose of rAAV particles is about 6×10¹⁰ vg/eye to about 7×10¹⁰ vg/eye, about 7×10¹⁰ vg/eye to about 8×10¹⁰ vg/eye, about 8×10¹⁰ vg/eye to about 9×10¹⁰ vg/eye, about 9×10¹⁰ vg/eye to about 10×10¹⁰ vg/eye, about 10×10¹⁰ vg/eye to about 1×10¹¹ vg/eye, or about 1×10¹¹ vg/eye to about 2×10¹¹ vg/eye of the rAAV particles. In some embodiments, the unit dose of rAAV particles is about 6×10¹⁰ vg/eye, about 7×10¹⁰ vg/eye, about 8×10¹⁰ vg/eye, about 9×10¹⁰ vg/eye, about 10×10¹⁰ vg/eye, about 1×10¹¹ vg/eye, or about 2×10¹¹ vg/eye of the rAAV particles. In some embodiments, the unit dose of rAAV particles is about 6×10¹⁰ vg/eye or about 2×10¹¹ vg/eye of the rAAV particles. In some embodiments, the unit dose of rAAV particles is about 6×10¹⁰ vg/eye of the rAAV particles. In some embodiments, the unit dose of rAAV particles is about 6 × 10¹⁰ vg/eye, about 2 × 10¹¹ vg/eye, or about 6× 10¹¹ vg/eye. In some embodiments, the unit dose of rAAV particles is about 6 × 10¹⁰ vg/eye. In some embodiments, the unit dose of rAAV particles is about 2 × 10¹¹ vg/eye. In some embodiments, the unit dose of rAAV particles is about 6× 10¹¹ vg/eye.

In some embodiments, the unit dose of rAAV particles administered to the one eye of the individual and the unit dose of rAAV particles administered to the contralateral eye of the individual are administered at the same time. In some embodiments, the unit dose of rAAV particles administered to the one eye of the individual and the unit dose of rAAV particles administered to the contralateral eye of the individual are administered at different times. In some embodiments, the unit dose administered to the contralateral eye is administered any of at least about 1 hour, at least about 2 hours, at least about 4 hours, at least about 8 hours, at least about 12 hours, at least about 24 hours, at least about 1 day, at least about 2 days, at least about 3 days, at least about 4 days, at least about 5 days, at least about 6 days, at least about 7 days, at least about 1 week, at least about 2 weeks, at least about 3 weeks, at least about 4 weeks, or more after administering of the unit dose to the one eye. In some embodiments, the unit dose administered to the contralateral eye is administered at least about 2 weeks after administering of the unit dose to the one eye.

In some embodiments, a single unit dose of rAAV particles is administered to the one eye and/or the contralateral eye of the individual. In some embodiments, the single unit dose of rAAV particles administered to the one eye and/or to the contralateral eye is a therapeutically effective dose. In some embodiments, more than one dose of rAAV particles (e.g., more than any of about 2, 3, 4, 5, or more unit doses) are administered to the one eye and/or the contralateral eye of the individual. In some embodiments, the more than one doses of rAAV particles administered to the one eye and/or to the contralateral are therapeutically effective doses.

In some embodiments, an anti-VEGF treatment, e.g., an IVT injection with an anti-VEGF agent such as aflibercept, is administered to the one eye and/or to the contralateral eye administered the rAAV particles at least about one week, e.g. at least about 7 days, prior to administration of the unit dose of rAAV particles (e.g., a unit dose of between about 2 × 10¹¹ vg/eye to about 6 × 10¹¹ vg/eye of rAAV particles). In some embodiments, an anti-VEGF treatment, e.g., an IVT injection with an anti-VEGF agent such as aflibercept, is administered to an eye on about Day 1 and the unit dose of rAAV particles, e.g., a unit dose of between about 2 × 10¹¹ vg/eye to about 6 × 10¹¹ vg/eye of rAAV particles, is administered to the eye on about Day 8. In some embodiments, the unit dose of rAAV particles is about 2 × 10¹¹ vg/eye or about 6 × 10¹¹ vg/eye of rAAV particles. In some embodiments, the ocular neovascular disease is diabetic macular edema.

### Pharmaceutical Formulations

In some embodiments, the unit dose of rAAV particles is in a pharmaceutical formulation. In some embodiments, the pharmaceutical formulation comprises the rAAV particles, one or more osmotic or ionic strength agents, one or more buffering agents, one or more surfactants, and one or more solvents. In some embodiments, the osmotic or ionic strength agent is sodium chloride. In some embodiments, the one or more buffering agents are sodium phosphate monobasic and/or sodium phosphate dibasic. In some embodiments, the surfactant is Poloxamer 188. In some embodiments, the solvent is water. In some embodiments, the pharmaceutical formulation comprises the rAAV particles, sodium chloride, sodium phosphate monobasic, sodium phosphate dibasic, and a surfactant.

In some embodiments, the pharmaceutical formulation comprises about 1×10¹⁰ vg/mL to about 1×10¹³ vg/mL of rAAV particles, about 150 mM to about 200 mM sodium chloride, about 1 mM to about 10 mM monobasic sodium phosphate, about 1 mM to about 10 mM dibasic sodium phosphate, and about 0.0005% (w/v) to about 0.005% (w/v) poloxamer 188, wherein the pharmaceutical formulation has a pH of about 7.0 to about 7.5. In some embodiments, the pharmaceutical formulation comprises about 6×10¹¹ vg/mL to about 6×10¹² vg/mL of rAAV particles, about 150 mM to about 200 mM sodium chloride, about 1 mM to about 10 mM monobasic sodium phosphate, about 1 mM to about 10 mM dibasic sodium phosphate, and about 0.0005% (w/v) to about 0.005% (w/v) poloxamer 188, wherein the pharmaceutical formulation has a pH of about 7.0 to about 7.5. In some embodiments, the pharmaceutical formulation comprises about 6×10¹¹ vg/mL of rAAV particles, about 150 mM to about 200 mM sodium chloride, about 1 mM to about 10 mM monobasic sodium phosphate, about 1 mM to about 10 mM dibasic sodium phosphate, and about 0.0005% (w/v) to about 0.005% (w/v) poloxamer 188, wherein the pharmaceutical formulation has a pH of about 7.0 to about 7.5. In some embodiments, the pharmaceutical formulation comprises about 6×10¹² vg/mL of rAAV particles, about 150 mM to about 200 mM sodium chloride, about 1 mM to about 10 mM monobasic sodium phosphate, about 1 mM to about 10 mM dibasic sodium phosphate, and about 0.0005% (w/v) to about 0.005% (w/v) poloxamer 188, wherein the pharmaceutical formulation has a pH of about 7.0 to about 7.5.

In some embodiments, the rAAV particles in the pharmaceutical formulation are present at a concentration of about 1×10¹⁰ vg/ml to about 1×10¹³ vg/ml. In some embodiments, the rAAV particles in the pharmaceutical formulation are present at a concentration of about 1×10⁰⁹ vg/ml to about 6×10¹⁴ vg/ml. In certain embodiments, the rAAV particles in the pharmaceutical formulation are present at a concentration of about 1×10⁰⁹ vg/ml to about 2×10⁰⁹ vg/ml, about 2×10⁰⁹ vg/ml to about 3×10⁰⁹, about 3×10⁰⁹ vg/ml to about 4×10⁰⁹, about 4×10⁰⁹ vg/ml to about 5×10⁰⁹, about 5×10⁰⁹ vg/ml to about 6×10⁰⁹, about 6×10⁰⁹ vg/ml to about 7×10⁰⁹, about 7×10⁰⁹ vg/ml to about 8×10⁰⁹, about 8×10⁰⁹ vg/ml to about 9×10⁰⁹, about 9×10⁰⁹ vg/ml to about 10×10⁰⁹, about 10×10⁰⁹ vg/ml to about 1×10¹⁰, about 1×10¹⁰ vg/ml to about 2×10¹⁰, about 2×10¹⁰ vg/ml to about 3×10¹⁰, about 3×10¹⁰ vg/ml to about 4×10¹⁰, about 4×10¹⁰ vg/ml to about 5×10¹⁰, about 5×10¹⁰ vg/ml to about 6×10¹⁰, about 6×10¹⁰ vg/ml to about 7×10¹⁰, about 7×10¹⁰ vg/ml to about 8×10¹⁰, about 8×10¹⁰ vg/ml to about 9×10¹⁰, about 9×10¹⁰ vg/ml to about 10×10¹⁰, about 10×10¹⁰ vg/ml to about 1×10¹¹, about 1×10¹¹ vg/ml to about 2×10¹¹, about 2×10¹¹ vg/ml to about 3×10¹¹, about 3×10¹¹ vg/ml to about 4×10¹¹, about 4×10¹¹ vg/ml to about 5×10¹¹, about 5×10¹¹ vg/ml to about 6×10¹¹, about 6×10¹¹ vg/ml to about 7×10¹¹, about 7×10¹¹ vg/ml to about 8×10¹¹, about 8×10¹¹ vg/ml to about 9×10¹¹, about 9×10¹¹ vg/ml to about 10×10¹¹, about 1×10¹² vg/ml to about 2×10¹², about 2×10¹² vg/ml to about 3×10¹², about 3×10¹² vg/ml to about 4×10¹², about 4×10¹² vg/ml to about 5×10¹², about 5×10¹² vg/ml to about 6×10¹², about 6×10¹² vg/ml to about 7×10¹², about 7×10¹² vg/ml to about 8×10¹², about 8×10¹² vg/ml to about 9×10¹², about 9×10¹² vg/ml to about 10×10¹², about 1×10¹³ vg/ml to about 2×10¹³, about 2×10¹³ vg/ml to about 3×10¹³, about 3×10¹³ vg/ml to about 4×10¹³, about 4×10¹³ vg/ml to about 5×10¹³, about 5×10¹³ vg/ml to about 6×10¹³, about 6×10¹³ vg/ml to about 7×10¹³, about 7×10¹³ vg/ml to about 8×10¹³, about 8×10¹³ vg/ml to about 9×10¹³, about 9×10¹³ vg/ml to about 10×10¹³, about 1×10¹⁴ vg/ml to about 2×10¹⁴, about 2×10¹⁴ vg/ml to about 3×10¹⁴, about 3×10¹⁴ vg/ml to about 4×10¹⁴, about 4×10¹⁴ vg/ml to about 5×10¹⁴, or about 5×10¹⁴ vg/ml to about 6×10¹⁴ vg/mL. In some embodiments, the pharmaceutical formulation comprises about 6×10¹¹ vg/mL to about 6×10¹² vg/mL of rAAV particles. In some embodiments, the pharmaceutical formulation comprises about 6×10¹² vg/mL of rAAV particles. In some embodiments, the pharmaceutical formulation comprises about 6×10¹¹ vg/mL of rAAV particles.

In some embodiments, the sodium chloride in the pharmaceutical formulation is present at a concentration of about 150 mM to about 200 mM. In certain embodiments, the sodium chloride in the pharmaceutical formulation is present at a concentration of about 150 mM, about 160 mM, about 170 mM, about 180 mM, about 190 mM, or about 200 mM. In certain embodiments, the sodium chloride in the pharmaceutical formulation is present at a concentration of about 180 mM.

In some embodiments, the sodium phosphate monobasic is present in the pharmaceutical formulation at a concentration of about 1 mM to about 10 mM. In some embodiments, the sodium phosphate monobasic is present in the pharmaceutical formulation at a concentration of any of about 1 mM, about 2 mM, about 3 mM, about 4 mM, about 5 mM, about 6 mM, about 7 mM, about 8 mM, about 9 mM, or about 10 mM. In certain embodiments, the sodium phosphate monobasic is present in the pharmaceutical formulation at a concentration of about 5 mM.

In some embodiments, the sodium phosphate dibasic is present in the pharmaceutical formulation at a concentration of about 1 mM to about 10 mM. In some embodiments, the sodium phosphate dibasic is present in the pharmaceutical formulation at a concentration of any of about 1 mM, about 2 mM, about 3 mM, about 4 mM, about 5 mM, about 6 mM, about 7 mM, about 8 mM, about 9 mM, or about 10 mM. In certain embodiments, the sodium phosphate dibasic is present in the pharmaceutical formulation at a concentration of about 5 mM.

In some embodiments, the Poloxamer 188 is present in the pharmaceutical formulation at a concentration of about 0.0005% (w/v) to about 0.005% (w/v). In some embodiments, the Poloxamer 188 is present in the pharmaceutical formulation at a concentration of any of about 0.0005% (w/v), about 0.0006% (w/v), about 0.0007% (w/v), about 0.0008% (w/v), about 0.0009% (w/v), about 0.001% (w/v), about 0.002% (w/v), about 0.003% (w/v), about 0.004% (w/v), or about 0.005% (w/v). In certain embodiments, the Poloxamer 188 is present in the pharmaceutical formulation at a concentration of about 0.001% (w/v).

In some embodiments, the pharmaceutical formulation has a pH of about 7.0 to about 7.5. In some embodiments, the pharmaceutical formulation has a pH of about 7.0, about 7.1, about 7.2, about 7.3, about 7.4, or about 7.5. In certain embodiments, the pharmaceutical formulation has a pH of about 7.3. In some embodiments, hydrochloric acid and sodium hydroxide are used to adjust the pH of the pharmaceutical formulation.

In some embodiments, the pharmaceutical formulation comprises about 6×10¹² vg/mL of rAAV particles, about 180 mM sodium chloride, about 5 mM monobasic sodium phosphate, about 5 mM dibasic sodium phosphate, and about 0.001% (w/v) poloxamer 188, wherein the pharmaceutical formulation has a pH of about 7.3. In some embodiments, the pharmaceutical formulation comprises about 6×10¹¹ vg/mL of rAAV particles, about 180 mM sodium chloride, about 5 mM monobasic sodium phosphate, about 5 mM dibasic sodium phosphate, and about 0.001% (w/v) poloxamer 188, wherein the pharmaceutical formulation has a pH of about 7.3.

In some embodiments, the pharmaceutical formulations are suitable for administration to the one eye and/or the contralateral eye of the individual, e.g., a human patient, via intravitreal (IVT) injection to achieve a desired therapeutic or prophylactic effect. In some embodiments, the pharmaceutical formulation is supplied as a reconstituted homogenous solution. In some embodiments, the solution is a suspension. In some embodiments, the pharmaceutical formulation is supplied as a frozen suspension, and is thawed prior to administration to the one eye and/or the contralateral eye of the individual. In some embodiments, the solution is isotonic.

In other embodiments, the pharmaceutical composition comprising e.g., an AAV2.7m8 vector that comprises a nucleic acid sequence encoding the anti-VEGF agent (e.g., aflibercept or a functional fragment or variant thereof), is supplied in a lyophilized form, and is reconstituted prior to administration to the one eye and/or the contralateral eye of the individual. In some embodiments, the methods provided herein further comprise the steps of reconstituting, dissolving, or solubilizing a lyophilized pharmaceutical composition comprising rAAV (e.g., AAV2.7m8) and encoding the anti-VEGF agent (e.g., aflibercept or a functional fragment or variant thereof) in a buffer prior to administration to the subject. In some embodiments, such lyophilized pharmaceutical composition comprises one or more of the following: a cryoprotectant, a surfactant, a salt, a stabilizer, or any combination thereof.

In some embodiments, the pharmaceutical formulation is a homogenous solution. In some embodiments, the homogenous solution is supplied in a pre-filled syringe. In some embodiments, the pharmaceutical formulation is supplied as a suspension. In some embodiments, a suspension is a solution. In some embodiments, the suspension is refrigerated. In some embodiments, the suspension is frozen. In some embodiments, methods provided herein further comprise the step of warming the refrigerated suspension to room temperature and/or agitating the suspension to ensure that the active ingredient(s) are dissolved and/or evenly distributed in solution prior to administering to the one eye and/or the contralateral eye of the individual (e.g., via IVT injection). In some embodiments, methods provided herein further comprise the step of thawing the frozen suspension and warming to room temperature and/or agitating the suspension to ensure that the active ingredient(s) are dissolved and/or evenly distributed in solution prior to administering to the one eye and/or the contralateral eye of the individual (e.g., via IVT injection). In some embodiments, the suspension is diluted prior to administration to the subject (e.g., via IVT injection). In some embodiments, the suspension is supplied as a pre-filled syringe.

In some embodiments, the pharmaceutical formulation is provided as a frozen suspension. In some embodiments, the suspension comprises a pharmaceutically acceptable excipient, e.g., surfactant, glycerol, non-ionic surfactant, buffer, glycol, salt, and any combination thereof.

In some embodiments, the suspension is a solution. In some embodiments, the suspension comprises micelles.

In some embodiments, for storage stability and convenience of handling, a pharmaceutical formulation, comprising rAAV (e.g., AAV2.7m8) and a nucleic acid sequence that encodes the anti-VEGF agent (e.g., aflibercept or a functional fragment or variant thereof), is formulated as a lyophilized, freeze dried, or vacuum dried powder that is reconstituted with saline, buffer, or water prior to administration to the one eye and/or the contralateral eye of the individual. Alternately, the pharmaceutical formulation is formulated as an aqueous solution, such as a suspension or a homogeneous solution. A pharmaceutical formulation can contain rAAV particles comprising a nucleic acid sequence that encodes aflibercept. Various excipients, such as phosphate, PBS, or Tris buffer, glycol, glycerol, saline, surfactant (e.g., pluronic or polysorbate), or any combination thereof, can be used to stabilize a pharmaceutical formulation. Additionally, cryoprotectants, such as alcohols can be used as a stabilizer under freezing or drying conditions. In some embodiments, the gene therapy is provided as a suspension, a refrigerated suspension, or a frozen suspension.

In some embodiments, a suspension of the pharmaceutical formulation as disclosed herein has a volume of any of about 20 µL, 30 µL, 40 µL, 50 µL, 60 µL, 70 µL, 80 µL, 90 µL, 100 µL, 200 µL, 300 µL, 400 µL, 500 µL, 600 µL, 700 µL, 800 µL, 900 µL, or 1000 µL. In some embodiments, a suspension of the pharmaceutical formulation as disclosed herein has a volume of about 250 µL. In some embodiments, the suspension of the pharmaceutical formulation as disclosed herein has a volume of between 0.1 to 0.5 mL, between 0.1 to 0.2 mL, between 0.3 to 0.5 mL, between 0.5-1.0 mL, between 0.5-0.7 mL, between 0.6 to 0.8 mL, between 0.8 to 1 mL, between 0.9 to 1.1 mL, between 1.0 to 1.2 mL, or between 1.0 to 1.5 mL. In other embodiments, the volume is no more than 0.1 mL, 0.2 mL, 0.3 mL, 0.4 mL, 0.5 mL, 0.6 mL, 0.7 mL, 0.8 mL, 0.9 mL, 1.0 mL, 1.1 mL, 1.2 mL, 1.3 mL, 1.4 mL, or 1.5 mL. In some embodiments, the suspension of the pharmaceutical formulation as disclosed herein has a volume of about 0.25 mL.

In some embodiments, a suspension of the pharmaceutical formulation as disclosed herein is provided as a sterile-filtered, frozen suspension in a sterile, ready-to-use vial (e.g., a 0.5 mL vial; e.g., a Crystal Zenith^{®} vial) with a ready-to-use stopper (e.g., a stopper made of chlorobutyl), and sealed (e.g., with a sterile aluminum tear-off seal). In some embodiments, a suspension of the pharmaceutical formulation as disclosed herein is provided as a sterile-filtered, frozen suspension in a sterile, ready-to-use vial (e.g., a 0.5 mL vial; e.g., a Crystal Zenith^{®} vial) with, a ready-to-use stopper (e.g., a stopper made of chlorobutyl), and sealed (e.g., with a sterile aluminum tear-off seal), wherein the vial contains a volume of between 0.1 to 0.5 mL, between 0.1 to 0.2 mL, between 0.2 to 0.3 mL, between 0.3 to 0.4 mL, or between 0.4 mL to 0.5 mL of the suspension of the pharmaceutical formulation. In some embodiments, a suspension of the pharmaceutical formulation as disclosed herein is provided as a sterile-filtered, frozen suspension in a sterile, ready-to-use vial (e.g., a 0.5 mL vial; e.g., a Crystal Zenith^{®} vial) with a ready-to-use stopper (e.g., a stopper made of chlorobutyl), and sealed (e.g., with a sterile aluminum tear-off seal), wherein the vial contains a volume of about 0.25 mL of the suspension of the pharmaceutical formulation.

In some embodiments, pharmaceutical formulations disclosed herein are designed, engineered, or adapted for administration to a primate (e.g., non-human primate and human subjects) via intravitreal or subretinal injection. In some embodiments, a pharmaceutical formulation comprising rAAV particles comprising a nucleic acid sequence that encodes the anti-VEGF agent (e.g., aflibercept) is formulated for intravitreal injection into an eye of an individual. In some embodiments, the pharmaceutical composition is formulated to or reconstituted to a concentration that allows intravitreal injection of a volume not more than about or not more than any of 25 µL, 30 µL, 35 µL, 40 µL, 45 µL, 50 µL, 55 µL, 60 µL, 65 µL, 70 µL, 75 µL, 80 µL, 85 µL, 90 µL, 95 µL, 100 µL, 110 µL, 120 µL, 130 µL, 140 µL, 150 µL, 160 µL, 170 µL, 180 µL, 190 µL, 200 µL, 210 µL, 220 µL, 230 µL, 240 µL, or 250 µL. In some embodiments, a unit dose of the pharmaceutical formulation comprises a volume not more than about or not more than any of 25 µL, 30 µL, 35 µL, 40 µL, 45 µL, 50 µL, 55 µL, 60 µL, 65 µL, 70 µL, 75 µL, 80 µL, 85 µL, 90 µL, 95 µL, 100 µL, 110 µL, 120 µL, 130 µL, 140 µL, 150 µL, 160 µL, 170 µL, 180 µL, 190 µL, 200 µL, 210 µL, 220 µL, 230 µL, 240 µL, or 250 µL. In some embodiments, methods disclosed herein comprise intravitreal injection of a volume of any of about 25 µL, 30 µL, 35 µL, 40 µL, 45 µL, 50 µL, 55 µL, 60 µL, 65 µL, 70 µL, 75 µL, 80 µL, 85 µL, 90 µL, 95 µL, 100 µL, 110 µL, 120 µL, 130 µL, 140 µL, 150 µL, 160 µL, 170 µL, 180 µL, 190 µL, 200 µL, 210 µL, 220 µL, 230 µL, 240 µL, or 250 µL of a solution or suspension of a pharmaceutical formulation comprising a rAAV (e.g., AAV2.7m8) and a nucleic acid sequence that encodes the anti-VEGF agent (e.g., aflibercept). In some embodiments, methods disclosed herein comprise intravitreal injection of a volume of about 30 µL or about 100 µL of a solution or suspension of a pharmaceutical formulation comprising a rAAV (e.g., AAV2.7m8) and a nucleic acid sequence that encodes the anti-VEGF agent (e.g., aflibercept). In some embodiments, methods disclosed herein comprise intravitreal injection of a volume of about 30 µL of a solution or suspension of a pharmaceutical formulation comprising a rAAV (e.g., AAV2.7m8) and a nucleic acid sequence that encodes the anti-VEGF agent (e.g., aflibercept). In some embodiments, methods disclosed herein comprise intravitreal injection of a volume of about 100 µL of a solution or suspension of a pharmaceutical formulation comprising a rAAV (e.g., AAV2.7m8) and a nucleic acid sequence that encodes the anti-VEGF agent (e.g., aflibercept).

In some embodiments, an AAV2.7m8 particle comprising a nucleic acid sequence of the anti-VEGF agent (e.g., aflibercept) transgene described herein is a component of a gene therapy pharmaceutical formulation. In some embodiments, a rAAV particle of any serotype comprising the 7m8 variant capsid protein as described herein is used to make a frozen suspension or a freeze-dried or lyophilized formulation composition. In some embodiments, the gene therapy is formulated as a refrigerated or frozen suspension. In some embodiments, the rAAV particle is rAAV2. In some embodiments, the lyophilized or suspension of the pharmaceutical formulation comprises rAAV2 comprising the 7m8 variant capsid protein and a DNA sequence that encodes the anti-VEGF agent (e.g., aflibercept). In some embodiments, the suspension is refrigerated or frozen.

In some embodiments, the administration of the unit dose of rAAV particles to the one eye and/or to the contralateral eye of the individual is by intravitreal (IVT) injection. For IVT injection, the rAAV particles can be delivered in the form of a suspension of a pharmaceutical formulation (e.g., as described herein). Initially, topical anesthetic is applied to the surface of the eye followed by an ophthalmic antiseptic solution. The eye is held open, with or without instrumentation, and the rAAV particles are injected through the sclera with a short, narrow needle, e.g., a 30-gauge needle, into the vitreous cavity of the one eye and/or the contralateral eye of the individual under direct observation. Typically, a volume of between about 25 µL to about 250 µL (e.g., any of about 25 µL, about 30 µL, about 40 µL about 50 µL, about 60 µL, about 70 µL, about 80 µL, about 90 µL, about 100 µL, about 110 µL, about 120 µL, about 130 µL, about 140 µL, about 150 µL, about 160 µL, about 170 µL, about 180 µL, about 190 µL, about 200 µL, about 210 µL, about 220 µL, about 230 µL, about 240 µL, or about 250 µL) of an rAAV particle suspension may be delivered to the eye by IVT injection. In some embodiments, the unit dose of rAAV particles comprises a volume of about 100 µL. In some embodiments, the unit dose of rAAV particles comprises a volume of about 30 µL. In some embodiments, the IVT injection is performed in combination with removal of vitreous fluid. In some embodiments, a vitrectomy may be performed, and the entire volume of vitreous gel is replaced by an infusion of the rAAV particle suspension (e.g., about 4 mL of the rAAV particle suspension). A vitrectomy is performed using a cannula of appropriate bore size (e.g., 20 gauge to 27 gauge), wherein the volume of vitreous gel that is removed is replaced by infusion of fluid, e.g., saline, an isotonic solution, a rAAV particle suspension, from the infusion cannula. IVT administration is generally well tolerated. At the conclusion of the procedure, there is sometimes mild redness at the injection site. There is occasional tenderness, but most patients do not report any pain. No eye patch or eye shield is necessary after this procedure, and activities are not restricted. Sometimes, an antibiotic eye drop is prescribed for several days to help prevent infection.

In some embodiments, the pharmaceutical formulation is a unit dose (e.g., a therapeutically effective dose) to be administered to the one eye and/or the contralateral eye of an individual (e.g., a human or non-human primate) via IVT injection for the treatment of an ocular disease or disorder characterized by abnormal (e.g., excessive) angiogenesis or neovascularization. In some embodiments, the pharmaceutical formulation comprises a unit dose (e.g., a therapeutically effective dose) as described in further detail elsewhere herein. In some embodiments, the volume of the unit dose (e.g., a therapeutically effective dose) of a viral vector (e.g., an rAAV vector disclosed herein) administered to the subject is no more than any one of about 25 µL, 30 µL, 35 µL, 40 µL, 45 µL, 50 µL, 55 µL, 60 µL, 65 µL, 70 µL, 75 µL, 80 µL, 85 µL, 90 µL, 95 µL, 100 µL, 110 µL, 120 µL, 130 µL, 140 µL, 150 µL, 160 µL, 170 µL, 180 µL, 190 µL, 200 µL, 210 µL, 220 µL, 230 µL, 240 µL, or 250 µL, including any range in between these values. Minimizing the volume of the unit dose to be administered to the subject may obviate or mitigate changes in ocular pressure and other adverse effects associated with IVT injection (e.g., elevated intraocular pressure, inflammation, irritation, or pain).

Pharmaceutical formulations suitable for ocular use include sterile aqueous solutions or dispersions and sterile powders for the extemporaneous preparation of sterile injectable solutions, suspension, or dispersion. For intravitreal administration, suitable carriers include physiological saline, bacteriostatic water, phosphate buffered saline (PBS), and/or an isotonic agent, e.g., glycerol. In certain embodiments, the pharmaceutical formulation is sterile and fluid to the extent that easy syringability or injectability exists. In certain embodiments, the pharmaceutical formulation is stable under the conditions of manufacture and storage and is preserved against the contaminating action of microorganisms such as bacteria and fungi. In some embodiments, the pharmaceutical composition can include an isotonic agent, such as a salt or glycerol. In some embodiments, a surfactant or a stabilizer is added to the pharmaceutical composition to prevent aggregation.

In some embodiments, the pharmaceutical formulation contains an excipient or a carrier. A carrier is a solvent or dispersion medium containing, for example, water, saline, ethanol, a polyol (for example, glycerol, propylene glycol, and liquid polyethylene glycol, and the like), and any combination thereof. The proper fluidity can be maintained, for example, by the use of a coating such as lecithin, by the maintenance of the required particle size in the case of dispersion and by the use of surfactants such as polysorbates (e.g., Tween^{™}, polysorbate 20, polysorbate 80), sodium dodecyl sulfate (sodium lauryl sulfate), lauryl dimethyl amine oxide, cetyltrimethylammonium bromide (CTAB), polyethoxylated alcohols, polyoxyethylene sorbitan, octoxynol (Triton X100^{™}), N,N-dimethyldodecylamine-N-oxide, hexadecyltrimethylammonium bromide (HTAB), polyoxyl 10 lauryl ether, Brij 721^{™}, bile salts (sodium deoxycholate, sodium cholate), pluronic acids (F-68, F-127), polyoxyl castor oil (Cremophor^{™}) nonylphenol ethoxylate (Tergitol^{™}), cyclodextrins, and ethylbenzethonium chloride (Hyamine^{™}). Prevention of the action of microorganisms can be achieved by various antibacterial and antifungal agents, for example, parabens, chlorobutanol, phenol, ascorbic acid, cresol, thimerosal, and the like. In many embodiments, isotonic agents are included in the pharmaceutical formulation, for example, sugars, polyalcohols such as mannitol, sorbitol, and/or sodium chloride. Prolonged absorption of the internal compositions can be brought about by including in the composition an agent that delays absorption, for example, aluminum monostearate and gelatin. In some embodiments, the pharmaceutical carrier includes sodium phosphate, sodium chloride, polysorbate, and sucrose. In some embodiments, a pharmaceutical formulation comprises a surfactant, e.g., non-ionic surfactant such as polysorbate, poloxamer, or pluronic. In some embodiments, the addition of a non-ionic surfactant reduces aggregation in the pharmaceutical composition.

Also provided herein are kits comprising at least one pharmaceutical formulation described herein. In some embodiments, the kit comprises a frozen suspension of a pharmaceutical formulation (e.g., one unit dose in a vial). In some embodiments, the kit comprises a lyophilized or freeze-dried pharmaceutical formulation (e.g., one unit dose in a vial) disclosed herein and a solution for dissolving, diluting, and/or reconstituting the lyophilized pharmaceutical composition. In some embodiments, the solution for reconstituting or dilution is supplied as a pre-filled syringe. In some embodiments, a kit comprises a freeze-dried or lyophilized pharmaceutical composition comprising rAAV (e.g., AAV2.7m8) and a solution for reconstituting the pharmaceutical composition to a desired concentration or volume. In some embodiments, the kit includes a buffer that helps to prevent aggregation upon reconstituting the pharmaceutical composition disclosed herein. In some embodiments, the pharmaceutical composition is provided in a pre-filled syringe. In some embodiments, a kit comprises a dual-chamber syringe or container wherein one of the chambers contains a buffer for dissolving or diluting the pharmaceutical composition. In some embodiments, the kit comprises a syringe for injection. In some embodiments, the reconstituted solution is filtered before administration. In some embodiments, the kit comprises a filter or a filter syringe for filtering the reconstituted pharmaceutical composition before administration to a patient. In some embodiments, the kit comprises a suspension of the pharmaceutical formulation comprising the rAAV particles as disclosed herein provided as a sterile-filtered, frozen suspension in a sterile, ready-to-use vial (e.g., a 0.5 mL vial; e.g., a Crystal Zenith^{®} vial) with a ready-to-use stopper (e.g., a stopper made of chlorobutyl), and sealed (e.g., with a sterile aluminum tear-off seal). In some embodiments, the kit comprises a suspension of the pharmaceutical formulation comprising the rAAV particles as disclosed herein provided as a sterile-filtered, frozen suspension in a sterile, ready-to-use vial (e.g., a 0.5 mL vial; e.g., a Crystal Zenith^{®} vial) with a ready-to-use stopper (e.g., a stopper made of chlorobutyl), and sealed (e.g., with a sterile aluminum tear-off seal), wherein the vial contains a volume of between 0.1 to 0.5 mL, between 0.1 to 0.2 mL, between 0.2 to 0.3 mL, between 0.3 to 0.4 mL, or between 0.4 mL to 0.5 mL of the suspension of the pharmaceutical formulation. In some embodiments, the kit comprises a suspension of the pharmaceutical formulation comprising the rAAV particles as disclosed herein provided as a sterile-filtered, frozen suspension in a sterile, ready-to-use vial (e.g., a 0.5 mL vial; e.g., a Crystal Zenith^{®} vial) with a ready-to-use stopper (e.g., a stopper made of chlorobutyl), and sealed (e.g., with a sterile aluminum tear-off seal), wherein the vial contains a volume of about 0.25 mL of the suspension of the pharmaceutical formulation. In some embodiments, the kit further comprises instructions for use, e.g., instructions for treating an ocular neovascular disease with the rAAV particles disclosed herein.

### Ocular Neovascular Diseases

In one aspect, the present disclosure provides methods for treating an ocular neovascular disease in an individual. In another aspect, the present disclosure provides methods for reducing retinal fluid in the eye of an individual with an ocular neovascular disease.

In some embodiments, the ocular neovascular disease is age-related macular degeneration (AMD), wet-AMD, retinal neovascularization, choroidal neovascularization diabetic retinopathy, proliferative diabetic retinopathy, retinal vein occlusion, central retinal vein occlusion, branched retinal vein occlusion, diabetic macular edema, diabetic retinal ischemia, ischemic retinopathy, diabetic retinal edema, or any combination thereof. In some embodiments, the ocular neovascular disease is active choroidal neovascularization (CNV) secondary to age-related macular degeneration (AMD).

In some embodiments, the ocular neovascular disease is recurrent and/or persistent wAMD. In some embodiments, the ocular neovascular disease is active subfoveal CNV secondary to AMD. In some embodiments, the active subfoveal CNV secondary to AMD occupies ≥ 50% of the total lesion size. In some embodiments, the active subfoveal CNV secondary to AMD occupies ≥ 50% of the total lesion size with evidence of leakage on fluorescein angiogram (FA), fluid on spectral domain optical coherence tomography (SD-OCT), and/or subretinal hemorrhage on color fundus photography. In some embodiments, the active subfoveal CNV secondary to AMD occupies ≥ 50% of the total lesion size with evidence of leakage on fluorescein angiogram (FA), fluid on spectral domain optical coherence tomography (SD-OCT), and/or subretinal hemorrhage on color fundus photography, and the entire dimension of the lesion does not exceed 12 macular photocoagulation study disc areas. In some embodiments, the one eye and/or the contralateral eye of the individual exhibited best corrected visual acuity (BCVA) based on an ETDRS letters assessment of 78-25 (e.g., less than any of about 78, about 75, about 70, about 65, about 60, about 55, about 50, about 45, about 40, about 35, about 30, or about 25) prior to administration of the unit dose of rAAV particles of the present disclosure. In some embodiments, the one eye and/or the contralateral eye of the individual exhibited best corrected visual acuity (BCVA) based on an ETDRS letters assessment of more than any of about 5, about 10, about 15, about 20, about 25, about 30, about 35, about 40, about 45, about 50, about 55, about 60, about 65, about 70, about 75, about 80, about 85, about 90, about 95, or about 100 prior to administration of the unit dose of rAAV particles of the present disclosure.

In some embodiments, the individual had polypoidal choroidal vasculopathy (PCV) in the one eye and/or the contralateral eye prior to administration of the unit dose of rAAV particles.

In some embodiments, ETDRS letters assessment is done at about 0.5 meters, about 1 meter, about 2 meters, about 3 meters, or about 4 meters. In some embodiments, ETDRS letters assessment is done at about 4 meters.

In some embodiments, the individual received at least one prior treatment (e.g., at least one, at least two, at least three, at least four, at least 5 or more treatments) with an anti-VEGF agent (e.g., bevacizumab, brolucizumab, ranibizumab, faricimab, abicipar pegol, conbercept, OPT-302, KSI-301, injectable sunitinib maleate (GB-102), PAN-90806 (PanOptica), and/or aflibercept) in about the last 12 weeks (e.g., about 3 or about 4 months) prior to administration of the unit dose of rAAV particles. In some embodiments, the individual received 2 or 3 prior treatments with an anti-VEGF agent (e.g., bevacizumab, brolucizumab, ranibizumab, faricimab, abicipar pegol, conbercept, OPT-302, KSI-301, injectable sunitinib maleate (GB-102), PAN-90806 (PanOptica), and/or aflibercept) in the one eye and/or in the contralateral eye during about the last 12 weeks (e.g., about 3 or about 4 months) prior to administration of the unit dose of rAAV particles to the one eye and/or the contralateral eye. In some embodiments, the individual received at least about 1, at least about 5, at least about 10, at least about 20, at least about 30, at least about 40, at least about 50, at least about 60, at least about 70, at least about 80, at least about 90, at least about 100, at least about 110, at least about 120, or more prior treatments with an anti-VEGF agent (e.g., bevacizumab, brolucizumab, ranibizumab, faricimab, abicipar pegol, conbercept, OPT-302, KSI-301, injectable sunitinib maleate (GB-102), PAN-90806 (PanOptica), and/or aflibercept) in the one eye and/or the contralateral eye. In some embodiments, the individual had a calculated anti-VEGF agent (e.g., bevacizumab, brolucizumab, ranibizumab, and/or aflibercept) injection interval in the one eye and/or the contralateral eye of about 2 weeks, about 3 weeks, 4 weeks, about 5 weeks, about 6 weeks, about 7 weeks, about 8 weeks, about 9 weeks, about 10 weeks, about 11 weeks, about 12 weeks, or more. In some embodiments, the individual had a calculated anti-VEGF (e.g., bevacizumab, brolucizumab, ranibizumab, and/or aflibercept) injection interval in the one eye and/or the contralateral eye of about 5-7 weeks, about 4-10 weeks, about 4-7 weeks, or about 4-6 weeks. In some embodiments, the individual received a prior treatment with an anti-VEGF agent (e.g., bevacizumab, brolucizumab, ranibizumab, faricimab, abicipar pegol, conbercept, OPT-302, KSI-301, injectable sunitinib maleate (GB-102), PAN-90806 (PanOptica), and/or aflibercept) in the one eye and/or in the contralateral eye any of at least about 5 days, at least about 6 days, at least about 7 days, at least about 8 days, at least about 9 days, at least about 10 days, at least about 11 days, at least about 12 days, at least about 13 days, at least about 14 days, at least about 15 days, at least about 16 days, at least about 17 days, at least about 18 days, at least about 19 days, or at least about 20 days prior to administration of the unit dose of rAAV particles to the one eye and/or the contralateral eye. In some embodiments, the individual received a prior treatment with an anti-VEGF agent (e.g., bevacizumab, brolucizumab, ranibizumab, faricimab, abicipar pegol, conbercept, OPT-302, KSI-301, injectable sunitinib maleate (GB-102), PAN-90806 (PanOptica), and/or aflibercept) in the one eye and/or the contralateral eye about 7 days, about 10 days, or about 14 days prior to administration of the unit dose of rAAV particles to the one eye and/or the contralateral eye. In some embodiments, the prior treatment comprises an intraocular, subretinal or intravitreal injection with an anti-VEGF agent. In some embodiments, the anti-VEGF agent is bevacizumab, brolucizumab, ranibizumab, faricimab, abicipar pegol, conbercept, OPT-302, KSI-301, injectable sunitinib maleate (GB-102), PAN-90806 (PanOptica), and/or aflibercept. In some embodiments, the anti-VEGF agent is aflibercept. In some embodiments, the individual received between 1 and 20 (e.g., any of 1, 2, 3, 4, 5, 6, 7, 8, 9, 10, 11, 12, 13, 14, 15, 16, 17, 18, 19, or 20) prior treatments with an anti-VEGF agent (e.g., bevacizumab, brolucizumab, ranibizumab, faricimab, abicipar pegol, conbercept, OPT-302, KSI-301, injectable sunitinib maleate (GB-102), PAN-90806 (PanOptica), and/or aflibercept) in the one eye and/or in the contralateral eye during the last about 12 months prior to administration of the unit dose of rAAV particles to the one eye and/or the contralateral eye. In some embodiments, the individual received about 9 or about 10 prior treatments with an anti-VEGF agent (e.g., bevacizumab, brolucizumab, ranibizumab, faricimab, abicipar pegol, conbercept, OPT-302, KSI-301, injectable sunitinib maleate (GB-102), PAN-90806 (PanOptica), and/or aflibercept) in the one eye and/or in the contralateral eye during the last about 12 months prior to administration of the unit dose of rAAV particles to the one eye and/or the contralateral eye.

In some embodiments, the individual demonstrated a meaningful response to a prior treatment with anti-VEGF agent. In some embodiments, the anti-VEGF agent is aflibercept, a functional variant thereof, or a functional fragment thereof. In some embodiments, the anti-VEGF agent comprises a polypeptide comprising an amino acid sequence with at least about 95% identity to the amino acid sequence of SEQ ID NO: 35. In some embodiments, the individual demonstrated a meaningful response to a prior anti-VEGF treatment (e.g., aflibercept, a functional variant thereof, or a functional fragment thereof) for the ocular neovascular disease in the one eye and/or in the contralateral eye prior to administration of the unit dose of rAAV particles to one eye and/or the contralateral eye. In some embodiments, the individual is determined to have a meaningful response to a prior anti-VEGF treatment (e.g., aflibercept, a functional variant thereof, or a functional fragment thereof) for the ocular neovascular disease if a reduction of ≥30% (e.g., any of at least 30%, at least 40%, at least 50%, at least 60%, at least 70%, at least 80%, at least 90%, or 100%) of central retinal thickness (CRT) or central subfield thickness (CST) is observed any of at least about 5 days, at least about 6 days, at least about 7 days, at least about 8 days, at least about 9 days, at least about 10 days, at least about 11 days, at least about 12 days, at least about 13 days, at least about 14 days, at least about 15 days, at least about 16 days, at least about 17 days, or more, after the anti-VEGF treatment, compared to the central retinal thickness (CRT) or central subfield thickness (CST) at the initial diagnosis in the one eye and/or the contralateral eye. In some embodiments, the individual is determined to have a meaningful response to a prior anti-VEGF treatment (e.g., aflibercept, a functional variant thereof, or a functional fragment thereof) for the ocular neovascular disease if a reduction of ≥30% (e.g., any of at least 30%, at least 40%, at least 50%, at least 60%, at least 70%, at least 80%, at least 90%, or 100%) in central retinal thickness (CRT) or central subfield thickness (CST) is observed more than any of about 7 days, about 10 days, or about 14 days after the anti-VEGF treatment, compared to the central retinal thickness (CRT) or central subfield thickness (CST) prior to administration of the prior anti-VEGF treatment in the one eye and/or the contralateral eye.

In some embodiments, the central subfield thickness and/or central retinal thickness is determined by SD-OCT in the one eye and/or the contralateral eye. Central subfield thickness is the mean thickness of the retina across the central subfield of an ETDRS grid, a 1 mm diameter circle centered on the fovea.

In some embodiments, the individual is determined to have a meaningful response to a prior treatment with anti-VEGF agent (e.g., aflibercept, a functional variant thereof, or a functional fragment thereof) for the ocular neovascular disease if a reduction of ≥20% (e.g., any of at least 20%, at least 30%, at least 40%, at least 50%, at least 60%, at least 70%, at least 80%, at least 90%, or 100%) in central subfield thickness and/or central retinal thickness is observed compared to the central subfield thickness and/or central retinal thickness prior to administration of the prior treatment with the anti-VEGF agent. In some embodiments, the individual is determined to have a meaningful response to a prior treatment with anti-VEGF agent (e.g., aflibercept, a functional variant thereof, or a functional fragment thereof) for the ocular neovascular disease if a reduction of ≥20% (e.g., any of at least 20%, at least 30%, at least 40%, at least 50%, at least 60%, at least 70%, at least 80%, at least 90%, or 100%) in central subfield thickness and/or central retinal thickness is observed any of at least about 5 days, at least about 6 days, at least about 7 days, at least about 8 days, at least about 9 days, at least about 10 days, at least about 11 days, at least about 12 days, at least about 13 days, at least about 14 days, at least about 15 days, at least about 16 days, at least about 17 days, or more, after administration of the prior treatment with an anti-VEGF agent, compared to the central subfield thickness and/or central retinal thickness prior to administration of the prior treatment with an anti-VEGF agent. In some embodiments, the individual is determined to have a meaningful response to a prior treatment with an anti-VEGF agent (e.g., aflibercept, a functional variant thereof, or a functional fragment thereof) for the ocular neovascular disease if a reduction of ≥20% (e.g., any of at least 20%, at least 30%, at least 40%, at least 50%, at least 60%, at least 70%, at least 80%, at least 90%, or 100%) in central subfield thickness and/or central retinal thickness is observed about 7 days, about 10 days, or about 14 days after administration of the prior treatment with the anti-VEGF agent, compared to the central subfield thickness and/or central retinal thickness prior to administration of the prior treatment with an anti-VEGF agent. In some embodiments, the central subfield thickness and/or central retinal thickness is determined by SD-OCT in the one eye and/or the contralateral eye. In some embodiments, the individual is determined to have a meaningful response to a prior treatment with an anti-VEGF agent (*e.g*., aflibercept, a functional variant thereof, or a functional fragment thereof) for the ocular neovascular disease if normalization of CST is observed with no observable vascular exudation after the anti-VEGF treatment in the one eye and/or the contralateral eye. Normalization refers to a CST value that is normal for that class of patient (e.g., based on age, sex, etc.)

In some embodiments, the individual is determined to have a meaningful response to a prior anti-VEGF treatment (e.g., aflibercept, a functional variant thereof, or a functional fragment thereof) for the ocular neovascular disease if a reduction of ≥20% (e.g., any of at least 20%, at least 30%, at least 40%, at least 50%, at least 60%, at least 70%, at least 80%, at least 90%, or 100%) in central retinal thickness (CRT) or central subfield thickness (CST) is observed any of at least about 5 days, at least about 6 days, at least about 7 days, at least about 8 days, at least about 9 days, at least about 10 days, at least about 11 days, at least about 12 days, at least about 13 days, at least about 14 days, at least about 15 days, at least about 16 days, at least about 17 days, or more, after the anti-VEGF treatment, compared to the central retinal thickness (CRT) or central subfield thickness (CST) prior to administration of the anti-VEGF treatment (e.g., aflibercept), as determined by SD-OCT in the one eye and/or the contralateral eye. In some embodiments, the individual is determined to have a meaningful response to a prior anti-VEGF treatment (e.g., aflibercept, a functional variant thereof, or a functional fragment thereof) for the ocular neovascular disease if a reduction of ≥20% (e.g., any of at least 20%, at least 30%, at least 40%, at least 50%, at least 60%, at least 70%, at least 80%, at least 90%, or 100%) in central retinal thickness (CRT) or central subfield thickness (CST) is observed about 7 days, about 10 days, or about 14 days after the anti-VEGF treatment, compared to the central retinal thickness (CRT) or central subfield thickness (CST) prior to administration of the anti-VEGF treatment, as determined by SD-OCT in the one eye and/or the contralateral eye.

CST is measured using a software configured to detect the membrane lines in the OCT images. Once the membrane lines are defined, the distance is measured at the central 1mm subfield between the retinal pigment epithelium (RPE) and the internal limiting membrane, as discussed in for example, Lai, et al. "Association between Retinal Thickness Variability and Visual Acuity Outcome during Maintenance Therapy Using Intravitreal Anti-Vascular Endothelial Growth Factor Agents for Neovascular Age-Related Macular Degeneration. J. Pers. Med. 2021, 11, 1024, which is incorporated by reference herein for its disclosure of methods of measuring CST.

In some embodiments, the individual has not received a prior treatment for an ocular neovascular disease. In some embodiments, the individual has not received a prior treatment in the one eye and/or the contralateral eye for an ocular neovascular disease. In some embodiments, the individual has not received a prior anti-VEGF treatment. In some embodiments, the individual has not received a prior anti-VEGF treatment in the one eye and/or the contralateral eye. In some embodiments, the individual has not received a prior aflibercept treatment. In some embodiments, the individual has not received a prior aflibercept treatment in the one eye and/or the contralateral eye.

In some embodiments, the ocular disease or disorder treated according to the methods described herein is diabetic macular edema. Diabetic macular edema (DME) is a swelling of the retina in diabetes mellitus due to leaking of fluid from blood vessels within the macula. The macula is the central portion of the retina, a small area rich in cones, the specialized nerve endings that detect color and upon which daytime vision depends. As macular edema develops, blurring occurs in the middle or just to the side of the central visual field. Visual loss from diabetic macular edema can progress over a period of months and make it impossible to focus clearly. Common symptoms of DME are blurry vision, floaters, double vision, and eventually blindness if it goes untreated. In some embodiments, methods and pharmaceutical compositions as disclosed herein are used to treat DME. In some embodiments, treatment of DME is assessed by measuring refraction or visual acuity (e.g., BCVA using ETDRS letters). In some embodiments, visual acuity is measured starting at a distance of about 4 meters prior to dilation of the one eye and/or the contralateral eye. In some embodiments, visual acuity is considered to be maintained if the individual loses fewer than 15 letters in the ETDRS score after administration of the unit dose of rAAV particles compared to prior to administration of the unit dose of rAAV particles. In some embodiments, treatment of DME is assessed by SD-OCT and/or OCT-A. In some embodiments, treatment of DME is assessed by measuring central subfield thickness and/or macular volume using SD-OCT. In some embodiments, treatment of DME is assessed by the number of treatments with an anti-VEGF agent (e.g., aflibercept) administered after administration of the unit dose of rAAV particles (e.g., aflibercept rescue treatments). In some embodiments, one or more aflibercept rescue treatments are administered after administration of the unit dose of rAAV particles if an increase in central subfield thickness (CST) of > 50 µm occurs, assessed by SD-OCT, compared to the lower of the two CST measurements recorded prior to administration of the unit dose of rAAV particles (e.g., on Day 1) and about 3 weeks after administration of the unit dose of rAAV particles (e.g., on Week 4). In some embodiments, one or more aflibercept rescue treatments are administered after administration of the unit dose of rAAV particles if loss of > 5 letters in BCVA occurs due to worsening DME disease activity compared to the higher of the two BCVA measurements recorded prior to administration of the unit dose of rAAV particles (e.g., on Day 1) and about 3 weeks after administration of the unit dose of rAAV particles (e.g., on Week 4). In some embodiments, treatment of DME is assessed using the Diabetic Retinopathy Severity Scale (DRSS), e.g., using ultra-wide field color fundus photography, compared to DRSS prior to administration of the unit dose of rAAV particles. In some embodiments, treatment of DME is assessed by the occurrence of vision threatening complications (e.g., anterior segment neovascularization, diabetic macular edema, high-risk PDR development, vitreous hemorrhage, or tractional retinal detachment). In some embodiments, vision threatening complications are assessed by ultra-wide field imaging and clinical examination. In some embodiments, the administering the unit dose of rAAV particles to the one eye and/or to the contralateral eye of the individual results in a 2-step or in a 3-step improvement in Diabetic Retinopathy Severity Scale (DRSS). In some embodiments, the administering the unit dose of rAAV particles to the one eye and/or to the contralateral eye of the individual results in a 2-step improvement in Diabetic Retinopathy Severity Scale (DRSS). In some embodiments, the administering the unit dose of rAAV particles to the one eye and/or to the contralateral eye of the individual results in a 3-step improvement in Diabetic Retinopathy Severity Scale (DRSS).

In some embodiments, the ocular disease or disorder treated according to the methods described herein is a retinal vein occlusion. Retinal vein occlusion is a blockage of the small veins that carry blood away from the retina. The retina is the layer of tissue at the back of the inner eye that converts light images to nerve signals and sends them to the brain. Retinal vein occlusion is most often caused by hardening of the arteries (atherosclerosis) and the formation of a blood clot. Blockage of smaller veins (branch veins or BRVO) in the retina often occurs in places where retinal arteries that have been thickened or hardened by atherosclerosis cross over and place pressure on a retinal vein. Symptoms of retinal vein occlusion can include a sudden blurring or vision loss in all or part of one eye.

In some embodiments, the ocular disease or disorder treated according to the methods described herein is choroidal neovascularization (CNV), also known as wet age-related macular degeneration (wAMD). Choroidal neovascularization can involve the growth of new blood vessels that originate from the choroid through a break in the Bruch membrane into the sub-retinal pigment epithelium (sub-RPE) or subretinal space, which can be a major cause of visual loss. CNV can create a sudden deterioration of central vision, noticeable within a few weeks. Other symptoms can include color disturbances, and metamorphopsia (distortions in which straight lines appears wavy). Hemorrhaging of the new blood vessels can accelerate the onset of symptoms of CNV. CNV may also include feeling of pressure behind the eye.

The advanced "wet" form (neovascular or exudative) of AMD may frequently cause a rapid and often substantial loss of central vision in patients. In the wet form of AMD, choroidal neovascularization forms and develops into a network of vessels that may grow under and through the retinal pigment epithelium. As this is accompanied by leakage of plasma and/or hemorrhage into the subretinal space, there could be severe sudden loss of central vision if this occurs in the macula. The present disclosure contemplates treatment or prevention of AMD, wet AMD. In some embodiments, methods and pharmaceutical compositions as disclosed herein are used to treat AMD.

In some embodiments, methods described herein are used to prevent or treat an ocular disease or disorder in a subject who has received prior treatment with bevacizumab, brolucizumab, ranibizumab, faricimab, abicipar pegol, conbercept, OPT-302, KSI-301, injectable sunitinib maleate (GB-102), PAN-90806 (PanOptica), and/or aflibercept. In some embodiments, methods described herein are used to prevent or treat an ocular disease or disorder that is responsive to treatment with bevacizumab, brolucizumab, ranibizumab, and/or aflibercept.

In some embodiments, the individual was diagnosed with the ocular neovascular disease at least 1 day, at least 1 week, at least 1 month, at least 2 months, at least 4 months, at least 6 months, at least 12 months, at least 18 months, at least 24 months, at least 30 months, at least 36 months, at least 42 months, at least 48 months, at least 54 months, at least 60 months, at least 66 months, at least 72 months, at least 78 months, at least 84 months, at least 90 months, 96 months, at least 102 months, at least 108 months, at least 114 months, at least 120 months, at least 126 months, at least 132 months, or more, prior to administration of the unit dose of rAAV particles to the one eye and/or the contralateral eye.

The following description is presented to enable a person of ordinary skill in the art to make and use the various embodiments. Descriptions of specific devices, techniques, and applications are provided only as examples. Various modifications to the examples described herein will be readily apparent to those of ordinary skill in the art, and the general principles defined herein may be applied to other examples and applications without departing from the spirit and scope of the various embodiments. Thus, the various embodiments are not intended to be limited to the examples described herein and shown, but are to be accorded the scope consistent with the claims.

### EXAMPLES

### Example 1: An open label Phase 1 study of AAV2.7m8-aflibercept in neovascular (wet) age-related macular degeneration at a dose of 6 × 10¹¹ vg/eye.

This example describes an open label Phase 1 study of AAV2.7m8-aflibercept, a rAAV vector containing the VEGF inhibitor aflibercept and the AAV2.7m8 protein capsid, for the treatment of age-related macular degeneration (AMD) with choroidal neovascularization (wet AMD; wAMD).

### I. Study Objectives

### A. Primary Objective

The primary objective of this study was to assess the safety and tolerability of a single intravitreal (IVT) injection of AAV2.7m8-aflibercept in subjects with wAMD.

### Primary Endpoints

The primary endpoints of this study were the type, severity, and incidence of ocular and systemic adverse events (AEs).

### B. Secondary Objectives

The secondary objectives of this study were:
- To evaluate the effect of AAV2.7m8-aflibercept on Best Corrected Visual Acuity (BCVA).
- To evaluate the effect of AAV2.7m8-aflibercept on central subfield thickness (CST), also known as central retinal thickness (CRT).
- To assess the need for rescue therapy from Week 4 to Week 104.
- To evaluate the effect of AAV2.7m8-aflibercept on the presence of intraretinal fluid (IRF) and subretinal fluid (SRF).
- To evaluate the effect of AAV2.7m8-aflibercept on pigment epithelial detachment (PED) resolution among patients with a PED at baseline.

### Secondary Endpoints

The secondary endpoints of this study were:
- The mean change in BCVA from baseline over time, as assessed by ETDRS letters over time from Day 8 to Week 104, compared to baseline.
- The percentage of subjects with a BCVA gain of ≥15 ETDRS letters from baseline over time, as assessed from Day 8 to Week 104, compared to baseline.
- The percentage of subjects with a BCVA decline of ≤15 ETDRS letters from baseline over time, as assessed from Day 8 to Week 104, compared to baseline.
- The mean change in CST and macular volume from Baseline over time, as assessed from Day 8 to Week 104, compared to baseline.
- The mean number of aflibercept injections over time, as assessed from Weeks 4-104.
- The percentage of subjects requiring aflibercept injections over time, as assessed from Weeks 4-104.
- The percentage of subjects without IRF over time, as assessed from Day 8 to Week 104.
- The percentage of subjects without SRF over time, as assessed from Day 8 to Week 104.
- The percentage of subjects without a PED over time, as assessed from Day 8 to Week 104 among subjects with a PED at baseline.

### II. Study Subjects

Subjects in this study were diagnosed with active choroidal neovascularization (CNV) secondary to age-related macular degeneration (AMD), had a history of recent responsiveness to anti-VEGF treatment, and required frequent injections of anti-VEGF therapy.

Only one eye was selected as the study eye for the duration of the study. If both eyes met all of the inclusion and exclusion criteria, the eye with the worst BCVA assessed at Screening was selected as the study eye. If both eyes met all of the inclusion and exclusion criteria and BCVA values were identical for both eyes, the subject chose to select his/her non-dominant eye for treatment, or by default the right eye was selected as the study eye.

### A. Inclusion Criteria

Subjects that met the following inclusion criteria were enrolled in this study:
- Male or female subjects, age ≥ 50 years of age.
- The study eye had prior or current evidence of active subfoveal CNV secondary to AMD occupying ≥ 50% of total lesion size with:
   o Leakage on fluorescein angiogram (FA), fluid on Spectral Domain Optical Coherence Tomography (SD-OCT), or subretinal hemorrhage on color fundus photo; and
   o The entire dimension of the lesion did not exceed 12 Macular Photocoagulation Study disc areas.
- Subjects were under active anti-VEGF treatment for wAMD with a minimum of 2 injections within 4 months prior to Screening.
- Vision of study eye at Screening visit (prior to aflibercept injection):
   o BCVA ETDRS of 78-25.
- Vision of non-study eye:
   o BCVA ETDRS of ≥35.
- Demonstrated a meaningful anti-VEGF response as confirmed by the Investigator and defined as:
   o Reduction from initial diagnosis in central subfield thickness by ≥ 30% as assessed using SD-OCT; or
   o Reduction from screening in central subfield thickness by ≥ 20% as assessed using SD-OCT; or
   o Normalization of CST with no observable vascular exudation.

VEGF responsiveness was confirmed by the Investigator for purposes of confirmation of anti-VEGF response at Day 1 visit prior to dosing with AAV2.7m8-aflibercept. Subjects determined not to have a meaningful anti-VEGF response failed screening and were not enrolled in this study.

### B. Exclusion Criteria

Subjects that met the following exclusion criteria were not enrolled in this study:

### Neutralizing Antibodies

- Although there is not an established correlation between levels of neutralizing antibodies (NAbs) to AAVs in serum and vitreous fluid (Lukason et al., (2011) Mol Ther 19(2):260-265), subjects were screened for NAbs to the AAV2.7m8 vector as a precaution. Subjects with documented anti-AAV2.7m8 neutralizing antibody titer levels > 1:125 within 6 months prior to dosing with AAV2.7m8-aflibercept were excluded from this study.

### CNV Lesion

- Known history or evidence of the following CNV lesion characteristics:
   o Fibrosis or atrophy, retinal epithelial tear in the center of the fovea in the study eye, or any condition preventing visual acuity improvement.
   o Scarring or fibrosis making up > 50% of total lesion area.
   o Lesion size > 12 Macular Photocoagulation Study disc areas (30.5 mm²), including blood, scars, and neovascularization as assessed by Fluorescein Angiography (FA).
   o Subretinal hemorrhage that was ≥ 50% of the total lesion area, or the presence of blood under the fovea that was ≥ 1 disc area in size in the study eye (if blood was under the fovea, then the fovea was surrounded 270 degrees by visible CNV).

### Ocular Conditions (Retina/Posterior Eye)

- Significant epiretinal membrane or vitreomacular traction (VMT) syndrome in the study eye at time of dosing with AAV2.7m8-aflibercept or history of a full thickness macular hole (Gass Stage 2 and above) in the study eye.
- History of retinal disease in the study eye other than wAMD, including diabetic retinopathy (in either eye), retinal vein occlusion, uveitis, suspected retinal angiomatous proliferation, polypoidal choroidopathy, or CNV due to other causes (e.g., ocular histoplasmosis, trauma, or pathologic myopia), or any other vascular disease in the eye (benign conditions of the vitreous or peripheral retina were non-exclusionary).
- History of retinal detachment (with or without repair) in the study eye.

### Other Conditions (Non-Retinal)

- Known history or evidence of significant non-retinal disease or media opacity in the study eye that could have compromised vision during the course of the study, required surgery, and/or precluded proper visualization or imaging of the retina (e.g., central corneal scarring, significant cataract, corneal dystrophy, scleromalacia).
- Uncontrolled ocular hypertension or glaucoma in the study eye at time of dosing with AAV2.7m8-aflibercept (defined as intraocular pressure [IOP] > 22 mmHg despite treatment with anti-glaucoma medication) or use of > 2 IOP lowering medications at the time of screening.
- Active or history of ocular or periocular infection in either eye within 4 weeks prior to dosing with AAV2.7m8-aflibercept.

### Ocular Surgeries/Procedures

- Any previous intraocular or periocular surgery on the study eye within 6 months of dosing with AAV2.7m8-aflibercept, or any planned major surgical procedure within 6 months of dosing with AAV2.7m8-aflibercept. Lid surgery > 1 month of dosing with AAV2.7m8-aflibercept was not an exclusion criterion.
- History of vitrectomy, trabeculectomy, or other filtration surgery in the study eye.
- Yttrium aluminum garnet (YAG) posterior capsulotomy within 3 months prior to dosing with AAV2.7m8-aflibercept.
- Any prior treatment with photodynamic therapy or retinal laser for the treatment of wAMD and any previous therapeutic radiation in the region of the study eye.

### General/Systemic Conditions

- History or evidence of any of the following cardiovascular diseases within 6 months of dosing unless specified:
   o Severe cardiac disease (e.g., New York Heart Association [NYHA] Functional Class III or IV) history or clinical evidence of unstable angina.
   o Acute coronary syndrome, myocardial infarction or coronary artery revascularization.
   o Ventricular tachyarrhythmias requiring ongoing treatment, or uncontrolled arrhythmia.
   o Uncontrolled hypertension defined as average systolic blood pressure (SBP) ≥160 mmHg or an average diastolic blood pressure (DBP) ≥100 mmHg, despite using BP-lowering medication within the screening period prior to dosing.
   o History of cerebrovascular accident or transient ischemic attack.
- Any history of ongoing bleeding disorders or international normalized ratio (INR) >3.0. The use of aspirin or other anticoagulants (e.g., Factor Xa inhibitors) was not an exclusion criterion. INR was repeated during the Screening period to confirm eligibility criteria were met.
- Evidence of uncontrolled diabetes with an HbAlc >7.0% within the screening period prior to dosing with AAV2.7m8-aflibercept.
- History of malignancy within the last 5 years except for the following, if adequately treated:
   o Local basal cell or squamous cell carcinoma of the skin.
   o Carcinoma *in situ* of the cervix or breast.
   o Papillary, noninvasive bladder cancer.
   o Prostate cancer Stage 1 and 2 for which observation was clinically indicated with stable prostate-specific antigen (PSA) for 6 months.
   o Any other cancer that was in complete remission for at least 2 years or considered surgically cured.
- Positive HIV, Hepatitis B, or Hepatitis C (unless treated with a documented cure).
- Within 36 hours prior to dosing with AAV2.7m8-aflibercept, evidence or suspicion of systemic active infection of any type deemed clinically significant by the Investigator based on clinical exam and/or temperature >38.5°C.
- Known serious allergies to:
   o Fluorescein dye or sodium fluorescein used in angiography (mild allergy amenable to treatment was allowable); or
   o Aflibercept.
- Women who were pregnant, breastfeeding, or intend to become pregnant during the study.
- Other significant laboratory abnormalities or medical conditions that compromised the subject's safety in the view of the Investigator.

### Medications

- Use of systemic anti-inflammatory steroids or immunosuppressant medications (other than protocol-specified prednisone) within 5 half-lives prior to dosing with AAV2.7m8-aflibercept. Inhaled or topical steroids and nonsteroidal anti-inflammatory drugs (NSAIDs) were permitted.
- Received any of the following:
   o Investigational medicinal product within 30 days or 5 half-lives prior to dosing with AAV2.7m8-aflibercept, whichever was longer.
   o Prior gene therapy.

### III. Investigational Medicinal Product

The investigational medicinal product (IMP), AAV2.7m8-aflibercept, was a recombinant, replication-deficient adeno-associated viral (rAAV) vector containing the AAV2.7m8 protein capsid derived by *in vivo* directed evolution on the AAV2 capsid (Dalkara et al., (2013) Sci Transl Med 5(189):189ra76; US2014/0364338). AAV2.7m8-aflibercept carried an expression cassette of a codon-optimized version of the aflibercept cDNA under the control of a ubiquitous chimeric promoter (FIG. 1A) (*See* WO2018170473A1). AAV2.7m8-aflibercept was manufactured using a baculovirus expression vector system in Sf9 cells where two different baculoviruses were used, one encoding the genes for AAV2 Rep and AAV2.7m8 Cap proteins, and the other encoding the human aflibercept cDNA expression cassette.

AAV2.7m8-aflibercept was supplied as a sterile-filtered, frozen suspension in a sterile, ready-to-use 0.5 mL Crystal Zenith vial which contained 0.25 mL of IMP, formulated as shown in **Table 1.**

**Table 1: Formulation of the AAV2.7m8-aflibercept investigational medicinal product for Cohort 1.**

| **Formulation (pH 7.3)** | | |
|---|---|---|
| **Components** | **Concentration** | **Function** |
| AAV2.7m8-aflibercept | 6 × 10¹² vg/mL | Active ingredient |
| Sodium Chloride | 180 mM | Osmotic/ionic strength agent |
| Sodium Phosphate Monobasic | 5 mM | Buffering agent |
| Sodium Phosphate Dibasic | 5 mM | Buffering agent |
| Poloxamer 188 | 0.001% (w/v) | Surfactant |
| Water for Injection | Quantum satis (Q.S.) | Solvent |
| Injection Volume | 100 µL | |

### IV. Study Design

### A. Dose and Method of Administration

### Cohort 1

Subjects in Cohort 1 were administered a single IVT injection of AAV2.7m8-aflibercept at Dose 1 of 6 × 10¹¹vg/eye in an injection volume of 100 µL.

AAV2.7m8-aflibercept vials were removed from the frozen storage at ≤ -60°C and thawed at room temperature. AAV2.7m8-aflibercept was administered via IVT injection. Aseptic technique with povidone-iodine was employed, along with topical or subconjunctival anesthesia. Post-injection care and medication regimen were given based on institutional standard of care.

### B. Study Visits

### Screening (Days -15 to -7)

As shown in **FIG. 1B****,** subjects received a single IVT injection of aflibercept 2 mg during Screening on Days -15 to -7 (e.g., Days -14- to -7), consistent with standard of care. Subjects received routine institutional standard post-injection care.

### Study Day 1

On Study Day 1 (between 7 and 15 days, e.g., between 7 and 14 days, after the IVT injection of aflibercept), study subjects underwent SD-OCT studies to confirm that they were responsive to anti-VEGF therapy, prior to dosing with AAV2.7m8-aflibercept. Anti-VEGF responsiveness was confirmed by the Investigator. Only subjects confirmed to have a meaningful anti-VEGF response, as described above (*See* Inclusion Criteria), were eligible to enroll in this study.

Subjects responsive to anti-VEGF therapy were sequentially enrolled into the study cohort and received a single AAV2.7m8-aflibercept IVT injection in the study eye. As described above, only one eye was selected as the study eye for the duration of the study.

### Post-AAV2.7m8-aflibercept Administration

Subjects returned on Days 3 and 8, during Weeks 2, 4, 6, and 8, and every 4 weeks thereafter (*i.e.,* weeks 12, 16, 20, and 24) for clinical evaluation and treatment (if necessary). A safety and efficacy analysis of Cohort 1 was performed at week 24.

Starting on Week 4, subjects were eligible to receive rescue injections of aflibercept 2 mg IVT if there was evidence of increased disease activity according to the retreatment criteria (see below). The presence of any one of the following warranted resumption of standard anti-VEGF treatment with 2 mg IVT aflibercept:
- Loss of ≥ 10 letters in BCVA (using the ETDRS protocol) from Baseline and intraretinal or subretinal fluid observed by SD-OCT and judged by the Investigator to be the cause of the BCVA loss.
- An increase in central subfield thickness > 75 µm from Baseline as assessed by SD-OCT.
- Presence of vision-threatening hemorrhage due to macular degeneration.

Subjects are evaluated again on week 52 following administration of AAV2.7m8-aflibercept for safety and efficacy. The follow up period continues until week 104 following administration of AAV2.7m8-aflibercept.

### C. Corticosteroid Regimen

To reduce the possible risk of post-injection ocular inflammation, subjects were administered a prophylactic corticosteroid regimen (e.g., prednisone) and monitored closely for ocular and systemic tolerability of the vector.

Subjects in Cohort 1 were administered a prophylactic 13-day oral corticosteroid regimen, starting with 60 mg/day of prednisone from 3 days before (day -3) to 3 days after AAV2.7m8-aflibercept treatment for a total of 6 days. This was followed by a 7-day prednisone taper. A summary of the oral prednisone regimen is provided in **Table 2.**

**Table 2: Oral prednisone regimen.**

| **Study Days** | **Total Number of Days** | **Prednisone Dose per Day** |
|---|---|---|
| Days -3 to +3 | 6 | 60 mg |
| Days 4-6 | 3 | 40 mg |
| Days 7-8 | 2 | 20 mg |
| Days 9-10 | 2 | 10 mg |
| Day 11 | 0 | STOP |

Initiating immunosuppression (*i.e.,* prednisone) prior to AAV2.7m8-aflibercept IVT injection was designed to limit the immune response upon exposure to capsid antigens. Subjects self-administered prednisone for the 13-day regimen.

Subjects received topical or oral corticosteroids (prednisone) as needed during weeks 2-24 of the study.

### D. Prohibited Medications and Treatments

The following medications were prohibited during the study:
- Any systemic anti-VEGF agent including bevacizumab.
- Any anti-VEGF agent in the study eye other than the study drug or aflibercept injection 2 mg, according to the rescue anti-VEGF injection criteria in this study.
- IVT steroids in the study eye.
- Immune suppression drugs. Systemic, inhaled or topical steroids and NSAIDs were allowed.
- Use of and participation in any other investigational studies.
- Cataract Surgery in the study eye could be performed if clinically indicated and was scheduled > 90 days after IVT administration and/or >7 days after the last injection of aflibercept.
- Subjects who developed AMD in the non-study eye could receive standard of care therapy in the non-study eye.

### E. Summary of Study Design for Cohort 1

Six subjects were enrolled into Cohort 1.

Subjects in Cohort 1 were administered a single IVT injection of AAV2.7m8-aflibercept at Dose 1 of 6 × 10¹¹vg/eye. The first (sentinel) subject enrolled in Cohort 1 received an IVT injection of AAV2.7m8-aflibercept and was evaluated for 29 days prior to dosing the subsequent 5 subjects (Subjects 2-6) within the cohort.

A summary of the study design for Cohort 1 is provided in **Table 3.**

**Table 3: Summary of study design for Cohort 1.**

| **Cohort** | **AAV2.7m8-aflibercept Dose** | **Corticosteroid Regimen** |
|---|---|---|
| Cohort 1 | 6 × 10¹¹ vg/eye | Prophylactic oral prednisone regimen. |

### F. Study Duration

Duration of subject participation in the study is approximately 108 weeks for each subject. This includes a screening period of 4 weeks and an additional 104-week study period.

Upon completion or discontinuation of the study, if appropriate, subjects are offered the opportunity to enroll in a long-term follow-up study to further assess the safety of this gene therapy.

### V. Study Assessments

### A. General Physical Examination and Vital Signs

Each subject's relevant medical and ophthalmic history was collected and recorded. A general physical examination consisted of height (at Screening only), body weight, and vital signs.

Vital signs consisted of blood pressure, pulse rate measurements, body temperature, and respiratory rate. A 12-lead electrocardiogram (ECG) was taken for each subject. The following clinical laboratory and antibody tests were performed: chemistry, complete blood count (CBC), coagulation studies, urinalysis, serological evidence of HIV or Hepatitis, and pregnancy testing.

At the Week 104, end of study (EOS), and/or Early Termination visit, a physical examination assesses if any changes in the subject's physical condition occurred since the Screening examination.

### B. Immune Response and Aflibercept Expression

Total anti-AAV2.7m8 antibodies were measured. Neutralizing anti-AAV2.7m8 antibodies in subject's serum were determined using a reporter gene-based transduction interference assay assessed by cutpoint.

The humoral immune response against anti-aflibercept antibodies was measured in serum using an ELISA-based cutpoint antibody assay.

Serum was collected to determine the presence of aflibercept protein.

Cellular immunity against AAV2.7m8 capsid proteins and aflibercept protein were measured using an ELISPOT assay.

### C. Full Ophthalmic Examination and Other Assessment Methods

Study assessments included an ophthalmologic exam, intraocular pressure (IOP), and indirect ophthalmoscopy.

The ophthalmic examination consisted of an external examination of the eye and adnexa, routine screening for eyelid/pupil responsiveness (including but not limited to blepharoptosis, abnormal pupil shape, unequal pupils, abnormal reaction to light, and afferent pupillary defect), and slit-lamp examination (eyelids, conjunctiva, cornea, lens, iris, anterior chamber). The slit-lamp examination examined the anterior ocular structures and was used for grading any findings. If any finding was noted during the slit-lamp examination, at any visit, the severity was graded by the Investigator and the finding was described as clinically significant or not clinically significant.

The IOP measurements were performed using a Goldmann applanation tonometer or Tono-pen^{™}. IOP measurements were performed prior to any IVT injection and prior to dilating eyes. Day 1 visit required pre-injection and post-injection (30 minutes after injection) IOP measurements.

The dilated indirect ophthalmoscopy examination included an evaluation of posterior segment abnormalities of the vitreous, optic nerve, peripheral retina, and retinal vasculature. If any finding was noted during the ophthalmoscopy, at any visit, the severity was graded by the Investigator and the finding was described as clinically significant or not clinically significant. Day 1 visit required pre-injection and post-injection indirect ophthalmoscopy assessments.

### Spectral Domain Optical Coherence Tomography (SD-OCT)

SD-OCT was used to obtain depth-resolved tissue structure information encoded in the magnitude and delay of the back-scattered light by spectral analysis of the interference fringe pattern.

### Fluorescein Angiography

Fluorescein angiography images were used to confirm patient eligibility for study enrollment, to assess the efficacy of CNV lesion growth, and to evaluate leakage compared to Baseline.

### Digital Color Fundus Photography

Color fundus images of the retina, optic disc, and macula were taken.

### Optical Coherence Tomography Angiography (OCT-A)

OCT-A imaging (swept-source or spectral-domain) was used to obtain volumetric, three-dimensional maps of the retina and choroid as well as information on blood flow.

### Refraction and Visual Acuity

Refraction and BCVA were measured by a trained and certified visual acuity examiner at the study sites. Visual acuity was measured at a starting distance of 4 meters, prior to dilating eyes.

### D. Safety Assessments

To mitigate the risks associated with the IVT administration of AAV2.7m8-aflibercept, subjects were closely monitored on the day of the IVT AAV2.7m8-aflibercept administration and thereafter post-treatment.

The safety of AAV2.7m8-aflibercept was assessed through the collection of AEs, vital signs, physical and eye examinations, ECG, pregnancy testing, and laboratory evaluations.

Intense monitoring of subjects was done in the first 8 weeks of the study, followed by regular safety assessments for safety and efficacy thereafter. All subjects had their visual acuity tested by Early Treatment Diabetic Retinopathy Study (ETDRS) letters assessments at each study visit, and standard of care aflibercept IVT injections were used as a rescue treatment.

The severity, or intensity, of an AE was rated on the following scale:
- Mild: the AE was noticeable but did not significantly impair the subject's daily activities.
- Moderate: the AE reduced or impaired normal daily activity but was not incapacitating.
- Severe: the AE was incapacitating and resulted in an inability to perform normal daily activity.

Safety is assessed over 104 weeks post-administration of study treatment. Upon completion of the End of Study (EOS) Visit, subjects are offered enrollment in a long-term extension study to further assess the safety and durability of transgene expression.

### E. Efficacy Assessments

The efficacy of AAV2.7m8-aflibercept in the treatment of wAMD was assessed by the following measures. A key assessment time point was at 24 weeks. The baseline values of BCVA and SD-OCT refer to the values taken during the screening visit on Days -15 to -7 (e.g., Days -14 to -7) prior to aflibercept injection. The baseline values were used to compare for analysis.

Vision was assessed primarily through the BCVA expressed as an ETDRS score (number of letters correctly read) (Vitale et al., (2016) JAMA Ophtalmol 134(9):1041-1047). Maintenance of vision was classified if the subject lost fewer than 15 letters in the ETDRS score compared to Baseline. Calculated endpoints included the mean change from Baseline, the percent gaining at least 15 letters compared to Baseline, and the percent losing 15 or more letters compared to Baseline.

FA was performed to assess CNV lesions using a standard technique to evaluate leakage compared to Baseline.

SD-OCT was performed using approved equipment and standard techniques to evaluate retinal thickness (e.g., central retinal thickness or central subfield thickness), macular volume, and the presence of fluid (e.g., subretinal fluid and intraretinal fluid) compared to baseline values.

The number of aflibercept injections given post AAV2.7m8-aflibercept treatment per subject, over time, from Week 4 to Week 104 were determined. In addition, the time to the first aflibercept injection post treatment with AAV2.7m8-aflibercept and the proportion of subjects who did not require an aflibercept rescue treatment were determined.

The proportion of subjects without IRF over time from week 4 to week 104 was determined.

The proportion of subjects without SRF over time from week 4 to week 104 was determined.

The proportion of subjects without a PED over time from week 4 to week 104 among subjects with a PED at baseline was determined.

### F. Statistics

The Safety population included all subjects who received AAV2.7m8-aflibercept and were analyzed according to the dose received.

All other safety parameters were summarized by cohort. AEs were coded using the Medical Dictionary for Regulatory Activities (MedDRA, version 21) classification to give a preferred term (PT) and system organ class (SOC) for each event. SAEs and AEs leading to study withdrawal were listed separately.

Efficacy analyses included all subjects. Efficacy analysis endpoints were evaluated, and descriptive statistics will be calculated by cohort. The key assessment time point was at 24 weeks. Efficacy was assessed according to dose received and in aggregate.

### VI. Results

### A. Subject Characteristics

All six subjects enrolled in Cohort 1 were diagnosed wAMD. At the time of enrollment, subjects had a high requirement for anti-VEGF treatment (e.g., required frequent anti-VEGF treatment), functional vision around 20/50, some excess central subfield thickness on OCT, and were undergoing regular IVT injections of anti-VEGF treatment and responding to therapy. Disease characteristics and treatment histories for all subjects are provided in **Tables 4-5.**

As shown in **Table 4,** subjects in Cohort 1 were diagnosed with wAMD between approximately one year (Subject 5) to approximately a decade or more (Subject 4) prior to being administered AAV2.7m8-aflibercept. Subjects had received a wide range of prior anti-VEGF IVT injections in the study eye, from 7 (Subject 5) to 109 (Subject 4) prior injections. The calculated average anti-VEGF IVT injection interval prior to this study ranged from every 4 weeks to every 10 weeks. All subjects received 2 or 3 anti-VEGF injections in the four months prior to screening for enrollment in this study. Subjects were administered AAV2.7m8-aflibercept 7 days (Subjects 1-3) or 14 days (Subjects 4-6) after the Screening anti-VEGF injection prior to the start of the study.

**Table 4: Baseline characteristics of subjects in Cohort 1.**

| **Characteristic** | **Value** |
|---|---|
| Mean age, years | 79.0 |
| Mean time since nAMD diagnosis, years | 3.3 |
| Mean number anti-VEGF injections since initial diagnosis (range) | 35.3 (7-109) |
| Mean number of anti-VEGF injections in 4 months prior to screening | 2.8 |
| Mean number anti-VEGF injections in 8 months prior to screening | 6.2 |
| Mean number anti-VEGF injections in 12 months prior to screening | 9.2 |
| Average annualized injection frequency | 9.3 |
| Baseline BCVA in study eye (ETDRS letters), mean | 65.8 |
| Approximate Snellen equivalent | 20/50 |
| Baseline mean CST study eye, µm | 369.2 |

**Table 5: Disease characteristics and treatment histories of study subjects in Cohort 1.**

| | **Subject 1** | **Subject 2** | **Subject 3** | **Subject 4** | **Subject 5** | **Subject 6** |
|---|---|---|---|---|---|---|
| Age | 76 years | 83 years | 87 years | 62 years | 88 years | 77 years |
| Gender | Male | Male | Male | Male | Male | Female |
| Time between neovascular AMD diagnosis and AAV2.7m8-aflibercept IVT injection: | 2.3 years | 1.1 years | 1.2 years | 11.0 years | 1.0 years | 7.0 years |
| Number of prior anti-VEGF injections in study eye: | 18 | 9 | 29 | 109 | 7 | 8 |
| Calculated anti-VEGF injection interval average pre-study: | 5-7 weeks | 4-10 weeks | 4-7 weeks | 4-6 weeks | 4-7 weeks | 4-6 weeks |
| Number of anti-VEGF injections received in 4 months prior to screening: | 3 | 2 | 3 | 3 | 2 | 4 |
| Number of anti-VEGF injections received in 8 months prior to screening: | 5 | 6 | 6 | 6 | 6 | 8 |
| Average annualized injection frequency | 7.5 | 9 | 9 | 9 | 9 | 12 |
| Days between Screening aflibercept IVT injection and AAV2.7m8-aflibercept IVT injection: | 7 days | 7 days | 7 days | 14 days | 14 days | 10 days |

### B. Safety

During the 24 weeks following administration of AAV2.7m8-aflibercept, no SAEs occurred, nor were there any AEs that met the criteria for dose limiting toxicity (DLT). No drug-related non-ocular AEs were observed. Ocular inflammation was observed in all subjects, and was manageable with topical steroids. In addition, no vasculitis, retinitis, or choroiditis were observed. Nineteen ocular AEs were observed that were deemed potentially related to AAV2.7m8-aflibercept; 14 were mild, and 5 were moderate (2 AEs were intermediate uveitis, 1 AE was vitreous cells, 2 AEs were anterior chamber cells). One patient had two anterior chamber cell events (one mild event and one moderate event). Mild-to-moderate intraocular inflammation, responsive to topical or oral corticosteroids, was frequently observed during early follow-up. OCT images showed resolution of persistent fluid in most subjects, with no signs of worsening. Visual acuity was generally stable. A summary of all safety events that were related to AAV2.7m8-aflibercept through 24 weeks is provided in **Table 6.**

**Table 6: All safety events related to AAV2.7m8-aflibercept.**

| **AAV2.7m8-aflibercept-related ocular AEs** | **No. of Patients, n** | **Number of Events, n** | | | | | | |
|---|---|---|---|---|---|---|---|---|
| | | Patient 1 | Patient 2 | Patient 3 | Patient 4 | Patient 5 | Patient 6 | Total |
| Anterior Chamber Cells | 3 | 0 | 1 | 0 | 1* | 0 | 2^{‡} | 4 |
| AC Flare | 3 | 1 | 0 | 2 | 0 | 1 | 0 | 4 |
| Vitreous Cells | 3 | 0 | 1* | 1 | 0 | 0 | 1 | 3 |
| Intermediate Uveitis | 2 | 0 | 1* | 0 | 0 | 0 | 1* | 2 |
| Keratic Precipitates | 1 | 0 | 0 | 2 | 0 | 0 | 0 | 2 |
| Poor Pupil Dilation | 1 | 0 | 0 | 0 | 0 | 1 | 0 | 1 |
| Ocular Floaters | 1 | 0 | 1 | 0 | 0 | 0 | 0 | 1 |
| Vitreous Debris | 1 | 0 | 0 | 0 | 0 | 0 | 1 | 1 |
| Vitreous Haze | 1 | 0 | 0 | 1 | 0 | 0 | 0 | 1 |
| * Moderate Event; All events were either mild (14) or moderate (5) in intensity. ^{‡} Patient had two anterior chamber cell events (one mild event and one moderate event). | | | | | | | | |

All subjects received 60 mg of oral prednisone for 6 days starting at day -3, followed by a 7-day tapering course of prednisone. Clinical assessment of ocular cellular inflammation revealed that no clinically significant inflammation occurred early post-AA V2.7m8-aflibercept. In addition, as shown in **FIG. 6****,** no worsening or new inflammation was observed when subjects were receiving steroid eye drops. By week 24, anterior chamber cellular inflammation was resolved or improving. Observed cellular inflammation was generally mild. The aqueous cell count categories shown in **FIG. 6** were based on the Standardization of Uveitis Nomenclature (SUN) criteria (Jabs, DA et al., J Ophthalmol. 2005;140:509-516), whereas vitreous cell count categories were based on National Institutes of Health (NIH) guidelines.

Overall, safety assessments showed that AAV2.7m8-aflibercept was well tolerated, with no DLTs or SAEs reported. All safety events were mild to moderate, and most were inflammation-related. Continued follow-up of AEs showed that mean visual acuity remained stable and no anti-VEGF rescue injections were required. Thus, the results indicate that AAV2.7m8-aflibercept administered as a single IVT injection at a dose of 6×10¹¹ vg/eye in wAMD patients previously requiring frequent anti-VEGF injections has an acceptable safety profile.

### C. Efficacy

Following AAV2.7m8-aflibercept administration, all six subjects showed stabilization of disease activity based on OCT assessments. As shown in **FIGS. 2A-2L****,** OCT images taken prior to and after treatment with AAV2.7m8-aflibercept indicated that a robust anatomical response was evident in all six subjects in Cohort 1. For example, subretinal fluid persisted with standard of care anti-VEGF treatment, but resolved and remained resolved following administration of AAV2.7m8-aflibercept in Subject 1 (*e.g.,* compare **FIGS. 2A** and **2B****),** Subject 2 (*e.g*., compare **FIGS. 2C** and **2D****),** Subject 3 (*e.g.,* compare **FIGS. 2E** and **2F****),** Subject 4 (*e.g.,* compare **FIGS. 2G** and **2H****),** and Subject 5 (*e.g.,* compare **FIGS. 21** and **2J****).** Subject 6 exhibited retinal morphology consistent with polypoidal choroidal vasculopathy (PCV) **(****FIG. 2K****),** and while some fluid persisted after treatment with AAV2.7m8-aflibercept, there was no evidence of disease progression **(****FIG. 2L****).** In addition, as shown in **FIG. 3****,** none of the subjects exhibited an increase in central subfield thickness (CST) and a mean decrease of -52.7 µm was observed (90% CI -86.5, -18.8). Increased CST is indicative of disease progression in wAMD. Importantly, none of the subjects were administered or required any standard of care aflibercept rescue injections ("rescue injections").

Best corrected visual acuity (BCVA) was measured based on ETDRS letters assessments in all subjects throughout the study. As shown in **FIG. 4****,** BCVA was stable across subjects in Cohort 1 with a mean decrease of -2 letters (90% CI -9.1, 5.1).

At a median follow up time of 34 weeks after administration of AAV2.7m8-aflibercept, no subjects exhibited signs of disease reactivation on OCT imaging **(****FIGS. 7A-7B****).** In addition, no subjects required any rescue anti-VEGF IVT injections and no subjects met retreatment criteria at any point during the follow-up period of up to 44 weeks. Finally, during the additional follow-up period beyond 24 weeks, BCVA was maintained (i.e., no patient lost or gained greater than 10 ETDRS letters), the anatomic improvements (*i.e.,* resolution of subretinal and intraretinal fluid, and reductions in CST) observed at week 24 were maintained, and no safety concerns arose.

A summary of safety and efficacy results evaluated at the median follow up time of 34 weeks is provided in **Table 7.** A summary of efficacy results evaluated at the median follow up time of 44 weeks is provided in **Table 8.** One patient experienced a macula-off retinal detachment unrelated to study treatment. For that patient, the last observations prior to detachment were used to calculate CST and BCVA mean values and ranges shown in **Table 8.**

**Table 7: Summary of safety and efficacy results at median follow up time of 34 weeks.**

| | **Value** |
|---|---|
| Median follow-up, weeks | 34.0 |
| Follow-up (min, max), weeks | 28, 44 |
| Grade 3 adverse events, n | 0 |
| Serious adverse events, n | 0 |
| Dose-limiting toxicities, n | 0 |
| Mean BCVA change from baseline, ETDRS letters | -1.5 |
| BCVA change from baseline (min, max), ETDRS letters | -9, +5 |
| Total number of rescue injections, n | 0 |

**Table 8: Summary of efficacy results at median follow up time of 44 weeks.**

| | **Value** |
|---|---|
| Mean BCVA change from baseline, ETDRS letters | -1.8 |
| BCVA change from baseline (min, max), ETDRS letters | -7, +7 |
| Mean CST change from baseline, µm | -18.5 |
| CST change from baseline (min, max), µm | -75, +32 |
| Total number of rescue injections, n | 0 |

A summary of an additional analysis of efficacy results at the median follow up time of 44 weeks (range of 40-52 weeks) after administration of AAV2.7m8-aflibercept is provided in **Table 9.**

**Table 9: Summary of efficacy results at the follow up time of 40-52 weeks (median 44 weeks).**

| | **Value** |
|---|---|
| Mean BCVA change from baseline, ETDRS letters | -1.0 |
| BCVA change from baseline (min, max), ETDRS letters | -7, +7 |
| Mean CST change from baseline, µm | -25.5 |
| CST change from baseline (min, max), µm | -117, +32 |
| Total number of rescue injections, n | 0 |

Cellular inflammatory events and steroid treatments given to each subject were analyzed up to the follow-up time of 52 weeks. As shown in **FIG. 8****,** Subjects 1 and 5 had no cellular reactions beyond day 3. Both subjects started topical steroids in the second 6 months of the study for aqueous cell (AC) flare and posterior synechiae. Both subjects received topical steroids and mydriatic eye drops, and Subject 1 has stopped receiving topical steroid eye drops. Subjects 2 and 6 exhibited early onset inflammation by the first month after administration of AAV2.7m8-aflibercept, with an AC response and some spillover to vitreous cells. The inflammation in both subjects resolved following treatment with topical steroids. Both subjects continued twice daily eye drops (BID). Subjects 3 and 4 experienced more delayed cellular inflammation onset, after topical steroids were discontinued (by months 2-3 after administration of AAV2.7m8-aflibercept). The inflammation in these subjects was responsive to topical steroids. Subject 3 has stopped receiving topical steroid eye drops. Subject 4 experienced an unrelated retinal detachment and is receiving BID eye drops.

Overall, inflammation was low grade, with no early clinically significant inflammation reported. All inflammation was responsive to topical steroids, with a trend towards resolution over time. Inflammation was primarily driven an aqueous cell process.

**FIGS. 9A-9B** show OCT imaging of Subjects 1-6 through follow-up weeks 40 to 52. No subjects exhibited signs of disease reactivation on OCT imaging, and BCVA and anatomic improvements were maintained. In addition, no subjects required any rescue anti-VEGF IVT injections or met retreatment criteria.

### D. Conclusions

The current standard of care therapy for wAMD is anti-VEGF IVT injections that are typically required long term, about every 4-8 weeks. Compliance with this regimen can be difficult for patients, caregivers, and healthcare systems, leading to suboptimal dosing and loss of vision due to undertreatment.

The results presented in this example show that AAV2.7m8-aflibercept provided a clear benefit for improving retinal anatomy and stabilizing vision, while exhibiting an acceptable safety profile. Specifically, patients maintained vision during the study, and AAV2.7m8-aflibercept was shown to be safe and well tolerated, with observed inflammation generally being mild and responsive to steroid eye drops.

It was noted that subjects in Cohort 1 of this study previously required frequent anti-VEGF injections **(Table 4)** in order to slow or prevent progression of their disease, however no anti-VEGF rescue injections were required during this study following administration of AAV2.7m8-aflibercept.

It was noted that subretinal fluid was present on OCT scans of subjects undergoing standard of care anti-VEGF treatment for more than 20 weeks, and continued 1-2 weeks after the Screening aflibercept IVT injection. This fluid was present even though subjects had been treated repeatedly and was thus refractory to standard of care IVT bolus of anti-VEGF protein. Unexpectedly, this refractory SRF resolved after treatment with AAV2.7m8-aflibercept, a result which would not have been predicted from preclinical studies.

The observed safety and efficacy of AAV2.7m8-aflibercept administered at a dose of 6×10¹¹ vg/eye led to the continuation of this Phase 1 study to assess the safety and efficacy of AAV2.7m8-aflibercept administered at lower doses and with topical corticosteroids, as described in Example 2.

### Example 2: An open label Phase 1 study of AAV2.7m8-aflibercept in neovascular (wet) age-related macular degeneration at doses lower than 6 × 10¹¹vg/eye and with topical corticosteroids.

The following example describes a continuation of the Phase 1 study described in Example 1 to assess the safety and efficacy of AAV2.7m8-aflibercept administered at doses lower than 6×10¹¹ vg/eye and with topical corticosteroids in subjects with wAMD.

### I. Study Objectives and Endpoints

The primary objective, secondary objectives, and primary and secondary endpoints are as described in Section I of Example 1.

### II. Study Subjects

The study subjects in this study are as described in Section II of Example 1.

### III. Investigational Medicinal Product

The investigational medicinal product is AAV2.7m8-aflibercept, as described in detail in Section III of Example 1, and as depicted in **FIG. 1A****,** with the exception that the concentration of AAV2.7m8-aflibercept was varied in order to maintain a suitable injection volume as described in **Table 10,** below.

**Table 10: Formulation of the AAV2.7m8-aflibercept investigational medicinal product for cohorts 2-4.**

| | **AAV2.7m8-aflibercept Concentration** | **Injection Volume** |
|---|---|---|
| Cohort 2 | 6 × 10¹² vg/mL | 30 µL |
| Cohort 3 | 6 × 10¹² vg/mL | 30 µL |
| Cohort 4a | 6 × 10¹² vg/mL | 100 µL |
| Cohort 4b | 11 6 × 10¹¹ vg/mL | 100 µL |

### IV. Study Design

### A. Dose and Method of Administration

AAV2.7m8-aflibercept at the doses described below was administered as described in Section IV of Example 1.

### Study Cohorts 2-4

**Cohort 2:** Six subjects who are diagnosed with wAMD are enrolled into Cohort 2. Subjects in Cohort 2 are administered a single IVT injection of AAV2.7m8-aflibercept at Dose 2 of 2 × 10¹¹vg/eye with a prophylactic oral prednisone regimen.

**Cohort 3:** Nine subjects who are diagnosed with wAMD are enrolled into Cohort 3. Subjects in Cohort 3 are administered a single IVT injection of AAV2.7m8-aflibercept at Dose 2 of 2 × 10¹¹vg/eye with a prophylactic topical corticosteroid regimen.

**Cohort 4:** Nine subjects who are diagnosed with wAMD are enrolled into Cohort 4. If signs of choroidal neovascularization exudation requiring rescue therapy are observed in a majority of subjects in Cohorts 2 and 3, the subjects in Cohort 4 are administered a single IVT injection of AAV2.7m8-aflibercept at Dose 3 of 6×10¹⁰ vg/eye with a topical corticosteroid regimen (Cohort 4b). If signs of choroidal neovascularization exudation requiring rescue therapy are not observed in a majority of subjects in Cohorts 2 and 3, the subjects in Cohort 4 are administered a single IVT injection of AAV2.7m8-aflibercept at Dose 1 of 6×10¹¹ vg/eye with a topical corticosteroid regimen (Cohort 4a).

### B. Study Visits

The study visits are as described in Section IV of Example 1 and **FIG. 1B****.**

### C. Corticosteroid Regimen

Subjects in Cohort 2 are administered a prophylactic 13-day oral corticosteroid regimen as described in detail for Cohort in Example 1:

**Cohort 2:** Subjects in Cohort 2 are administered a single IVT injection of AAV2.7m8-aflibercept at Dose 2 of 2 × 10¹¹vg/eye with a prophylactic 13-day oral corticosteroid regimen, starting with 60 mg of prednisone 3 days before and 3 days after AAV2.7m8-aflibercept treatment for a total of 6 days. This is followed by a 7-day prednisone taper. A summary of the oral prednisone regimen for Cohort 2 is provided in **Table 2** of Example 1.

Subjects in Cohorts 3 and 4 are administered a prophylactic tapering regimen of topical corticosteroid (difluprednate 0.05% drops) as described below:

**Cohort 3:** Subjects in Cohort 3 are administered a single IVT injection of AAV2.7m8-aflibercept at Dose 2 of 2 × 10¹¹vg/eye with a prophylactic topical 4-week tapering corticosteroid regimen.

**Cohort 4**: Subjects in Cohort 4b are administered a single IVT injection of AAV2.7m8-aflibercept at Dose 3 of 6×10¹⁰ vg/eye with a prophylactic topical 4-week tapering corticosteroid regimen that can be extended at the discretion of the treating physician. Subjects in Cohort 4a are administered a single IVT injection of AAV2.7m8-aflibercept at Dose 3 of 6×10¹¹ vg/eye with a prophylactic topical 4-week tapering difluprednate regimen that can be extended at the discretion of the treating physician.

A summary of the topical difluprednate regimen is provided in **Table 11A,** below.

**Table 11A: Summary of topical difluprednate regimen.**

| **Study Weeks** | **Difluprednate administrations per day** |
|---|---|
| Week 1 | 4 times |
| Week 2 | 3 times |
| Week 3 | 2 times |
| Week 4 | 1 time |
| The difluprednate regimen can be extended at the discretion of the treating physician. | |

Subjects in Cohorts 3 and 4 may also be administered a 6-week prophylactic corticosteroid regimen that can be extended at the discretion of the treating physician. A summary of the 6-week prophylactic corticosteroid regimen is provided in **Table 11B.**

**Table 11B. Summary of the 6-week topical difluprednate regimen.**

| **Study Days** | **Total Number of Days** | **Difluprednate administrations per day** |
|---|---|---|
| Days 1 to 21 | 21 | 4 times |
| Days 22 to 28 | 7 | 3 times |
| Days 29 to 35 | 7 | 2 times |
| Days 36 to 42 | 7 | 1 time |
| Day 43 and beyond | 0 | The difluprednate regimen can be extended at the discretion of the treating physician. |

Initiating immunosuppression immediately after exposure to AAV2.7m8-aflibercept IVT injection is designed to limit the immune response upon exposure to capsid antigens. Subjects self-administer one drop of difluprednate 0.05% solution (2.5 µg difluprednate) to the conjunctival sac of the treated eye 4 times per day for the first week beginning on the day of injection of AAV2.7m8-aflibercept (Day 1, post-injection). This is followed by 3 times per day for second week, 2 times per day for the third week, and 1 time per day for the fourth week. The tapering corticosteroid regimen may be extended at the discretion of the treating physician.

### D. Prohibited Medications and Treatments

The prohibited medications and treatments for this study are as described in Section IV of Example 1.

### E. Summary of Study Design for Cohorts 2-4

A summary of the study design for Cohorts 2-4 is provided in **Table 12.**

**Table 12: Summary of study design for Cohorts 2-4.**

| **Cohort** | **AAV2.7m8-aflibercept Dose** | **Corticosteroid Regimen** |
|---|---|---|
| Cohort 2 | 2 × 10¹¹ vg/eye | Prophylactic oral prednisone regimen. |
| Cohort 3 | 2 × 10¹¹ vg/eye | Prophylactic topical corticosteroid. |
| Cohort 4a | 6 × 10¹¹ vg/eye | Prophylactic topical corticosteroid. |
| Cohort 4b | 6 × 10¹⁰ vg/eye | Prophylactic topical corticosteroid. |

### F. Study Duration

The duration of subject participation in this study is as described in Section IV of Example 1.

### V. Study Assessments

Assessments performed in this study are as described in Section V of Example 1. In addition, samples are taken of aqueous and vitreous humors.

### Aqueous Humor Sampling

Aqueous humor samples are obtained at screening (prior to aflibercept injection) and on Day 1 (prior to AAV2.7m8-aflibercept injection), Weeks 12, 24, 36, 52, 76, 88, 104, and at any visit requiring the first aflibercept (Eylea) rescue treatment, or early termination. Aqueous humor samples are analyzed for aflibercept, VEGF-A, and NAb concentrations.

### Vitreous Humor Sampling

Vitreous humor samples are obtained at any point during the study when a vitrectomy is performed. Vitreous humor samples are analyzed primarily for aflibercept concentrations. Remaining samples are analyzed for VEGF-A concentrations.

### VI. Results

### A. Subject Characteristics

Disease characteristics and treatment histories for all subjects are recorded. In particular, the date or year of neovascular AMD diagnosis, the number of prior anti-VEGF injections in the study eye, the calculated anti-VEGF injection interval average pre-study, the number of anti-VEGF injections received in the 4 months prior to screening, the date of Screening aflibercept IVT dose, and the date of AAV2.7m8-aflibercept injection are recorded.

### B. Safety

Subjects are closely monitored on the day of the IVT AAV2.7m8-aflibercept administration and thereafter post-treatment.

The safety of AAV2.7m8-aflibercept is assessed through the collection of vital signs, physical and eye examinations, ECG, pregnancy testing, and laboratory evaluations.

The incidence, severity, and relationship to treatment of AEs, SAEs, and DLTs are assessed in all subjects.

The occurrence, severity, and relationship to treatment of intraocular inflammation is assessed in all subjects.

Visual acuity is assessed in all subjects through BCVA expressed as an ETDRS score (number of letters correctly read) (Vitale et al., (2016) JAMA Ophtalmol 134(9):1041-1047).

The requirement for anti-VEGF rescue injections is monitored in all subjects.

The existence of a correlation between preexisting anti-AAV NAb-levels in subject's sera and AEs, SAEs, and DLTs is determined.

The effect of steroid prophylaxis (topical or oral) on the occurrence and resolution of AEs, SAEs, DLTs, and intraocular inflammation is assessed.

Standard of care aflibercept IVT injections are used as a rescue treatment.

### C. Efficacy Assessments

Disease stabilization is assessed in all subjects. Retinal thickness, central subfield thickness, fluid and other anatomical features are measured (e.g., by OCT imaging) prior to and after administration of study treatment.

The efficacy of AAV2.7m8-aflibercept in the treatment of wAMD is assessed by the following measures. A key assessment time point is at 24 weeks. The baseline values of BCVA and SD-OCT refer to the values taken during the screening visit on Days -15 to -7 (e.g., Days -14 to -7) prior to aflibercept injection. The baseline values are used to compare for analysis.

Vision is assessed primarily through BCVA expressed as an ETDRS score (number of letters correctly read) (Vitale et al., (2016) JAMA Ophtalmol 134(9):1041-1047). Maintenance of vision is classified if the subject loses fewer than 15 letters in the ETDRS score compared to Baseline. Calculated endpoints include the mean change from Baseline, the percent gaining at least 15 letters compared to Baseline, and the percent losing 15 or more letters compared to Baseline.

FA is performed to assess CNV lesions using a standard technique to evaluate leakage compared to Baseline.

SD-OCT is performed using approved equipment and standard techniques to evaluate retinal thickness (e.g., central retinal thickness or central subfield thickness), macular volume, and the presence of fluid (e.g., subretinal fluid and intraretinal fluid) and fluid compared to Baseline values.

The number of aflibercept injections given post AAV2.7m8-aflibercept treatment per subject, over time, from Week 4 to Week 104 are determined. In addition, the time to the first aflibercept injection post treatment with AAV2.7m8-aflibercept and the proportion of subjects who did not require an aflibercept retreatment are determined.

The proportion of subjects without IRF over time from week 4 to week 104 is determined.

The proportion of subjects without SRF over time from week 4 to week 104 is determined.

The proportion of subjects without a PED over time from week 4 to week 104 among subjects with a PED at baseline is determined.

### Example 3: Results of an open label Phase 1 study of AAV2.7m8-aflibercept in neovascular (wet) age-related macular degeneration at doses lower than 6 × 10¹¹vg/eye and with topical corticosteroids.

This Example describes the results of the Phase 1 study described in Example 2 that assessed the safety and efficacy of AAV2.7m8-aflibercept administered at doses lower than 6×10¹¹ vg/eye and with topical corticosteroids in subjects with wAMD.

### Results

### Cohort 2

The baseline characteristics of subjects in Cohort 2 are provided in **Table 13.**

**Table 13. Baseline characteristics of subjects in Cohort 2.**

| **Characteristic** | **Value** |
|---|---|
| Mean age, years | 79.8 |
| Mean time since nAMD diagnosis, years | 4.0 |
| Mean number anti-VEGF injections since initial diagnosis (range) | 34.0 (4-69) |
| Mean number anti-VEGF injections in 12 months prior to screening | 9.2 |
| Baseline BCVA in study eye (ETDRS letters), mean | 64.7 |
| Approximate Snellen equivalent | 20/50 |
| Baseline mean CST study eye, µm | 307.7 |

As described in Example 2, subjects in Cohort 2 were administered a single IVT injection of AAV2.7m8-aflibercept at Dose 2 of 2 × 10¹¹vg/eye with a prophylactic 13-day oral corticosteroid regimen, starting with 60 mg of prednisone 3 days before and 3 days after AAV2.7m8-aflibercept treatment for a total of 6 days. This was followed by a 7-day prednisone taper. A summary of the oral prednisone regimen for Cohort 2 is provided in **Table 2** of Example 1.

As shown in **FIG. 10****,** 24 weeks after administration of AAV2.7m8-aflibercept, BCVA was stable across subjects in Cohort 2, with a mean decrease of -4.8 ETDRS letters. In addition, as shown in **FIG. 11****,** subjects exhibited improved central retinal thickness on OCT, with an average decrease in CST of 27.8 µm. Four out of six subjects (66%) had not received a rescue anti-VEGF injection at the follow-up time of 24 weeks.

Cellular inflammatory events and steroid treatments given for each subject were analyzed up to a follow-up time of 24 weeks. As shown in **FIG. 12****,** inflammation was responsive to topical steroids. Subjects 4 and 5 had no clinically relevant cellular inflammation (Subject 5 had <5 AC cells at week 20) and required no steroid treatments. Subjects 2 and 3 showed 1/2 + aqueous and vitreous cell grades, or between 1 and 5 cells observed in the aqueous and between 1 and 10 cells observed in the vitreous that resolved following a short course of topical steroids. Subject 1 had an early inflammatory response following cessation of oral steroids that responded quickly to steroid eye drops; the subject remained on steroid eye drops four times per day (QID). Subject 6 had an AC cellular response that worsened on BID drops and resolved on QID drops. Aqueous cell grade categories were based on the Standardization of Uveitis Nomenclature (SUN) criteria (Jabs, DA et al. J Ophthalmol. 2005;140:509-516). Vitreous cell grade categories were based on National Institutes of Health (NIH) guidelines.

Overall, inflammation was generally mild. Oral steroids did not provide long enough coverage in certain subjects. However, all inflammation events were responsive to topical steroids.

OCT imaging at 24 weeks after administration of AAV2.7m8-aflibercept showed anatomic improvements and maintenance of visual acuity (BCVA). As shown in **FIGS. 13A-13B****,** Subjects 1, 2, 4, and 6 exhibited resolution of fluid. Subject 3 received 3 rescue aflibercept injections, all for a loss of 10 BCVA letters attributable to retinal fluid. Subject 5 received 3 rescue aflibercept injections, all for a CST increase of 75 µm.

Through week 24 after administration of AAV2.7m8-aflibercept, four out of six subjects had not received any anti-VEGF rescue injections. As shown in the Swimmer's Lane Plot in **FIG. 14****,** Subjects 3 and 5 received three anti-VEGF IVT injections each during the 24-week follow-up period.

Overall, the results for Cohort 2 at 24 weeks after administration of AAV2.7m8-aflibercept show that AAV2.7m8-aflibercept has robust efficacy. In particular, mean BCVA was maintained and retinal anatomy and CST were improved in subjects not requiring rescue anti-VEGF injections. In addition, AAV2.7m8-aflibercept was safe and well tolerated, with low-grade inflammation being responsive to steroid eye drops.

### Example 4: Overview of results of an open label Phase 1 study of AAV2.7m8-aflibercept in neovascular (wet) age-related macular degeneration.

This Example provides an overview of the results of the open label Phase 1 study of AAV2.7m8-aflibercept for the treatment of age-related macular degeneration (AMD) with choroidal neovascularization described in Examples 1-3.

### Safety

Results available for Cohort 1 up to the median follow up time of 44 weeks and for Cohort 2 up to the follow up time of 24 weeks show that AAV2.7m8-aflibercept was well tolerated. Specifically, no AAV2.7m8-aflibercept or procedure-related serious adverse events have been reported. In addition, no AAV2.7m8-aflibercept-related systemic adverse events or adverse events meeting the criteria for dose-limiting toxicities have been reported. AAV2.7m8-aflibercept-related adverse events were mild (71%) or moderate (29%). Low-grade inflammation was commonly reported and was responsive to steroid eye drops. No vasculitis, retinitis, or choroiditis were reported. One serious adverse event of spontaneous, pseudophakic retinal detachment unrelated to AAV2.7m8-aflibercept was reported. The retinal detachment was surgically repaired and the subject remains under follow-up.

### Efficacy

Results available for Cohort 1 up to the median follow up time of 44 weeks and for Cohort 2 up to the follow up time of 24 weeks show that AAV2.7m8-aflibercept had robust efficacy. In Cohort 1, no subjects were administered an anti-VEGF rescue injection. In Cohort 2, four out of six subjects were not administered anti-VEGF rescue injections. Overall, in the 10 out of 12 subjects (83%) in Cohorts 1 and 2 that were not administered anti-VEGF rescue injections, the mean BCVA was maintained and mean CST was improved.

A summary of the available safety and efficacy results for Cohorts 1 and 2 is provided in **Table 14.**

**Table 14. Summary of safety and efficacy results for subjects in Cohorts 1 and 2.**

| **Results Following a Single AAV2.7m8-aflibercept Dose:** | **Cohort 1** | **Cohort 2** | |
|---|---|---|---|
| Subjects | 6 | 6 | |
| Dose of AAV2.7m8-aflibercept | Higher Dose | Lower Dose | |
| | 6 x 10¹¹ vg/eye | 2 x 10¹¹ vg/eye | |
| | | | |

| **Rescue Injections:** | | | |
|---|---|---|---|
| Follow-up | 50 weeks (median) | 24 weeks (median) | |
| Number of subjects requiring anti-VEGF rescue injections | 0/6 subjects | 2/6 subjects | |
| Total anti-VEGF rescue injections | 0 injections | 6 injections | |
| | | | |
| **Safety:** | | | |
| Follow-up | 50 weeks (median) | 24 weeks (median) | |
| Systemic adverse events | 0 | 0 | |
| Dose-limiting toxicities (DLTs) | 0 | 0 | |
| Serious adverse events (SAEs)¹ | 1 | 0 | |
| Drug/procedure related SAEs | 0 | 0 | |
| | | | |

| **Change in BCVA²:** | | Full cohort | Rescue-free subjects |
|---|---|---|---|
| Follow-up | 44 weeks (median) | 24 weeks | 24 weeks |
| Mean (ETDRS letters)³ | -1.0 | -4.8 | -0.8 |
| Range (ETDRS letters) | -7 / +7 | -19 / +16 | -14 / +16 |
| | | | |

| **Change in CRT³:** | | | |
|---|---|---|---|
| Follow-up | 44 weeks (median) | 24 weeks | 24 weeks |
| Mean (µm)⁴ | -25.5 | -27.8 | -30.8 |
| Range (µm)⁴ | -117 / +32 | -61 / -8 | -61 / -8 |
| ¹ This event was deemed unrelated to AAV2.7m8-aflibercept or any study procedure. | | | |
| ² Best corrected visual acuity (BCVA) as measured by Early Treatment Diabetic Retinopathy Study (ETDRS) (i.e., sight charts). | | | |
| ³ Central retinal thickness (CRT), also referred to as central subfield thickness (CST) assessed using Optical Coherence Tomography (OCT) imaging. | | | |
| ⁴ BCVA and CST values for subject with retinal detachment (unrelated to study treatment) used last observations prior to detachment. | | | |

### Example 5: Additional results of an open label Phase 1 study of AAV2.7m8-aflibercept in neovascular (wet) age-related macular degeneration.

This Example describes results for Cohorts 1, 2, and 3 of the Phase 1 study described in Examples 1-4 that assessed the safety and efficacy of a single intravitreal injection of AAV2.7m8-aflibercept in subjects with wAMD. Safety and efficacy results are provided for Cohort 1 at a median follow up time of 60 weeks (range of 52-64 weeks), for Cohort 2 at a median follow up time of 36 weeks (range of 32-40 weeks), and for Cohort 3 at a follow up time of up to 20 weeks.

### Results

Subjects in Cohort 1 were administered a single IVT injection of AAV2.7m8-aflibercept at a dose of 6 × 10¹¹vg/eye. Subjects in Cohort 2 were administered a single IVT injection of AAV2.7m8-aflibercept at a dose of 2 × 10¹¹vg/eye. Subjects in Cohorts 1 and 2 were also administered a prophylactic oral prednisone regimen (*see* **Table 2).**

Subjects in Cohort 3 were administered a single IVT injection of AAV2.7m8-aflibercept at a dose of 2 × 10¹¹vg/eye with a 6-week prophylactic topical corticosteroid regimen (*see* **Table 11B).** The topical corticosteroid regimen consisted of corticosteroid eye drops (0.05% difluprednate [2.5µg]) administered four times per day for 3 weeks (QID for 3 weeks), starting on the same day as the administration of AAV2.7m8-aflibercept (administered after the IVT injection of AAV2.7m8-aflibercept), followed by a 3-week taper which consisted of corticosteroid eye drops administered three times per day for one week (TID for 1 week), followed by corticosteroid eye drops administered two times per day for one week (BID for 1 week), followed by corticosteroid eye drops administered once per day for one week (QD for 1 week).

### Subject Characteristics

The baseline subject characteristics for subjects in Cohorts 1, 2, and 3 are provided in **Table 15.**

**Table 15. Baseline characteristics of subjects in Cohorts 1-3.**

| **Baseline Characteristics** | **Cohort 1 (N=6)** | **Cohort 2 (N=6)** | **Cohort 3 (N=9)** |
|---|---|---|---|
| Mean age, years | 79.0 | 79.8 | 77.4 |
| Mean years since nAMD diagnosis | 3.5 | 4.1 | 3.3 |
| Mean (range) number anti-VEGF injections since initial diagnosis | 35.3 (7-109) | 34.0 (4-69) | 24.8 (9-70) |
| Mean number anti-VEGF injections in 12 months prior to AAV2.7m8-aflibercept | 9.2 | 9.2 | 9.1 |
| Mean BCVA study eye, ETDRS letters | 65.8 | 64.7 | 65.9 |
| Approximate Snellen equivalent | 20/50 | 20/50 | 20/50 |
| Mean CST study eye, µm | 369.2 | 307.7 | 472.3 |

As shown in **Table 15,** subjects in Cohorts 1-3 previously required frequent anti-VEGF injections to maintain vision. The baseline characteristics for subjects in Cohorts 1-3 were well balanced, with similar ages, similar mean prior anti-VEGF injections, and a similar need for frequent injections (e.g., over 9 injections in the year prior to administration of AAV2.7m8-aflibercept) to maintain a relatively high baseline vision of about 65 ETDRS letters across cohorts. Subjects in Cohort 3 were, on average, younger and had fewer prior anti-VEGF injections since initial diagnosis and a thicker average baseline CST.

### Safety Results

As shown in **Table 16,** across Cohorts 1-3, no AAV2.7m8-aflibercept- or procedure-related serious adverse events (SAEs) occurred. In addition, no AAV2.7m8-aflibercept-related systemic adverse events (AEs) were observed. An SAE of retinal detachment that was unrelated to study treatment occurred and was surgically repaired and resolved (a previous cataract extraction and artificial lens implantation were key risk factors). Two subjects had adverse events of intraocular pressure (IOP) elevation that resolved. One subject had two mild IOP elevation events (highest IOP was 24mmHg) that were both treated with Combigan^{®} eye drops. The second subject was taking Combigan^{®} for ocular hypertension, and the IOP AE spontaneously resolved within a month with no change to treatment.

Subjects in Cohort 3 (administered steroid eye drop prophylaxis) had a low number of ocular adverse events.

**Table 16. Adverse events in Cohorts 1, 2, and 3.**

| Category | Cohort 1 (n=6) | | Cohort 2 (n=6) | | Cohort 3 (n=9) | |
|---|---|---|---|---|---|---|
| | Subjects | Events | Subjects | Events | Subjects | Events |
| **Ocular AE (Total)** Serious AE AAV2.7m8-aflibercept-related** | 6 | 49 | 5 | 32 | 7 | 16 |
| | 1 | 1* | 0 | 0 | 0 | 0 |
| | 6 | 29 | 5 | 21 | 4 | 8 |
| **Non-ocular AE (Total)†** Serious AE‡ | 5 | 17 | 5 | 5 | 4 | 6 |
| | 1 | 1 | 0 | 0 | 2 | 2 |
| * Retinal detachment (unrelated to AAV2.7m8-aflibercept); **AAV2.7m8-aflibercept-related ocular events were mild (69%) or moderate (31%); †None of the non-ocular AEs were AAV2.7m8-aflibercept-related; ‡Serious non-ocular AEs included degenerative intervertebral disc disease (1) in Cohort 1, and COPD exacerbation (1) and stable angina pectoris (1) in Cohort 3. | | | | | | |

As shown in **FIGS. 15A-15C****,** mild and low-grade inflammation, primarily affecting the anterior segment, was common across Cohorts 1-3 and was responsive to topical steroids. No clinical or fluorescein (fluorescein angiography of posterior pole) evidence of vasculitis, retinitis, or choroiditis were observed.

Three subjects in Cohort 1 continue to be managed with topical steroids, but only 1 subject has any evidence of inflammatory cells. Subject 2 is on topical steroids once per day; Subject 4 is on topical steroids twice per day; and Subject 6 is on topical steroids once per day. Overall, none of the subjects in Cohort 1 ever had inflammatory cell counts above 2+. In addition, subjects in Cohort 1 only had inflammatory cell counts of 2+ for one time point.

Two subjects in Cohort 2 continue to be managed with topical steroids and have evidence of mild inflammatory cells. Subject 1 is on topical steroids once per day and Subject 6 is on topical steroids once per day. Subjects 1 and 6 had some breakthrough inflammation of up to 3+ inflammatory cell counts, but both subjects rapidly resolved with topical steroid treatment.

Subjects in Cohort 3 were administered a 6-week prophylactic steroid eye drop regimen. This regimen minimized early ocular inflammation **(****FIG. 15C****).** Certain subjects in Cohort 3 also continue to be managed with topical steroids. Subject 3 in Cohort 3 is receiving topical steroids four times per day. Subject 5 is no longer receiving topical steroids but was administered topical steroids as follows: four times per day for 3 weeks following administration of AAV2.7m8-aflibercept, then twice per day for one week, then four times per day for a week starting at Week 5, and then was decreased from three times per day for a week, to twice per day for a week, to once per day for a week. Subjects 7-9 of Cohort 3 continue on the 6-week prophylactic steroid eye drop regimen. In addition to the prophylactic steroid regimen described, Subject 6 was hospitalized for chronic obstructive pulmonary disease (COPD) following treatment with AAV2.7m8-aflibercept and was prescribed oral steroids. The COPD was found not to be related to AAV2.7m8-aflibercept.

### Efficacy

**FIG. 16A** shows the mean BCVA (ETDRS letters) from baseline up to Week 52 for subjects in Cohort 1. At a median follow up time of 60 weeks (range of 52-64 weeks), subjects in Cohort 1 had a mean BCVA change from baseline of -2.7 letters and 100% of subjects (6/6) had not received a rescue anti-VEGF injection. The apparent decrease in mean BCVA at Weeks 44 and 48 was due to one subject that experienced an SAE of retinal detachment. **FIG. 16B** shows the mean CST (µm) from baseline up to Week 52 for subjects in Cohort 1. At a median follow up time of 60 weeks (range of 52-64 weeks), subjects in Cohort 1 had a mean CST change from baseline of -26.2 µm.

**FIG. 17A** shows the mean BCVA (ETDRS letters) from baseline up to Week 36 for subjects in Cohort 2. At a median follow up time of 36 weeks (range of 32-40 weeks), subjects in Cohort 2 had a mean BCVA change from baseline of -2.8 letters and 67% of subjects (4/6) had not received a rescue anti-VEGF injection. The subjects that had not received a rescue anti-VEGF injection had a mean BCVA change from baseline of +2.3 letters. **FIG. 17B** shows the mean CST (µm) from baseline up to Week 36 for subjects in Cohort 2. At a median follow up time of 36 weeks (range of 32-40 weeks), subjects in Cohort 2 had a mean CST change from baseline of -40.8 µm. In addition, the subjects that had not received a rescue anti-VEGF injection had a mean CST change from baseline of -30.0 µm. Overall, in Cohort 2, CST improvements were maintained out to Week 36.

**FIG. 18A** shows the mean BCVA (ETDRS letters) from baseline up to Week 20 for five subjects in Cohort 3. At a follow up time of 20 weeks, the five subjects from Cohort 3 had a mean BCVA change from baseline of +6.8 letters and 80% (4/5) of the five subjects from Cohort 3 had not received a rescue anti-VEGF injection. The subjects that had not received a rescue anti-VEGF injection had a mean BCVA change from baseline of +8.8 letters. Overall, in the five subjects in Cohort 3, BCVA improved through Week 20 (*i.e.,* a 6.8 letter mean improvement for the five subjects in Cohort 3). **FIG. 18B** shows the mean CST from baseline up to Week 20 for five subjects in Cohort 3. At a follow up time of 20 weeks, the five subjects from Cohort 3 had a mean CST change from baseline of -137.8 µm. In addition, the subjects that had not received a rescue anti-VEGF injection had a mean CST change from baseline of -149.8 µm. Overall, in the five subjects in Cohort 3, the mean CST was improved through Week 20, with a substantial decrease in CST (*i.e.,* a mean decrease of 137.8 µm for the five subjects in Cohort 3).

As shown in the Swimmer's Lane Plot in **FIG. 19****,** no subjects in Cohort 1 received a rescue anti-VEGF injection at a median follow up time of 60 weeks (range of 52-64 weeks). Subjects 3 and 5 in Cohort 2 each received 4 anti-VEGF rescue injections (aflibercept). Subject 3 in Cohort 3 received 2 anti-VEGF rescue injections (aflibercept).

### Subject Case Studies

At baseline, Subject 4 in Cohort 1 (62 year old male) had received 109 prior anti-VEGF injections (9 in the last 12 months prior to AAV2.7m8-aflibercept administration) and had persistent subretinal fluid even with frequent anti-VEGF injections **(****FIG. 20A****).** As shown in **FIG. 20B****,** some reduction in subretinal fluid after the screening IVT aflibercept injection was observed in Subject 4. After administration of AAV2.7m8-aflibercept, the subretinal fluid continued to resolve, and the retina was dry by Week 12. This effect continued with no signs of fluid recurrence until the subject experienced an unrelated retinal detachment involving the macula that was diagnosed at Week 44. Due to the macular involvement, the vision was reduced and the subject underwent a successful retinal detachment repair surgery with vitrectomy and gas bubble. However, due to the gas bubble, OCT imaging was not possible at Week 48 and the subject was unable to read any letters on the sight chart. As the gas bubble dissolved, the vision improved, and there have been no signs of wet AMD disease activity or recurrence of fluid. The fundus topography has changed somewhat due to the retinal detachment and the foveal center has been manually located to match pre-operative reference scans in the retinal thickness maps presented in **FIG. 20B** at weeks 44, 52, and 60. At Week 60, the retina remained dry, vision was within 10 letters of baseline (BCVA), and the subject remained rescue injection-free.

At baseline, Subject 5 in Cohort 3 (82 year old male) had received 19 prior anti-VEGF injections (9 in the last 12 months prior to AAV2.7m8-aflibercept administration) and had persistent subretinal fluid even with frequent anti-VEGF injections **(****FIG. 21A****).** As shown in **FIG. 21B****,** Subject 5 exhibited responsiveness to the screening anti-VEGF injection, however, the subretinal fluid returned within 2 weeks. Thus, Subject 5 is an example of a subject that was responsive to anti-VEGF treatment but required frequent injections to keep the fluid away. Based on the history of Subject 5, the subject would have required bolus anti-VEGF injections on a monthly basis (e.g., about once every 4 weeks). However, one week after AAV2.7m8-aflibercept administration, the subretinal fluid had not increased, and no fluid was observed at Weeks 12, 16, and 20 after AAV2.7m8-aflibercept administration. These results demonstrate that the course of the disease in Subject 5 was altered by a single administration of AAV2.7m8-aflibercept.

### Discussion

The results described in this Example showed that AAV2.7m8-aflibercept was well tolerated and showed robust efficacy across Cohorts 1, 2, and 3. In particular, these results showed that a single dose of AAV2.7m8-aflibercept reduced the anti-VEGF injection burden in subjects in Cohorts 1, 2, and 3.

The results for subjects in Cohort 1, which were administered AAV2.7m8-aflibercept at a dose of 6x10¹¹vg/eye, showed that zero subjects required anti-VEGF rescue injections at 1 year and beyond after administration of AAV2.7m8-aflibercept, thus demonstrating the long-term durability of the efficacy of AAV2.7m8-aflibercept. In addition, the majority of subjects in Cohorts 2 and 3, which were administered AAV2.7m8-aflibercept at a dose of 2x10¹¹vg/eye (3-fold lower dose than Cohort 1), also remained anti-VEGF rescue injection-free.

Overall, out of 17 subjects administered AAV2.7m8-aflibercept in Cohorts 1-3 (including 5 subjects from Cohort 3 that were followed for 20 weeks), 14 (82%) remained anti-VEGF rescue injection-free, maintained mean BCVA, and had improvements in mean CST.

The results from Cohorts 1-3 also showed all adverse events related to AAV2.7m8-aflibercept were mild to moderate, and that ocular inflammation observed following administration of AAV2.7m8-aflibercept was low grade and responsive to steroid eye drops. In addition, subjects in Cohort 3, which were administered a 6-week prophylactic steroid eye drop regimen, exhibited minimal inflammation, thus demonstrating that the 6-week prophylactic steroid eye drop regimen was effective at minimizing early ocular inflammation. No events of vasculitis, retinitis, or choroiditis were observed.

### Example 6: A Phase 2, Multi-Center, Randomized, Double-Masked, Active Controlled Study of AAV2.7m8-aflibercept in Subjects with Diabetic Macular Edema.

This Example describes a Phase 2, multi-center, randomized, double-masked, active controlled study that evaluated the durability of a single intravitreal (IVT) injection of AAV2.7m8-aflibercept in subjects with diabetic macular edema.

### I. Study Objectives and Endpoints

### A. Primary Objective

The primary objective of this study is to assess the durability of a single IVT injection of AAV2.7m8-aflibercept.

### B. Secondary Objectives

Secondary objectives of this study include:
- Assessment of the safety and tolerability of AAV2.7m8-aflibercept.
- Evaluation of the effect of AAV2.7m8-aflibercept on macular edema.
- Evaluation of the effect of AAV2.7m8-aflibercept on Best Corrected Visual Acuity (BCVA).
- Evaluation of the effect of AAV2.7m8-aflibercept on Diabetic Retinopathy Severity Scale (DRSS) score.
- Assessment of the need for rescue aflibercept (2 mg IVT).
- Assessment of the effect of a preceding dose of aflibercept (2 mg IVT) prior to AAV2.7m8-aflibercept administration.
- Evaluation of the effect of AAV2.7m8-aflibercept on development of vision threatening complications (anterior segment neovascularization, vitreous hemorrhage, or tractional retinal detachment).

### C. Primary Endpoints

The primary endpoint of this study is the time to worsening of diabetic macular edema (DME) disease activity in the study eye, as defined by the occurrence of either of:
- An increase in central subfield thickness (CST) > 50 µm as assessed by SD-OCT compared to the lower of the two CST measurements recorded at Day 1 or Week 4.
- A loss of > 5 letters in BCVA due to worsening DME disease activity compared to the higher of the two BCVA measurements recorded at Day 1 or Week 4.

### D. Secondary Endpoints

The secondary endpoints for this study are based on outcome measures for the study eye (unless otherwise specified) and include:
- Incidence and severity of ocular and non-ocular adverse events (AEs).
- Change from Baseline in CST and macular volume over time through Week 48.
- Change from Baseline in BCVA over time through Week 48.
- Frequency of rescue aflibercept (2 mg IVT) in the study eye over time during the study.
- Incidence of 2-step and 3-step improvement in DRSS score over time through Week 48.
- Incidence of 2-step and 3-step worsening in DRSS score over time through Week 48.
- Occurrence of vision threatening complication (anterior segment neovascularization, vitreous hemorrhage, or any other high-risk proliferative diabetic retinopathy (DR), or tractional retinal detachment) over time through Week 48.
- Incidence of CST <300 µm over time through Week 48.
- Incidence of clinically significant findings via physical examinations, ocular examinations, imaging, and laboratory evaluation over time through Week 48.

### II. Study Population

### A. Inclusion Criteria

Subjects with newly diagnosed DME *(i.e.,* DME diagnosis within 6 months of screening) that have received up to 2 prior injections of anti-VEGF therapy in the study eye are included in this study if they meet the following inclusion criteria:
- Age ≥ 18 years of age.
- Type 1 or 2 diabetes mellitus.
- Vision impairment due to center involving diabetic macular edema.
- Vision at Screening:
   o Study Eye: BCVA 78 to 50 ETDRS letters, inclusive (approximate Snellen equivalent 20/32 to 20/100).
   ∘ Non-study eye: BCVA 35 ETDRS letters or more (approximate Snellen equivalent of 20/200 or better).
- CST of study eye at Screening visit of ≥ 325µm using Heidelberg Spectralis^{®} with center-involving IRF (center 1 mm).
- A decrease in vision in the study eye determined to be primarily due to DME.
- Initial DME diagnosis within 6 months from screening.
- Up to 2 prior injections (0, 1, or 2) of anti-VEGF in the study eye.
   ∘ If a prior anti-VEGF has been administered to the study eye, there must have been a meaningful CST response (e.g., ≥ 10% reduction) and no adverse reaction to anti-VEGF (e.g., inflammation).
- A minimum 60-day interval between the last anti-VEGF injection in the study eye and randomization on Day 1.

### B. Exclusion Criteria

Subjects meeting any of the following criteria are excluded from this study:
- Documented anti-AAV2.7m8 neutralizing antibody titer > 1:125 within 6 months prior to randomization.
- Prior ocular gene therapy.
- History of allergy to aflibercept, corticosteroid, or fluorescein dye or sodium fluorescein used in angiography (mild allergy amenable to treatment is allowable).
- History or evidence of any of the following cardiovascular disease within 6 months of dosing:
   ∘ Severe cardiac disease (e.g., New York Heart Association [NYHA] Functional Class III or IV) or clinical evidence of unstable angina.
   ∘ Acute coronary syndrome, myocardial infarction or coronary artery revascularization, cerebrovascular accident (CVA), transient ischemic attack (TIA).
   ∘ Ventricular tachyarrhythmias requiring ongoing treatment, or uncontrolled arrhythmia.
   ∘ Uncontrolled hypertension defined as systolic blood pressure (SBP) >160 mmHg or a diastolic blood pressure (DBP) >100 mmHg, despite using BP-lowering medication within the screening period. If BP-lowering medications are required, subject should be on a stable dose of the same medication continuously for 30 days prior to randomization.
- Any history of ongoing bleeding disorders. The use of aspirin or other anticoagulants (e.g., Factor Xa inhibitors) is not an exclusion criterion.
- Uncontrolled diabetes defined as HbA1C >10%; or history of diabetic ketoacidosis within 3 months prior to randomization; or subjects who, within the last 3 months, initiated intensive insulin treatment (a pump or multiple daily injection) or plan to do so in the next 3 months.
- History of systemic autoimmune disease requiring treatment with systemic steroids or immunosuppressive treatments (e.g. methotrexate, adalimumab).
- Systemic drugs known to cause macular edema (e.g., fingolomod, tamoxifen, chloroquine/hydroxychloroquine) or any prior systemic anti-VEGF therapy.
- Known to be positive for HIV or active viral hepatitis (unless documented cure after treatment for Hepatitis C); known history of syphilis
- Known severe renal impairment, as indicated by estimated CrCl <30 mL/min (by Cockcroft-Gault calculation); need or anticipated need for hemodialysis during the study period.
- Any febrile illness within 1 week prior to randomization.

In addition, subjects meeting any of the following ocular exclusion criteria in the study eye are excluded from this study:
- High-risk proliferative diabetic retinopathy (PDR) at time of screening, defined as: any vitreous or preretinal hemorrhage, neovascularization elsewhere >1/2-disc area within an area equivalent to standard ETDRS 7-field on clinical examination, or neovascularization of disc > 1/3-disc area on clinical examination.
- Any prior focal or grid laser photocoagulation or any prior pan retinal photocoagulation (PRP) in the study eye.
- Any anti-VEGF therapy in the preceding 60 days prior to randomization (up to 2 prior anti-VEGF injections are allowed but they cannot have occurred in the prior 60 days).
- History of anterior segment neovascularization (e.g., neovascularization of the iris [NVI] or neovascular glaucoma [NVG]), significant vitreous hemorrhage, fibrovascular proliferation or tractional retinal detachment.
- Examination evidence of structural abnormalities at the fovea (e.g., dense hard exudates, pigment abnormalities, foveal atrophy, vitreomacular traction or epiretinal membrane), either on clinical examination or OCT, thought to be contributing to macular edema or visual impairment.
- History of retinal disease in the study eye other than diabetic retinopathy including age-related macular degeneration (in either eye), retinal vein occlusion, retinal arterial occlusion, pathologic myopia, etc.
- Any current or history of ocular disease other than DME that could reduce the potential for visual improvement, confound assessment of the macula or require medical or surgical intervention during the study (e.g. significant cataract, macular traction) or any evidence of posterior subcapsular cataract.
- History of cataract extraction or Yttrium Aluminum Garnet (YAG) capsulotomy within 3 months before Day 1.
- History of retinal detachment (with or without repair) in the study eye.
- History of trabeculectomy or glaucoma shunt or minimally invasive glaucoma surgery (MIGS).
- History of vitrectomy or other filtration surgery.
- Aphakia or presence of an anterior chamber intraocular lens.
- Uncontrolled ocular hypertension or glaucoma in the study eye at time of randomization (defined as IOP >22 mmHg despite treatment with anti-glaucoma medication) or current use of >2 IOP lowering medications.
- Any history of intraocular or periocular steroid treatment for any ocular condition (e.g., IVT Triesence, Iluvien or Ozurdex).
- Refractive surgery within the 90 day period prior to Screening.
- Previous penetrating keratoplasty, endothelial keratoplasty, or ocular radiation.
- Any prior vitreoretinal surgery.

In addition, subjects meeting any of the following ocular exclusion criteria in the study eye or in the non-study eye (*i.e.,* the "fellow eye") are excluded from this study:
- Any history of uveitis or intraocular inflammation (grade trace or above) except mild anticipated post-operative inflammation that resolved.
- History of IOP elevation related to topical steroid administration.
- Known history of ocular Herpes Simplex Virus (HSV), Varicella-zoster virus (VZV), or Cytomegalovirus (CMV) including viral uveitis, retinitis or keratitis.
- Evidence of external ocular infection, including conjunctivitis, chalazion or significant blepharitis.
- History of ocular toxoplasmosis.

### III. Study Design

### A. Study Treatment

This is a multi-center, randomized, double-masked, controlled, parallel-group study to evaluate the efficacy, safety and tolerability of a single 0.10 mL IVT injection of AAV2.7m8-aflibercept. Two doses of AAV2.7m8-aflibercept are investigated.

Subjects with initial diagnosis of DME within 6 months of screening and that have received up to 2 prior injections of anti-VEGF therapy are eligible for enrollment.

Approximately 33 eligible subjects are randomized to receive one of two doses of AAV2.7m8-aflibercept (6 × 10¹¹ vg/eye or 2 × 10¹¹ vg/eye), or to a control arm to receive a sham ocular injection with a preceding aflibercept injection. Subjects who are assigned to receive AAV2.7m8-aflibercept are further randomized to receive a preceding aflibercept or sham ocular injection. The study arms are summarized below:
- Arm 1 (n=6): Subjects receive AAV2.7m8-aflibercept at a dose of 6 × 10¹¹ vg/eye with a preceding aflibercept dose.
- Arm 2 (n=6): Subjects receive AAV2.7m8-aflibercept at a dose of 6 × 10¹¹ vg/eye without a preceding aflibercept dose.
- Arm 3 (n=6): Subjects receive AAV2.7m8-aflibercept at a dose of 2 × 10¹¹ vg/eye with a preceding aflibercept dose.
- Arm 4 (n=6): Subjects receive AAV2.7m8-aflibercept at a dose of 2 × 10¹¹ vg/eye without a preceding aflibercept dose.
- Arm 5 (n=9): Subjects receive aflibercept only (active control).

To maintain masking of the treatment assignment, subjects assigned to the arms with no preceding aflibercept on Day 1 or to the arm with no AAV2.7m8-aflibercept on Day 8 receive a sham ocular injection on the corresponding visit. Only one eye per subject is selected as the study eye. If both eyes are eligible, the eye with the worse BCVA is selected as the study eye.

Both AAV2.7m8-aflibercept and aflibercept are administered via IVT injection. IVT injections are not performed if active inflammation is present. Aseptic technique with povidone-iodine is used with topical or subconjunctival anesthesia. The sham ocular injection procedure is done under the same conditions but with an empty syringe without a needle (using the blunt end) pressed against the eye to mimic an injection.

A summary of the arms in this study is provided in **Table 17.**

**Table 17. Study Arms.**

| Arm | N | Day 1 IVT Aflibercept or Sham | Day 8 AAV2.7m8-aflibercept Dose (vg/eye) |
|---|---|---|---|
| 1 | 6 | Aflibercept | 6 × 10¹¹ |
| 2 | 6 | Sham | 6 × 10¹¹ |
| 3 | 6 | Aflibercept | 2 × 10¹¹ |
| 4 | 6 | Sham | 2 × 10¹¹ |
| 5 | 9 | Aflibercept | Sham |

After the assigned IVT injections on Day 1 and Day 8, all subjects are followed up on Week 2, Week 4, and then every 4 weeks up to Week 48 after Day 1 (e.g., Week 8, Week 12, Week 16, etc.). To maintain masking, both subjects and personnel conducting assessments are masked to the treatment assignments throughout the study.

All subjects are followed for 48 weeks after randomization.

An overview of the study design is provided in **FIG. 29****.**

### B. Prophylactic Topical Steroid Regimen

All subjects are administered a prophylactic 7-week topical corticosteroid regimen of difluprednate (0.05%; for example., Durezol) starting on Day 1. Subjects are instructed to self-administer difluprednate four times per day (QID) for 4 weeks (*i.e.*, from Day 1 to Day 28), followed by three times per day (TID) for 1 week (*i.e.,* for 7 days), followed by two times per day (BID) for 1 week (*i.e.*, 7 days), and finally once per day (QD) for 1 week (*i.e.,* 7 days). Tapering is not commenced in the presence of active inflammation. This regimen is prolonged should signs of inflammation occur. A summary of the difluprednate regimen is provided in **Table 18.**

**Table 18. Difluprednate Regimen.**

| **Study Days** | **Total Number of Days** | **Difluprednate Administrations per Day** |
|---|---|---|
| Days 1 to 28 | 28 | 4 times |
| Days 29 to 35 | 7 | 3 times |
| Days 36 to 42 | 7 | 2 times |
| Days 43 to 49 | 7 | 1 time |
| Day 50 | 0 | STOP - if no signs of inflammation |

### C. Rescue Treatment

Starting at Week 8, subjects receive rescue aflibercept (2 mg IVT) if they meet any of the following:
- Increase in CST > 50 µm as assessed by SD-OCT compared to the lower of the two CST measurements recorded at Day 1 or Week 4.
- Loss of > 5 letters in BCVA due to worsening DME disease activity compared to the higher of the two BCVA measurements recorded at Day 1 or Week 4.

Aflibercept is not injected in eyes with active inflammation. A minimum of 21 days is required between rescue aflibercept injections.

### D. Medications and Treatments

The following medications are prohibited during the study:
- Any systemic anti-VEGF agent, including bevacizumab.
- Systemic drugs known to cause macular edema (e.g., fingolomod, tamoxifen, chloroquine/hydroxychloroquine).
- Any anti-VEGF agent in the study eye other than the study drug or aflibercept IVT 2 mg.
- IVT steroids in the study eye (e.g., Ozurdex or Illuvien Triesence).
- Systemic immunosuppressive drugs (e.g., intravenous steroids, methotrexate, azathioprine, ciclosporin, adalimumab, infliximab, etanercept). Inhaled or topical steroids and NSAIDs are allowed.

Subjects who develop high-risk PDR in the study eye receive panretinal photocoagulation (PRP) after receiving rescue aflibercept.

Subjects with visually significant cataract are not enrolled in the study, but if a cataract develops during the study, cataract surgery in the study eye may be performed if clinically indicated and is scheduled > 90 days after AAV2.7m8-aflibercept administration and/or > 7 days after the last injection of aflibercept.

Subjects who develop DME in the fellow (non-study) eye may receive standard of care therapy.

### IV. Study Assessments

### A. General Physical Examination and Vital Signs

A general physical examination (PE) is conducted at the screening and End of Study (EOS) or Early Termination visit. It consists of body system examination for general appearance, neurologic, HEENT (head, eyes, ears, nose, and throat), neck, cardiovascular, respiratory, abdomen, extremities, skin, weight, and height. At the EOS or Early Termination visit, the physical examination assesses if any changes in the subject's physical condition have occurred since the Screening examination. A targeted physical examination is conducted as needed for the evaluation of AEs.

Vital signs consist of blood pressure, pulse rate, body temperature, and respiratory rate. A 12-lead Electrocardiogram (ECG) is taken for each subject at Screening and EOS or Early Termination Visit.

### B. Laboratory Tests, Vector Expression and Immune Response

The following Clinical Laboratory Tests are conducted for the study: chemistry, complete blood count, HbA1C, urinalysis, and HLA-B27 genotyping.

Subjects' samples (both blood and/or aqueous humor) are collected to measure the following:
- Total antibodies to AAV2.7m8: serum for total anti-AAV2.7m8 antibodies are measured in an ELISA assay.
- Neutralizing antibodies to AAV2.7m8: serum for neutralizing anti-AAV2.7m8 antibodies are measured in a cell-based assay.
- Anti-aflibercept antibodies: serum for the humoral immune response against aflibercept is measured in an ELISA assay.
- Aflibercept protein expression: Serum and aqueous humor samples are collected for the presence of aflibercept protein to be measured in a MesoScale Discovery assay.
- Cell-mediated immune response: cellular immunity against AAV2.7m8 capsid and aflibercept protein are measured in an ELISPOT assay.

### C. Full Ophthalmic Examination

Study assessments include an ophthalmologic exam, Intraocular Pressure (IOP), and indirect ophthalmoscopy.

The ophthalmic examination consists of an external examination of the eye and adnexa, routine screening for eyelid/pupil responsiveness (including but not limited to blepharoptosis, abnormal pupil shape, unequal pupils, abnormal reaction to light, and afferent pupillary defect), and slit-lamp examination (eyelids, conjunctiva, cornea, lens, iris, anterior chamber). The slit-lamp examination examines the anterior ocular structures and is used for grading any findings. If any finding is noted during the slit-lamp examination, at any visit, the severity is graded and the finding is described as clinically significant or not clinically significant.

IOP measurements are performed using a Goldmann applanation tonometer or Tono-pen^{™}. The same method of IOP measurement is used throughout the study for each individual subject. IOP measurements are performed prior to any IVT injection and prior to dilating eyes, using the same method throughout the study. Day 1 and Day 8 visits require pre-injection and post-injection (30 minutes after injection) IOP measurements.

The dilated indirect ophthalmoscopy examination includes an evaluation of posterior segment abnormalities of the vitreous, optic nerve, peripheral retina, and retinal vasculature. If any finding is noted during the ophthalmoscopy, at any visit, the severity is graded and the finding is described as clinically significant or not clinically significant. Day 1 and Day 8 visits require pre-injection and post-injection indirect ophthalmoscopy assessments.

### D. Refraction and Visual Acuity

Refraction and BCVA are measured. Visual acuity measurements are measured at a starting distance of 4 meters, prior to dilating eyes.

### E. Imaging

Spectral Domain Optical Coherence Tomography (SD-OCT) is an interferometric technique that provides depth-resolved tissue structure information encoded in the magnitude and delay of the back-scattered light by spectral analysis of the interference fringe pattern. If a subject received anti-VEGF injections at visits prior to study randomization, OCTs from those visits are collected and delivered to the central reading center.

Optical Coherence Tomography Angiography (OCT-A) is an imaging technology that provides volumetric, three-dimensional maps of the retina and choroid as well as information on blood flow. There are two types of OCT-A, swept-source and spectral-domain. Swept-source imaging is used where available. If a swept-source instrument is not available and a spectral-domain instrument is available, then the spectral domain instrument is used.

Standardized procedures for the collection of ultra-wide field fundus digital photographic images of the retina, optic disc, and macula are followed. In addition, photographs of the iris are taken prior to dilation.

A standardized procedure for examining the retinal circulation and vessel permeability using a dye-tracing method is followed. This involves injection of sodium fluorescein into the systemic circulation, after which an angiogram is obtained by digitally photographing the fluorescence emitted after illumination of the retina with blue light at a wavelength of 490 nm.

### F. Laboratory, Biomarker and Other Biological Specimens

Aqueous humor samples are collected and analyzed for levels of aflibercept, VEGF-A, neutralizing antibodies (NAbs), and additional biomarkers. Vitreous humor samples are obtained and analyzed for aflibercept concentrations and other biomarkers.

### G. Safety

After study treatment administration, all clinically significant adverse events (AEs) are reported. Each subject is followed until a) the end of the AE reporting period at 30 days after the last study visit or b) for any ongoing study treatment related AEs and/or serious AEs (SAEs) until resolved or stable. Any clinically significant safety assessment that is associated with DME is not reported as an AE or SAE, unless judged to be more severe than expected for the subject's condition. Progression of the disease under study is captured as an efficacy outcome.

Adverse events of special interest for this study include:
- Sight-threatening adverse events: an adverse event is considered to be sight-threatening if it meets one or more of the following criteria:
   ∘ Causes a decrease of ≥ 30 letters in BCVA compared with the prior visit.
   ∘ Requires surgical or medical intervention (i.e., conventional surgery, vitrectomy) to prevent permanent loss of sight.
   ∘ Causes severe intraocular inflammation (i.e., endophthalmitis, 4+ anterior chamber cell/flare, or 4+ vitreous cells).

All of the above-listed sight-threatening adverse events are reported as serious adverse events, listing the underlying cause (if known) of the event as the primary event term.

### H. Efficacy

The efficacy of AAV2.7m8-aflibercept in the treatment of DME is assessed by the following measures. Baseline values for BCVA and SD-OCT endpoints refer to pre-treatment measurements taken on the Day 1 visit when aflibercept IVT or sham ocular injection are administered.
- BCVA: Vision is assessed primarily through BCVA expressed as an ETDRS score (number of letters correctly read). Maintenance of vision is classified if the subject has lost fewer than 15 letters in the ETDRS score compared to Baseline. Calculated endpoints include the mean change from Baseline, the percent gaining at least 15 letters compared to Baseline, and the percent losing 15 or more letters compared to Baseline.
- Central subfield thickness: SD-OCT is performed using approved equipment and standard techniques to evaluate thickness and fluid compared to Baseline values. Endpoints include CST and macular volume.
- Aflibercept re-treatments: The incidence and timing of aflibercept injections given post-AAV2.7m8-aflibercept treatment over time.
- For each timepoint, Diabetic Retinopathy Severity Scale (DRSS) is determined using ultra-wide field color fundus photography and compared to Day 1.
- Vision threatening complications (anterior segment neovascularization, diabetic macular edema, high-risk PDR development, vitreous hemorrhage, or tractional retinal detachment), as determined by ultra-wide field imaging and clinical examination.

### I. Statistical Analyses

The main analysis population includes all randomized subjects who receive the study treatment on Day 8 (AA V2.7m8-aflibercept IVT or Sham ocular injection). All safety and efficacy variables are summarized descriptively by treatment arm. Mean, standard deviation (SD), median and range are provided for continuous variables; and frequency counts and percentages are provided for categorical variables. Confidence intervals of the means and percentages are provided at both 90% and 95% levels. Kaplan-Meier survival analysis is utilized to derive median time to the first occurrence of DME disease worsening. All rescue aflibercept (2 mg IVT) received by each subject during the study are summarized using statistical models for recurrent events. Mean cumulative function (MCF) curve over time is plotted for the mean cumulative number of injections. Mixed-effect models for repeated measures (MMRM) are employed to explore the treatment effect on the change over time in BCVA and CST. The treatment effect on DRSS changes over time is explored using generalized mixed models for categorical outcomes. An interim analysis (IA) occurs after all subjects have been followed for 24 weeks.

### Example 7: Additional results of an open label Phase 1 study of AAV2.7m8-aflibercept in neovascular (wet) age-related macular degeneration.

This Example describes results for Cohorts 1, 2, 3, and 4 of the Phase 1 study described in Examples 1-5 that assessed the safety and efficacy of a single intravitreal injection of AAV2.7m8-aflibercept in subjects with wAMD. Safety and efficacy results are provided for Cohort 1 at median follow up time of 72 weeks (range of 64-84 weeks), for Cohort 2 at a median follow up time of 52 weeks (range of 52-56 weeks), for Cohort 3 at a median follow up time of 36 weeks (range 20-40 weeks), and for Cohort 4 at a median follow up time of 4 weeks (range 2-8 weeks).

### Results

Subjects in Cohort 1 were administered a single IVT injection of AAV2.7m8-aflibercept at a dose of 6 × 10¹¹vg/eye. Subjects in Cohort 2 were administered a single IVT injection of AAV2.7m8-aflibercept at a dose of 2 × 10¹¹vg/eye. Subjects in Cohorts 1 and 2 were also administered a prophylactic oral prednisone regimen (*see* **Table 2**).

Subjects in Cohort 3 were administered a single IVT injection of AAV2.7m8-aflibercept at a dose of 2 × 10¹¹vg/eye. Subjects in Cohort 4 were administered a single IVT injection of AAV2.7m8-aflibercept at a dose of 6 × 10¹¹vg/eye. Subjects in Cohorts 3 and 4 were administered a 6-week prophylactic topical corticosteroid regimen (*see* Example 5 and **Table 11B**).

### Subject Characteristics

The baseline characteristics for subjects in Cohorts 1-4 were well balanced, with similar ages and mean prior anti-VEGF injections, and a similar need for frequent injections to maintain baseline vision of about 65 ETDRS letters across cohorts. Some subjects in Cohort 4 were diagnosed more recently than in other cohorts, e.g., less than 12 months prior to administration of AAV2.7m8-aflibercept, therefore the mean number of prior anti-VEGF injections is lower. Overall, subjects in Cohorts 1-4 had good vision, and were difficult to treat, with some fluid with frequent treatment with anti-VEGF injections. The baseline subject characteristics for subjects in Cohorts 1-4 are provided in **Table 19.**

**Table 19. Baseline characteristics of subjects in Cohorts 1-4.**

| **Baseline Characteristics** | **Cohort 1 (N=6)** | **Cohort 2 (N=6)** | **Cohort 3 (N=9)** | **Cohort 4 (N=9)** |
|---|---|---|---|---|
| Mean (range) age, years | 79.0 (62-88) | 79.8 (74-90) | 77.4 (65-90) | 79.9 (68-88) |
| Mean (range) years since nAMD diagnosis | 3.5 (0.9-10.6) | 4.1 (0.5-6.8) | 3.3 (0.7-8.0) | 3.2 (0.2-8.0) |
| Mean (range) number anti-VEGF injections since initial diagnosis* | 32.2 (7-109) | 34.0 (4-69) | 24.8 (9-70) | 28.5 (2-58)** |
| Mean (range) number anti-VEGF injections in 12 months prior to AAV2.7m8-aflibercept | 9.2 (8-11) | 9.2 (5-11) | 9.1 (7-10) | 6.8 (3-12)** |
| Mean (range) BCVA, ETDRS letters | 65.8 (57-77) | 64.7 (53-72) | 65.9 (53-75) | 65.0 (54-77) |
| Approximate Snellen equivalent | 20/50 | 20/50 | 20/50 | 20/50 |
| Mean (range) CST, µm | 369.2 (293-561) | 307.7 (235-339) | 473.4 (301-857) | 398.6 (255-538) |
| *Not including the mandated aflibercept at Screening; **Excluding Subject 2 with incomplete prior anti-VEGF data due to relocation. | | | | |
| BCVA, best corrected visual acuity; CST, central subfield thickness; ETDRS, Early Treatment Diabetic Retinopathy Study; nAMD, neovascular age-related macular degeneration; VEGF, vascular endothelial growth factor. | | | | |

### Safety

No deaths, treatment discontinuations, or AAV2.7m8-aflibercept-related non-ocular adverse events have occurred.

When observed, inflammation was responsive to and managed by topical steroids. No clinical or fluorescein (fluorescein angiography of posterior pole) evidence of posterior inflammation has been observed. In addition, no vasculitis, retinitis, choroiditis, vascular occlusions, or endophthalmitis have occurred.

All AAV2.7m8-aflibercept-related ocular adverse events were mild (78%) or moderate (22%).

One adverse event of special interest (AESI) of recurrent moderate uveitis occurred and was deemed related to AAV2.7m8-aflibercept (Cohort 1). The recurrent moderate uveitis AESI was responsive to steroid eye drops. In addition, a treatment-unrelated ocular serious adverse event (SAE) of retinal detachment occurred and was surgically repaired and resolved (Cohort 1).

Two subjects had mild adverse events of mild intraocular pressure (IOP) elevation that resolved. One subject from Cohort 1 had two mild IOP elevations (highest of 24mmHg) that were both treated with Combigan^{®} eye drops. The second subject was taking Combigan^{®} for ocular hypertension, and the IOP adverse event spontaneously resolved within a month with no change to treatment.

Overall, AAV2.7m8-aflibercept has been well tolerated and there have been no systemic adverse events attributed to AAV2.7m8-aflibercept.

A summary of safety results in Cohorts 1-4 of this study is provided in **Table 20.**

**Table 20. Adverse events in Cohorts 1-4.**

| **Adverse events** | **Cohort 1 (N=6)** 6×10¹¹ vg/eye Oral steroids 13-day prophylaxis | | **Cohort 2 (N=6)** 2×10¹¹ vg/eye Oral steroids 13-day prophylaxis | | **Cohort 3 (N=9)** 2×10¹¹ vg/eye Steroid eye drops 6-week prophylaxis | | **Cohort 4 (N=9)** 6×10¹¹ vg/eye Steroid eye drops 6-week prophylaxis | |
|---|---|---|---|---|---|---|---|---|
| **Ocular** | Subjects | Events | Subjects | Events | Subjects | Events | Subjects | Events |
| Serious | 2 | 2* | 0 | 0 | 0 | 0 | 0 | 0 |
| Related ** | 6 | 30 | 5 | 21 | 5 | 14 | 5 | 12 |
| Total ocular | 6 | 51 | 5 | 32 | 8 | 26 | 7 | 15 |
| **Non-ocular†** | Subjects | Events | Subjects | Events | Subjects | Events | Subjects | Events |
| Serious ‡ | 1 | 1 | 0 | 0 | 2 | 2 | 0 | 0 |
| Total non-ocular^{†} | 5 | 18 | 6 | 7 | 4 | 9 | 1 | 1 |
| *Retinal detachment (unrelated to AAV2.7m8-aflibercept) and recurrent moderate uveitis (likely related to AAV2.7m8-aflibercept). | | | | | | | | |
| ** AA V2.7m8-aflibercept-related ocular events were mild (78%) or moderate (22%). | | | | | | | | |
| † None of the non-ocular AEs were AAV2.7m8-aflibercept-related. | | | | | | | | |
| ‡ Serious non-ocular AEs included degenerative intervertebral disc disease (1) in Cohort 1; and COPD exacerbation (1), and stable angina pectoris (1) in Cohort 3. | | | | | | | | |

AAV2.7m8-aflibercept-related adverse events that occurred in Cohorts 1-4, reported by MedDRA preferred term, are provided in **Table 21.**

**Table 21. AAV2.7m8-aflibercept-related AEs by preferred term in Cohorts 1-4.**

| **MedDRA Preferred Term** | **Cohort 1 (N=6)** | | **Cohort 2 (N=6)** | | **Cohort 3 (N=9)** | | **Cohort 4 (N=9)** | |
|---|---|---|---|---|---|---|---|---|
| | Events | Subjects | Events | Subjects | Events | Subjects | Events | Subjects |
| Any Events | 30 | 6 | 21 | 5 | 14 | 5 | 12 | 5 |
| Anterior chamber cell | 4 | 3 | 7 | 4 | 2 | 2 | 4 | 4 |
| Anterior chamber flare | 5 | 3 | 3 | 2 | 0 | 0 | 2 | 2 |
| Vitreal cells | 4 | 3 | 2 | 2 | 1 | 1 | 2 | 2 |
| Keratic precipitates | 2 | 1 | 3 | 2 | 2 | 2 | 1 | 1 |
| Anterior chamber inflammation | 3 | 1 | 0 | 0 | 2 | 1 | 0 | 0 |
| Iris hyperpigmentation | 2 | 2 | 1 | 1 | 1 | 1 | 0 | 0 |
| Anterior chamber pigmentation | 0 | 0 | 1 | 1 | 2 | 1 | 0 | 0 |
| Uveitis | 3 | 2 | 0 | 0 | 0 | 0 | 0 | 0 |
| Vitreous floaters | 2 | 2 | 0 | 0 | 0 | 0 | 1 | 1 |
| Iris adhesions | 0 | 0 | 0 | 0 | 2 | 2 | 0 | 0 |
| Iris transillumination defect | 0 | 0 | 0 | 0 | 1 | 1 | 1 | 1 |
| Vitreous haze | 1 | 1 | 1 | 1 | 0 | 0 | 0 | 0 |
| Iridocyclitis | 0 | 0 | 1 | 1 | 0 | 0 | 0 | 0 |
| Iris bombe | 1 | 1 | 0 | 0 | 0 | 0 | 0 | 0 |
| Iritis | 0 | 0 | 0 | 0 | 1 | 1 | 0 | 0 |
| Lenticular pigmentation | 0 | 0 | 1 | 1 | 0 | 0 | 0 | 0 |
| Miosis | 1 | 1 | 0 | 0 | 0 | 0 | 0 | 0 |
| Pupil fixed | 1 | 1 | 0 | 0 | 0 | 0 | 0 | 0 |
| Retinal pigmentation | 1 | 1 | 0 | 0 | 0 | 0 | 0 | 0 |
| Visual acuity reduced | 0 | 0 | 1 | 1 | 0 | 0 | 0 | 0 |
| Vitritis | 0 | 0 | 0 | 0 | 0 | 0 | 1 | 1 |

Ocular adverse events that occurred with a frequency of ≥2 events, reported by MedDRA preferred term, are provided in **Table 22.**

**Table 22. Ocular AEs by preferred term (in ≥2 events).**

| **MedDRA Preferred Term** | **Cohort 1 (N=6)** | | **Cohort 2 (N=6)** | | **Cohort 3 (N=9)** | | **Cohort 4 (N=9)** | |
|---|---|---|---|---|---|---|---|---|
| | Events | Subjects | Events | Subjects | Events | Subjects | Events | Subjects |
| Any Events* | 51 | 6 | 32 | 5 | 26 | 8 | 15 | 7 |
| Anterior chamber cell | 5 | 4 | 7 | 4 | 2 | 2 | 4 | 4 |
| Anterior chamber flare | 5 | 3 | 3 | 2 | 0 | 0 | 2 | 2 |
| Conjunctival haemorrhage | 3 | 3 | 2 | 2 | 1 | 1 | 3 | 3 |
| Vitreal cells | 4 | 3 | 2 | 2 | 1 | 1 | 2 | 2 |
| Keratic precipitates | 2 | 1 | 3 | 2 | 2 | 2 | 1 | 1 |
| Vitreous floaters | 4 | 3 | 0 | 0 | 2 | 2 | 1 | 1 |
| Anterior chamber inflammation | 3 | 1 | 0 | 0 | 2 | 1 | 0 | 0 |
| Anterior chamber pigmentation | 0 | 0 | 1 | 1 | 3 | 2 | 0 | 0 |
| Iris hyperpigmentation | 2 | 2 | 1 | 1 | 1 | 1 | 0 | 0 |
| Posterior capsule opacification | 0 | 0 | 2 | 2 | 2 | 2 | 0 | 0 |
| Intraocular pressure increased | 2 | 1 | 0 | 0 | 1 | 1 | 0 | 0 |
| Iris adhesions | 1 | 1 | 0 | 0 | 2 | 2 | 0 | 0 |
| Macular oedema | 0 | 0 | 3 | 1 | 0 | 0 | 0 | 0 |
| Retinal haemorrhage | 2 | 1 | 0 | 0 | 1 | 1 | 0 | 0 |
| Uveitis | 3 | 2 | 0 | 0 | 0 | 0 | 0 | 0 |
| Dry eye | 2 | 2 | 0 | 0 | 0 | 0 | 0 | 0 |
| Iris transillumination defect | 0 | 0 | 0 | 0 | 1 | 1 | 1 | 1 |
| Lenticular pigmentation | 1 | 1 | 1 | 1 | 0 | 0 | 0 | 0 |
| Retinal pigmentation | 1 | 1 | 0 | 0 | 1 | 1 | 0 | 0 |
| Vitreous haze | 1 | 1 | 1 | 1 | 0 | 0 | 0 | 0 |
| *Includes terms reported in only one event. | | | | | | | | |

Non-ocular adverse events that occurred with a frequency of ≥2 events, reported by MedDRA preferred term, are provided in **Table 23.**

**Table 23. Non-ocular AEs by preferred term (in ≥2 events).**

| **MedDRA Preferred Term** | **Cohort 1 (N=6)** | | **Cohort 2 (N=6)** | | **Cohort 3 (N=9)** | | **Cohort 4 (N=9)** | |
|---|---|---|---|---|---|---|---|---|
| | Events | Subjects | Events | Subjects | Events | Subjects | Events | Subjects |
| Any Events* | 18 | 5 | 7 | 6 | 9 | 4 | 1 | 1 |
| Arthritis | 1 | 1 | 1 | 1 | 0 | 0 | 0 | 0 |
| Atrial fibrillation | 1 | 1 | 1 | 1 | 0 | 0 | 0 | 0 |
| Bronchitis | 0 | 0 | 0 | 0 | 2 | 1 | 0 | 0 |
| Chronic obstructive pulmonary disease | 0 | 0 | 0 | 0 | 2 | 1 | 0 | 0 |
| Hypertension | 0 | 0 | 2 | 2 | 0 | 0 | 0 | 0 |
| Nasopharyngitis | 2 | 1 | 0 | 0 | 0 | 0 | 0 | 0 |
| Post-traumatic pain | 0 | 0 | 0 | 0 | 2 | 1 | 0 | 0 |
| *Includes terms reported in only one event. | | | | | | | | |

A summary of ocular and non-ocular serious, mild, and moderate adverse events observed in Cohorts 1-4 of this study is provided in **Table 24.**

**Table 24. Adverse events in Cohorts 1-4.**

| **Adverse events** | **Cohort 1 (N=6)** | | **Cohort 2 (N=6)** | | **Cohort 3 (N=9)** | | **Cohort 4 (N=9)** | |
|---|---|---|---|---|---|---|---|---|
| | 6×10¹¹ vg/eye Oral steroids | | 2×10¹¹ vg/eye Oral steroids | | 2×10¹¹ vg/eye Steroid eye drops | | 6×10¹¹ vg/eye Steroid eye drops | |
| | 13-day prophylaxis | | 13-day prophylaxis | | 6-week prophylaxis | | 6-week prophylaxis | |
| **Ocular** | Subjects | Events | Subjects | Events | Subjects | Events | Subjects | Events |
| Serious | 2 | 2* | 0 | 0 | 0 | 0 | 0 | 0 |
| Related** | 6 | 30 | 5 | 21 | 5 | 14 | 5 | 12 |
| Mild | 6 | 22 | 4 | 14 | 5 | 12 | 5 | 12 |
| Moderate | 3 | 8 | 2 | 7 | 2 | 2 | 0 | 0 |
| Total ocular | 6 | 51 | 5 | 32 | 8 | 26 | 7 | 15 |
| **Non-ocular†** | Subjects | Events | Subjects | Events | Subjects | Events | Subjects | Events |
| Serious ^{‡} | 1 | 1 | 0 | 0 | 2 | 2 | 0 | 0 |
| Total non-ocular^{†} | 5 | 18 | 6 | 7 | 4 | 9 | 1 | 1 |
| *Retinal detachment (unrelated to study treatment) and recurrent moderate uveitis (likely related to study treatment). | | | | | | | | |
| **Study treatment-related ocular events were mild (78%) or moderate (22%). | | | | | | | | |
| †None of the non-ocular AEs were related to study treatment. | | | | | | | | |
| ‡Serious non-ocular AEs included degenerative intervertebral disc disease (1) in Cohort 1; and COPD exacerbation (1), and stable angina pectoris (1) in Cohort 3. | | | | | | | | |

As shown in **FIGS. 22A-22D****,** when observed, inflammation was primarily in the anterior segment and responsive to and managed by topical steroids. No evidence of posterior inflammation has been observed, and there have been no cases of vasculitis.

As shown in **FIG. 22A****,** two subjects (Subjects 2 and 6) in Cohort 1 who were previously on QD steroid eye drops with no active inflammation subsequently discontinued drops. Subject 2 had recurrent moderate uveitis following discontinuation and restarted steroid eye drops. Inflammation improved and the subject resumed tapering down steroid eye drops. At the time of data cut, Subject 4 was receiving steroid eye drops BID and had trace inflammation after retinal detachment surgery. Subject 6 was not receiving any steroid eye drops and had no inflammation. Overall, inflammation in subjects in Cohort 1 was resolving, with only 2 out of 6 subjects continuing on steroid eye drop treatment at the time of data cut. The results of Cohort 1 have shown that all subjects were responsive to topical steroids and trending towards tapering of the steroids at the time of data cut.

In Cohort 2, 4 out of 6 subjects were inflammation-free and 2 out of 6 subjects continued to receive steroid eye drops at the time of data cut (**FIG. 22B**). Subject 3 received topical steroids around Week 46 after cataract surgery. At the time of data cut, Subject 1 was receiving steroid eye drops QD, and Subject 6 had low grade inflammation and continued on steroid eye drops. Overall, inflammation in subjects in Cohort 2 was resolving with all subjects past the 52-week (1 year) milestone at the time of data cut.

In Cohort 3, 6 out of 9 subjects were inflammation-free at the time of data cut (**FIG. 22C**), and all were beyond the 20-week milestone. At the time of data cut, Subjects 2, 3 and 4 were receiving steroid eye drops. Subject 4 started durezol treatment BID at Week 24 due to poor pupil dilation and posterior synechiae likely related to subclinical inflammation. 3 out of 9 subjects continued to receive steroid eye drops at the time of data cut. Difluprednate ophthalmic emulsion 0.05% is a generic version of durezol.

As shown in **FIG. 22D****,** subjects in Cohort 4 have shown minimal to zero early inflammation with steroid eye drops, consistent with the results observed for Cohort 3. Subjects 1, 2 and 3 had anterior inflammation and continued to receive steroid eye drops at the time of data cut.

The 6-week prophylactic topical steroid regimen administered to Cohorts 3 and 4 showed evidence of less active inflammation (**FIGS. 22C-22D**) compared to the oral steroid regimen administered to Cohorts 1 and 2 (**FIGS. 22A-22B**).

### Efficacy

**FIG. 23A** shows the mean BCVA (ETDRS letters) from baseline up to Week 72 for subjects in Cohort 1. At a median follow up time of 72 weeks (range of 64-84 weeks), subjects in Cohort 1 had a mean BCVA change from baseline of -3.2 letters and 100% of subjects (6/6) had not received a rescue anti-VEGF injection. The apparent decrease in mean BCVA at Weeks 44 and 48 was due to one subject that experienced an SAE of retinal detachment. Overall, BCVA scores in subjects in Cohort 1 remained stable out to 72 weeks. **FIG. 23B** shows the mean CST (µm) from baseline up to Week 72 for subjects in Cohort 1. At a median follow up time of 72 weeks (range of 64-84 weeks), subjects in Cohort 1 had a mean CST change from baseline of -21.0 µm. Overall, CST measurements in Cohort 1 showed that anatomy was maintained out to week 72 for all subjects. The observed drop and change in the error bars at Weeks 68 and 72 in **FIG. 23B** was due to exclusion of 1 subject that has always had a very thick retina.

**FIG. 24A** shows the mean BCVA (ETDRS letters) from baseline up to Week 52 for subjects in Cohort 2. At a median follow up time of 52 weeks (range of 52-56 weeks), subjects in Cohort 2 had a mean BCVA change from baseline of -2.0 letters and 50% of subjects (3/6) had not received a rescue anti-VEGF injection. The subjects that had not received a rescue anti-VEGF injection had a mean BCVA change from baseline of +0 letters. Overall, BCVA scores in subjects in Cohort 2 remained stable beyond 52 weeks. **FIG. 24B** shows the mean CST (µm) from baseline up to Week 52 for subjects in Cohort 2. At a median follow up time of 52 weeks (range of 52-56 weeks), subjects in Cohort 2 had a mean CST change from baseline of -24.8 µm. In addition, the subjects that had not received a rescue anti-VEGF injection had a mean CST change from baseline of -8.3 µm. Overall, CST measurements in Cohort 2 showed that anatomy was maintained beyond week 52.

**FIG. 25A** shows the mean BCVA (ETDRS letters) from baseline up to Week 20 for subjects in Cohort 3. At a median follow up time of 36 weeks (range 20-40 weeks), subjects in Cohort 3 had a mean BCVA change from baseline of +4.0 letters and 78% (7/9) of subjects from Cohort 3 had not received a rescue anti-VEGF injection. The subjects that had not received a rescue anti-VEGF injection had a mean BCVA change from baseline of +6.4 letters. Overall, BCVA scores in subjects in Cohort 3 improved. **FIG. 25B** shows the mean CST (µm) from baseline up to Week 20 for subjects in Cohort 3. At a median follow up time of 36 weeks (range 20-40 weeks), subjects in Cohort 3 had a mean CST change from baseline of -118.6 µm. In addition, the subjects that had not received a rescue anti-VEGF injection had a mean CST change from baseline of -152.7 µm. Overall, the mean CST continued to improve in all 9 subjects in Cohort 3.

As shown in the Swimmer's Lane Plot in **FIG.26****,** no subjects in Cohort 1 received a rescue anti-VEGF injection at a median follow up time of 72 weeks (range of 64-84 weeks). In Cohort 2 (median follow up time of 52 weeks; range of 52-56 weeks), Subject 2 received one anti-VEGF rescue injection (aflibercept), and Subjects 3 and 5 each received six anti-VEGF rescue injections (aflibercept). In Cohort 3 (median follow up time of 36 weeks; range 20-40 weeks), Subject 2 received one anti-VEGF rescue injection (aflibercept), and Subject 3 received four anti-VEGF rescue injections (aflibercept). Overall, 10 out of 15 subjects in Cohorts 2-3 remained rescue injection-free. Overall, even with rescue injections in certain cohorts, there was a dramatic reduction in the anti-VEGF injection burden.

A substantial reduction in the annualized anti-VEGF injection rate was observed in subjects in Cohorts 1-3 (**FIGS. 27A-27B**). Specifically, no subjects in Cohort 1 have received any rescue anti-VEGF injections (100% reduction), and subjects in Cohorts 2 and 3 combined had an 87% reduction in the mean annual rate of anti-VEGF injections (**FIG. 27A**).

### Case Studies

At baseline, Subject 5 in Cohort 3 (82 year old male with wAMD in the right eye) had received 19 prior anti-VEGF injections (9 in the last 12 months prior to AAV2.7m8-aflibercept administration). Subject 5 had persistent subretinal fluid even with frequent anti-VEGF injections every 5 weeks (**FIG. 28A**). The white area in the center of the thickness maps in **FIG. 28A** reflects a very thick macula.

As shown in **FIG. 28B****,** Subject 5 received a screening aflibercept injection 2 weeks prior to the planned AAV2.7m8-aflibercept injection. Subject 5 showed a good response to the screening aflibercept injection, with resolution of IRF and improvement of SRF. Thus, Subject 5 was responsive to anti-VEGF treatment but required very frequent injections to keep the fluid away. One week after the AAV2.7m8-aflibercept injection, fluid improved. If this subject was only on bolus aflibercept injections, the fluid would have started returning within 4-5 weeks and the OCT images would look like the baseline OCT image, with significant fluid. However, as shown in **FIG. 28B,** at 12 weeks, 16 weeks, 20 weeks, 28 weeks, and 36 weeks following AAV2.7m8-aflibercept injection, there was no fluid and vision improved. Thus, in Subject 5, AAV2.7m8-aflibercept injection addressed the treatment burden by eliminating the need for injections, improved vision, and controlled disease activity. These results demonstrate that the course of the disease in Subject 5 was altered by a single administration of AAV2.7m8-aflibercept.

### Discussion

The results described in this Example showed that AAV2.7m8-aflibercept continues to be well tolerated and has a favorable safety profile in all cohorts (n = 30). All AAV2.7m8-aflibercept-related ocular adverse events were mild (78%) or moderate (22%), and ocular inflammation, when observed, was responsive to steroid eye drops.

In addition, the results described in this Example showed that AAV2.7m8-aflibercept has robust and sustained efficacy.

A clear dose response was observed, with all subjects administered AAV2.7m8-aflibercept at a dose of 6 × 10¹¹vg/eye remaining rescue anti-VEGF injection-free, and 10 out of 15 subjects administered AAV2.7m8-aflibercept at a dose of 2 × 10¹¹vg/eye remaining rescue anti-VEGF injection-free. All subjects in Cohort 1 (dose of 6 × 10¹¹vg/eye) remain rescue injection-free beyond 15 months after administration of AAV2.7m8-aflibercept.

The results described in this Example also showed that a single IVT injection of AAV2.7m8-aflibercept resulted in a substantial reduction in the annualized anti-VEGF injection rate. Mean BCVA was generally maintained to improved and the mean CST generally improved.

Results from Cohort 4 showed that the 6-week prophylactic regimen of steroid eye drops was effective at minimizing early ocular inflammation, consistent with results observed in Cohort 3.

Although the present disclosure has been described in some detail by way of illustration and example for purposes of clarity of understanding, the descriptions and examples should not be construed as limiting the scope of the present disclosure. The disclosures of all patent and scientific literature cited herein are expressly incorporated in the entirety by reference.

Certain embodiments of the invention are set out in the following numbered paragraphs:
1. A method for reducing fluctuation of central subfield thickness (CST) or central retinal thickness (CRT) in an eye of an individual with an ocular neovascular disease, the method comprising administering a unit dose of rAAV particles to one eye of the individual, wherein the individual is a human, and wherein the rAAV particles comprise:
   a) a nucleic acid encoding a polypeptide comprising an amino acid sequence with at least about 95% identity to the amino acid sequence of SEQ ID NO: 35 and flanked by AAV2 inverted terminal repeats (ITRs), and
   b) an AAV2 capsid protein comprising an amino acid sequence LGETTRP (SEQ ID NO: 14) inserted between positions 587 and 588 of the capsid protein, wherein the amino acid residue numbering corresponds to an AAV2 VP1 capsid protein.
2. The method of embodiment 1, wherein the individual has received at least one treatment of an anti-VEGF agent in about 1 to 12 weeks prior to administration of the unit dose of rAAV particles.
3. The method of embodiment 1 or embodiment 2, wherein the level of fluctuation in the CST or CRT in the one eye of the individual is refractory to prior treatment with an anti-VEGF agent.
4. The method of embodiment 2 or embodiment 3, wherein the anti-VEGF agent is aflibercept.
5. The method of embodiment 1, wherein the fluctuation level of CST or CRT after administration of rAAV particles in the one eye is reduced by about 40% to about 90% compared to the fluctuation level of CST or CRT in the one eye of the individual prior to administration of the rAAV to the individual.
6. The method of embodiment 5, wherein the reduction is about 45% to about 85%..
7. The method of embodiment 5, wherein the reduction is about 50 to about 90%.
8. The method of any one of embodiments 1-7, wherein the unit dose of rAAV particles is about 6 x 10¹⁰ to about 6 × 10¹¹ vector genomes per eye (vg/eye) or less.
9. The method of embodiment 10, wherein the rAAV particle is about 2 x 10¹¹ vg/eye or 6 x 10¹¹.
10. A method for improving visual acuity in an individual suffering from ocular neovascular disease, the method comprising:
   (a) administering an anti-VEGF agent to one eye of the individual; and
   (b) administering a unit dose of about 6 × 10¹⁰ to about 6 × 10¹¹ vector genomes (vg) or less of recombinant adeno-associated virus (rAAV) particles to the one eye of the individual, wherein the individual is a human, and wherein the rAAV particles comprise:
      (i) a nucleic acid encoding a polypeptide comprising an amino acid sequence with at least about 95% identity to the amino acid sequence of SEQ ID NO: 35 and flanked by AAV2 inverted terminal repeats (ITRs), and
      (ii) an AAV2 capsid protein comprising an amino acid sequence LGETTRP (SEQ ID NO: 14) inserted between positions 587 and 588 of the capsid protein, wherein the amino acid residue numbering corresponds to an AAV2 VP1 capsid protein.
11. The method of embodiment 10, comprising administering the unit dose of rAAV particles to the one eye of the individual about 1 to 12 weeks after administration of the anti-VEGF agent.
12. The method of embodiment 10 or embodiment 11, comprising administering the anti-VEGF agent to the one eye of the individual on Day 1, and administering the unit dose of rAAV particles to the one eye of the individual on Day 8.
13. The method of any one of embodiments 10-12, wherein the anti-VEGF agent comprises aflibercept.
14. The method of embodiment 13, where the aflibercept is administered at a dose of about 2 mg by intravitreal injection.
15. The method of any one of embodiments 1-14, wherein the unit dose of rAAV particles is between about 6 × 10¹⁰ to about 6 × 10¹¹ vector genomes per eye (vg/eye).
16. The method of any one of embodiments 1-15, wherein the unit dose of rAAV particles is between about 6 × 10¹⁰ to about 2 × 10¹¹ vector genomes per eye (vg/eye).
17. The method of any one of embodiments 1-16, wherein the unit dose of rAAV particles is between about 2 × 10¹¹ to about 6 × 10¹¹ vector genomes per eye (vg/eye).
18. The method of embodiment 17, wherein the unit dose of rAAV particles is about 2 × 10¹¹ or about 6 × 10¹¹ vector genomes per eye (vg/eye).
19. The method of embodiment 18, wherein the unit dose of rAAV particles is about 2 × 10¹¹ vector genomes per eye (vg/eye).
20. The method of embodiment 18, wherein the unit dose of rAAV particles is about 6 × 10¹¹ vector genomes per eye (vg/eye).
21. The method of any one of embodiments 1-20, wherein the individual has one or more symptoms of an ocular neovascular disease in the contralateral eye.
22. The method of any one of embodiments 1-21, further comprising administering a unit dose of rAAV particles to the contralateral eye of the individual.
23. The method of embodiment 22, wherein the administering the unit dose of rAAV particles to the contralateral eye is up to about 2 weeks after administering the unit dose of rAAV particles to the one eye.
24. The method of embodiment 23, wherein:
   (a) the administering the unit dose of rAAV particles to the contralateral eye is on the same day as the administering the unit dose of rAAV particles to the one eye; or
   (b) the administering the unit dose of rAAV particles to the contralateral eye is between about 1 day to about 14 days after administering the unit dose of rAAV particles to the one eye.
25. The method of embodiment 23 or embodiment 24, wherein the unit dose of rAAV particles administered to the contralateral eye of the individual comprises the same or less vector genomes per eye (vg/eye) than the unit dose of rAAV particles administered to the one eye of the individual.
26. The method of embodiment 22, wherein the administering the unit dose of rAAV particles to the contralateral eye is at least about 2 weeks after administering the unit dose of rAAV particles to the one eye.
27. The method of embodiment 26 wherein the unit dose of rAAV particles administered to the contralateral eye of the individual comprises more vector genomes per eye (vg/eye) than the unit dose of rAAV particles administered to the one eye of the individual.
28. The method of any one of embodiments 1-27, wherein the nucleic acid comprises the nucleic acid sequence of SEQ ID NO: 40 or a sequence having at least 85% identity thereto.
29. The method of any one of embodiments 1-28, wherein the polypeptide comprises the amino acid sequence of SEQ ID NO: 35.
30. The method of any one of embodiments 1-29, wherein the polypeptide comprises the amino acid sequence of SEQ ID NO: 41.
31. The method of any one of embodiments 1-30, wherein the polypeptide is aflibercept.
32. The method of any one of embodiments 1-31, wherein the nucleic acid further comprises a first enhancer region, a promoter region, a 5'UTR region, a second enhancer region, and a polyadenylation site.
33. The method of any one of embodiments 1-32, wherein the nucleic acid comprises, in the 5' to 3' order:
   (a) a first enhancer region;
   (b) a promoter region;
   (c) a 5'UTR region;
   (d) a nucleic acid encoding a polypeptide comprising an amino acid sequence with at least about 95% identity to the amino acid sequence of SEQ ID NO: 35;
   (e) a second enhancer region; and
   (f) a polyadenylation site;
   and flanked by AAV2 inverted terminal repeats (ITRs).
34. The method of embodiment 32 or embodiment 33, wherein the first enhancer region comprises a CMV sequence comprising the sequence of SEQ ID NO: 22 or a sequence having at least 85% identity thereto.
35. The method of any one of embodiments 32-34, wherein the promoter region comprises a CMV sequence comprising the sequence of SEQ ID NO: 23 or a sequence having at least 85% identity thereto.
36. The method of any one of embodiments 33-35, wherein the nucleic acid encoding a polypeptide comprises the nucleic acid sequence of SEQ ID NO: 40 or a sequence having at least 85% identity thereto.
37. The method of any one of embodiments 33-36, wherein the polypeptide comprises the amino acid sequence of SEQ ID NO: 35 or a sequence having at least 95% identity thereto.
38. The method of any one of embodiments 33-37, wherein the polypeptide comprises the amino acid sequence of SEQ ID NO: 41 or a sequence having at least 95% identity thereto.
39. The method of any one of embodiments 33-38, wherein the polypeptide is aflibercept.
40. The method of any one of embodiments 32-39, wherein the 5'UTR region comprises, in 5' to 3' order, a TPL sequence comprising the sequence of SEQ ID NO: 24 or a sequence having at least 85% identity thereto, and an eMLP sequence comprising the sequence of SEQ ID NO: 25 or a sequence having at least 85% identity thereto.
41. The method of any one of embodiments 32-40, wherein the second enhancer region comprises a full EES sequence comprising the sequence of SEQ ID NO: 26 or a sequence having at least 85% identity thereto.
42. The method of any one of embodiments 32-41, wherein the polyadenylation site comprises a HGH polyadenylation site comprising the sequence of SEQ ID NO: 27 or a sequence having at least 85% identity thereto.
43. The method of any one of embodiments 1-31, wherein the nucleic acid further comprises (a) a first enhancer region comprising a CMV sequence comprising the sequence of SEQ ID NO: 22 or a sequence having at least 85% identity thereto; (b) a promoter region, comprising a CMV sequence comprising the sequence of SEQ ID NO: 23 or a sequence having at least 85% identity thereto; (c) a 5'UTR region comprising, in 5' to 3' order, a TPL sequence comprising the sequence of SEQ ID NO: 24 or a sequence having at least 85% identity thereto, and an eMLP sequence comprising the sequence of SEQ ID NO: 25 or a sequence having at least 85% identity thereto; (d) a second enhancer region comprising a full EES sequence comprising the sequence of SEQ ID NO: 26 or a sequence having at least 85% identity thereto; and (e) a HGH polyadenylation site comprising the sequence of SEQ ID NO: 27 or a sequence having at least 85% identity thereto.
44. The method of any one of embodiments 1-43, wherein the nucleic acid comprises the sequence of SEQ ID NO: 39 or a sequence having at least 85% identity thereto.
45. The method of any one of embodiments 1-44, wherein the AAV2 capsid protein comprises the amino acid sequence LALGETTRPA (SEQ ID NO: 1) inserted between positions 587 and 588 of the capsid protein, wherein the amino acid residue numbering corresponds to an AAV2 VP1 capsid protein.
46. The method of any one of embodiments 1-45, wherein the AAV2 capsid protein comprises the amino acid sequence LGETTRP (SEQ ID NO: 14) inserted between positions 587 and 588 of the AAV2 VP1 comprising the sequence of SEQ ID NO: 13.
47. The method of any one of embodiments 1-46, wherein the AAV2 capsid protein comprises the amino acid sequence LALGETTRPA (SEQ ID NO: 1) inserted between positions 587 and 588 of the AAV2 VP1 comprising the sequence of SEQ ID NO: 13.
48. The method of any one of embodiments 1-45, wherein the rAAV particles comprise an AAV2 VP1 capsid protein comprising a GH loop that comprises the amino acid sequence of SEQ ID NO: 38 or an amino acid sequence having at least 90% sequence identity to SEQ ID NO: 38.
49. The method of any one of embodiments 1-48, wherein the administration of the unit dose of rAAV particles to the one eye and/or the contralateral eye is by intravitreal administration.
50. The method of any one of embodiments 1-49, wherein the unit dose of rAAV particles is in a pharmaceutical formulation.
51. The method of embodiment 50, wherein the pharmaceutical formulation comprises the rAAV particles, sodium chloride, sodium phosphate and a surfactant.
52. The method of embodiment 51, wherein the pharmaceutical formulation comprises about 150 to about 200 mM sodium chloride, about 1 to about 10 mM monobasic sodium phosphate, about 1 to about 10 mM dibasic sodium phosphate, about 0.0005% (w/v) to about 0.005% (w/v) poloxamer 188, and about 6 × 10¹³ to about 6 × 10¹⁰ vector genomes (vg) per mL (vg/mL) of the rAAV particles, wherein the pharmaceutical formulation has a pH of about 7.0 to about 7.5.
53. The method of embodiment 52, wherein the pharmaceutical formulation comprises about 180 mM sodium chloride, about 5 mM monobasic sodium phosphate, about 5 mM dibasic sodium phosphate, about 6 × 10¹² vg/mL of the rAAV particles, and about 0.001% (w/v) poloxamer 188, wherein the pharmaceutical formulation has a pH of about 7.3.
54. The method of embodiment 52, wherein the pharmaceutical formulation comprises about 180 mM sodium chloride, about 5 mM monobasic sodium phosphate, about 5 mM dibasic sodium phosphate, about 2 ×10¹² vg/mL of the rAAV particles, and about 0.001% (w/v) poloxamer 188, wherein the pharmaceutical formulation has a pH of about 7.3.
55. The method of embodiment 52, wherein the pharmaceutical formulation comprises about 180 mM sodium chloride, about 5 mM monobasic sodium phosphate, about 5 mM dibasic sodium phosphate, about 6 ×10¹¹ vg/mL of the rAAV particles, and about 0.001% (w/v) poloxamer 188, wherein the pharmaceutical formulation has a pH of about 7.3.
56. The method of any one of embodiments 1-55, wherein the unit dose of rAAV particles administered to the one eye and/or to the contralateral eye in a volume of about 25 µL to about 250 µL.
57. The method of embodiment 56, wherein the unit dose of rAAV particles administered to the one eye and/or to the contralateral eye comprises a volume of about 100µL.
58. The method of embodiment 56, wherein the unit dose of rAAV particles administered to the one eye and/or to the contralateral eye comprises a volume of about 30µL.
59. The method of any one of embodiments 1-58, wherein the individual received prior treatment for the ocular neovascular disease with an anti-VEGF agent.
60. The method of embodiment 59, wherein the individual has received 1 or 2 injections of an anti-VEGF agent in the one eye and/or in the contralateral eye prior to administration of the rAAV particles in the one eye and/or in the contralateral eye.
61. The method of any one of embodiments 1-60, wherein the individual has not received prior treatment for the ocular neovascular disease with an anti-VEGF agent.
62. The method of any one of embodiments 59-61, wherein the anti-VEGF agent is aflibercept.
63. The method of any one of embodiments 1-62, wherein the ocular neovascular disease is wet age-related macular degeneration (AMD), retinal neovascularization, choroidal neovascularization diabetic retinopathy, proliferative diabetic retinopathy, retinal vein occlusion, central retinal vein occlusion, branched retinal vein occlusion, diabetic macular edema, diabetic retinal ischemia, ischemic retinopathy, diabetic retinal edema, or any combination thereof.
64. The method of any one of embodiments 1-63, wherein the unit dose of rAAV particles is administered in combination with steroid treatment.
65. The method of embodiment 64, wherein the steroid treatment is a corticosteroid treatment.
66. The method of embodiment 64 or embodiment 65, wherein the steroid treatment is a systemic steroid treatment.
67. The method of any one of embodiments 64-66, wherein the steroid treatment is an oral steroid treatment.
68. The method of any one of embodiments 64-67, wherein the steroid treatment is a prednisone treatment.
69. The method of embodiment 64 or embodiment 65, wherein the steroid treatment is a topical steroid treatment.
70. The method of embodiment 69, wherein the steroid treatment is a difluprednate treatment.
71. The method of any one of embodiments 64-70, wherein the steroid is administered before, during and/or after administration of the unit dose of rAAV particles to the one eye and/or to the contralateral eye.
72. The method of any one of embodiments 69-71, wherein the steroid treatment is a topical steroid treatment and the topical steroid treatment is a daily steroid treatment for up to about 4 weeks, up to about 6 weeks, or up to about 8 weeks from administering the unit dose of rAAV particles.
73. The method of embodiment 72, wherein the topical steroid treatment comprises about four administrations of topical steroid per day on about week 1, about three administrations of topical steroid per day on about week 2, about two administrations of topical steroid per day on about week 3, and about one administration per day of topical steroid on about week 4; timing starting with and following administration of the unit dose of rAAV particles.
74. The method of embodiment 72, wherein the topical steroid treatment comprises about four administrations of topical steroid per day for about 3 weeks after administration of the unit dose of rAAV particles, followed by about 3 administrations of topical steroid per day for about 1 week, followed by about 2 administrations of topical steroid per day for about 1 week, and followed by about 1 administration of topical steroid per day for about 1 week.
75. The method of embodiment 72, wherein the topical steroid treatment comprises about four administrations of topical steroid per day for about four weeks, followed by about three administrations of topical steroid per day for about one week, followed by about two administrations of topical steroid per day for about one week, and followed by about one administration of topical steroid per day for about one week; timing starting at about one week prior to administration of the unit dose of rAAV particles.
76. The method of any one of embodiments 10-63, further comprising administering a topical steroid treatment.
77. The method of embodiment 76, wherein the topical steroid treatment is a difluprednate treatment.
78. The method embodiment 76 or embodiment 77, wherein the topical steroid treatment comprises about four administrations of topical steroid per day for about four weeks, followed by about three administrations of topical steroid per day for about one week, followed by about two administrations of topical steroid per day for about one week, and followed by about one administration of topical steroid per day for about one week; timing starting with and following administration of the anti-VEGF agent.
79. The method of any one of embodiments 70-78, wherein the topical steroid comprises difluprednate 0.05% at a dose of about 1µg to about 3 µg.
80. The method of any one of embodiments 70-79, wherein the topical steroid comprises difluprednate 0.05% at a dose of about 2.5µg.
81. The method of any one of embodiments 1-80, wherein the administering the unit dose of rAAV particles to the one eye and/or to the contralateral eye of the individual results in maintenance or a decrease of retinal thickness compared to the retinal thickness prior to administration of the unit dose of rAAV particles.
82. The method of embodiment 81, wherein the administering the unit dose of rAAV particles to the one eye and/or to the contralateral eye of the individual results in a decrease in retinal thickness compared to the retinal thickness prior to administration of the unit dose of rAAV particles.
83. The method of embodiment 82, wherein the decrease in retinal thickness is at least about 10% compared to the retinal thickness prior to administration of the unit dose of rAAV particles.
84. The method of any one of embodiments 81-83, wherein retinal thickness is central subfield thickness (CST) or central retinal thickness (CRT).
85. The method of any one of embodiments 1-84, wherein the administering the unit dose of rAAV particles to the one eye and/or to the contralateral eye of the individual results in maintenance or a decrease in macular volume compared to the macular volume prior to administration of the unit dose of rAAV particles.
86. The method of embodiment 85, wherein the administering the unit dose of rAAV particles to the one eye and/or to the contralateral eye of the individual results in a decrease in macular volume compared to the macular volume prior to administration of the unit dose of rAAV particles.
87. The method of embodiment 86, wherein the decrease in macular volume is at least about 10% compared to the macular volume prior to administration of the unit dose of rAAV particles.
88. The method of any one of embodiments 1-87, wherein the administering the unit dose of rAAV particles to the one eye and/or to the contralateral eye of the individual results in maintenance or an improvement of visual acuity compared to the visual acuity prior to administration of the unit dose of rAAV particles.
89. The method of embodiment 88, wherein the administering the unit dose of rAAV particles to the one eye and/or to the contralateral eye of the individual results in an improvement of visual acuity compared to the visual acuity prior to administration of the unit dose of rAAV particles.
90. The method of embodiment 88 or embodiment 89, wherein visual acuity is best corrected visual acuity (BCVA).
91. The method of any one of embodiments 1-90, wherein administration of the unit dose of rAAV particles to the one eye and/or to the contralateral eye of a plurality of individuals results in at least about 50% of the individuals in the plurality not requiring an anti-VEGF rescue treatment.
92. The method of embodiment 91, wherein at least about 50% of the individuals in the plurality do not require an anti-VEGF rescue treatment for at least about 20 weeks, at least about 36 weeks, at least about 52 weeks, at least about 56 weeks, or more after administration of the unit dose of rAAV particles.
93. The method of any one of embodiments 1-90, wherein administration of the unit dose of rAAV particles to the one eye and/or to the contralateral eye of a plurality of individuals results in at least about 67% of the individuals in the plurality not requiring an anti-VEGF rescue treatment.
94. The method of embodiment 93, wherein at least about 67% of the individuals in the plurality do not require an anti-VEGF rescue treatment for at least about 20 weeks, at least about 36 weeks, at least about 52 weeks, at least about 60 weeks, at least about 64 weeks, or at least about 66 weeks after administration of the unit dose of rAAV particles.
95. The method of any one of embodiments 1-90, wherein administration of the unit dose of rAAV particles to the one eye and/or to the contralateral eye of a plurality of individuals results in at least about 78% of the individuals in the plurality not requiring an anti-VEGF rescue treatment.
96. The method of embodiment 95, wherein at least about 78% of the individuals in the plurality do not require an anti-VEGF rescue treatment for at least about 20 weeks, at least about 36 weeks, or more after administration of the unit dose of rAAV particles.
97. The method of any one of embodiments 1-90, wherein administration of the unit dose of rAAV particles to the one eye and/or to the contralateral eye of a plurality of individuals results in 100% of the individuals in the plurality not requiring an anti-VEGF rescue treatment.
98. The method of embodiment 97, wherein 100% of the individuals in the plurality do not require an anti-VEGF rescue treatment for at least about 64 weeks, at least about 68 weeks, at least about 72 weeks, at least about 76 weeks, at least about 80 weeks, at least about 84 weeks, or more after administration of the unit dose of rAAV particles.
99. The method of any one of embodiments 1-98, wherein administration of the unit dose of rAAV particles to the one eye and/or to the contralateral eye of a plurality of individuals results in a reduction in the annualized anti-VEGF injection rate of at least about 80%, at least about 85%, at least about 87%, at least about 90%, at least about 95%, at least about 99%, or 100% compared to the annualized anti-VEGF injection rate prior to administration of the unit dose of rAAV particles.
100. The method of any one of embodiments 1-99, wherein the ocular neovascular disease is wet age-related macular degeneration (AMD).
101. The method of any one of embodiments 1-100, wherein the ocular neovascular disease is diabetic macular edema (DME).
102. The method of embodiment 101, wherein the administering the unit dose of rAAV particles to the one eye and/or to the contralateral eye of the individual results in a 2-step or in a 3-step improvement in Diabetic Retinopathy Severity Scale (DRSS).
104. A unit dose of about 6 × 10¹¹ vector genomes (vg) or less of recombinant adeno- associated virus (rAAV) particles for use in a method for treating an ocular neovascular disease in an individual, the method comprising administering said unit dose to one eye of the individual, wherein the individual is a human, and wherein the rAAV particles comprise:
   a) a nucleic acid encoding a polypeptide comprising an amino acid sequence with at least about 95% identity to the amino acid sequence of SEQ ID NO: 35 and flanked by AAV2 inverted terminal repeats (ITRs), and
   b) an AAV2 capsid protein comprising an amino acid sequence LGETTRP (SEQ ID NO: 14) inserted between positions 587 and 588 of the capsid protein, wherein the amino acid residue numbering corresponds to an AAV2 VP1 capsid protein.
105. A unit dose of rAAV particles for use in a method for reducing fluctuation of CST or CRT in an eye of an individual with an ocular neovascular disease, the method comprising administering said unit dose to one eye of the individual, wherein the individual is a human, and wherein the rAAV particles comprise:
   a) a nucleic acid encoding a polypeptide comprising an amino acid sequence with at least about 95% identity to the amino acid sequence of SEQ ID NO: 35 and flanked by AAV2 inverted terminal repeats (ITRs), and
   b) an AAV2 capsid protein comprising an amino acid sequence LGETTRP (SEQ ID NO: 14) inserted between positions 587 and 588 of the capsid protein, wherein the amino acid residue numbering corresponds to an AAV2 VP1 capsid protein.
106. The unit dose for use according to embodiment 104 or embodiment 105 wherein said method further comprises administering an anti-VEGF agent to said one eye of the individual before administration of said unit dose.

## Claims

1. A unit dose of recombinant adeno-associated virus (rAAV) particles for use in a method of reducing fluctuation of central subfield thickness (CST) or central retinal thickness (CRT) in a human individual with an ocular neovascular disease, wherein the unit dose is administered to one eye of the individual and the rAAV particles comprise:
a) a nucleic acid encoding a polypeptide comprising an amino acid sequence with at least about 95% identity to the amino acid sequence of SEQ ID NO: 35 and flanked by AAV2 inverted terminal repeats (ITRs), and
b) an AAV2 capsid protein comprising an amino acid sequence LGETTRP (SEQ ID NO: 14) inserted between positions 587 and 588 of the capsid protein, wherein the amino acid residue numbering corresponds to an AAV2 VP1 capsid protein;
optionally wherein said method further comprises administering an anti-VEGF agent to said one eye of the individual before administration of said unit dose.

2. The unit dose for use according to claim 1, wherein:
the individual has received at least one treatment of an anti-VEGF agent in about 1 to 12 weeks prior to administration of the unit dose of rAAV particles; and/or
the level of fluctuation in the CST or CRT in the one eye of the individual is refractory to prior treatment with an anti-VEGF agent;
optionally wherein the anti-VEGF agent comprises aflibercept, for example at a dose of about 2 mg by intravitreal injection.

3. The unit dose for use according to claim 1, wherein the fluctuation level of CST or CRT after administration of rAAV particles in the one eye is reduced by about 40% to about 90% compared to the fluctuation level of CST or CRT in the one eye of the individual prior to administration of the rAAV to the individual, optionally wherein the reduction is about 45% to about 85% or about 50% to about 90%.

4. The unit dose for use according to any one of the preceding claims, wherein the unit dose of rAAV particles is to be administered to one eye of the individual after administration of the anti-VEGF agent to the one eye, and the unit dose of rAAV particles is administered to the one eye of the individual about 1 week to 12 weeks after administration of the anti-VEGF agent, or administering the anti-VEGF agent to the one eye of the individual on Day 1, and administering the unit dose of rAAV particles to the one eye of the individual on Day 8; optionally wherein the anti-VEGF agent comprises aflibercept, for example at a dose of about 2 mg by intravitreal injection.

5. The unit dose for use according to any one of the preceding claims, wherein the unit dose of rAAV particles is:
(a) between about 1 × 10¹⁰ to about 6 × 10¹¹ vector genomes per eye (vg/eye); or
(b) between about 6 × 10¹⁰ to about 6 × 10¹¹ vg/eye; or
(c) between about 6 × 10¹⁰ to about 2 × 10¹¹ vg/eye; or
(d) between about 2 × 10¹¹ to about 6 × 10¹¹ vg/eye; or
(e) about 2 × 10¹¹ or about 6 × 10¹¹ vg/eye; or
(f) about 2 × 10¹¹ vg/eye; or
(g) about 6 × 10¹¹ vg/eye; or
(h) about 6 x 10¹⁰ vg/eye.

6. The unit dose for use according to any one of the preceding claims, wherein:
(a) the individual has one or more symptoms of an ocular neovascular disease in the contralateral eye; and/or
(b) the unit dose of rAAV particles is administered to the contralateral eye of the individual, optionally wherein:
(i) the unit dose of rAAV particles is administered to the contralateral eye up to about 2 weeks after administration of the unit dose of rAAV particles to the one eye, for example wherein the unit dose of rAAV particles is administered to the contralateral eye on the same day as the unit dose of rAAV particles administered to the one eye, or wherein the unit dose of rAAV particles is administered to the contralateral eye between about 1 day to about 14 days after administration of the unit dose of rAAV particles to the one eye, and/or
(ii) the unit dose of rAAV particles administered to the contralateral eye of the individual comprises the same or less vector genomes per eye (vg/eye) than the unit dose of rAAV particles administered to the one eye of the individual; or
(iii) the unit dose of rAAV particles is to be administered to the contralateral eye at least about 2 weeks after administration of the unit dose of rAAV particles to the one eye, optionally wherein the unit dose of rAAV particles administered to the contralateral eye of the individual comprises more vector genomes per eye (vg/eye) than the unit dose of rAAV particles administered to the one eye of the individual.

7. The unit dose for use according to any one of the preceding claims, wherein: (a) the nucleic acid comprises the nucleic acid sequence of SEQ ID NO: 40 or a sequence having at least 85% identity thereto; and/or (b) the polypeptide comprises the amino acid sequence of SEQ ID NO: 35 or SEQ ID NO: 41; and/or (c) the polypeptide is aflibercept.

8. The unit dose for use according to any one of the preceding claims, wherein:
(a) the nucleic acid further comprises a first enhancer region, a promoter region, a 5'UTR region, a second enhancer region, and a polyadenylation site; optionally wherein the first enhancer region comprises a CMV sequence comprising the sequence of SEQ ID NO: 22 or a sequence having at least 85% identity thereto, the promoter region comprises a CMV sequence comprising the sequence of SEQ ID NO: 23 or a sequence having at least 85% identity thereto, the 5'UTR region comprises, in 5' to 3' order, a TPL sequence comprising the sequence of SEQ ID NO: 24 or a sequence having at least 85% identity thereto and an eMLP sequence comprising the sequence of SEQ ID NO: 25 or a sequence having at least 85% identity thereto, the second enhancer region comprises a full EES sequence comprising the sequence of SEQ ID NO: 26 or a sequence having at least 85% identity thereto, and the polyadenylation site comprises an HGH polyadenylation site comprising the sequence of SEQ ID NO: 27 or a sequence having at least 85% identity thereto; and/or
(b) the nucleic acid comprises, in the 5' to 3' order:
(i) a first enhancer region, optionally wherein the first enhancer region comprises a CMV sequence comprising the sequence of SEQ ID NO: 22 or a sequence having at least 85% identity thereto,
(ii) a promoter region, optionally wherein the promoter region comprises a CMV sequence comprising the sequence of SEQ ID NO: 23 or a sequence having at least 85% identity thereto,
(iii) a 5'UTR region, optionally wherein the 5'UTR region comprises, in 5' to 3' order, a TPL sequence comprising the sequence of SEQ ID NO: 24 or a sequence having at least 85% identity thereto and an eMLP sequence comprising the sequence of SEQ ID NO: 25 or a sequence having at least 85% identity thereto,
(iv) a nucleic acid encoding a polypeptide comprising an amino acid sequence with at least about 95% identity to the amino acid sequence of SEQ ID NO: 35, optionally wherein the nucleic acid encoding the polypeptide comprises the nucleic acid sequence of SEQ ID NO: 40 or a sequence having at least 85% identity thereto, for example wherein the polypeptide comprises the amino acid sequence of SEQ ID NO: 35, the amino acid sequence of SEQ ID NO: 41 or a sequence having at least 95% identity thereto, or is aflibercept,
(v) a second enhancer region, optionally wherein the second enhancer region comprises a full EES sequence comprising the sequence of SEQ ID NO: 26 or a sequence having at least 85% identity thereto, and
(vi) a polyadenylation site, optionally wherein the polyadenylation site comprises a HGH polyadenylation site comprising the sequence of SEQ ID NO: 27 or a sequence having at least 85% identity thereto,
and flanked by AAV2 inverted terminal repeats (ITRs); and/or
(c) the nucleic acid comprises the sequence of SEQ ID NO: 39 or a sequence having at least 85% identity thereto.

9. The unit dose for use according to any one of the preceding claims, wherein:
(a) the AAV2 capsid protein comprises the amino acid sequence LALGETTRPA (SEQ ID NO: 1) inserted between positions 587 and 588 of the capsid protein, wherein the amino acid residue numbering corresponds to an AAV2 VP1 capsid protein; and/or
(b) the AAV2 capsid protein comprises the amino acid sequence LGETTRP (SEQ ID NO: 14) inserted between positions 587 and 588 of the AAV2 VP1 comprising the sequence of SEQ ID NO: 13; and/or
(c) the AAV2 capsid protein comprises the amino acid sequence LALGETTRPA (SEQ ID NO: 1) inserted between positions 587 and 588 of the AAV2 VP1 comprising the sequence of SEQ ID NO: 13.

10. The unit dose for use according to any one of the preceding claims, wherein the rAAV particles comprise an AAV2 VP1 capsid protein comprising a GH loop that comprises the amino acid sequence of SEQ ID NO: 38 or an amino acid sequence having at least 90% sequence identity to SEQ ID NO: 38.

11. The unit dose for use according to any one of the preceding claims, wherein the unit dose of rAAV particles:
(a) is to be administered to the one eye and/or the contralateral eye by intravitreal administration; and/or
(b) is in a pharmaceutical formulation, optionally wherein the pharmaceutical formulation comprises the rAAV particles, sodium chloride, sodium phosphate and a surfactant, for example wherein the pharmaceutical formulation comprises:
(i) about 150 to about 200 mM sodium chloride, about 1 to about 10 mM monobasic sodium phosphate, about 1 to about 10 mM dibasic sodium phosphate, about 0.0005% (w/v) to about 0.005% (w/v) poloxamer 188, and about 6 × 10¹³ to about 6 × 10¹⁰ vector genomes (vg) per mL (vg/mL) of the rAAV particles, wherein the pharmaceutical formulation has a pH of about 7.0 to about 7.5;
(ii) about 180 mM sodium chloride, about 5 mM monobasic sodium phosphate, about 5 mM dibasic sodium phosphate, about 6 ×10¹² vg/mL of the rAAV particles, and about 0.001% (w/v) poloxamer 188, wherein the pharmaceutical formulation has a pH of about 7.3;
(iii) about 180 mM sodium chloride, about 5 mM monobasic sodium phosphate, about 5 mM dibasic sodium phosphate, about 2 ×10¹² vg/mL of the rAAV particles, and about 0.001% (w/v) poloxamer 188, wherein the pharmaceutical formulation has a pH of about 7.3; or
(iv) about 180 mM sodium chloride, about 5 mM monobasic sodium phosphate, about 5 mM dibasic sodium phosphate, about 6 ×10¹¹ vg/mL of the rAAV particles, and about 0.001% (w/v) poloxamer 188, wherein the pharmaceutical formulation has a pH of about 7.3.

12. The unit dose for use according to any one of the preceding claims, wherein the unit dose of rAAV particles administered to the one eye and/or to the contralateral eye is in a volume of about 25 µL to about 250 µL, optionally wherein the unit dose of rAAV particles administered to the one eye and/or to the contralateral eye comprises a volume of about 100 µL or about 30 µL.

13. The unit dose for use according to any one of claims 1 and 3-12, wherein:
(a) the individual received prior treatment for the ocular neovascular disease with an anti-VEGF agent, optionally wherein the individual has received 1 or 2 injections of an anti-VEGF agent in the one eye and/or in the contralateral eye prior to administration of the rAAV particles in the one eye and/or in the contralateral eye; or
(b) the individual has not received prior treatment for the ocular neovascular disease with an anti-VEGF agent,
optionally wherein the anti-VEGF agent is aflibercept.

14. The unit dose for use according to any one of the preceding claims, wherein the unit dose of rAAV particles is administered in combination with steroid treatment, optionally wherein:
(a) the steroid treatment is a topical steroid treatment, for example wherein the steroid treatment is a difluprednate treatment, e.g., difluprednate 0.05% at a dose of about 1µg to about 3 µg, or at a dose of about 2.5µg; and/or
(b) the steroid is administered before, during and/or after administration of the unit dose of rAAV particles to the one eye and/or to the contralateral eye; and/or
(c) the steroid treatment is a topical steroid treatment, and wherein: (i) the topical steroid treatment is a daily steroid treatment for up to about 4 weeks, up to about 6 weeks, or up to about 8 weeks from administration of the unit dose of rAAV particles, for example wherein the topical steroid treatment comprises about four administrations of topical steroid per day on about week 1, about three administrations of topical steroid per day on about week 2, about two administrations of topical steroid per day on about week 3, and about one administration per day of topical steroid on about week 4; timing starting with and following administration of the unit dose of rAAV particles; or (ii) the topical steroid treatment comprises about four administrations of topical steroid per day for about 3 weeks after administration of the unit dose of rAAV particles, followed by about 3 administrations of topical steroid per day for about 1 week, followed by about 2 administrations of topical steroid per day for about 1 week, and followed by about 1 administration of topical steroid per day for about 1 week; (iii) the topical steroid treatment comprises about four administrations of topical steroid per day for about four weeks, followed by about three administrations of topical steroid per day for about one week, followed by about two administrations of topical steroid per day for about one week, and followed by about one administration of topical steroid per day for about one week; timing starting at about one week prior to administration of either the unit dose of rAAV particles or, if administered, the anti-VEGF agent; or (iv) the topical steroid treatment comprises about four administrations of topical steroid per day for about four weeks, followed by about three administrations of topical steroid per day for about one week, followed by about two administrations of topical steroid per day for about one week, and followed by about one administration of topical steroid per day for about one week; timing starting with and following administration of the anti-VEGF agent.

15. The unit dose for use according to any one of the preceding claims, wherein the method is for treating an ocular neovascular disease or for improving visual acuity in an individual suffering from ocular neovascular disease, optionally wherein the ocular neovascular disease is selected from: wet age-related macular degeneration (AMD), retinal neovascularization, choroidal neovascularization diabetic retinopathy, proliferative diabetic retinopathy, retinal vein occlusion, central retinal vein occlusion, branched retinal vein occlusion, diabetic macular edema (DME), diabetic retinal ischemia, ischemic retinopathy, diabetic retinal edema, or any combination thereof; further optionally wherein the ocular neovascular disease is wet age-related macular degeneration (AMD) or diabetic macular edema (DME).
